# EUROPEAN PATENT APPLICATION

(11) **EP 4 209 492 A1**
(43) Date of publication of application: **12.07.2023**
(21) Application number: 21847386.6
(22) Date of filing: 26.07.2021
(51) Int. Cl.: C07D 471/04, C07D 491/048, C07D 493/04, C07D 495/04, C07D 209/12, C07D 307/79, C07D 311/04, C07D 333/56, A61K 31/343, A61K 31/381, A61K 31/40, A61K 31/519, A61P 9/00

(54) **COMPOUND FOR TREATING THROMBOTIC DISEASES**

(30) Priority: 24.07.2020 CN 202010724532
(71) Applicant: Shanghai Institute of Materia Medica, Chinese Academy of Sciences, Pudong, Shanghai 201203 (CN); Ruijin Hospital, Shanghai Jiaotong University School of Medicine, Shanghai 200025 (CN); Suzhou Institute of Materia Medica China Science And Technology, Suzhou, Jiangsu 215123 (CN)
(72) Inventor: LUO, Cheng, Shanghai 201203 (CN); XI, Xiaodong, Shanghai 200025 (CN); ZHOU, Bing, Shanghai 201203 (CN); ZHU, Kongkai, Shanghai 201203 (CN); MAO, Jianhua, Shanghai 200025 (CN); LIU, Jingqiu, Shanghai 201203 (CN); YANG, Yaxi, Shanghai 201203 (CN); MEI, Lianghe, Suzhou, Jiangsu 215123 (CN); RUAN, Zheng, Shanghai 200025 (CN); JIANG, Hao, Shanghai 201203 (CN); XI, Wenda, Shanghai 200025 (CN); LONG, Zhangbiao, Shanghai 200025 (CN); XIAO, Bing, Shanghai 200025 (CN); HUANG, Jiansong, Shanghai 200025 (CN); JIANG, Hualiang, Shanghai 201203 (CN)
(74) Representative: Mewburn Ellis LLP
(86) International application number: PCT/CN2021/108368
(87) International publication number: WO 2022/017531

(57) **Abstract**

The present invention relates to a compound for treating thrombotic diseases. Specifically, the present invention provides a compound represented by formula I, or a pharmaceutically acceptable salt, or an enantiomer, or a diastereoisomer, or an atropisomer, or a racemate, or a polymorph, or a solvate, or an isotopically labeled derivative thereof. The compound disclosed in the present invention can be specifically combined with SH3 domain protein of Src kinase and then interfere the combination of integrin αIIbβ3 and Src kinase, so that outside-to-inside signal transduction is selectively inhibited and inside-to-outside signal transduction is not influenced; thus, while resisting thrombus, the compound of the present invention does not affect the normal physiological hemostatic function, prevents the occurrence of hemorrhagic side effect, and can be used as a new generation of effective medicine for preventing and treating thrombosis-related cardiovascular and cerebrovascular diseases.

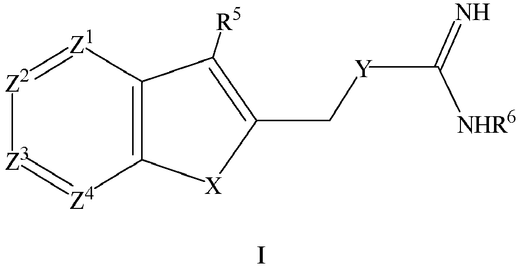

## Description

### TECHNICAL FIELD

The invention relates to the field of medicine, in particular to a compound for the treatment of thrombotic diseases.

### BACKGROUND OF THE INVENTION

At present, cardio-cerebrovascular thrombotic diseases such as myocardial infarction and cerebral infarction have become the most important causes of serious threats to human life and health, featuring high morbidity and mortality. According to the statistics of the World Health Organization, about 12 million people are prematurely killed by cardio-cerebrovascular diseases every year. The mortality rate of cardio-cerebrovascular diseases is the highest among all kinds of diseases, and the number of deaths from cardio-cerebrovascular diseases accounts for about 1/3 of the global deaths. Platelets play a key role in cardio-cerebrovascular thrombosis. When the vascular endothelium is damaged or the atheromatous plaque is ruptured, platelets activate and cause pathological thrombosis through a series of functions such as adhesion, extension and aggregation, causing ischemic necrosis of the heart and brain tissues in the distribution area of the affected vessels, which can seriously endanger the life of patients. Therefore, anti-platelet therapy has become the first choice for the treatment of cardio-cerebrovascular thrombotic diseases. The most direct cause of pathological thrombosis in thrombotic diseases is the abnormal activation and aggregation of platelets. The final common pathway of platelet aggregation and thrombosis caused by various platelet agonists in the body is mediated by integrin αIIbβ3(GPIIb/IIIa). Therefore, integrin αIIbβ3 has become an ideal target for anti-platelet therapy. However, at present, the difficulty of antithrombotic drugs is that they inhibit the normal physiological hemostatic function at the same time, and often lead to bleeding side effects.

Integrin αIIbβ3 is a member of the integrin family. Integrin is a kind of transmembrane protein widely existing in cells, which mediates interaction between cells (by binding cadherin and selectin), and between cells and extracellular matrix (by binding extracellular matrix such as fibronectin, collagen, laminin) , involved in a series of physiological and pathological processes such as cell signaling, adhesion, extension, tumor migration, hemostasis and thrombosis. Integrin is a heterodimer formed by α subunits and β subunits. At present, 18 α subunits are found in mammals, which form 24 different integrins with 8 β subunits. Among them, integrin β3 can form two integrins with αIIb and αv subunits: αIIbβ3 and αvβ3. Integrin αIIbβ3 is mainly expressed on the surface of platelets and megakaryocytes. It is the main membrane receptor on the surface of platelets and plays an important role in thrombosis and hemostasis; while integrin αvβ3 is widely distributed in vivo, such as endothelial cells, osteoclasts, tumour cells, smooth muscle cells, fibroblasts, etc., mediating various pathophysiological processes such as angiogenesis, bone resorption and tumour metastasis.

Integrin αIIbβ3 is a transmembrane heterodimer composed of the two subunits of αIIb and β3 through non-covalent bonds. It is mainly expressed on the surface of platelets and megakaryocytes. It is the main membrane receptor on the surface of platelets. It can mediate bidirectional signaling of platelets, so it plays a key role in platelet activation, maintenance of normal platelet function and thrombosis. Platelet activators such as thrombin and ADP interact with the corresponding receptors and cause a conformational change in integrin αIIbβ3 and an increase in affinity for its ligand, i.e. soluble fibrinogen, etc. This process is inside-out signaling, with the hallmark events being unstable platelet adhesion, free fibrinogen binding and reversible aggregation, etc. The integrin αIIbβ3 is activated and bound to the ligand, activating the outside-in signaling with the hallmark events being stable platelet adhesion, extension, irreversible aggregation, fibrin clot retraction, etc., which eventually promote the aggregation of platelets and form a more stable thrombus to complete the physiological or pathological process of hemostasis and thrombosis. The current consensus is that the realization of hemostasis and thrombosis requires the participation of inside-out and outside-in signaling, and the pathological process of thrombosis needs to increase platelet emboli under the condition of resisting high blood flow impact. Therefore, thrombogenesis is relatively more dependent outside-in signaling.

Integrin αIIbβ3 is the final common pathway that mediates platelet activation and aggregation and thrombosis, so it is the main target of anti-thrombotic drug research. In fact, the research on integrin αIIbβ3 as the target of anti-thrombotic drugs has made important progress. At present, it mainly focuses on integrin αIIbβ3 receptor antagonists and has achieved good clinical effects. At present, there are three kinds of integrin αIIbβ3 receptor antagonist anti-platelet drugs approved by the U.S. Food and Drug Administration (FDA) for clinical anti-thrombotic therapy, namely abciximab, eptifibatide and tirofiban. Such αIIbβ3 receptor antagonists specifically exert anti-thrombotic effects by interfering with the interaction of integrin αIIbβ3 with its ligand. However, there are still obvious problems with this strategy. αIIbβ3 receptor antagonist drugs block bidirectional signaling by preventing integrin αIIbβ3 from binding to its ligand, that is, it affects normal hemostasis while exerting anti-thrombotic effects. Clinical trial review statistics show that about 2% of patients treated with integrin αIIbβ3 antagonists have severe intracranial hemorrhage, about 15% have gastrointestinal hemorrhage, and about 5-10% have peritoneal hemorrhage. In addition, about 60-80% of the tested patients have obvious hemorrhage at the femoral artery puncture point. Like the classic anti-thrombotic drugs aspirin and clopidogrel, the integrin αIIbβ3 antagonist not only exerts an effective anti-thrombotic effect, but also leads to an increase in the risk of bleeding in patients. This is currently the most common and most important side effect of anti-thrombotic drugs. Therefore, the choice of clinical anti-thrombotic drug dose should also take into account the dedication of its bleeding side effects, which makes it difficult to achieve better anti-thrombotic effect by increasing the dose of anti-thrombotic drugs. Therefore, by developing a new generation of antithrombotic drugs that do not affect normal hemostatic function, it will be possible to obtain stronger antithrombotic effects under the premise of low risk, which represents the development direction of antithrombotic drugs.

Integrin β3 is the main subunit that binds to platelet intracellular signal molecules and regulates bidirectional signaling. The interaction of intracellular segments with signaling molecules involved in the regulation of bidirectional signaling has received widespread attention from scholars. In recent years, a large number of research results suggest that selective inhibition of integrin αIIbβ3 outside-in signaling can inhibit pathological thrombosis, and does not cause the risk of spontaneous bleeding. Therefore, in order to achieve a more reasonable new antithrombotic strategy, a scientific and reasonable idea should avoid direct antagonism of the αIIbβ3 receptor but selectively regulate the outside-in signaling by interfering with the interaction of integrin β3 and platelet intracellular signal molecules to achieve effective antithrombotic while preserving physiological hemostasis as much as possible. Therefore, the key problem currently facing is a way to achieve clinical transformation and application. The most feasible strategy is small-molecule interference strategy, which can achieve effective antithrombotic strategy in a real sense without affecting normal hemostatic function by selectively regulating the outside-in signaling of human platelet integrin without affecting the inside-out signaling of platelet integrin basically.

Integrin αIIbβ3 bidirectional signaling is two signal pathways that are connected and distinct from each other. In recent years, great progress has been made in the study of integrin β3 cytoplasmic segment regulating bidirectional signaling through interaction with cytoplasmic signal protein molecules, for example, β3-endonexin, ILK, talin head domain, kindlin-3, etc. which participate in the regulation of inside-out signaling, and c-Src, Gα13, Talin, VPS33B, Shc, JAM-A, Dok-1, etc. which participate in the regulation of outside-in signaling. Among them, the function and mechanism of the interaction between platelet integrin β3 and c-Src are the most clear. The c-Src that constitutively bind to the RGT sequence at the end of integrin β3 receiving activation signals is to modulate kinase activity and then phosphorylate downstream signal molecules, which is the most basic regulatory pathway for outside-in signaling that has been confirmed. It has been confirmed that the defects of Src family gene such as c-Src, Hck, Fgr, Lyn, etc. (especially c-Src) or under the action of Src kinase inhibitors, integrin β3^{T762A} mutation and β3^{Δ760-762} deletion mutation can inhibit platelet function mediated by outside-in signals. The three amino acids RGT at the end of the β3 subunit can interact directly with c-Src, and the synthetic RGT polypeptide can specifically interfere with the interaction between integrin β3 and c-Src in platelets, and selectively inhibit platelet outside-in signal transduction. Therefore, β3/ c-Src interaction plays a very important role in platelet integrin αIIbβ3-mediated outside-in signaling, which has become a consensus in academia. Selective inhibition of outside-in signaling by targeting β3/ c-Src interaction through synthetic molecules has the potential to develop into a new generation of antithrombotic drugs.

In the resting state of platelets, there are about 80000 integrins αIIbβ3 on the surface of the platelet membrane. When activated, platelet α particles release integrins αIIbβ3 while the intracellular pipeline system opens and transfers to the membrane, increasing the number to enhance hemostasis function. Integrin αIIbβ3 is composed of αIIb subunit and β3 subunit combined by non-covalent bonds. The molecular weight of αIIb subunit is 140kD, which is composed of a heavy chain (containing 871 amino acids) and a light chain (containing 137 amino acids), which are connected by disulfide bonds. The molecular weight of β3 subunit is 105kD which is a transmembrane polypeptide composed of 762 amino acids. Both αIIb and β3 subunits are composed of longer extracellular segments, transmembrane α helices and shorter intracellular segments. The extracellular segment of integrin can bind to ligands such as fibrinogen and vWF factor. Its binding ability with ligands is not only regulated by the extracellular segment, but the three parts of extracellular, transmembrane, and intracellular are all involved in regulation. Electron microscopy found that the extracellular segments of the two subunits have an N-terminal and ligand-bound "head", connected to the longer C-terminal "leg", and then connected to the transmembrane segment and cytoplasmic segment.

Crystal diffraction further found that the "head" of the α subunit consists of seven blade-like folds to form the β-propeller-like domain (β-propeller), and the "leg" consists of "thigh", "Calf-1" and "Calf-2" domains, and forms the knee of the leg between the two domains of "thigh" and "Calf-1. The "head" of the β subunit consists of the β A domain and the " Hybrid " domain of immunoglobulins like protein. The "leg" consists of a PSI(plexin-semaphorin-integrin) domain, four EGF(epidermal growth factor) domains and a β tail domain, and the knee of the leg is formed between the "EGF-1" and "EGF-2" domains. When the integrin is resting, the legs of the two subunits are in a "V"-shaped buckling state, making the head of the subunit close to the cell membrane, obscuring the binding site with the ligand, and integrin αIIbβ3 had a low affinity for the ligand. When platelet agonists such as thrombin and adenosine diphosphate interact with the corresponding receptors on the platelet membrane, the talin head binds to the proximal membrane cytoplasmic segment of integrin β3 through a series of signaling, disrupting the salt bridge that maintains the resting state αIIbβ3 between αIIbR995-β3D723. The spatial conformation of integrin αIIbβ3 changes from "buckling" to "stretching". When the ligand binding site is exposed, the affinity for the ligand increases. This process is called "inside-out" signaling, which is functionally manifested as platelet binding to soluble fibrinogen, platelet initial adhesion and one-phase aggregation. After the conformational change, the integrin αIIbβ3 receptor clusters, aggregates and binds with kinases and anchor proteins, leading to a series of signaling such as calcium influx, protein tyrosine phosphorylation, and skeleton protein reorganization. This process is called outside-in signaling, which is functionally manifested as stable adhesion and extension of platelets, two-phase aggregation, and fibrin clot retraction. Integrin αIIbβ3 completes its role in thrombosis and hemostasis through such bidirectional signaling. The inside-out signaling completes the initial activation of integrin, the initial adhesion and reversible aggregation, and basically realizes the hemostatic function, while the outside-in signaling realizes the extension and irreversible aggregation of platelets, and the formation of fibrin clots to form a firm thrombus, which plays a more important role in thrombosis.

The cytoplasmic segment of integrin αIIbβ3 is very short, but it can bind to many intracellular proteins and kinases to play an important role in regulating integrin αIIbβ3 signaling. The proteins that interact with the β3 cytoplasmic segment include cytoskeletal protein talin, α-actin, filament protein and myosin, etc. Kinases include Src kinase family, integrin-linked kinase (ILK), focal adhesion kinase (FAK), etc., regulatory proteins include β3-inline protein and cell attachment protein-1. In recent years, the role of Src kinase in integrin αIIbβ3 bidirectional signaling, especially in outside-in signaling, has attracted the interest of many scholars. Src kinase is a member of the SFK(Src family kinase protein) family. The SFK family contains 9 members, of which Src, Fyn and Yes are widely present in various tissues in the body and play an important regulatory role in cell growth, development and differentiation. When mice lack these three kinases, embryos die due to developmental defects. Src kinase is composed of SH3, SH2, kinase region and C- terminal region domain from N-terminal to C- terminal. Csk kinase can bind to SH2 domain and phosphorylate Y527 on the C-terminal of Src kinase, thus maintaining the closed conformation of Src kinase and making it in an inactive state. Under certain signal stimuli, Csk kinase can leave the SH2 domain of Src kinase, Y527 is dephosphorylated, and Y416 of Src kinase is phosphorylated, so that Src has kinase activity and can phosphorylate downstream substrate, participating in cell signaling.

The current research shows that the SH3 domain of Src kinase forms a constitutive binding to the three amino acids of RGT at the end of integrin β3 C. In the resting state of platelets, β3-Src(SH3)-Src(SH2)-Csk forms a complex that forms the basis of the integrin αIIbβ3 outside-in signaling. When the mouse platelets were deficient in Src kinase, the outside-in signaling of integrin αIIbβ3 was inhibited, and the outside-in signaling was also significantly inhibited when Src kinase inhibitors were acted on the platelets. When the integrin-activated outside-in signaling, Src kinase interacts with the RGT sequence at the C-terminal of β 3 , and phosphorylation of the β 3 cytoplasmic segments Y747 and Y759 occurs, which becomes an important event of outside-in signaling. When αIIbβ33Δ759 is transferred into CHO cells, the outside-in signal is significantly inhibited than that of αIIbβ3 in CHO cells, while the outside-in signal is significantly impaired, and the phosphorylation of Y747 and Y759 is inhibited when the integrin αIIbβ3-RGT sequence is knocked out in mice. Ablooglu, A. J. et al. established integrin αIIbβ3Δ759 transgenic mice, namely RGT knockout mice. The results showed compared with control mice, the extension of RGT knockout mice platelets on the solid phase fibrinogen was inhibited; fibrin clot retraction was also inhibited; the phosphorylation of Y747 was impaired; but free fibrinogen binding in PAR4 and glycoprotein VI as agonists was not affected. In vivo experiments confirmed that RGT knockout mice can avoid thrombosis caused by FeCl₃ stimulating carotid artery. In the tail resection experiment, some mice had prolonged bleeding time, but there was no spontaneous hemorrhage, massive hemorrhage after operation, bloody stool, hematuria, anemia, etc. Knockout of the RGT sequence of integrin αIIbβ3 can affect the αIIbβ3 signaling involved in c-Src kinase, and can avoid arterial thrombosis; it inhibits the outside-in signaling of integrin αIIbβ3, while the inside-out signaling is only partially affected.

Experimental studies have found that an oligopeptide (RGT peptide) that mimics the C-terminal RGT sequence of integrin β3 is synthesized and modified by tetradecane acylation (Myr-RGT peptide) to enable the oligopeptide to obtain the ability to penetrate the cell membrane, and the RGT peptide acts on human platelets, and then observes the adhesion, extension, aggregation, free fibrinogen binding ability of platelets, etc., which are functions related to outside-in and inside-out signaling of integrin αIIbβ3. The tetradecane acylation-modified RGT peptide dose-dependently inhibited the stable adhesion and extension of normal human platelets on solid-phase fibrinogen; co-incubation of Myr-RGT peptide with platelets inhibited fibrin clot retraction. In addition, Myr-RGT peptides can inhibit the two-phase aggregation (irreversible aggregation) of platelets induced by ADP, thrombin and ristomycin, but have basically no effect on one-phase aggregation (reversible aggregation). The expression of CD 62P on the membrane surface of platelets incubated with Myr-RGT peptide was significantly reduced after thrombin stimulation. These results indicate that the related function of platelet integrin αIIbβ3 mediated by outside-in signaling is inhibited after Myr-RGT peptide treatment. At the same time, related platelet functions mediated by integrin αIIbβ3 inside-out signaling are not affected, including platelet one-phase aggregation and free fibrinogen binding. These studies show that after Myr-RGT peptide action, the inside-out signaling pathway of integrin αIIbβ in platelets is not blocked, and synthetic peptides simulating the three amino acids (RGT) sequence at the end of integrin β3 cytoplasmic segment can selectively inhibit outside-in signaling without affecting inside-out signaling (CN200610117991.7). Recent work by the inventors has further demonstrated that the synthetic RGT peptide inhibits platelet thrombosis under high shear in the fluid state and that this synthetic RGT peptide, which targets the SH3 structural domain of c-Src, does not directly affect the activity of c-Src and therefore does not affect the activity of other intracellular c-Src that are not in the integrin β3 signaling pathway (201811037039.5).

Through Myr-RGT, it is expounded that interfering with the binding of integrin αIIbβ3 RGT sequence and Src kinase SH3 domain can selectively inhibit outside-in signaling, and the mechanism research lays a foundation for applied research. However, due to the limitation of low efficiency of mimetic peptide, it is difficult to apply to the body, which has become a major obstacle to pharmacization and even clinical application. Therefore, it is urgent to explore a new group of small molecule compounds that can target this binding site and be applied efficiently enough in vivo. Therefore, by artificially synthesized small molecule compounds targeting β3/ c-Src interaction to selectively inhibit outside-in signaling without affecting inside-out signaling, it has the potential to develop into a new generation of antithrombotic drugs.

To sum up, the biggest problem in the research and development of antithrombotic drugs is that the difficulty in the research and development of such drugs is to separate the simultaneous platelet thrombosis and hemostatic functions, that is, to distinguish the outside-in signaling and the inside-out signaling of platelets for inhibition. Although some oligopeptides or peptides have been found to selectively inhibit platelet outside-in signalling (related to thrombosis) and have less effect on inside-out signalling (related to hemostasis), the poor membrane permeability and low utilisation efficiency of these mimetic peptides make them difficult to be applied in vivo and have poor druggability, making them a major obstacle to pharmacological and even clinical application. At present, there are no reported inhibitors of β3 and c-Src protein-protein interaction.

Therefore, it is necessary to develop a drug aimed at Src kinase target, which interferes with the binding of integrin αIIbβ3 and Src kinase, thereby selectively inhibiting outside-in signalling, and does not affect inside-out signaling, so that the drug is anti-thrombotic without affecting the normal physiological hemostatic function at the same time, and avoiding the appearance of bleeding side effects, while targeting the integrin target allowing the drug to have multiple therapeutic effects.

### SUMMARY OF THE INVENTION

It is an object of the present invention to provide a structurally novel compound that is antithrombotic without affecting normal physiological hemostatic function.

In the first aspect of the present invention, it provides a compound of formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof: 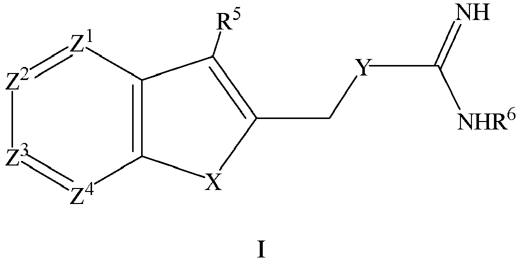
Z¹ is N or CR¹;
Z² is N or CR²;
Z³ is N or CR³;
Z⁴ is N or CR⁴;
with the proviso that 0, 1, or 2 of Z¹, Z², Z³ and Z⁴ are each independently N;
R¹, R², R³, and R⁴ are each independently hydrogen, hydroxyl, amino, C₁-C₆ alkyl, substituted or unsubstituted C₃-C₁₀ cycloalkyl, substituted or unsubstituted C₃-C₁₀ cycloalkenyl, C₂-C₆ alkenyl, C₂-C₆ alkynyl, halogen, C₁-C₆ haloalkyl, G^{a}, -OR^{a}, -OC(O)R^{d}, -OC(O)NR^{b}R^{c}, -SR^{a}, -S(O)₂R^{d}, -S(O)₂NR^{b}R^{c}, -C(O)R^{d}, -C(O)OR^{a}, -C(O)NR^{b}R^{c}, -NR^{b}R^{c}, -N(R^{e})C(O)R^{d}, -N(R^{e})S(O)₂R^{d}, -N(R^{e})C(O)OR^{d}, -N(R^{e})C(O)NR^{b}R^{c}, -N(R^{e})S(O)₂NR^{b}R^{c}, -(C₁-C₆ alkylene)-G^{a}, -(C₁-C₆ alkylene)-OR^{a}, -(C₁-C₆ alkylene)-OC(O)R^{d}, -(C₁-C₆ alkylene)-OC(O)NR^{b}R^{c}, -(C₁-C₆ alkylene)-S(O)₂R^{d}, -(C₁-C₆ alkylene)-S(O)₂NR^{b}R^{c}, -(C₁-C₆ alkylene)-C(O)R^{d}, -(C₁-C₆ alkylene)-C(O)OR^{a}, -(C₁-C₆ alkylene)-C(O)NR^{b}R^{c}, -(C₁-C₆ alkylene)-NR^{b}R^{c}, -(C₁-C₆ alkylene)-N(R^{e})C(O)R^{d}, -(C₁-C₆ alkylene)-N(R^{e})S(O)₂R^{d}, - (C₁-C₆ alkylene)-N(R^{e})C(O)OR^{a}, -(C₁-C₆ alkylene)-N(R^{e})C(O)NR^{b}R^{c}, -(C₁-C₆ alkylene)-N(R^{e})S(O)₂NR^{b}R^{c}, -(C₁-C₆ alkylene)-CN, substituted or unsubstituted C₆-C₁₆ aryl, substituted or unsubstituted 5-16-membered heteroaryl, substituted or unsubstituted C₆-C₁₆ aryl-C₁-C₄ alkyl-, substituted or unsubstituted 5-16-membered heteroaryl-C₁-C₄ alkyl-, wherein the substituted means that one or more (preferably 1, 2, 3 or 4) hydrogens on the group are each independently substituted by a substituent selected from the group consisting of C₁-C₆ alkyl, halogen, C₁-C₆ haloalkyl, and C₃-C₆ cycloalkyl; or, Z³ is CR³, Z⁴ is CR⁴, R³ and R⁴ are connected to form a C6-C12 aromatic ring; or, Z¹ is CR¹, Z² is CR², and R¹ and R² are connected to form a C6-C12 aromatic ring;
R⁵ is -CN, -C(O)OR^{a}, -C(O)NR^{b}R^{c}, or -N(R^{e})C(O)R^{d};
X is independently -NR^{x}-, -O-, or -S-; wherein, R^{x} is hydrogen, C₁-C₆ alkyl, C₂-C₆ alkenyl, C₂-C₆ alkynyl, C₁-C₆ haloalkyl, C₆-C₁₂ aryl, 5-12 membered heteroaryl, G^{b}, or -(C₁-C₆ alkylene)-G^{b};
Y is independently -NH-, -O- or -S-;
R⁶ is hydrogen, C₁-C₆ alkyl, C₁-C₆ haloalkyl, or G^{b};
R^{a}, R^{b}, R^{c} and R^{e} are each independently hydrogen, C₂-C₆ alkenyl, C₂-C₆ alkynyl, C₁-C₆ haloalkyl, G^{b}, C₁-C₆ alkyl, C₃-C₈ cycloalkyl, C₆-C₁₂ aryl, 5-12 membered heteroaryl, or C₁-C₆ alkyl substituted by a substituent, wherein the substituent is -OR^{z1}, -NR^{z1}R^{z2}, -C(O)OR^{z1}, -C(O)NR^{z1}R^{z2}, -S(O)₂R^{z1}, -S(O)₂NR^{z1}R^{z2}, or G^{b};
R^{d} is C₂-C₆ alkenyl, C₂-C₆ alkynyl, C₁-C₆ haloalkyl, G^{b}, C₁-C₆ alkyl, or C₁-C₆ alkyl substituted by a substituent, and the substituent is selected from -OR^{z1}, -NR^{z1}R^{z2}, -C(O)OR^{z1}, -C(O)NR^{z1}R^{z2}, -S(O)₂R^{z1}, -S(O)₂NR^{z1}R^{z2}, or G^{b};
R^{z1} and R^{z2} are each independently hydrogen, C₁-C₆ alkyl, or C₁-C₆ haloalkyl;
G^{a} and G^{b} are each independently C₆-C₁₂ aryl, 5-12-membered heteroaryl, 3-10-membered heterocyclyl, C₃-C₁₀ cycloalkyl, or C₃-C₁₀- cycloalkenyl, and each is independently unsubstituted or substituted by 1, 2, 3, 4, or 5 R^{v};
R^{v} is C₁-C₆ alkyl, C₂-C₆ alkenyl, C₂-C₆ alkynyl, halogen, C₁-C₆ haloalkyl, -CN, oxo, -OR^{h}, -OC(O)R', -OC(O) NR^{j}R^{k}, -SR^{h}, -S(O)₂R^{h}, -S(O)₂NR^{j}R^{k}, -C(O)R^{h}, -C(O)-5-12 membered monocyclic heterocyclyl, -C(O)-5-12 membered monocyclic heteroaryl, -C(O)OR^{h}, -C(O) NR^{j}R^{k}, -NR^{j}R^{k}, -N(R^{h})C(O)Rⁱ, -N(R^{h})S(O)₂Rⁱ, -N(R^{h})C(O)ORⁱ, -N(R^{h})C(O) NR^{j}R^{k}, -(C₁-C₆ alkylene)-OR^{h}, -(C₁-C₆ alkylene)-OC(O)Rⁱ, -(C₁-C₆ alkylene)-OC(O)NR^{j}R^{k}, -(C₁-C₆ alkylene)-S(O)₂R^{h}, -(C₁-C₆ alkylene)-S(O)₂NR^{j}R^{k}, -(C₁-C₆ alkylene)-C(O)R^{h}, -(C₁-C₆ alkylene)-C(O)OR^{h}, -(C₁-C₆ alkylene)-C(O)NR^{j}R^{k}, -(C₁-C₆ alkylene)-NR^{j}R^{k}, -(C₁-C₆ alkylene)-N(R^{h})C(O)Rⁱ, -(C₁-C₆ alkylene)-N(R^{h})S(O)₂Rⁱ, -(C₁-C₆ alkylene)-N(R^{h})C(O)ORⁱ, -(C₁-C₆ alkylene)-N(R^{h})C(O)NR^{j}R^{k}, or -(C₁-C₆ alkylene)-CN;
R^{z1} and R^{z2} are each independently hydrogen, C₁-C₆ alkyl, or C₁-C₆ haloalkyl;
R^{h}, R^{j}, and R^{k} are each independently hydrogen, C₁-C₆ alkyl, or C₁-C₆ haloalkyl; and Rⁱ is independently C₁-C₆ alkyl, or C₁-C₆ haloalkyl at each occurrence;
Rⁱ is C₁-C₆ alkyl, or C₁-C₆ haloalkyl;
each heterocycle of the heterocyclyl and heteroaryl independently has 1 to 4 (preferably 1, 2, 3 or 4) heteroatoms selected from N, O or S.

In another preferred embodiment, **Z¹** is N or CR¹.

In another preferred embodiment, **Z²** is N or CR².

In another preferred embodiment, **Z³** is N or CR³.

In another preferred embodiment, **Z⁴** is N or CR⁴.

In another preferred embodiment, at least one of R¹, R², R³ and R⁴ is not hydrogen.

In another preferred embodiment, at least one of Z¹, Z², Z³, and Z⁴ is not N.

In another preferred embodiment, R¹, R², R³, and R⁴ are each independently hydrogen, hydroxyl, amino, C1-C4 alkyl-O-, C6-C12 aryl-O-, 5-12-membered heteroaryl-O-, C6-C16 aryl, C6-C16 aryl substituted by C1-C4 alkyl, halogenated C6-C16 aryl, 5-16-membered heteroaryl substituted by C1-C4 alkyl, halogenated 5-16-membered heteroaryl, 5-16-membered heteroaryl, C₃-C₈ cycloalkyl-O-, halogen, C₃-C₈ cycloalkenyl, halogenated or unsubstituted C₃-C₈ cycloalkyl, C₆-C₁₆ aryl-C₁-C₄ alkyl-, 5-12-membered heteroaryl-C₁-C₄ alkyl-, benzo-C5-C8 cycloalkyl, or benzo-C5-C8 cycloalkenyl; or, Z³ is CR³, Z⁴ is CR⁴, R³ and R⁴ are connected to form a C6-C12 aromatic ring; or, Z¹ is CR¹, Z² is CR², and R¹ and R² are connected to form a C6-C12 aromatic ring.

In another preferred embodiment, R¹, R², R³ and R⁴ are each independently hydrogen, hydroxyl, amino, C1-C4 alkoxy, naphthyl substituted by C1-C4 alkyl, C₆-C₁₂ aryl, 5-16-membered heteroaryl, halogenated naphthyl (preferably single halogenated naphthyl), C₃-C₈ cycloalkyl-O-, C₆-C₁₂ aryl-O-, 5-12-membered heteroaryl-O-, anthryl, halogen, benzopyranyl, C₃-C₈ cycloalkenyl, dihalogenated or unsubstituted C₃-C₈ cycloalkyl, C₆-C₁₂ aryl-C₁-C₄ alkyl-, benzo-C5-C6 cycloalkyl; or, Z³ is CR³, Z⁴ is CR⁴, R³ and R⁴ are connected to form a C6-C12 aromatic ring; or, Z¹ is CR¹, Z² is CR², and R¹ and Rare connected to form a C6-C12 aromatic ring.

In another preferred embodiment, R¹, R², R³, and R⁴ are each independently hydrogen, hydroxyl, amino, methoxy, monomethylnaphthyl, isoquinolinyl, quinolinyl, mono-halogenated naphthyl, phenyl, naphthyl, cyclopentyl-O-, cyclohexyl-O-, anthryl, halogen (such as bromine), benzopyranyl, 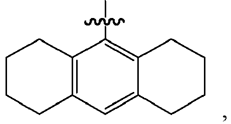 cyclopentenyl, cyclohexenyl, cycloheptenyl, cyclopropyl, cyclopentyl, cyclohexyl, cycloheptyl, benzyl, benzocyclopentyl, benzocycloheptenyl, dihalocyclohexyl, pyridyl, pyridyl-O-; or, Z³ is CR³, Z⁴ is CR⁴, R³ and R⁴ are connected to form benzene ring; or, Z¹ is CR¹, Z² is CR², and R¹ and R² are connected to form benzene ring.

In another preferred embodiment, the naphthyl substituted by C1-C4 alkyl is naphthyl monosubstituted by C1-C4 alkyl.

In another preferred embodiment, the naphthyl is

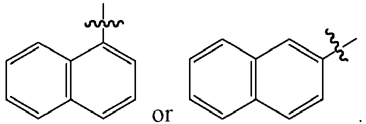

In another preferred embodiment, the monomethylnaphthyl is

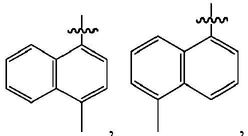

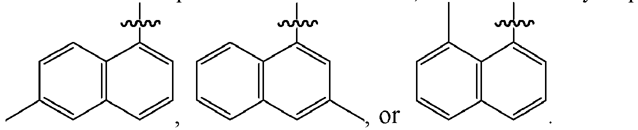

In another preferred embodiment, the isoquinolinyl

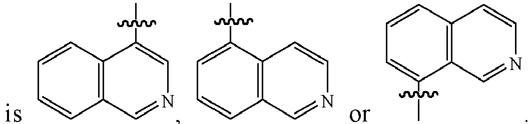

In another preferred embodiment, the quinolinyl is 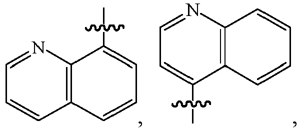 or

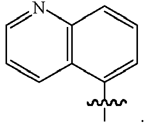

In another preferred embodiment, the mono-halogenated naphthyl is

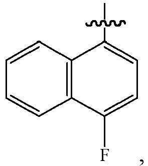

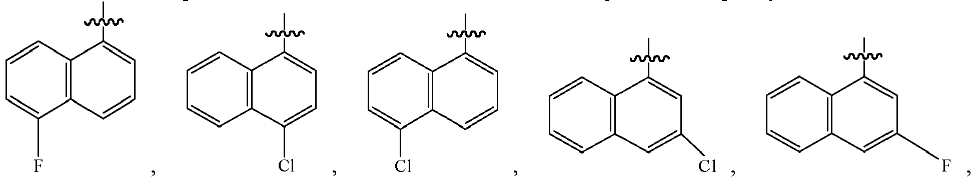

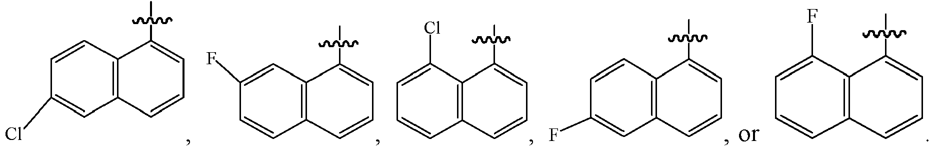

In another preferred embodiment, the anthryl

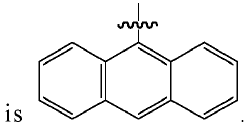

In another preferred embodiment, the benzopyranyl- has a structure of

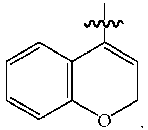

In another preferred embodiment the benzocyclopentyl is

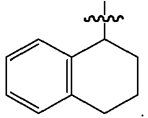

In another preferred embodiment, the dihalogenated cyclohexyl is

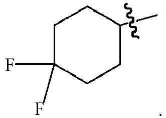

In another preferred embodiment, the cycloheptyl is

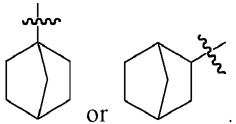

In another preferred embodiment, the benzocycloheptenyl is

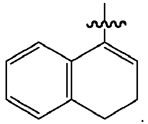

In another preferred embodiment, the substituted means that one or more (preferably 1, 2, 3 or 4) of the hydrogen on the group are each independently substituted by a substituent.

In another preferred embodiment, the C6-C16 aryl is C6-C12 aryl.

In another preferred embodiment, the 5-16-membered heteroaryl is 5-12-membered heteroaryl.

As used herein, " 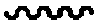 " is the substitution site.

In another preferred embodiment, X is independently -NR^{x}-, -O-, or -S-; wherein R^{x} is hydrogen, C₁-C₆ alkyl, C₆-C₁₂ aryl, or 5-12 membered heteroaryl.

In another preferred embodiment, X is independently -NR^{x}-, -O-, or -S-; R^{x} is hydrogen, methyl, propyl, or phenyl.

In another preferred embodiment, R^{a} is C₁-C₆ alkyl.

In another preferred embodiment, R^{a} is ethyl.

In another preferred embodiment, R^{b}, R^{c} and R^{e} are each independently hydrogen, or C₁-C₆ alkyl.

In another preferred embodiment, R^{b}, R^{c} and R^{e} are each independently hydrogen or methyl.

In another preferred embodiment, R^{d} is C₁-C₆ alkyl.

In another preferred embodiment, R^{d} is methyl.

In another preferred embodiment, R⁵ is -CN, -C(O)OR^{a}, -C(O)NR^{b}R^{c}, or -N(R^{e})C(O)R^{d}; wherein, R^{a} is C₁-C₆ alkyl; R^{b}, R^{c} and R^{e} are each independently hydrogen, or C₁-C₆ alkyl; R^{d} is C₁-C₆ alkyl.

In another preferred embodiment, R⁵ is -CN, -C(O)OR^{a}, -C(O)NR^{b}R^{c}, or -N(R^{e})C(O)R^{d}; wherein, R^{a} is C₁-C₄ alkyl; R^{b}, R^{c} and R^{e} are each independently hydrogen, or C₁-C₄ alkyl; R^{d} is C₁-C₄ alkyl.

In another preferred embodiment, **Y** is independently -NH- or -S-.

In another preferred embodiment, **R⁶ is** hydrogen, C₁-C₄ alkyl, or C₁-C₄ haloalkyl.

In another preferred embodiment, **R⁶** is hydrogen or ethyl.

In another preferred embodiment, Z¹, Z², Z³, Z⁴, X, R⁵, Y and R⁶ are each the corresponding group or substituent of the specific compound as described in Table 1.

In another preferred embodiment, the compound has one or more features selected from the group consisting of:
R¹, R², R³, and R⁴ are each independently hydrogen, hydroxyl, amino, methoxy, monomethylnaphthyl, isoquinolinyl, quinolinyl, mono-halogenated naphthyl, phenyl, naphthyl, cyclopentyl-O-, cyclohexyl-O-, anthryl, halogen (such as bromine, benzopyranyl, 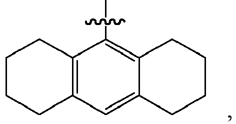 cyclopentenyl, cyclohexenyl, cycloheptenyl, cyclopropyl, cyclopentyl, cyclohexyl, cycloheptyl, benzyl, benzocyclopentyl, benzocycloheptenyl, dihalocyclohexyl, pyridyl, pyridyl-O-; or, Z³ is CR³, Z⁴ is CR⁴, R³ and R⁴ are connected to form benzene ring; or, Z¹ is CR¹, Z² is CR², and R¹ and R² are connected to form benzene ring;
X is independently -NR^{x}-, -O-, or -S-; R^{x} is hydrogen, methyl, propyl, or phenyl;
R^{a} is C₁-C₆ alkyl;
R^{b}, R^{c} and R^{e} are each independently hydrogen, C₁-C₆ alkyl;
R^{d} is C₁-C₆ alkyl;
R⁵ is -CN, -C(O)OR^{a}, -C(O)NR^{b}R^{c}, -N(R^{e})C(O)R^{d}; wherein R^{a} is C₁-C₄ alkyl; R^{b}, R^{c} and R^{e} are each independently hydrogen, C₁-C₄ alkyl; R^{d} is C₁-C₄ alkyl;
Y is independently -NH- or -S-;
R⁶ is hydrogen, C₁-C₄ alkyl, C₁-C₄ haloalkyl.

In another preferred embodiment, the compound is selected from the group consisting of:

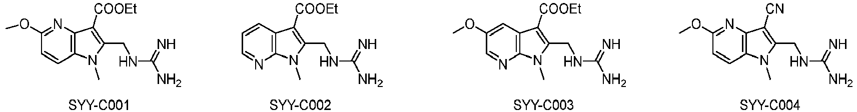

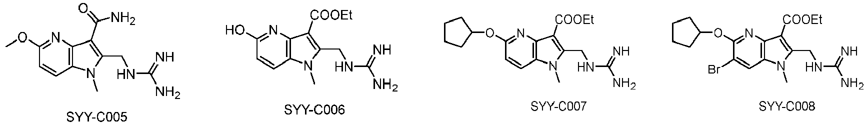

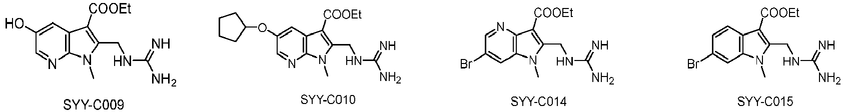

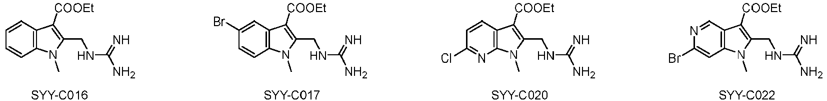

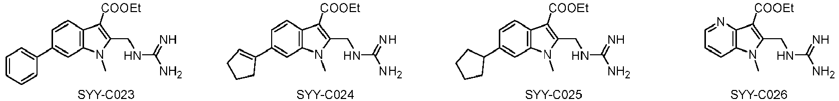

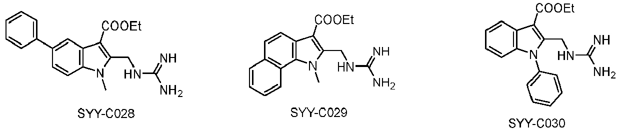

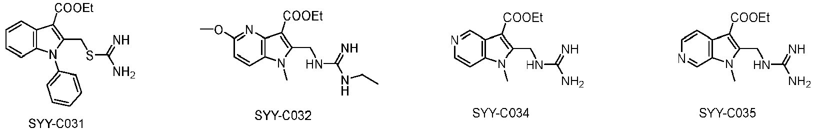

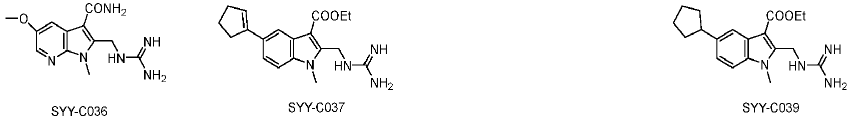

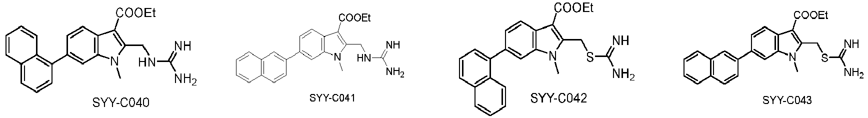

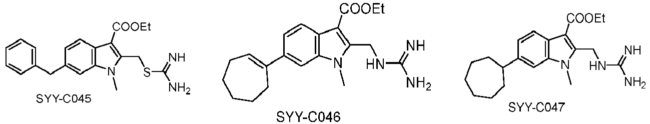

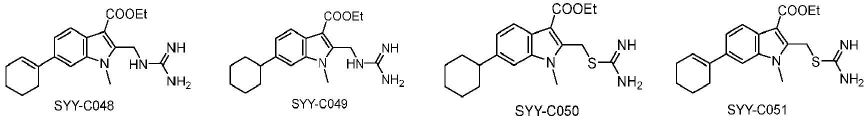

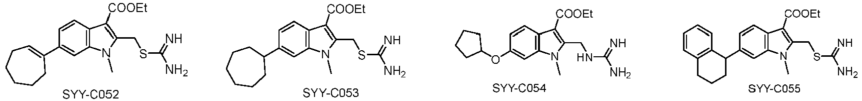

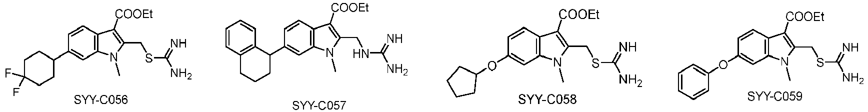

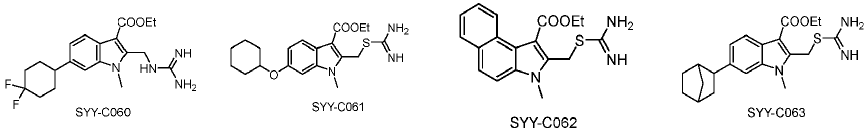

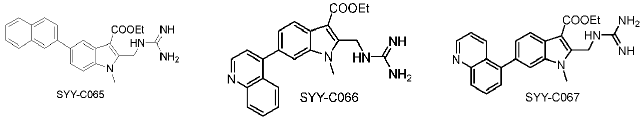

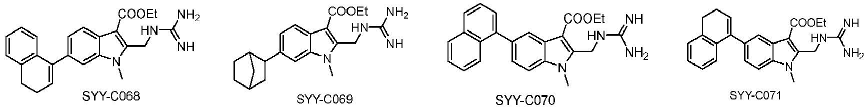

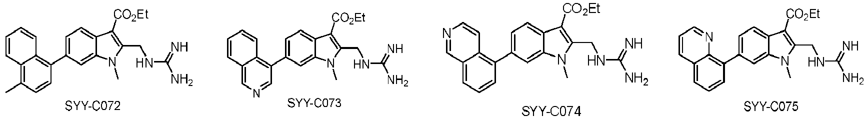

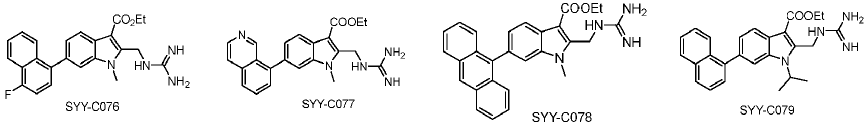

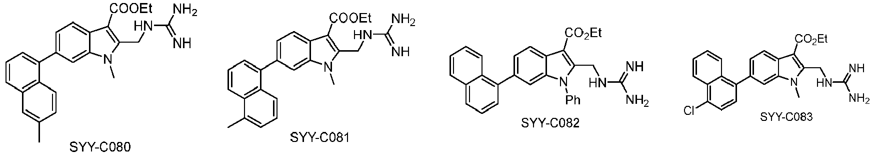

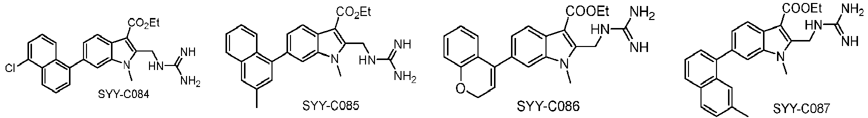

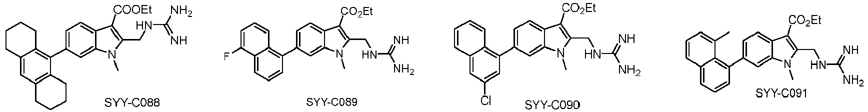

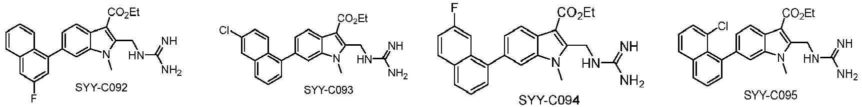

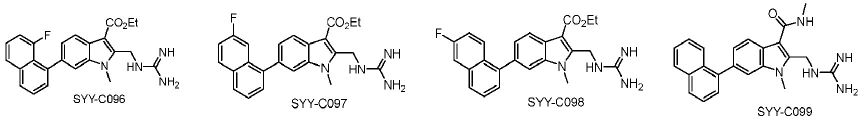

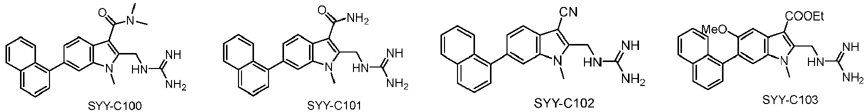

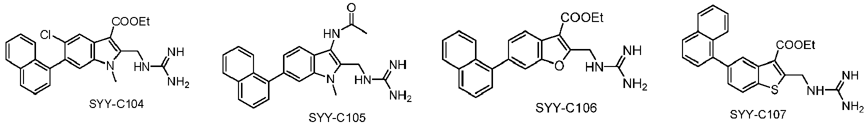

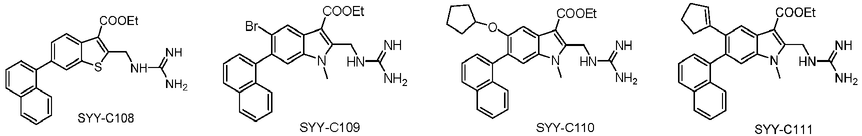

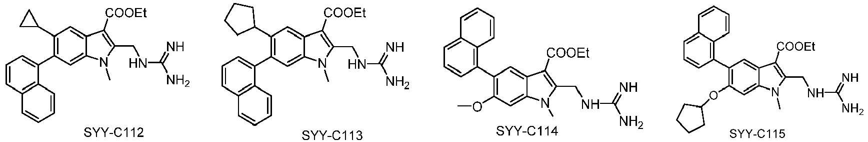

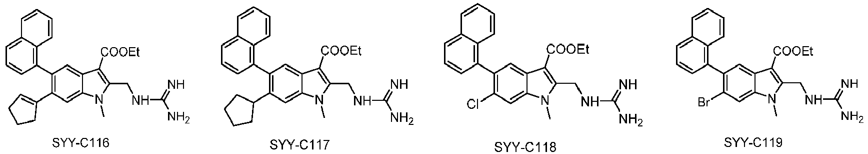

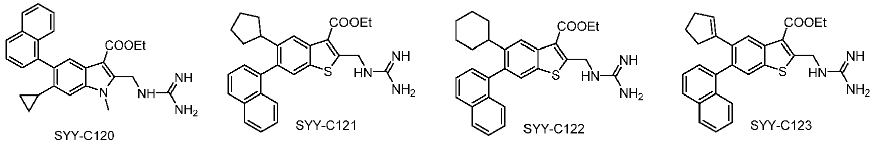

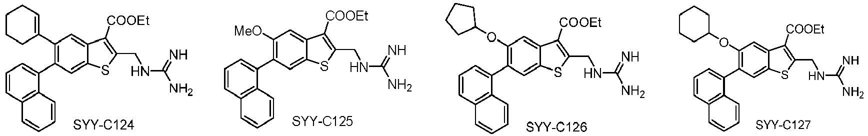

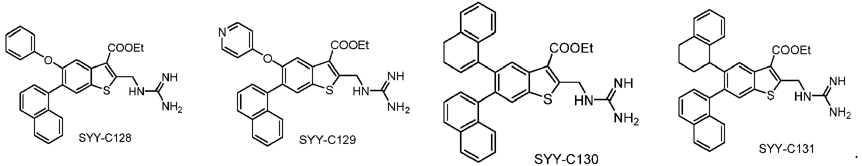

In the second aspect of the present invention, it provides a pharmaceutical composition comprising:
(a) the compound of formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof of the first aspect of the present invention; and
(b) pharmaceutically acceptable carriers.

In another preferred embodiment, the content of the compound of formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof is 0.01-99.99 wt.%, preferably 0.1-99.9 wt.%, more preferably 1-99 wt.%, more preferably 5-95 wt.%, more preferably 10-90 wt.%, more preferably 20-80 wt.%, most preferably 30-70 wt.%, by the weight of the composition.

In another preferred embodiment, the pharmaceutical composition further comprises a medicament for preventing and/or treating thrombosis.

In another preferred embodiment, the dosage form of the pharmaceutical composition is an oral or parenteral preparation (e. g., an injection, an infusion) .

In another preferred embodiment, the dosage form of the pharmaceutical composition is selected from the group consisting of injections, capsules, tablets, pills, powders, granules, aerosols, suppositories, film agents, drop pills, and topical liniments.

In another preferred embodiment, the pharmaceutical composition is a controlled-release formulation, sustained-release formulation or nano-formulation.

In the third aspect of the present invention, it provides a use of the compound of formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof of the first aspect of the present invention for the preparation of a composition or a formulation for one or more uses selected from the group consisting of: (1) selective inhibition of outside-in signaling mediated by the interaction of integrin β3 with Src kinase; (2) prevention and/or treatment of diseases related to outside-in signaling mediated by the interaction of integrin β3 with Src kinase; and (3) prevention and/or treatment of thrombosis.

In another preferred embodiment, the inhibition of the interaction of integrin β3 with Src kinase comprises inhibiting the interaction of integrin β3 with the SH3 domain of Src kinase.

In another preferred embodiment, the selective inhibition of the outside-in signaling mediated by the interaction of integrin β3 with Src kinase refers to the selective inhibition of the outside-in signaling mediated by the interaction of integrin β3 with Src kinase without affecting the inside-out signaling.

In another preferred embodiment, the interaction of integrin β3 with Src kinase refers to the interaction of integrin β3 in platelets with c-Src kinase.

In another preferred embodiment, the integrin β3 is β3 of integrin αIIbβ3 and/or β3 of integrin αvβ3.

In another preferred embodiment, the diseases related to outside-in signaling mediated by the interaction of integrin β3 with Src kinase is selected from the group consisting of thrombus, tumor, osteoporosis, endothelial cell-mediated angiogenesis, and a combination thereof.

In another preferred embodiment, the prevention and/or treatment of thrombosis does not affect bleeding or improve bleeding while achieving anti-thrombosis.

In another preferred embodiment, the improve bleeding includes inhibiting bleeding, not increasing bleeding risk, reducing bleeding risk, not causing bleeding side effects, and/or not affecting hemostatic function.

In another preferred embodiment, the hemostatic function comprises a platelet hemostatic function.

In another preferred embodiment, the hemostasis comprises physiological hemostasis.

In another preferred embodiment, the thrombosis is a cardio-cerebrovascular thrombus, and the cardio-cerebrovascular thrombus is selected from the group consisting of: myocardial infarction thrombus, cerebral infarction thrombus, ischemic stroke, atherosclerotic thrombus, and a combination thereof.

In another preferred embodiment, the prevention and/or treatment of thrombosis includes one or more ways selected from the group consisting of:
(3-1) inhibition of the extension function of platelets on solid phase fibrinogen;
(3-2) inhibition of platelet aggregation, adhesion and/or extension;
(3-3) inhibition of fibrin clot retraction;
(3-3) not affecting the binding function of platelet binding to free fibrinogen.

In another preferred embodiment, the inhibition of platelet aggregation comprises inhibiting one-phase or two-phase aggregation of platelets.

In another preferred embodiment, the composition or formulation is a pharmaceutical composition or formulation.

In another preferred embodiment, the composition or formulation further comprises a pharmaceutically acceptable carrier.

In another preferred embodiment, the composition or formulation further comprises other antithrombotic drugs, other anti-tumor drugs, other drugs for treating osteoporosis, and/or other anti-angiogenic drugs.

In another preferred embodiment, the composition or formulation is in the form of a tablet, an injection, an infusion, a paste, a gel, a solution, a microsphere or a film.

In the fourth aspect of the present invention, it provides a method for preventing and/or treating thrombosis comprising the steps of:
administrating of the compound of formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof of the first aspect of the present invention to a subject in need thereof so as to prevent and/or treat thrombosis.

In another preferred embodiment, the subject is a human and non-human mammal (rodent, rabbit, monkey, domestic animal, dog, cat, etc.).

It should be understood that in the present invention, any of the technical features specifically described above and below (such as in the Example) can be combined with each other, thereby constituting new or preferred technical solutions. Limited by space, it will not be repeated here.

### DESCRIPTION OF FIGURES

Figure 1 shows the experimental results of the surface plasmon resonance (SPR) of the compounds.
Figure 2 shows the inhibitory effect of the compound on platelet extension. The compound can concentration-dependently inhibit platelet extension.

### DETAILED DESCRIPTION OF THE INVENTION

After long-term and in-depth research, through a large number of screening, the inventor accidentally found that a class of compounds shown in formula I that can specifically interfere with the binding of integrin αIIbβ3 to Src kinase after binding to the SH3 structural domain protein of Src kinase, thereby selectively inhibiting outside-in signaling (related to thrombosis) and not affecting inside-out signaling (related to hemostatic function), thus allowing the compounds of the present invention to be anti-thrombotic without affecting normal physiological hemostatic function and avoiding bleeding side effects. On this basis, the inventor completed the present invention.

### Terms

Unless otherwise defined, all technical and scientific terms used herein have the same meaning as generally understood by those of ordinary skilled in the art to which the present invention belongs.

As used herein, the terms "include", "contain" and "comprise" are used interchangeably, including not only open definitions, but also semi-closed, and closed definitions. In other words, the term includes "consisting of" and "essentially consisting of".

As used herein, "Src kinase" is Src kinase, a non-receptor tyrosine kinase.

As used herein, "SH3", "SH3 domain", "SH3 domain protein", "SH3 protein" are used interchangeably.

It is to be understood that those of ordinary skilled in the art may select the substituents and substitution patterns on the compounds of the present invention to produce chemically stable compounds which may be synthesized by techniques known in the art and by the methods set forth below. If substituted by more than one (more) substituent group, it should be understood that the more groups may be on the same carbon or on different carbons, as long as a stable structure is produced.

As used herein, the term "substitution" or "substituted" is the substitution of a hydrogen atom on a group by a non-hydrogen atom group, but it is necessary to meet its valence requirements and to produce chemically stable compounds from the substitution, i .e. compounds that do not spontaneously undergo transformations such as cyclization, elimination, etc.

As used herein, "R₁", "R1", and "R¹" have the same meanings and can be substituted for each other, and other similar definitions have the same meanings.

As used herein, " 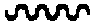 " denotes the attachment site of the group.

As used herein, the term "alkyl" refers to a linear (i. e. unbranched) or branched saturated hydrocarbon group containing only carbon atoms, or a combination of linear and branched groups. When the number of carbon atoms before the alkyl is defined (e. g., C1-C6 alkyl), it refers to that the alkyl contains 1-6 carbon atoms, for example, C1-C6 alkyl contains 1-6 carbon atoms. Representative examples include but are not limited to methyl, ethyl, propyl, isopropyl, butyl, isobutyl, sec-butyl, tert-butyl, or the like.

As used herein, the term "alkylene" refers to a group formed by the removal of one hydrogen from an alkyl, which is as defined herein above. Representative examples of C₁-C₆ alkylene include, but are not limited to, methylene, ethylene, propylene, isopropylene, butylene, isobutylene, sec-butylene, tert-butylene, or the like.

As used herein, the term "alkenyl" refers to a linear or branched carbon chain group having at least one carbon-carbon double bond. When the number of carbon atoms before the alkenyl is defined (such as C₂-C₆), it refers to that the alkenyl contains 2-6 carbon atoms. For example, C₂-C₆ alkenyl refers to an alkenyl containing 2-6 carbon atoms, including vinyl, propenyl, 1,2-butenyl, 2,3-butenyl, butadienyl, or the like.

As used herein, the term "alkynyl" refers to an aliphatic hydrocarbon group having at least one carbon-carbon triple bond. The alkynyl may be linear or branched, or a combination thereof. When the number of carbon atoms before the alkynyl is defined (such as C₂-C₆ alkynyl), it refers to that alkynyl contains 2-6 carbon atoms. For example, the term "C₂-C₆ alkynyl" refers to a linear or branched alkynyl having 2-6 carbon atoms, including ethynyl, propynyl, isopropynyl, butynyl, isobutynyl, sec-butynyl, t-butynyl, or the like.

In the present invention, the term "halogen" refers to F, Cl, Br or I.

In the present invention, the term "halogenated" refers to substitution by halogen.

As used herein, the term "haloalkyl" refers to the substitution of one or more (preferably 1, 2, 3 or 4) hydrogen of an alkyl with halogen, the alkyl and halogen are as defined above, when the number of carbon atoms before the alkyl is defined (such as C1-C6 haloalkyl), it refers to that the alkyl contains 1-6 carbon atoms, for example, C1-C6 haloalkyl refers to a haloalkyl containing 1-6 carbon atoms. Representative examples include, but are not limited to, -CF3, -CHF₂, monofluoroisopropyl, difluorobutyl, or the like.

As used herein, the term "cycloalkyl" refers to a cyclic group having a saturated monocyclic, bicyclic or polycyclic (fused, bridged or spiro) ring system. When the number of carbon atoms before a certain cycloalkyl is defined (e. g., C3-C12), it refers to that the cycloalkyl has 3-12 ring carbon atoms. In some preferred embodiments, the term "C3-C8 cycloalkyl" refers to a saturated monocyclic or bicyclic cycloalkyl having 3-8 cyclic carbon atoms, including cyclopropyl, cyclobutyl, cyclopentyl, cycloheptyl, or the like. "Spirocycloalkyl" refers to a bicyclic or polycyclic group that shares a carbon atom (called a spiro atom) between monocyclic rings which can contain one or more double bonds, but none of the rings have a fully conjugated π electron system. "Fused cycloalkyl" refers to an all-carbon bicyclic or polycyclic group in which each ring in the system shares an adjacent pair of carbon atoms with the other rings in the system, where one or more rings may contain one or more double bonds, but none of the rings have a fully conjugated π electron system. "Bridged cycloalkyl" refers to an all-carbon polycyclic group in which any two rings share two carbon atoms that are not directly connected. These rings can contain one or more double bonds, but none of the rings have a fully conjugated π electron system. The naphthenic ring of the cycloalkyl can be fused to the aromatic ring, but the connection site is located on the naphthenic ring, not on the aromatic ring.

The following are representative examples of cycloalkyl, including but not limited:

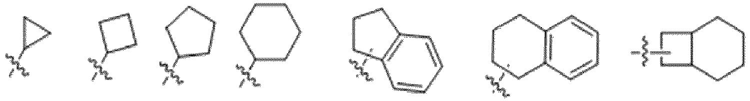

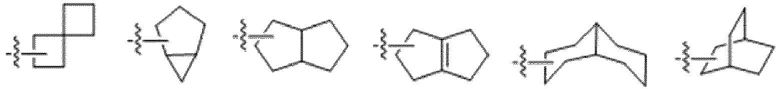

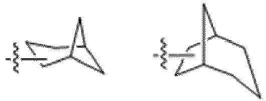

As used herein, the term "cycloalkenyl" refers to that the cycloalkyl ring has one or more double bonds on the naphthenic ring, but the double bonds cannot form a ring with a conjugated π-electron system, and the connection site is located on the carbon atom of a double bond. The cycloalkyl is as defined above, the ring of the cycloalkenyl may be fused to the aromatic ring, but the connection site cannot be on the aromatic ring.

As used herein, the term "halocycloalkyl" refers to the substitution of one or more (preferably 1, 2, 3 or 4) hydrogens of cycloalkyl with halogen, and the cycloalkyl and halogen are as defined above. When the number of carbon atoms before the cycloalkyl is defined (such as C3-C8 haloalkyl), it means that the cycloalkyl contains 3-8 ring carbon atoms, for example, C3-C8 haloalkyl refers to halocycloalkyl containing 3-6 carbon atoms, representative examples include, but are not limited to, monofluoro cyclopropyl, monochloro cyclobutyl, monofluoro cyclopentyl, difluoro cycloheptyl, or the like.

The term "alkoxy" refers to a group of R-O-, wherein R is alkyl which is as defined above, when the number of carbon atoms before the alkoxy is defined, such as C1-C4 alkoxy refers to that the alkyl in the alkoxy has 1 to 4 carbon atoms. Representative examples of alkoxy include, but are not limited to, methoxy, ethoxy, n-propoxy, isopropoxy, tert-butoxy, or the like.

As used herein, the term "haloalkoxy" refers to haloalkyl-O-, and the haloalkyl is as defined above, for example, C1-C6 haloalkoxy refers to haloalkoxy having 1 to 6 carbon atoms, representative examples include, but are not limited to, monofluoromethoxy, monofluoroethoxy, difluorobutoxy, or the like.

The term "heterocyclyl" is also referred to as a "heterocycloalkyl" and refers to a fully saturated or partially unsaturated ring (including, but not limited to, such as a 3-7-membered monocyclic ring, a 7-11 membered bicyclic ring, or an 8-16-membered tricyclic system) in which at least one heteroatom is present in a ring having at least one carbon atom. When the number of member before the heterocyclyl is defined, it refers to the number of ring atoms of the heterocyclyl. For example, a 3-16-membered heterocyclyl refers to a heterocyclyl having 3-16 ring atoms. Each heterocyclyl containing heteroatoms may carry one or more (e. g., 1, 2, 3, or 4) heteroatoms, each independently selected from nitrogen atom, oxygen atom or sulfur atom, wherein the nitrogen atom or sulfur atom can be oxidized, and the nitrogen atom can also be quaternized. The heterocyclic ring can be attached to any heteroatom or carbon atom residue of a ring or ring system molecule. Typical monocyclic heterocycloalkyl rings include but are not limited to azetidine, oxetane, imidazoline, imidazolidine, tetrahydrofuran, piperidine, piperazine, 2-oxo piperazine, 2-oxo piperidine, 4-piperidone, tetrahydropyran, morpholine, thiomorpholine, thiomorpholine sulfoxide, thiomorpholine sulfone, 1, 3-dioxane and tetrahydro-1, 1-dioxythiophene, etc. Polycyclic heterocycloalkyl ring includes spiro, fused and bridged heterocyclic ring; the spiro, fused and bridged heterocyclic ring involved are optionally connected to other rings by single bond, or are further fused to other naphthenic rings and heterocyclic rings by any two or more atoms on the ring.

The term "aromatic ring" refers to an all-carbon monocyclic or fused polycyclic ring with a conjugated π electron system (that is, a ring that shares adjacent pairs of carbon atoms) which is an aromatic cyclic hydrocarbon. When the number of carbon atoms before the aromatic ring is defined, such as the C6-C12 aromatic ring, it means that the aromatic ring has 6-12 ring carbon atoms, such as benzene ring and naphthalene ring. The aromatic ring may be fused to other rings (including saturated or unsaturated rings), but cannot contain heteroatoms such as nitrogen, oxygen, or sulfur, and the point of connection to the parent must be on a carbon atom of the ring with a conjugated π-electron system. The following are representative examples of aromatic rings, including but not limited to: benzene ring, naphthalene ring, anthracene ring, or the like.

The term "aryl" refers to an all-carbon monocyclic or fused polycyclic ring with a conjugated π electron system (that is, a ring that shares adjacent pairs of carbon atoms) which is an aromatic cyclic hydrocarbon. When the number of carbon atoms before the aromatic ring is defined, such as the C6-C12 aryl, it means that the aryl has 6-12 ring carbon atoms, such as phenyl and naphthyl. The aryl may be fused to other rings (including saturated or unsaturated rings), but cannot contain heteroatoms such as nitrogen, oxygen, or sulfur, and the point of connection to the parent must be on a carbon atom of the ring with a conjugated π-electron system. The following are representative examples of aryl, including but not limited to:

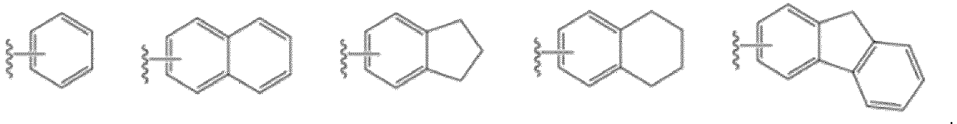

The term "heteroaryl" refers to an aromatic heterocyclic group having one to more (preferably 1, 2, 3 or 4) heteroatoms, which may be monocyclic or polycyclic (bicyclic, tricyclic or polycyclic) fused together or covalently connected, each heterocyclic ring containing heteroatoms may have one or more (e. g., 1, 2, 3, 4) heteroatoms each independently selected from the group consisting of oxygen, sulfur, and nitrogen. When the number of member before heteroaryl is defined, it refers to the number of ring atoms of heteroaryl. For example, 5-12-membered heteroaryl refers to heteroaryl with 5-12 ring atoms. Examples include but are not limited to: pyrrolyl, pyrazolyl, imidazolyl, oxazolyl, isoxazolyl, thiazolyl, thiadiazolyl, isothiazolyl, furanyl, pyridyl, pyrazinyl, pyrimidinyl, pyridazinyl, triazinyl, triazolyl and tetrazolyl. The heteroaromatic ring can be fused to other cyclic groups (including saturated or unsaturated rings), and the point of connecting the parent must be on the heteroaromatic ring.

As used herein, the term "amino" (alone or as part of another substituent) refers to -NH₂.

As used herein, the term "nitro" (alone or as part of another substituent) refers to -NO₂.

As used herein, the term "hydroxyl" (alone or as part of another substituent) refers to -OH.

In this specification, it should be interpreted that all substituents are unsubstituted, unless explicitly described herein as "substituted". The term "substitution" refers to the substitution of one or more hydrogen atoms on a specific group by specific substituent. The specific substituent is the substituent described above, or the substituent appearing in each example. Preferably, the substitution is one or more (preferably 1, 2, 3, 4 or 5) hydrogen atoms on the ring or group are substituted by a substituent selected from the group consisting of C1-C8 alkyl, C3-C8 cycloalkyl, C1-C8 haloalkyl, C3-C8 halocycloalkyl, halogen, nitro, -CN, hydroxyl, mercapto, amino, C1-C4 carboxyl, C2-C4 ester group, C2-C4 amido, C1-C8 alkoxy, C1-C8 alkylthio, C1-C8 haloalkoxy, C1-C8 haloalkylthio, C6-C12 aryl, 5-10 membered heteroaryl, and 5-10 membered heterocycloalkyl. Unless otherwise specified, an arbitrary substituted group may have a substituent selected from a specific group at any substitutable position of the group, and the substituent may be the same or different at each position.

In the present invention, the term "prevention" means a method of preventing the onset of a disease and/or its accompanying symptoms or of protecting a subject from acquiring a disease. The "prevention" used herein also includes delaying the onset of the disease and/or its accompanying symptoms and reducing the risk of acquiring a disease of the subject.

The "treatment" described in the present invention includes delaying and terminating the progression of the disease, or eliminating the disease, and does not require 100% inhibition, elimination and reversal. In some embodiments, compared to the level observed in the absence of the composition, kit, food kit or health care kit, active ingredient combination of the present invention, the compositions or pharmaceutical compositions of the present invention reduce, inhibit and/or reverse related diseases (such as tumors) and their complications by inhibiting the mitochondrial oxidative phosphorylation pathway, for example, at least about 10%, at least about 30%, at least about 50%, or at least about 80%.

### Active ingredient

As used herein, "compound of the present invention", "compound shown in formula I", "compound of formula I", "compound of formula I of the present invention", or "compound shown in formula I of the present invention" can be used interchangeably, refers to the compound shown in formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof, 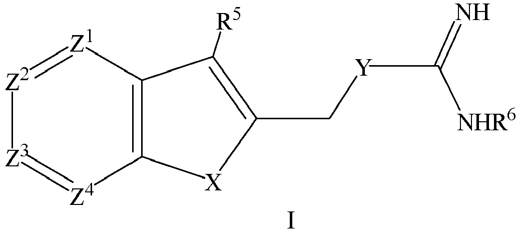 wherein, the definition of each group is as described in the first aspect of the invention above. It should be understood that the term also includes a mixture of the above components.

In another preferred embodiment, the compound is preferably the compound prepared in the Example.

The term "pharmaceutically acceptable salt" refers to a salt formed by a compound of the invention with an acid or base suitable for use as a medicament. Pharmaceutically acceptable salt comprises inorganic salt and organic salt. A preferred class of salt is a salt formed by a compound of the present invention with an acid, The acids suitable for salt formation include but are not limited to mineral acids such as hydrochloric acid, hydrobromic acid, hydrofluoric acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulfonic acid, and benzenesulfonic acid; and acidic amino acids such as aspartic acid and glutamic acid. A preferred class of salts are metal salts formed by the compounds of the invention with bases, suitable bases for salt formation include (but are not limited to): inorganic bases such as sodium hydroxide, potassium hydroxide, sodium carbonate, sodium bicarbonate, sodium phosphate, etc., the organic bases such as ammonia, triethylamine, diethylamine, etc.

The compound of formula I described in the present invention can be converted into a pharmaceutically acceptable salt thereof by a conventional method. For example, a solution of a corresponding acid can be added to the solution of the above-mentioned compound, and the corresponding salt of the compound of the present invention can be obtained by removing the solvent after the salt is completely formed.

### Preparation method

The preparation method of compound shown in formula (I) of the present invention is more specifically described below, but these specific methods do not constitute any limitation of the invention. The compound of the invention may also optionally be conveniently prepared by combining the various synthetic methods described in this specification or known in the art, such a combination may be easily performed by a skilled person in the art to which the invention belongs.

Typically, the preparation process for the compounds of the present invention is as follows, in which the raw materials and reagents used may be commercially purchased unless otherwise specified.

Exemplarily, the compound shown in formula I of the present invention is prepared as follows:

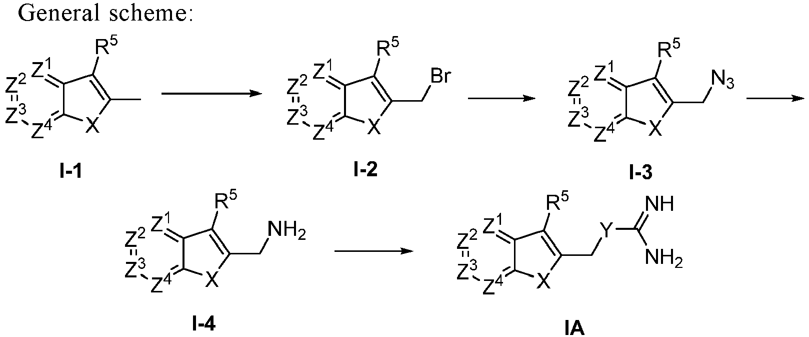

The method for preparing compounds of general formula **IA** is given in the scheme, wherein, the definitions of Z¹, Z², Z³, Z⁴, X, Y and R⁵ are as described above.

Synthesis of **1-2:** a substrate (formula **1-1**), a brominating reagent, and an initiator are reacted in an organic solvent. Examples of the brominating reagent include, but are not limited to, elemental bromine, inorganic bromides (hydrobromic acid, sodium bromide, etc.), N-bromosuccinimide (NBS), Pyridine Hydrobromide Perbromide (PHP), dibromohydantoin (DBDMH), tetrabromocycloketone (TBCO), etc., preferably NBS. Examples of the initiators include, but are not limited to, the organic peroxides, such as cyclohexanone peroxide, benzoyl peroxide (BPO), tert-butyl hydrogen peroxide, etc., azo-based initiators, such as azobisisobutyronitrile, 2,2'-azobis-(2,4-dimethylvaleronitrile), etc., preferably BPO. Examples of organic solvents include acetonitrile, chloroform, carbon tetrachloride, etc., preferably chloroform. Examples of reaction temperatures include from room temperature to reflux, preferably reflux.

Synthesis of **1-3: 1-2** and sodium azide are reacted in an organic solvent. The organic solvent such as, but not limited to, N,N-dimethylformamide (DMF), tetrahydrofuran (THF), acetonitrile, dioxane, etc., preferably N,N-dimethylformamide (DMF). Examples of reaction temperatures include from -20 °C to reflux, preferably room temperature.

Synthesis of **1-4: 1-3** is used as a substrate and I-4 is prepared according to conventional methods well known in the art. According to the specific substrate, Pd/C catalytic hydrogenation reaction or staudinger reaction is preferred, the organic solvent is but not limited to N,N-dimethylformamide (DMF), tetrahydrofuran (THF), acetonitrile, dioxane, methanol, ethanol, preferably THF. Examples of reaction temperatures include from room temperature to reflux, preferably room temperature or reflux temperature.

Synthesis of **IA:** the substrate (formula **I-4**), 1H-pyrazol-1- formamidine hydrochloride (Y is defined as NH), and'base are reacted in an organic solvent. Examples of the bases include, but are not limited to, potassium carbonate, sodium carbonate, cesium carbonate, sodium hydroxide, potassium hydroxide, potassium tert-butoxide, sodium tert-butoxide, sodium hydrogen (NaH), triethylamine, diisopropylethylamine, pyridine, 1,5-diazabicyclo [5.4.0] undec-5-ene, 4-dimethylaminopyridine (DMAP), preferably potassium carbonate; the organic solvent is but not limited to N,N-dimethylformamide (DMF), tetrahydrofuran (THF), acetonitrile, dioxane, dichloromethane (DCM), dichloroethane (DCE), chloroform, methanol, ethanol, etc., preferably acetonitrile. Examples of reaction temperatures include from room temperature to reflux, preferably room temperature. Or the substrate (formula **I-4**), and thiourea (Y is defined as S) are heated to react in an organic solvent such as but not limited to methanol, ethanol, N,N-dimethylformamide (DMF), tetrahydrofuran (THF), acetonitrile, dioxane, dichloromethane (DCM), dichloroethane (DCE), chloroform, etc., preferably ethanol. Examples of reaction temperatures include from room temperature to reflux, preferably reflux.

### The synthesis of 1-1 has the following route:

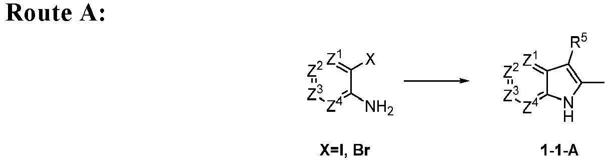

The preparation of compounds of formula **1-2-1** is given in route A.

Indole ring is closing, wherein the definitions of Z¹, Z², Z³, Z⁴ and R⁵ are consistent with the previous one.

Synthesis of 1-1-A: substrate, CuI, ethyl acetoacetate, and tetrazolazole acetic acid and base are reacted in an organic solvent. Examples of the base in the reaction include, but are not limited to, potassium carbonate, sodium carbonate, cesium carbonate, sodium hydroxide, potassium hydroxide, potassium tert-butoxide, sodium tert-butoxide, sodium hydrogen (NaH), triethylamine, diisopropylethylamine, pyridine, 1,5-diazabicyclo [5.4.0] undec-5-ene, 4-dimethylaminopyridine (DMAP), preferably potassium carbonate; the organic solvent is but not limited to, dimethyl sulfoxide (DMSO), N-methylpyrrolidone (NMP), N,N-dimethylformamide (DMF), tetrahydrofuran (THF), acetonitrile, dioxane, etc., preferably DMSO. Examples of reaction temperatures include from 50 °C to reflux, preferably 100 °C.

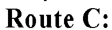

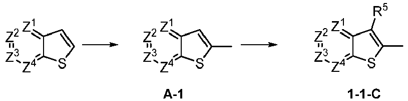

The preparation of compounds of formula **1-2-3** is given in route C.
Methylation --- alkylation
wherein, the definitions of Z¹, Z², Z³, Z⁴ and R⁵ are consistent with the previous one.

Synthetic A-1: At -60 °C, the substrate and base are placed in an organic solvent, and methyl iodide is added dropwise. Examples of the base in the reaction include, but are not limited to, lithium diisopropylamide (LDA), n-butyl lithium (n-BuLi), and the like, preferably LDA; the organic solvent is but not limited to, tetrahydrofuran (THF), acetonitrile, dichloromethane (DCM), and the like, preferably THF. Examples of reaction temperatures include from -60 °C to reflux, preferably added dropwise at -60 °C, followed by stirring at room temperature.

Synthesis of 1-1-C: A- 1, AlCl3 and acetyl chloride are reacted in an organic solvent. The organic solvent is but not limited to, acetonitrile, dichloromethane (DCM), dichloroethane (DCE), chloroform, etc., preferably DCM. Examples of reaction temperatures include from 0 °C to reflux, preferably 0 °C.

### The synthesis of 1-4 has the following route:

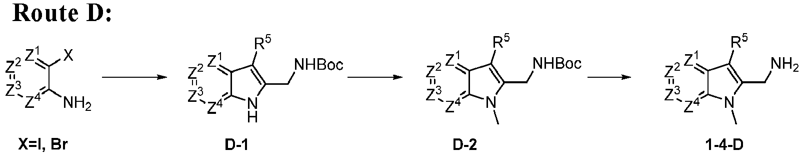

The preparation of compounds of formula 1-4-1 is given in route D.
Indole ring-closing --- methylation --- deprotection
wherein, the definitions of Z1, Z2, Z3, Z4 and R5 are consistent with the previous one.

Synthetic D-1: The substrate, 4-(tert-butoxycarbonylamino)-ethyl 3-oxobutyrate, tetrazolazeacetic acid, cuprous iodide and base are placed in an organic solvent. Examples of the base in the reaction include, but are not limited to, potassium carbonate, sodium carbonate, cesium carbonate, sodium hydroxide, potassium hydroxide, potassium tert-butoxide, sodium tert-butoxide, sodium hydrogen (NaH), triethylamine, diisopropylethylamine, pyridine, 1,5-diazabicyclo [5.4.0] undec-5-ene, 4-dimethylaminopyridine (DMAP), preferably potassium carbonate; the organic solvent is but not limited to, dimethyl sulfoxide (DMSO), N-methylpyrrolidone (NMP), N,N-dimethylformamide (DMF), tetrahydrofuran (THF), acetonitrile, dioxane, etc., preferably DMSO. Examples of reaction temperatures include from 50 °C to reflux, preferably 100 °C.

Synthetic D-2: D-1, methylation reagent and base are placed in organic solvent. Examples of the methylation reagents in the reaction include, but are not limited to, methyl iodide, dimethyl sulfate, diazomethane, etc., preferably methyl iodide. Examples of the base in the reaction include, but are not limited to, potassium carbonate, sodium carbonate, cesium carbonate, sodium hydroxide, potassium hydroxide, potassium tert-butoxide, sodium tert-butoxide, sodium hydrogen (NaH), triethylamine, diisopropylethylamine, pyridine, 1,5-diazabicyclo [5.4.0] undec-5-ene, 4-dimethylaminopyridine (DMAP), preferably cesium carbonate; the organic solvent is but not limited to, dimethyl sulfoxide (DMSO), N-methylpyrrolidone (NMP), N,N-dimethylformamide (DMF), tetrahydrofuran (THF), acetonitrile, dioxane, etc., preferably DMSO. Examples of reaction temperatures include from 0 °C to reflux, preferably room temperature.

Synthesis of 1-4-D: 1-3 is used as a substrate, 1-4-D is prepared according to conventional deprotection methods well known in the art, the preferred deprotection reagent is trifluoroacetic acid (TFA), and the preferred solvent is DCM.

The optimal reaction conditions and reaction time for each individual step can be changed depending on the specific reactants used and the substituents present in all reactants. Unless otherwise specified, solvents, temperatures and other reaction conditions can be easily selected by those skilled in the art. Specific steps are provided in the synthetic example section. The reaction can be further processed in a conventional manner, for example by removing the solvent from the residue and further purification according to methods generally known in the art, such as, but not limited to, crystallization, distillation, extraction, grinding and chromatography. Unless otherwise stated that the starting materials and reactants are commercially available or can be prepared by the skilled person from the available materials using the methods described in the chemical literature.

Conventional tests, including appropriate adjustment of reaction conditions, reaction agents and sequences of synthesis routes, protection of any chemical functional groups, which may not be compatible with reaction conditions, and deprotection at appropriate points in the reaction sequence of the method, are included in the scope of the present invention. Appropriate protection groups and methods of protecting and deprotecting different substituents using such appropriate protection groups are well known to those skilled in the art; examples of which are found in T.Greene and P. Wuts, Protecting Groups in Chemical Synthesis (Third Edition), John Wiley & Sons, NY (1999), which are incorporated herein as a whole by reference. The synthesis of the compounds of the present invention can be achieved by methods similar to those described in the synthesis schemes described above and in the specific examples.

The starting material, if not available from commercial sources, may be prepared by a step selected from the group consisting of standard organic chemistry techniques, techniques similar to the synthesis of known structural analogs, or techniques similar to the steps described in the above scheme or the synthetic examples section. When an optically active form of a compound of the invention is desired, it may be obtained by performing one of the steps described herein using an optically active starting material (e. g., prepared by asymmetric induction of an appropriate reaction step), or by resolving a stereoisomer mixture of the compound or intermediate using a standard step (e. g., chromatographic separation, recrystallization, or enzymatic resolution).

Similarly, when a pure geometric isomer of a compound of the invention is desired, it can be obtained by performing one of the above steps using the pure geometric isomer as the starting material, or by using standard steps such as chromatographic separation and resolution of a mixture of geometric isomers of a compound or intermediate.

### Src and c-Src

Src, as an oncogene protein, was first discovered in Rous sarcoma retrovirus (retrovirus rous sarcoma virus), and then it was found that highly conserved and homologous v-Src are common in cells.

Src kinase family is a protein with protein tyrosine kinase (PTK) activity, of which c-Src is an important component of Src kinase family.

### Use

The present invention provides a use of a compound of the present invention, for (1) selectively inhibiting the outside-in signaling mediated by the interaction of integrin β3 with Src kinase; (2) preventing and/or treating diseases related to the outside-in signaling mediated by the interaction of integrin β3 with Src kinase; and/or (3) preventing and/or treating thrombosis.

In another preferred embodiment, the inhibition of the interaction of integrin β3 with Src kinase comprises inhibiting the interaction of integrin β3 with the SH3 domain of Src kinase.

In another preferred embodiment, the selective inhibition of the outside-in signaling mediated by the interaction of integrin β3 with Src kinase refers to the selective inhibition of the outside-in signaling mediated by the interaction of integrin β3 with Src kinase without affecting the inside-out signaling.

In another preferred embodiment, the interaction of integrin β3 with Src kinase refers to the interaction of integrin β3 in platelets with c-Src kinase.

In another preferred embodiment, the integrin β3 is β3 of integrin αIIbβ3 and/or β3 of integrin αvβ3.

In another preferred embodiment, the diseases related to outside-in signaling mediated by the interaction of integrin β3 with Src kinase include (but are not limited to) thrombus, tumor, osteoporosis, endothelial cell-mediated angiogenesis, and a combination thereof.

In another preferred embodiment, the prevention and/or treatment of thrombosis includes anti-thrombosis and improvement of bleeding.

In another preferred embodiment, the improvement of bleeding includes inhibiting bleeding, not increasing bleeding risk, reducing bleeding risk, not causing bleeding side effects, and/or not affecting hemostatic function.

In another preferred embodiment, the hemostatic function comprises a platelet hemostatic function.

In another preferred embodiment, the hemostasis comprises physiological hemostasis.

In another preferred embodiment, the thrombosis is a cardio-cerebrovascular thrombus, and the cardio-cerebrovascular thrombus is selected from the group consisting of: myocardial infarction thrombus, cerebral infarction thrombus, ischemic stroke, atherosclerotic thrombus, and a combination thereof.

In another preferred embodiment, the prevention and/or treatment of thrombosis includes one or more ways selected from the group consisting of:
(3-1) inhibition of the stretching function of platelets on solid phase fibrinogen;
(3-2) inhibition of platelet aggregation, adhesion and/or extension;
(3-3) inhibition of fibrin clot retraction;
(3-3) not affecting the binding function of platelet binding to free fibrinogen.

### Compositions or formulations, combinations of active ingredients and kits and methods of administration

The present invention also provides a composition comprising a compound of formula I of the present invention.

The composition of the present invention is preferably a pharmaceutical composition. The composition of the present invention may include a pharmaceutically acceptable carrier.

As used herein, "pharmaceutically acceptable carrier" refers to one or more compatible solid, semi-solid, liquid or gel fillers that are suitable for human or animal use and must be of sufficient purity and sufficiently low toxicity. "Compatibility" refers to the components of the pharmaceutical composition can be mixed with the active ingredients of the drug and can be mixed with each other without significantly reducing the efficacy of the drug.

It is to be understood that in the present invention, the pharmaceutically acceptable carriers described are not particularly limited and may be materials commonly used in the art, or made by conventional methods, or purchased from the market. Examples of pharmaceutically acceptable carriers include cellulose and derivatives thereof (such as methyl cellulose, ethyl cellulose, hydroxypropyl methyl cellulose, sodium carboxymethyl cellulose, etc.), gelatin, talcum powder, solid lubricants (such as stearic acid, magnesium stearate), calcium sulfate, vegetable oil (such as soybean oil, sesame oil, peanut oil, olive oil, etc.), polyols (such as propylene glycol, glycerin, mannitol, sorbitol, etc.), emulsifiers (such as Tween), wetting agents (such as sodium dodecyl sulfate), buffer, chelating agent, thickener, pH regulator, transdermal enhancer, colorant, flavoring agent, stabilizer, antioxidant, preservative, bacteriostatic agent, pyrogen-free water, etc.

In the present invention, dosage forms of compositions and formulations include, but are not limited to, oral formulations, injectable formulations, and topical formulations.

Typically, the dosage forms of the compositions and formulations include, but are not limited to: tablets, injections, infusion agents, ointments, gels, solutions, microspheres, and films.

Typically, the injection is an intratumoral injection.

The pharmaceutical formulation should match the method of administration. The preferred method of administration is oral administration, injection administration (e. g. intratumoral injection), and when used, a therapeutically effective amount of the drug is administered to the subject (e. g. human or non-human mammal) in need thereof. As used herein, the term "therapeutically effective amount" refers to an amount that produces function or activity to humans and/or animals and is acceptable to humans and/or animals. Those of ordinary skill in the art will understand that the "therapeutically effective amount" may vary depending on the form of the pharmaceutical composition, the route of administration, the excipient of the drug used, the severity of the disease, and the combination with other drugs.

In one mode of administration, the safe and effective daily dose of the first active ingredient is usually at least about 0.1mg, and in most cases does not exceed about 2500mg. Preferably, the amount is 1 mg - 500 mg. The safe and effective amount of the second active ingredient is typically at least about 0.01 mg, and in most cases, less than 2500mg. Preferably, the amount is 0.1 mg to 2500mg. Of course, the particular dose should also depend on various factors, such as the route of administration, healthy status of a patient, which are all well within the skills of an experienced physician.

### The main advantages of the present invention include

1.The invention accidently develops a compound shown in formula I, and the compound disclosed in the present invention can be specifically combined with SH3 domain protein of Src kinase and then interfere the combination of integrin αIIbβ3 and Src kinase, so that outside-in signaling is selectively inhibited and inside-out signaling is not influenced; thus, while resisting thrombus, the compound of the present invention does not affect the normal physiological hemostatic function, prevents the occurrence of hemorrhagic side effect, and can be used as a new generation of effective medicine for preventing and treating thrombosis-related cardio-cerebrovascular diseases.
2. The compound of the invention is a small molecule compound, which has the advantages of good druggability and low side effects.

The present invention will be further illustrated below with reference to the specific examples. It should be understood that these examples are only to illustrate the invention but not to limit the scope of the invention. The experimental methods without specific conditions in the following examples usually follow conventional conditions, or according to the conditions recommended by the manufacturer. Unless otherwise stated, percentage and parts are calculated by weight.

### Example

### Example 1: Preparation of Compound SYY-C001

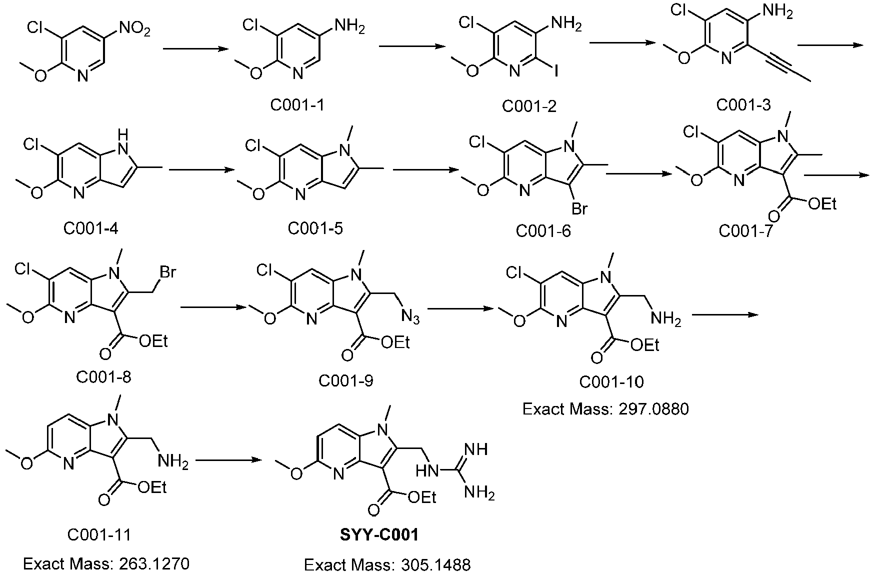

### Step 1:

Under mechanical stirring, 2-methoxy-3-chloro-5-nitropyridine (37.0g, 196.2 mmol) was added to ethanol (500 ml), iron powder (40.0g,716.2 mmol), ammonium chloride (40.0g,747.8mmol) and water (100 ml) were added at room temperature, and the mixture was heated to 90°C to react for 5 hours. TLC showed the reaction was completed. The reaction solution was filtered, the filtrate was concentrated to about 150 mL, ethyl acetate was added, washed with saturated salt water, dried over anhydrous sodium sulfate, and concentrated and the crude product was purified by silica gel column chromatography to obtain C001-1 as a grey solid (30.5 g, yield 98%).

### Step 2:

**C001-1**(30.5g,192.3 mmol) was dissolved in acetonitrile (400 ml), cooled to 0°C, and NIS(47.1g,209.4 mmol, dissolved in 300 ml acetonitrile) was added dropwise for 1 hour, maintained at 0-2°C. TLC monitored (a small amount of raw materials remained unreacted). The reaction solution was added with saturated sodium bicarbonate aqueous solution, extracted with ethyl acetate, washed with saturated salt water, dried over anhydrous sodium sulfate, and concentrated, and the crude product was purified by silica gel column chromatography to obtain **C001-2 as a** brown solid (38.0g, yield 69%).

### Step 3:

1,4-Dioxane/triethylamine (640 ml /160 ml) was added to the 2.0 L three-neck flask with nitrogen bubbling for 30 minutes. Substrate **C001-2**(38.0g,133.6 mmol), Pd(PPh₃)₂Cl₂(9.8g,13.36 mmol), CuI(5.1g,26.78 mmol), and TBAF.3 H₂O(63.1g,200.0 mmol) were added at room temperature, and replaced by nitrogen for three times, 1-(trimethylsilyl) propyne (20 ml,133.6 mmol) was added, and the mixture was heated to 80°C to react for 2 hours, and additional 1-(trimethylsilyl) propyne (20 ml,133.6 mmol) was added to continue to react overnight at 80°C. TLC monitored (raw material reaction was completed). The reaction solution was concentrated, extracted with ethyl acetate, the organic phases were combined, washed with saturated salt water, dried over anhydrous sodium sulfate, and concentrated, and the crude product was purified by silica gel column chromatography to obtain **C001-**3(30.5g) as a dark brown solid.

### Step 4:

Potassium tert-butoxide (52.2g,465.2 mmol) was dissolved in dry DMF(400 ml), a solution of crude **C001-3**(30.5g,155.1 mmol) in DMF(200 ml) was added dropwise at room temperature, and the mixture was stirred overnight at room temperature after addition. TLC detection showed that the reaction was completed. The reaction solution was added with water, extracted with ethyl acetate, dried over anhydrous sodium sulfate, and concentrated and the crude product was purified by silica gel column chromatography to obtain **C001-4**(20.0g, yield for two steps: 76%) as a brown-yellow solid.

### Step 5:

C001-4(5.0g,25.43 mmol) was dissolved in dry DMF(20 mL), cooled to about 0°C under nitrogen, sodium hydride solid (915 mg,38.13 mmol) was added in batches. After addition, it is restored to room temperature to react for half an hour, then cooled to about 0°C again, a solution of methyl iodide (7.2g,50.72 mmol) in DMF(5 mL) was added dropwise, the reaction was continued at 0°C for 1 hour after addition. TLC detection showed that the raw materials were reacted completely. The reaction solution was quenched with ice water, extracted with ethyl acetate, the organic phases were combined, washed with saturated salt water, dried over anhydrous sodium sulfate, and concentrated, and the crude product was purified by silica gel column chromatography to obtain **C001-5**(4.5g, yield 83%) as a yellow solid.

### Step 6:

**C001-5**(4.0g,18.99 mmol) was dispersed in acetonitrile (40 mL), cooled to about 0°C under the protection of nitrogen, and NBS(3.4g,19.10 mmol) was added in batches, and the reaction was continued at 0°C for half an hour after addition. TLC detection showed that the raw materials were reacted completely. The reaction solution was quenched with water, extracted with ethyl acetate, the organic phases were combined, washed with saturated salt water, dried over anhydrous sodium sulfate, and concentrated, and the crude product was purified by silica gel column chromatography to obtain **C001-6(**4.0 g, yield 73%) as a light yellow solid.

### Step 7:

**C001-6**(1.08g,3.73 mmol) was dissolved in tetrahydrofuran (15 mL), cooled to -70°C under the protection of nitrogen, n-butyl lithium solution (2.2 mL,5.50 mmol,2.5 M) was added dropwise for about half an hour, and ethyl cyanoformate (554 mg,5.59 mmol) was added, and the reaction was continued for half an hour after addition. TLC detection showed the reaction was completed. The reaction solution was quenched with saturated ammonium chloride aqueous solution, extracted with ethyl acetate, washed with saturated salt water, dried over anhydrous sodium sulfate, and concentrated, and the crude product was purified by silica gel column chromatography to obtain **C001-7**(490 mg, yield 50%) as an off-white solid.

### Step 8:

**C001-7**(490 mg,1.73 mmol) and NBS(308 mg,1.73 mmol) were dissolved in carbon tetrachloride (10 mL), BPO(59 mg,0.17 mmol) was added at room temperature, and the mixture was heated to 80°C to react for 2h. TLC detection showed that the raw materials were reacted completely. The reaction solution was cooled to room temperature and purified by silica gel column chromatography to obtain **C001-8**(350 mg, yield 56%) as an off-white solid.

### Step 9:

**C001-8**(170 mg,0.47 mmol) and sodium azide (34 mg,0.52 mmol) were heated in DMF(3mL) to 60°C to react for 3 hours. TLC detection showed that the raw materials were reacted completely. The reaction solution was cooled to room temperature, added with water, and filtered to obtain **C001-9** as an off-white solid which was directly used in the next reaction.

### Step 10:

The crude product **C001-9** was dissolved in tetrahydrofuran (5 mL) and water (1 mL), triphenylphosphine (325 mg,1.24 mmol) was added at room temperature, and the reaction solution was heated to 60°C to react for 3 hours. LCMS detection showed that the reaction was completed. The reaction solution was concentrated, and the crude product was purified by silica gel column chromatography to obtain **C001-10** (80 mg, yield for two steps: 57%) as a white solid. LCMS: m/z = 298.1 [M+H]⁺.

### Step 11:

C001-10 (80 mg,0.27 mmol) was dissolved in methanol (3 mL) and ethyl acetate (3 mL), palladium hydroxide (80 mg) was added at room temperature, and the mixture was heated to 60°C to react for 2 hours after nitrogen replacement for 3 times. TLC detection showed that the raw materials were reacted completely. The reaction solution was cooled to room temperature, filtered with diatomite, and the filtrate was concentrated to obtain **C001-11**(45 mg) as an off-white solid. LCMS: m/z = 264.2 [M+H]⁺.

### Step 12:

**C001-11** (45 mg,0.17 mmol) and pyrazole formimidamide hydrochloride (38 mg,0.26 mmol) were dispersed in acetonitrile (3 mL), DIPEA(88 mg,0.68 mmol) was added, and the mixture was reacted at room temperature for 2 days. TLC detection showed that a small amount of raw materials did not completely react. The reaction solution was concentrated and purified to obtain an off-white solid (28 mg, yield for two steps: 34%). ¹H NMR: (DMSO-*d₆*, 400 MHz): *δ* 1.36 (s, 3H), 3.85 (s, 3H), 3.92 (s, 3H), 4.31 (s, 2H), 4.89 (s, 2H), 6.75 (s, 1H), 7.17-7.75 (m, 3H), 8.04 (s, 1H), 8.41 (s, 1H); LCMS: m/z = 306.2 [M+H]⁺.

### Example 2: Preparation of Intermediates C- IN-002 and C- IN-003

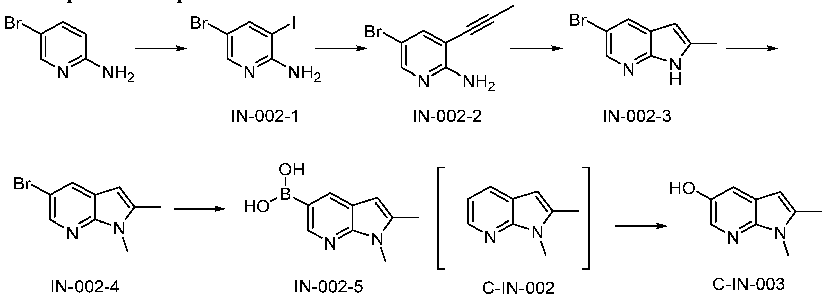

### Step 1:

Under the protection of N₂, the raw material 2-amino -5-bromopyridine (30.0g ,173.40 mmol) was dissolved in DMF(200 mL), trifluoroacetic acid (14.2 mL,191.17 mmol) and NIS(43.0g, 191.13 mmol) were added at room temperature, and the mixture was heated to 50°C and stirred for 3 hours. TLC showed the raw material was consumed completely. The reaction solution was cooled to room temperature, the reaction solution was slowly poured into water, extracted with ethyl acetate. The organic phases were combined, washed with 10% NaS₂SO₃ solution and saturated salt water, dried over anhydrous sodium sulfate, and concentrated to obtain IN-002-1(45g) as a yellow solid.

### Step 2:

Under the protection of N₂, IN-002-1(2.0g,6.69 mmol) and Et₃N(1.2 mL,8.63 mmol) were dissolved in THF(30 mL), trimethylsilylpropyne (2.0 mL,13.51 mmol) was added in batches at room temperature. After addition, PdCl₂(PPh₃)₂(240 mg,0.34 mmol) and CuI(65 mg,0.34 mmol) were continued to be added, and the mixture was stirred at room temperature for 5 minutes, TBAF.3 H₂O(2.2g,6.97 mmol) was added and stirred at room temperature for 18 hours. TLC showed the raw materials were reacted completely. The reaction solution was concentrated, and the crude product was purified by silica gel column chromatography to obtain IN-002-2(1.3g, yield for two steps: 67%) as a yellow solid. ¹H NMR (400 MHz, CDCl₃): *δ* 2.12 (s, 3H), 5.08 (br, 1H), 7.56 (d, *J* = 2.4 Hz, 1H), 8.01 (d, *J* = 2.4 Hz, 1H). LCMS: m/z = 211.0/213.0 [M +H]⁺.

### Step 3:

Under the protection of N₂, IN-002-2(11.0g,52.12 mmol) and t-BuOK(17.6g,156.85mmol) were dispersed into DMF(170 mL), and the mixture was stirred at room temperature for 18 hours. TLC detection showed that the reaction was completed. The reaction solution was added with water (500mL), extracted with ethyl acetate, the organic phases were combined, washed with saturated salt water, dried over anhydrous sodium sulfate, and concentrated to obtain IN-002-3(10.0g) as a brown solid. ¹H NMR (400 MHz, CDCl₃): *δ* 2.55 (s, 3H), 6.15 (br, 1H), 7.94 (d, *J* = 2.0 Hz, 1H), 8.24 (d, *J=* 1.8 Hz, 1H), 10.66 (br, 1H); LCMS: m/z = 211.0/213.0 [M +H]⁺.

### Step 4:

Under the protection of N₂, IN-002-3(10.0g,47.38 mmol) was dissolved in DMF(100 mL), NaH(2.9g,72.50 mmol,60%) was added in batches under ice water bath, and the mixture was stirred for 15 minutes after addition. MeI(6.0 mL,96.38 mmol) was added and stirred for 0.5 hours at room temperature. TLC showed the raw materials were reacted completely. Under ice water bath, the reaction solution was slowly poured into water, extracted with ethyl acetate, and the organic phases were combined, washed with saturated salt water, dried over anhydrous sodium sulfate, and concentrated, and the crude product was purified by silica gel column chromatography to obtain IN-002-4(9.0g, yield 84%) as a white solid. ¹H NMR (400 MHz, CDCl₃): *δ* 2.46 (s, 3H), 3.76 (s, 3H), 6.16 (s, 1H), 7.89 (d, *J=* 2.0 Hz, 1H), 8.25 (d, *J=* 2.0 Hz, 1H); LCMS: m/z = 225.0/227.0 [M +H]⁺.

### Step 5:

Under the protection of N₂, IN-002-4(8.5g ,37.76 mmol) was dissolved in THF(130 mL), cooled to -65°C under dry ice acetone bath, n-BuLi(18.2 mL,45.5 mmol,2.5M) was slowly added, and stirred at -65°C for 1.0 hour. Then trimethyl borate (43.0 mL,385.68 mmol) was added at one time, and the mixture was continued to be stirred at -65°C for 1 hour, then slowly heated to room temperature to stirr for 2 hours. TLC showed the raw materials disappeared. The reaction solution was quenched with saturated ammonium chloride solution, extracted with ethyl acetate, and the organic phases were combined, washed with saturated ammonium chloride solution, washed with saturated salt water, dried over anhydrous sodium sulfate, and concentrated to obtain a light yellow solid IN-002-5(8.0g)(containing about 5-10% debromination byproduct C- IN-002). LCMS:m/z = 191.1 [M + H]⁺, by-product C-IN-002 LCMS:m/z = 147.2 [M + H]⁺.

### Step 6:

Under the protection of N₂, IN-002-5(7.2g,37.89 mmol) was dissolved in DCM(210 mL), 30% H₂O₂(13.0 mL,129.58 mmol) was added, and the mixture was stirred at room temperature for 0.5 h. TLC showed the raw materials disappeared. Under ice water bath, Na₂SO₃(15.0g,119.01 mmol) aqueous solution (150 mL) was added, the mixture was stirred for 0.5 h, starch KI test paper did not show blue color, and liquid separation was carried out. The organic layer was washed with saturated sodium chloride solution, dried over anhydrous sodium sulfate, and concentrated, and the crude product was purified by silica gel column chromatography to obtain **C-IN-003**(3.8g, yield 62%) as a brown solid. LCMS:m/z = 163.1 [M + H]⁺.

### Example 3: Preparation of Compound SYY-C002

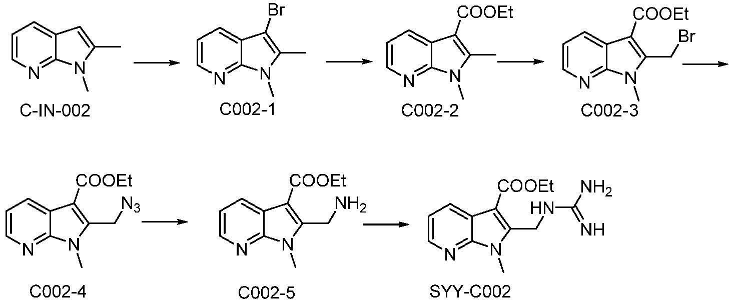

### Step 1:

Under the protection of N₂, C- IN-002(1.2g,8.21 mmol) was dissolved in DMF(20 mL), NBS(1.46g,8.20 mmol) was added under an ice water bath, and the mixture was reacted for 10 minutes. TLC showed the raw materials were reacted completely. The reaction solution was added with water, extracted with ethyl acetate, the organic phases were combined, washed with saturated salt water, dried over anhydrous sodium sulfate, and concentrated, and the crude product was purified by silica gel column chromatography to obtain C002-1(1.5g, yield 81%) as a yellow solid. ¹H NMR (400 MHz, CDCl₃) *δ* 2.48 (s, 3H), 3.83 (s, 3H), 7.10 (dd, *J=* 7.8, 4.8 Hz, 1H), 7.76 (dd, *J=* 7.8, 1.5 Hz, 1H), 8.29 (dd, *J* = 4.8, 1.5 Hz, 1H).

### Step 2:

Under the protection of N₂, C002-1(1.5g ,6.66 mmol) was dissolved in THF(30 mL), cooled to -65°C, n-BuLi(2.8 mL,7.00 mmol, 2.5 M) was slowly added dropwise and stirred at -65°C for 1 hour after addition. Ethyl cyanoformate (0.85 mL,8.60 mmol) was added and continued to stir for 1 hour after addition, and the mixture was naturally heated to room temperature to stir for 1 hour. TLC showed the raw materials were reacted completely. The reaction solution was quenched with saturated ammonium chloride solution, stratified, water layer was extracted with ethyl acetate, and the organic phases were combined, washed with saturated salt water, dried over anhydrous sodium sulfate, and concentrated, and purified by silica gel column chromatography to obtain C002-2(1.0g, yield 69%) as a light yellow solid. 1H NMR (400 MHz, CDCl3): *δ* 1.46 (t, *J=* 7.2 Hz, 3H), 2.83 (s, 3H), 3.85 (s, 3H), 4.41 (q, *J =* 7.2 Hz, 2H), 7.18 (dd, *J=* 7.6, 4.8 Hz, 1H), 8.31 (dd, *J=* 4.8, 1.6 Hz, 1H), 8.34 (dd, *J =* 7.8, 1.6 Hz, 1H). LCMS: m/z=219.1[M+H]+.

### Step 3:

Under the protection of N₂, C002-2(700 mg,3.21 mmol) was dissolved in CCl₄(15 mL), BPO(70%)(70 mg,0.20 mmol) was added and stirred. After heating to 80°C, NBS(580 mg,3.26 mmol) was added, and continued to react for 1.5 h. TLC showed the raw materials were reacted completely. The reaction solution was cooled to room temperature, and the crude product was purified by silica gel column chromatography to obtain C002-3(800 mg, yield 84%) as a yellow solid. ¹H NMR (400 MHz, CDCl₃) *δ* 1.49 (t, *J* = 7.2 Hz, 3H), 3.98 (s, 3H), 4.46 (q, *J* = 7.2 Hz, 2H), 5.19 (s, 2H), 7.24 (dd, *J* = 7.6, 4.8 Hz, 1H), 8.45-8.38 (m, 2H).

### Step 4:

Under the protection of N₂, C002-3(500 mg,1.68 mmol) was dissolved in DMF (10 mL), NaN₃(220 mg,3.38 mmol) was added at room temperature, and the mixture was heated to 50°C, stirred for 1 hour, and cooled to room temperature to stir overnight. TLC showed the raw materials were reacted completely. The reaction solution was quenched with water, extracted with ethyl acetate. The organic phases were combined, washed with saturated salt water, dried over anhydrous sodium sulfate, and concentrated to obtain C002-4(437 mg) which was directly used for the next step.

### Step 5:

Under the protection of N₂, C002-4 (437 mg) was dissolved in THF/H₂O (10mL/2mL), PPh₃(885 mg,3.37 mmol) was added at room temperature, and the mixture was heated to 50°C and stirred for 2 hours. TLC showed the raw materials and intermediates disappeared. The reaction solution was cooled to room temperature, and concentrated, and the crude product was purified by silica gel column chromatography to obtain C002-5(350 mg, yield for two steps: 89%) as a light yellow solid. ¹H NMR (400 MHz, DMSO_d₆): *δ* 1.37 (t, *J* = 7.2 Hz, 3H), 1.92 (br, 2H), 3.90 (s, 3H), 4.21 (s, 2H), 4.32 (q, *J=* 7.2 Hz, 2H), 7.26 (dd, *J=* 8.0, 4.8 Hz, 1H), 8.28 (dd, *J=* 8.0, 1.6 Hz, 1H), 8.33 (dd, *J* = 4.8, 1.6 Hz, 1H). LCMS:m/z = 234.1 [M+H]⁺.

### Step 6:

Under the protection of N₂, C002-5(250 mg,1.07 mmol) and DIEA(831mg,6.43 mmol) were dispersed into MeCN(10 mL), stirred to dissolve clear, 1H-1-methyl-formamidine hydrochloride (315 mg,2.15 mmol) was added, the mixture was stirred at room temperature for 18 hours, and a large amount of solids were precipitated. TLC showed a little raw material was left. The reaction solution was filtered, and the filter cake was washed with acetonitrile, and dried to obtain **SYY-C002** (220 mg, yield 75%) as a white solid. ¹H NMR (400 MHz, DMSO_d₆) *δ* 1.39 (t, *J=* 7.2 Hz, 3H), 3.92 (s, 3H), 4.37 (q, *J=* 7.2 Hz, 2H), 4.99 (s, 2H), 7.23-7.87 (m, 5H), 8.34 (dd, *J* = 7.6, 1.6 Hz, 1H), 8.42 (dd, *J* = 4.4, 1.6 Hz, 1H). LCMS:m/z = 276.2 [M+H]⁺.

### Example 4: Preparation of Compound SYY-C003

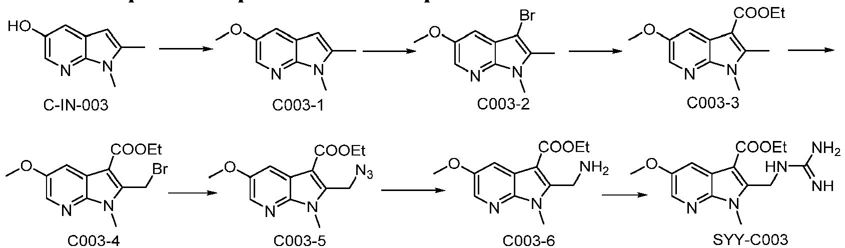

### Step 1:

Under the protection of N₂, C- IN-003(1.9g,11.71 mmol) was dissolved in DMF(20 mL), NaH(60%)(705 mg,17.63 mmol) was added under an ice bath, and the mixture was stirred for 15 minutes. Then MeI(2.0g, 14.1 mmol) was added, and stirred for 0.5 h at room temperature. TLC showed the raw materials were reacted completely. Under an ice bath, the reaction solution was slowly added into water, extracted with ethyl acetate, the organic phases were combined, washed with saturated salt water, dried over anhydrous sodium sulfate, and concentrated, and the crude product was purified by silica gel column chromatography to obtain C003-1(1.9g). LCMS:m/z = 177.1 [M + H]⁺.

### Step 2:

Under the protection of N₂, C003-1(1.90g) was dissolved in DMF(20 mL), NBS(1.92g,10.78 mmol) was added under an ice bath, and the mixture was stirred for 5 minutes. TLC showed the raw materials were reacted completely. The reaction solution was added to water, extracted with ethyl acetate, the organic phases were combined, washed with saturated salt water, dried over anhydrous sodium sulfate, and concentrated, and the crude product was purified by silica gel column chromatography to obtain C003-2(2.30g, yield for two steps: 77%) as a yellow solid.

### Step 3:

Under the protection of N₂, C003-2(2.3g,9.02 mmol) was dissolved in THF(45 mL), cooled to -65°C, n-BuLi(2.5M)(4.3 mL,10.75mmol, 2.5M) was slowly added dropwise and stirred at -65°C for 1 hour after addition. Ethyl cyanoformate (1.2 mL,12.14 mmol) was added, and continued stirring for 1 hour, and the mixture was naturally heated to room temperature to stir for 1 hour. TLC showed the raw materials were reacted completely. The reaction solution was quenched with saturated ammonium chloride solution, stratified, water layer was extracted with ethyl acetate, and the organic phases were combined, dried over anhydrous sodium sulfate, concentrated, and purified by silica gel column chromatography to obtain C003-3(800 mg, yield 36%) as a light yellow solid. ¹H NMR (400 MHz, CDCl₃): *δ* 1.46 (t, *J =* 7.2 Hz, 3H), 2.80 (s, 3H), 3.81 (s, 3H), 3.93 (s, 3H), 4.41 (q, *J* = 7.2 Hz, 2H), 7.90 (d, *J* = 2.8 Hz, 1H), 8.07 (d, *J* = 2.8 Hz, 1H). LCMS: m/z = 249.1[M+H]⁺.

### Step 4:

Under the protection of N₂, C003-3(600 mg,2.42 mmol) was dissolved in CCl₄(20 mL), BPO(70%)(70 mg,0.20 mmol) was added at room temperature. After heating to 80°C, NBS(480 mg,2.70 mmol) was added, and continued to react for 1.5 h. TLC showed the raw materials were reacted completely. The reaction solution was cooled to room temperature, and the crude product was purified by silica gel column chromatography to obtain C003-4(700 mg, yield 88%) as a yellow solid. ¹H NMR (400 MHz, CDCl₃) *δ* 1.49 (t, *J=* 7.2 Hz, 3H), 3.93 (s, 3H), 3.94 (s, 3H), 4.46 (q, *J=* 7.2 Hz, 2H), 5.16 (s, 2H), 7.92 (d, *J* = 2.8 Hz, 1H), 8.18 (d, J = 2.8 Hz, 1H).

### Step 5:

Under the protection of N₂, C003-4(450 mg,1.38 mmol) was dissolved in DMF (10 mL), NaN₃(180 mg,2.77 mmol) was added at room temperature, and the mixture was heated to 50°C, stirred for 1 hour, and cooled to room temperature to stir overnight. TLC showed the raw materials were reacted completely. The reaction solution was cooled to room temperature, quenched with water, extracted with ethyl acetate, and the organic phases were combined, washed with saturated salt water, dried over anhydrous sodium sulfate, concentrated, and purified by silica gel column chromatography to obtain C003-5(398 mg).

### Step 6:

Under the protection of N₂, C003-5 (398 mg) was dissolved in THF/H₂O (10mL/2mL), PPh₃(723mg,2.76mmol) was added at room temperature, and the mixture was heated to 50°C and stirred for 2 hours. TLC showed the raw materials and intermediates disappeared. The reaction solution was cooled to room temperature, and concentrated, and the crude product was purified by silica gel column chromatography to obtain C003-6(250 mg, yield 69%) as a light yellow solid. ¹H NMR (400 MHz, DMSO_d₆): *δ*1.37 (t, *J =* 7.2 Hz, 3H), 1.94 (, 2H), 3.85 (s, 3H), 3.86 (s, 3H), 4.16 (s, 2H), 4.30 (q, *J* = 7.2 Hz, 2H), 7.78 (d, *J* = 2.8 Hz, 1H), 8.07 (d, *J* = 2.8 Hz, 1H). LCMS:m/z = 264. 1[M+H]⁺.

### Step 7:

Under the protection of N₂ , C003-6(250mg,0.95mmol) and DIEA(491mg,3.8mmol) were dispersed into MeCN(10 mL), stirred to dissolve clear, 1H-1-methyl-formamidine hydrochloride (279 mg,1.90 mmol) was added, the mixture was stirred at room temperature for 18 hours, and a large amount of solids were precipitated. TLC showed a little raw material was left, and the reaction solution was filtered, and the filter cake was washed with acetonitrile, dried to obtain **SYY-C003** (200 mg, yield 69%) as a white solid. ¹H NMR (400 MHz, DMSO_d₆):δ 1.39 (t, *J =* 7.2 Hz, 3H), 3.88 (s, 6H), 4.36 (q, *J =* 7.2 Hz, 2H), 4.95 (s, 2H), 7.49 (br, 3H), 7.82 (d, *J =* 2.8 Hz, 1H), 8.17 (d, *J =* 2.8 Hz, 1H), 8.39 (br, 1H). LCMS: m/z = 306.1[M+H]⁺.

### Example 5: Preparation of Intermediate C- IN-001

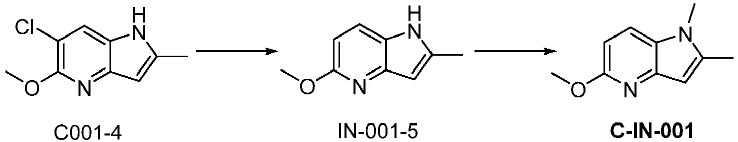

### Step 1:

C001-4(23.0g,116.97 mmol) was dissolved in methanol (300 mL), palladium hydroxide (10.0g) was added, replaced with hydrogen for 3 times, and the mixture was heated to 50°C to react for 4 hours. TLC detection showed that the reaction was basically completed. The reaction solution was cooled to room temperature, filtered with diatomite, and the filtrate was concentrated to obtain IN-001-5(25g) as a light yellow solid.

### Step 2:

Under the protection of nitrogen, IN-001-5(16.9g,104.20 mol) was dissolved in dry THF(170 ml), cooled under an ice water bath, 60% NaH(7.5g,187.5 mmol) was added in batches at 0°C, and the mixture was stirred for 0.5 h at room temperature after addition, cooled under an ice water bath, methyl iodide (29.6g,208.54 mmol) was added dropwise for 30 minutes at 0°C, and the reaction was continued for 1 hour after addition. TLC detection showed that the raw materials were reacted completely. Water was added, the mixuture was extracted with ethyl acetate, the organic phases were combined, washed with saturated salt water, dried over anhydrous sodium sulfate, and concentrated, and the crude product was added into 40 ml of mixed solvent (PE/EA = 20/1), stirred for 1h, and filtered to obtain **C-IN-001**(11.5 g, yield 63%) as a brown solid.

### Example 6: Preparation of Compound SYY-C004 and SYY-C005

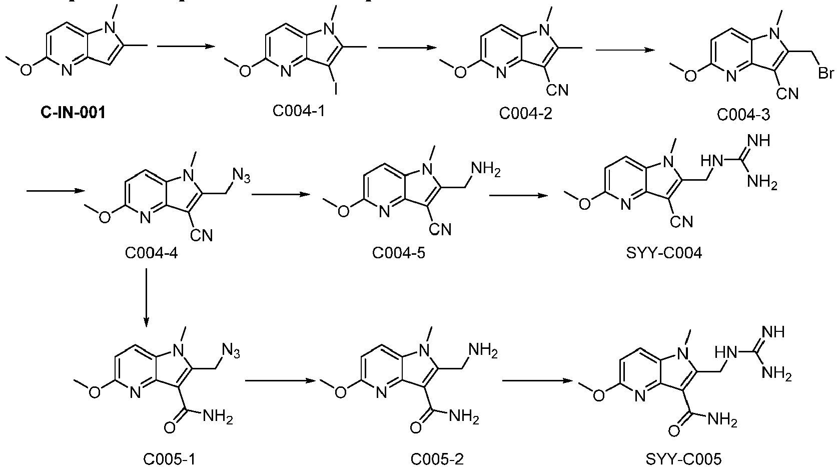

### Step 1:

C- IN-001(1.0g,5.67 mmol) was dissolved in acetonitrile (10 mL), cooled to about 0°C under an ice water bath, NIS(1.28g,5.69 mmol) was added in batches, and the reaction solution was reacted at 0 °C for 1 hour. TLC showed the reaction was completed. Water was added dropwise into the reaction solution, stirred at 0 °C for 10 minutes, filtered, and the filter cake was washed and dried to obtain C004-1(1.7g, crude) as a yellow solid.

### Step 2:

C004-1(900 mg, crude) was dissolved in DMF(15 mL), zinc cyanide (525 mg,4.47 mmol) and Pd(PPh₃)₄(344 mg,0.30 mmol) were added at room temperature, replaced with nitrogen for three times, and the mixture was heated to 130 °C to react for 5 hours. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C004-2(280 mg, yield for two steps: 47%) as a white solid. LCMS: m/z = 202.1 [M+H]⁺.

### Step 3:

C004-2(193 mg,0.96 mmol), NBS(171 mg,0.96 mmol), and BPO(35 mg,0.14 mmol) were dispersed in carbon tetrachloride (10 mL), and the mixture was heated to 80 °C for 2h under the protection of nitrogen. TLC detection showed that the raw materials were reacted completely. The reaction solution was cooled to room temperature, and concentrated, and the crude product was purified by silica gel column chromatography to obtain C004-3(255 mg, yield 95%) as a white solid. LCMS: m/z = 280.0/282.0[M + H]⁺.

### Step 4:

C004-3(627 mg,2.24 mmol) was dissolved in DMF(5 mL), sodium azide (218 mg,3.35 mmol) was added at room temperature, and the mixture was heated to 60 °C to react for 4 hours. TLC detection showed that the reaction was completed. The reaction solution was cooled to room temperature, added with water, extracted with ethyl acetate, and the organic phases were combined, washed with saturated salt water, dried over anhydrous sodium sulfate, and concentrated to obtain C004-4(500 mg, crude) as a yellow solid. LCMS: m/z = 243.1 [M+H]⁺.

### Step 5:

C004-4(500 mg,2.06 mmol) was dissolved in THF(10 mL) and water (2 mL), triphenylphosphine (881 mg,3.36 mmol) was added at room temperature, and the mixture was heated to 60 °C to react for 3 hours. LCMS detection showed the reaction was completed. The reaction solution was cooled to room temperature, concentrated to a small amount, added with water, extracted with ethyl acetate, the organic phases were combined, washed with saturated salt water, dried over anhydrous sodium sulfate, and concentrated, and the crude product was purified to obtain C004-5(110 mg, yield for two steps: 23%) as a yellow solid . LCMS: m/z = 217.2 [M+H]⁺.

### Step 6:

C004-5(80 mg,0.37 mmol) was dispersed in acetonitrile (3 mL), clarified by adding DIEA (191 mg, 1.48 mmol), then pyrazole formamidine hydrochloride (60 mg, 0.41 mmol) was added, after addition, the reaction solution was reacted at room temperature for 2 days, and the solid was precipitated, and the reaction solution was filtered and dried to obtain SYY-C004 ( 50 mg, yield 52%) as an off-white solid. ¹H NMR: (DMSO-*d₆*, 400MHz): δ3.80 (s, 3H), 3.91 (s, 3H), 4.80 (s, 2H), 6.80 (d, *J=* 8.8 Hz, 1H), 7.50 (br, 4H), 8.06 (d, *J* = 8.8 Hz, 1H). LCMS: m/z = 259.1[M+H]⁺.

### Step 7:

C004-4(100 mg,0.41 mmol) was dispersed in ethanol (2 mL), hydrogen peroxide (0.5 mL) was added at room temperature, potassium hydroxide aqueous solution (0.5 mL,1.0 mmol, 2 M) was added at one time, and the mixture was heated to 80 °C for 2 hours. TLC and LCMS detection showed the reaction was completed. The reaction solution was cooled to room temperature, added with water, extracted with ethyl acetate, washed with saturated sodium sulfite aqueous solution, dried over anhydrous sodium sulfate, and concentrated, and the crude product was purified to obtain C005-1(60 mg, yield 56%) as a white solid. LCMS: m/z = 261.1[M + H]⁺.

### Step 8:

C005-1(60 mg,0.23 mmol) was dispersed in methanol (5 mL), palladium carbon (10%,60 mg) was added, replaced with hydrogen for three times, and the mixture was heated to 60 °C to react for 1 hour. TLC detection showed the reaction was completed. The reaction solution was cooled to room temperature, filtered with diatomite, washed, and the filtrate was concentrated. The crude product was purified to obtain C005-2(42 mg, total yield 78%) as a light yellow solid. LCMS: m/z = 235.1 [M+H]⁺.

### Step 9:

C005-2(40 mg,0.17 mmol) was dispersed in acetonitrile (3 mL), DIEA(88 mg,0.68 mmol) and pyrazole formamidine hydrochloride (28 mg,0.19 mmol) were added at room temperature, and the mixture was reacted for 2 days at room temperature. The reaction solution was filtered, and the filter cake was washed and dried to obtain **SYY-C005** (45 mg, yield 96%) as a white solid. ¹H NMR: (DMSO-*d₆*, 400 MHz): *δ* 3.89(s, 3H),3.92 (s, 3H), 4.97 (s, 2H), 6.80 (d, *J* = 9.12 Hz, 1H), 7.19 (br, 2H), 7.90(s, 1H), 8.07 (d, *J* = 8.4 Hz, 1H), 8.08 (br, 1H), 8.53 (s, 1H), 8.88 (br, 1H). LCMS: m/z = 277.1 [M+H]⁺.

### Example 7: Preparation of Intermediate C- IN-005

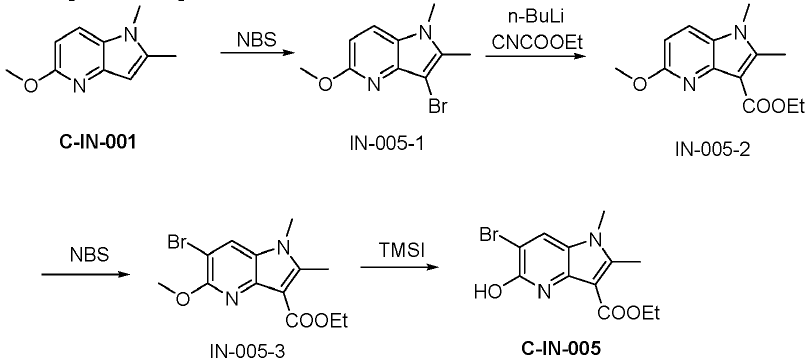

### Step 1:

Under the protection of nitrogen, C-IN-001(16.6g,94.20 mmol) was dissolved in acetonitrile (170 ml), cooled under an ice water bath, NBS(16.8g,94.39 mmol) was added in batches in 30 minutes at 0°C, and the mixture was reacted at 0°C for 0.5 h. TLC detection showed that the raw materials were reacted completely. The reaction solution was added with water, extracted with ethyl acetate, the organic phases were combined, washed with saturated salt water, dried over anhydrous sodium sulfate, and concentrated, and the crude product was purified by silica gel column chromatography to obtain IN-005-1(13.7g, yield 57%) as a light yellow solid.

### Step 2:

Under the protection of nitrogen, IN-005-1(13.7g,53.70 mmol) was dissolved in dry THF(200 ml), cooled to -60 ∼-70°C under dry ice-acetone bath, n-butyl lithium (28.0 ml,70.0 mmol,2.5mol/L) was added dropwise in 30 minutes, and the mixture was reacted at this temperature for 1 hour, and then ethyl cyanoacetate (8.0g,80.73 mmol, diluted with 10 ml THF) was added, and reacted at -60 ∼-70°C for 1h after addition, and the mixture was slowly heated to room temperature to react for 1h. TLC detection showed that the raw materials were reacted completely. The reaction solution was quenched with water, extracted with ethyl acetate, the organic phase was washed with saturated salt water, dried over anhydrous sodium sulfate, and concentrated, and purified by silica gel column chromatography to obtain crude product, mixed solution (PE/EA = 40/1) (50 ml) was added and stirred for 30 minutes, filtered, and the filter cake was washed and dried to obtain IN-005-2(9.3g, yield 70%) as a white solid.

### Step 3:

Under the protection of nitrogen, IN-005-2(2.0g,8.06 mmol) was dissolved in carbon tetrachloride (40 ml), NBS(1.43g,8.06 mmol) and BPO(195 mg,0.81 mmol) were added at room temperature, and the mixture was heated to 50°C to react for 1.0 hour. TLC showed raw materials were not reacted completely, and additional NBS(143 mg,0.8 mmol) was added, and the reaction was continued at 50°C for 0.5 h. TLC showed raw materials were not reacted completely. The reaction solution was cooled to room temperature, and the crude product was purified by silica gel column chromatography to obtain IN-005-3(2.4 g, yield 91%) as a yellow solid. LCMS:m/z = 327.0/329.0 [M + H]⁺.

### Step 4:

Under the protection of nitrogen, IN-005-3(2.78g,8.50 mmol) was dissolved in DCM(40 ml), TMSI(4.8 ml,33.73 mmol, diluted with 5 ml DCM) was added dropwise at room temperature, and the mixture was reacted at room temperature for 30 minutes after addition, heated to 40°C and refluxed for 20 minutes. TLC showed the raw materials were reacted completely. The reaction solution was cooled to room temperature, saturated sodium bicarbonate aqueous solution was added for quenching, extracted with ethyl acetate, the organic phases were combined, washed with saturated salt water, dried over anhydrous sodium sulfate, and concentrated, and the crude product was purified by silica gel column chromatography to obtain **C-IN-005**(2.57g, yield 97%) as a yellow solid. ¹H NMR (400 MHz, DMSO_d6): *δ* 1.30 (t, *J =* 7.2 Hz, 3H), 2.57 (s, 3H), 3.65 (s, 3H), 4.31 (q, *J* = 7.2 Hz, 2H), 8.40 (s, 1H), 10.63 (br, 1H). LCMS: m/z = 313.0/315.0 [M+H]⁺.

### Example 8: Preparation of Compound SYY-C006

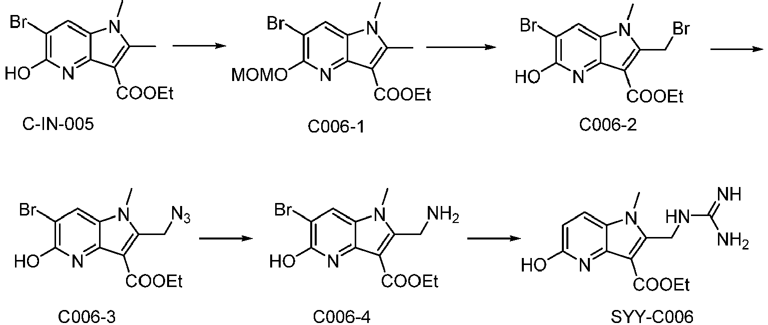

### Step 1:

Under the protection of nitrogen, C- IN-005(1.72g,5.49 mmol) was dissolved in DCM(50 ml),N,N-diisopropylethylamine(2.83g,21.90 mmol) was added, cooled to 0°C under ice water bath, chloromethyl methyl ether (1.1g, 13.66 mmol, diluted with 2 ml DCM) was added dropwise, and the mixture was reacted at 40°C for 5 hours after addition. TLC monitored (raw materials were not completely reacted), additional N,N-diisopropylethylamine(1.42g,10.99 mmol) and chloromethyl methyl ether (0.57g,7.08 mmol, diluted with 2 ml DCM) were added, and the mixture was heated to 40°C to react for 6 hours. The reaction solution was cooled to room temperature, washed with saturated salt water, dried over anhydrous sodium sulfate, and concentrated, and the crude product was purified by silica gel column chromatography to obtain C006-1(860 mg, yield 44%) as a light yellow solid. ¹H NMR (400 MHz, CDCl₃) *δ* 7.89 (s, 1H), 6.01 (s, 2H), 4.38 (d, *J* = 7.2 Hz, 2H), 3.63 (s, 3H), 3.20 (s, 3H), 2.51 (s, 3H), 1.40 (t, *J =* 7.2 Hz, 3H). LCMS: m/z=357.0/359.0 [M+H]⁺.

### Step 2:

C006-1(500 mg,1.40 mmol), NBS(250 mg,1.40 mmol) and BPO(34 mg,0.14 mmol) were dissolved in CCl₄(10 ml), and heated to 80°C to react for 1 hour. TLC detection showed that the raw materials were reacted completely. The reaction solution was purified by silica gel column chromatography to obtain C006-2(300 mg, yield 55%) as a yellow solid.

### Step 3:

C006-2(300 mg,0.77 mmol) and NaN₃(75 mg,1.15 mmol) were dissolved in DMF(6 ml), heated to 50°C to react for 1.5 hours. LCMS detection showed that the raw materials were reacted completely. The reaction solution was cooled to room temperature, added with water, extracted with ethyl acetate, the organic phases were combined, washed with saturated salt water, dried over anhydrous sodium sulfate, and concentrated to obtain C006-3(180 mg) which was directly used in the next step. LCMS:m/z = 354.0/356.0 [M + H]⁺.

### Step 4:

C006-3(180 mg,0.51 mmol) was dissolved in methanol (10 ml), 10% palladium hydroxide/carbon (150 mg) was added, replaced with hydrogen for three times, and the mixture was heated to 60°C to react for 1 hour. TLC detection showed that the reaction was completed. The reaction solution was cooled to room temperature, filtered with diatomite, the filtrate was concentrated to obtain a pink solid, neutralized with saturated sodium bicarbonate aqueous solution, extracted with ethyl acetate, the organic phase was discarded, the aqueous phase was concentrated, and (DCM/MeOH = 10/1) was added to slurry, filtered, the filtrate was concentrated, and the crude product was purified to obtain C006-4(57 mg, yield 45%) as a white solid.

### Step 5:

C006-4(57 mg,0.23 mmol), 1H-pyrazol-1-formamidine hydrochloride (40 mg,0.27 mmol), and DIEA(118 mg,0.91mmol) were dispersed in acetonitrile (4 ml) and reacted at room temperature for 2 days. The reaction solution was filtered and the filter cake was washed with 1 ml acetonitrile to obtain **SYY-C006(45** mg, yield 67%) as a white solid. ¹H NMR: (DMSO-*d₆*, 400 MHz): *δ* 1.32 (t, *J=* 7.2 Hz, 3H), 3.78 (s, 3H), 4.35 (q, *J=* 7.2 Hz, 2H), 4.77 (s, 2H), 6.23 (d, *J* = 9.6 Hz, 1H), 7.46 (br, 3H), 7.91 (d, *J=* 9.2 Hz, 1H), 8.27 (br, 1H), 10.36 (br, 1H). LCMS: m/z = 292.2 [M+H]⁺.

### Example 9: Preparation of Compounds SYY-C007 and SYY-C008

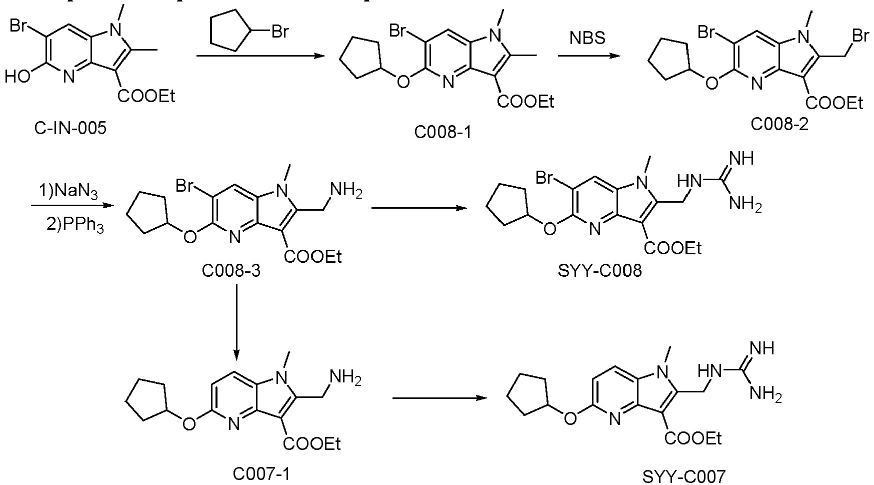

### Step 1:

C- IN-005(450 mg,1.44 mmol), cyclopentane bromide (428 mg,2.87 mmol) and potassium carbonate (595 mg,4.31 mmol) were dissolved in DMF(10 ml), and the mixture was heated to 80°C to react for 4 hours. TLC detection showed that raw materials did not completely react. Additional cyclopentane bromide (214 mg,1.44 mmol) was added, and the reaction was continued at 80°C for 2 hours, and TLC detection showed that raw materials were reacted completely. The reaction solution was cooled to room temperature, added with water, extracted with DCM, washed with saturated salt water, dried over anhydrous sodium sulfate, concentrated, and purified by silica gel column chromatography to obtain C008-1(460 mg, yield 84%) as a yellow solid. ¹H NMR (400 MHz, CDCl₃) *δ* 1.47 (t, *J =* 7.2 Hz, 3H), 1.61-1.68 (m, 2H), 1.86-1.95 (d, 4H), 2.05 -2.14 (m, 2H), 2.76 (s, 3H), 3.66 (s, 3H), 4.41 (q, *J =* 7.2 Hz, 2H), 5.59-5.62 (m, 1H), 7.71 (s, 1H). LCMS: m/z = 381.0/383.0 [M+H]⁺.

### Step 2:

C008-1(400 mg,1.05 mmol), NBS(205 mg,1.15 mmol) and BPO(25 mg,0.10 mmol) were dissolved in CCl₄(10 ml), and the mixture was heated to 80°C to react for 2 hours. TLC detection showed that the raw materials were reacted completely. The reaction solution was cooled to room temperature, and concentrated, and the residue was purified by silica gel column chromatography to obtain C008-2(515 mg) as a light yellow solid.

### Step 3:

C008-2(510 mg,1.11 mmol) and NaN₃(108 mg,1.66 mmol) were dissolved in DMF(8 ml), and heated to 50°C to react for 1.5 hours. TLC detecetion showed that the raw materials were reacted completely. The reaction solution was cooled to room temperature, added with water, extracted with ethyl acetate, washed with saturated salt water, dried over anhydrous sodium sulfate and concentrated, the residue was dissolved in a mixed solvent of THF(12 ml) and water (4 ml), PPh₃(434 mg,1.65 mmol) was added, and the mixture was reacted overnight at room temperature. LCMS detection showed that the raw materials were reacted completely. The reaction solution was concentrated, and the residue was purified by column chromatography to obtain C008-3(370 mg, yield 84%) as a yellow solid. LCMS:m/z = 379.0/81.0 [M-NH₂]⁺.

### Step 4:

C008-3(110 mg,0.28 mmol), 1H-pyrazol-1-formamidine hydrochloride (53 mg,0.36 mmol), and DIEA(143 mg,1.11 mmol) were dissolved in acetonitrile (3ml), reacted at room temperature for 2 days, solids were precipitated, filtered, and the filter cake was washed with 1ml acetonitrile to obtain **SYY-C008**(91 mg, yield 75%) as a white solid. ¹H NMR (DMSO-*d₆*, 400 MHz) *δ*1.36 (t, *J =* 7.2 Hz, 3H), 1.56-1.79 (m, 6H), 1.99-2.11 (m, 2H), 3.84 (s, 3H), 4.31 (q, *J* = 7.2 Hz, 2H), 4.88 (s, 2H), 5.47 (s, 1H), 7.45 (br, 3H), 8.29-8.44 (m, 2H). LCMS: m/z = 440.1 [M+H]⁺.

### Step 5:

C008-3(200 mg,0.50 mmol) was dissolved in methanol (5 ml), palladium hydroxide/carbon (200 mg) was added, replaced with hydrogen for three times, and heated to 60°C to react for 1 hour. TLC detection showed that the reaction was completed. The reaction solution was cooled to room temperature, filtered with diatomite, and the filtrate was concentrated to obtain C007-1(145 mg) as a light yellow solid.

### Step 6:

C007-1(145 mg,0.46 mmol), 1H-pyrazol-1-formamidine hydrochloride (80 mg,0.55 mmol), and DIEA(236 mg,1.83 mmol) were dissolved in acetonitrile (6 ml) and reacted at room temperature for 2 days with solid precipitation. The reaction solution was filtered and the filter cake was washed with 1ml acetonitrile to obtain **SYY-C007**(110 mg, yield for two steps: 60%) as a white solid. ¹H NMR: (DMSO-*d₆*, 400MHz): *δ*1.36 (t, *J* = 7.2 Hz, 3H), 1.54-1.77 (m, 6H), 2.00-2.10 (m, 2H), 3.84 (s, 3H), 4.31 (q, *J=* 7.2 Hz, 2H), 4.89 (s, 2H), 5.41-5.46 (m, 1H), 6.68 (d, *J* = 8.9 Hz, 1H), 7.46 (br, 3H), 7.96 (d, *J* = 8.9 Hz, 1H), 8.37 (br, 1H). LCMS: m/z = 360.2 [M+H]⁺.

### Example 10: Preparation of Compound SYY-C009

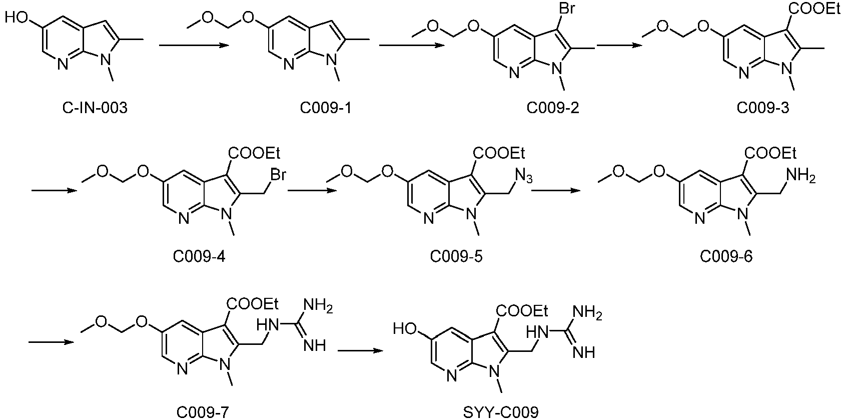

### Step 1:

Under the protection of N₂, C-IN-003(1.9g,8.54 mmol) was dissolved in DMF(30mL), NaH(60%)(938 mg,23.45 mmol) was added under ice water bath, and the mixture was stirred for 15 minutes, MOMCl(1.0 mL,13.17 mmol) was added, and the mixture was stirred for 0.5 h at room temperature. TLC showed the raw materials were reacted completely. Under an ice bath, the reaction solution was slowly added into saturated NH₄Cl solution, extracted with ethyl acetate, the organic phases were combined, washed with saturated salt water, dried over anhydrous sodium sulfate, and concentrated, and the crude product was purified by silica gel column chromatography to obtain C009-1(2.0g, yield 83%) as a light yellow oil. ¹H NMR (400 MHz, CDCl₃): *δ* 2.43 (d, *J* = 0.8 Hz, 3H), 3.53 (s, 3H), 3.74 (s, 3H), 5.17 (s, 2H), 6.13 (d, *J=* 1.0 Hz, 1H), 7.52 (d, *J=* 2.4 Hz, 1H), 8.08 (d, *J =* 2.4 Hz, 1H).

### Step 2:

Under the protection of N₂, C009-1(2.0g,9.70 mmol) was dissolved in DMF(20 mL), NBS(1.73g,9.70 mmol) was added under an ice bath, and the mixture was stirred for 2 minutes. TLC showed the raw materials were reacted completely. The reaction solution was added to water, extracted with ethyl acetate, the organic phases were combined, washed with saturated salt water, dried over anhydrous sodium sulfate, and concentrated, and the crude product was purified by silica gel column chromatography to obtain C009-2(2.3 g, yield 83%) as a yellow semisolid. ¹H NMR (400 MHz, CDCl₃) *δ* 2.43 (d, *J* = 0.8 Hz, 3H), 3.53 (s, 3H), 3.74 (s, 3H), 5.17 (s, 2H), 7.52 (d, *J=* 2.8 Hz, 1H), 8.10 (d, *J* = 2.8 Hz, 1H).

### Step 3:

Under the protection of N₂, C009-2(2.0g ,7.01 mmol) was dissolved in THF(40 mL), cooled to -65°C, n-BuLi(2.5 M)(3.1 mL,7.75 mmol) was slowly added dropwise, stirred for 1 hour at -65°C after addition, CNCOOEt(0.78 mL,7.90 mmol) was added, and the mixture was continued to be stirred for 1 hour after addition, and naturally heated to room temperature and stirred for 1 hour. TLC showed the raw materials were reacted completely. The reaction solution was quenched by adding saturated NH₄Cl solution, stratified, water layer was extracted with ethyl acetate, and the organic phases were combined, dried over anhydrous sodium sulfate, concentrated, and purified by silica gel column chromatography to obtain C009-3(800 mg, yield 41%) as a light yellow solid.

### Step 4:

Under the protection of N₂, C009-3(300 mg,1.08 mmol) was dissolved in CCl₄(10 mL), BPO(70%)(20 mg,0.058 mmol) was added at room temperature. After heating to 80°C, NBS(200mg,1.12 mmol) was added, and continued to react for 1 h. TLC showed the raw materials were reacted completely. The reaction solution was cooled to room temperature, and the crude product was directly purified by silica gel column chromatography to obtain C009-4(340 mg, yield 88%) as a light yellow solid. ¹H NMR (400 MHz, CDCl₃): δ 1.48 (t, *J=* 7.2 Hz, 3H), 3.55 (s, 3H), 3.94 (s, 3H), 4.45 (q, *J=* 7.2 Hz, 2H), 5.17 (s, 2H), 5.24 (s, 2H), 8.11 (d, *J=* 2.7 Hz, 1H), 8.24 (d, *J=* 2.8 Hz, 1H).

### Step 5:

Under the protection of N₂, C009-4(340 mg,0.95 mmol) was dissolved in DMF (10 mL), NaN₃(124 mg,1.91 mmol) was added at room temperature, and the mixture was heated to 50°C, stirred for 1 hour, and stirred overnight at room temperature. TLC showed the raw materials were reacted completely. The reaction solution was quenched with water, extracted with ethyl acetate. The organic phases were combined, washed with saturated salt water, dried over anhydrous sodium sulfate, and concentrated to obtain C009-5(306 mg, crude).

### Step 6:

Under the protection of N₂, C009-5 (306 mg, crude) was dissolved in THF/H₂O (8 mL/ 1.6 mL), PPh₃(500 mg,1.91 mmol) was added at room temperature, and the mixture was heated to 50°C and stirred for 2 hours. TLC showed the raw materials and intermediates disappeared. The reaction solution was cooled to room temperature, and concentrated, and the crude product was purified by silica gel column chromatography to obtain C009-6(190 mg, yield for two steps: 68%) as a light brown solid.

### Step 7:

Under the protection of N₂, C009-6(190 mg ,0.65 mmol) and DIEA (418 mg,3.23 mmol) were dispersed into acetonitrile (8 mL), stirred to dissolve clear, 1H-1-methyl-formamidine hydrochloride (191 mg,1.30 mmol) was added, and the mixture was stirred at room temperature for 18 hours. TLC showed the raw materials were reacted completely (the product was dissolved in acetonitrile and no solid was precipitated). The reaction solution was concentrated and the crude product was purified to obtain C009-8(190 mg, yield 88%) as a white solid.

### Step 8:

Under the protection of N₂, C009-8(190 mg,0.57 mmol) was dissolved in MeCN/MeOH (8 mL/1 mL), HCl/dioxane(1.0 mL) was added, and the mixture was stirred at room temperature for 3 hours. TLC showed the raw materials were reacted completely (a large amount of solids were precipitated). The reaction solution was filtered, and the filter cake was washed with acetonitrile, and dried to obtain white solid SYY-C009 hydrochloride (100 mg, yield 49%). ¹H NMR: (DMSO-*d₆*, 400 MHz): *δ* 1.38 (t, *J=* 7.2 Hz, 3H), 3.85(s, 3H),4.33 (q, *J* = 7.2 Hz, 2H), 4.94 (d, *J* = 4.8 Hz, 2H), 7.40 (br, 6H), 7.76 (d, *J=* 2.8 Hz, 1H), 8.03 (d, *J=* 2.8 Hz, 1H). LCMS: m/z = 292.2 [M+H]⁺.

### Example 11: Preparation of Compound SYY-C010

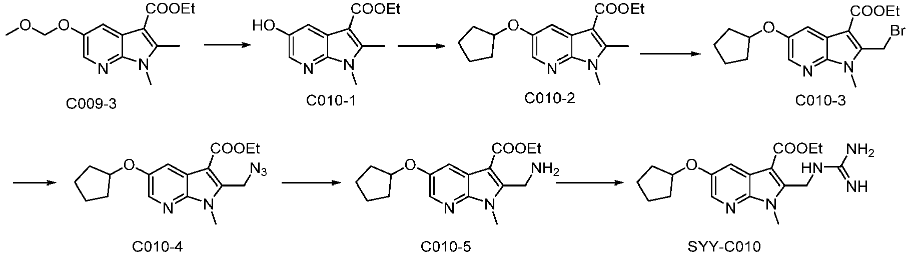

### Step 1:

Under the protection of N₂, C009-3(400 mg,1.44 mmol) was dissolved in DCM(10 mL), HCl/dioxane(1 mL,4 M) was added, and the mixture was stirred for 18 hours. TLC showed the raw materials were reacted completely. The reaction solution was concentrated to obtain C010-1(334 mg, crude), which was directly used in the next step.

### Step 2:

Under the protection of N₂, C010-1(334 mg, crude) was dissolved in DMF(10 mL), K₂CO₃(397 mg,2.87 mmol) and bromocyclopentane (425 mg,2.85 mmol) were added, and the mixture was stirred at room temperature for 18 hours. TLC showed about 2/3 raw materials were left, and additional K₂CO₃(397 mg,2.87 mmol) and bromocyclopentane (425 mg, 2.85 mmol) were added, heated to 50°C and stirred for 6 hours. TLC showed the raw materials basically were reacted completely. The reaction solution was cooled to room temperature, slowly added into water, extracted with ethyl acetate, the organic phases were combined, washed with saturated salt water, dried over anhydrous sodium sulfate, and concentrated, and the crude product was purified by silica gel column chromatography to obtain C010-2(200 mg, yield for two steps: 46%).

### Step 3:

Under the protection of N₂, C010-2(200 mg,0.66 mmol) was dissolved in CCl₄(8 mL), BPO(70%)(20 mg,0.06 mmol) was added at room temperature. After heating to 80°C, NBS(118 mg,0.66 mmol) was added, and continued to react for 2 h. TLC showed the raw materials were reacted completely. The reaction solution was cooled to room temperature, and the crude product was directly purified by silica gel column chromatography to obtain C010-3(200 mg, yield 79%) as a light yellow solid. ¹H NMR (400 MHz, cdcl₃) δ 1.49 (t, *J* = 7.2 Hz, 3H), 1.61-1.69 (m, 2H), 1.83-1.89 (m, 2H), 1.93-1.98 (m, 2H), 4.45 (q, *J* = 7.2 Hz, 2H), 3.93 (s, 3H), 4.80-4.87 (m, 1H), 5.16 (s, 2H), 7.90 (d, *J* = 2.8 Hz, 1H), 8.13 (d, *J* = 2.8Hz, 1H).

### Step 4:

Under the protection of N₂, C010-3(200 mg,0.52 mmol) was dissolved in DMF (10 mL), NaN₃(70 mg,1.08mmol) was added at room temperature, and the mixture was heated to 50°C, stirred for 1 h, and stirred overnight at room temperature. TLC showed the raw materials were reacted completely. The reaction solution was cooled to room temperature, quenched with water, extracted with ethyl acetate, and the organic phases were combined, washed with saturated salt water, dried over anhydrous sodium sulfate, and concentrated to obtain C010-4(182mg, crude).

### Step 5:

Under the protection of N₂, C010-4 (182 mg, crude) was dissolved in THF/H₂O (6 mL/ 1.2 mL), PPh₃(275 mg,1.05mmol) was added at room temperature, and the mixture was heated to 50°C and stirred for 2 hours. TLC showed the raw materials and intermediates disappeared. The reaction solution was cooled to room temperature, and concentrated, and the crude product was purified by silica gel column chromatography to obtain C010-5(150 mg, yield for two steps: 90%) as a light brown semisolid.

### Step 6:

Under the protection of N₂, C010-5(150 mg,0.47 mmol) and DIEA(305mg,2.36 mmol) were dispersed into MeCN(8 mL), stirred to dissolve clear, 1H-1-methyl-formamidine hydrochloride (140 mg,0.95 mmol) was added, and the mixture was stirred at room temperature for 18 h. TLC showed the raw materials were reacted completely (the product was dissolved in acetonitrile and no solid was precipitated). The reaction solution was concentrated and the crude product was purified by Pre-TLC to obtain SYY-C010(150 mg, yield 88%) as an off-white foam solid. 1H NMR (DMSO-d6, 400MHz) δ1.39 (t, J = 7.2 Hz, 3H), 1.58-1.65 (m, 2H), 1.69-1.82 (m, 4H), 1.88-2.00(m, 2H), 3.87 (s, 3H), 4.35 (q, J = 7.2 Hz, 2H), 4.88-4.91 (m, 1H), 4.94 (d, J = 5.2 Hz, 2H), 7.46 (br, 3H), 7.80 (d, J = 2.8 Hz, 1H), 8.12 (d, J = 2.8 Hz, 1H), 8.38 (s, 1H). LCMS: m/z=360.2[M+H]⁺

### Example 12: Preparation of Compound SYY-C014

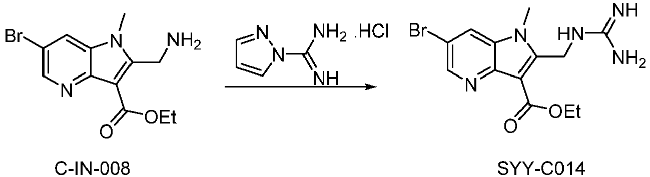

C- IN-008(100 mg,0.32 mmol), pyrazole formamidine hydrochloride (52 mg,0.35 mmol) and DIEA(165 mg,1.28 mmol) were dispersed into acetonitrile (5mL), the mixture was stirred overnight at room temperature, and white solids were precipitated. The reaction solution was filtered, and the filter cake was washed and dried to obtain an off-white solid (50 mg, HPLC purity 75%), which was combined with the mother liquor, and concentrated, and the crude product was purified by Pre-TLC to obtain a white solid (60 mg, yield 53%). ¹H NMR: (DMSO_d6, 400MHz): δ1.33 (t, J = 7.2 Hz, 3H), 3.88 (s, 3H), 4.14 (s, 3H), 4.36 (q, J = 7.2 Hz, 2H), 4.94 (s, 2H), 7.51-7.85(m, 4H), 8.48(d, J =2.0Hz, 1H), 8.61(d, J =2.0Hz, 1H).LC-MS: m/z = 356.1[M+H]⁺ (93.17% purity, 220 nm).

### Example 13: Preparation of Compound SYY-C015

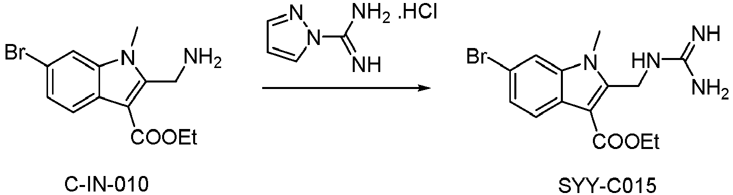

C-IN-010(150 mg ,0.48 mmol), 1H-pyrazole-1-formamidine hydrochloride (92 mg,0.63 mmol) and DIEA(187mg, 1.45 mmol) were dispersed into acetonitrile (4 ml) and reacted overnight at room temperature, and a large amount of solids were precipitated. The reaction solution was filtered, and the filter cake was washed with 1ml acetonitrile, and dried to obtain SYY-C015(135 mg, yield 79%) as a white solid. ¹H NMR: (DMSO-*d₆*, 400MHz): δ1.37 (t, J = 7.2 Hz, 3H), 3.85 (s, 3H), 4.35 (q, J = 7.2 Hz, 2H), 4.93 (s, 2H), 7.27-7.65 (m, 4H), 7.93-7.96 (m, 2H), 8.39 (br, 1H). LC-MS: m/z=355.1[M+H]+ (98.96% purity, 254 nm)

### Example 14: Preparation of Compound SYY-C016

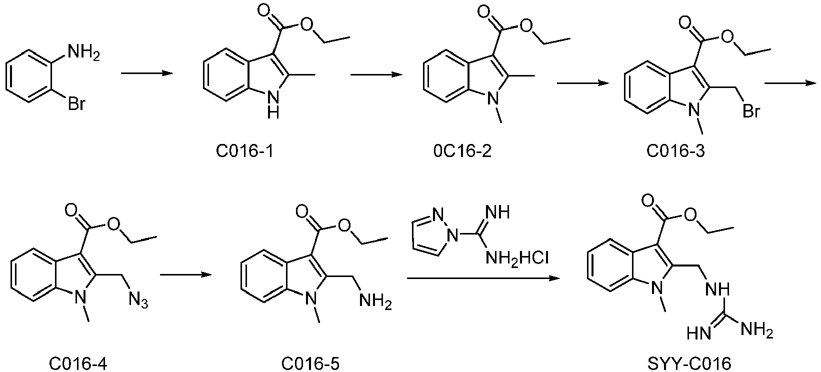

### Step 1

O-bromoaniline (10.0g, 58.13 mmol), ethyl acetoacetate (45.39g, 0.35 mol), Cs₂CO₃(37.88g, 0.12 mol), Tetrazoleacetic acid (1.49g, 11.63 mmol) and cuprous iodide (1.11g, 5.83 mmol) were dispersed into DMSO solution (50 mL), and the mixture was heated to 100 °C to react for 3 hours under the protection of nitrogen. TLC showed the reaction was completed. The reaction solution was cooled to room temperature, quenched with water, extracted with ethyl acetate, the organic phases were combined, washed with saturated salt water, dried over anhydrous sodium sulfate, and concentrated, and the crude product was purified by silica gel column chromatography to obtain C016-1(8.2g, yield 69%) as a light yellow solid, LC-MS:m/z = 204.1[M + H]⁺.

### Step 2:

Under an ice bath, 60% NaH(885 mg, 22.1 mmol) was added to a solution of C016-1(2.0g, 9.84 mmol) in DMF(20 ml), stirred for 15 min, methyl iodide (3.14g, 22.1 mmol) was added, and the mixture was stirred for 30min. TLC showed the reaction was completed. The reaction solution was quenched with ice water, solid was precipitated, filtered, and the filter cake was dissolved with DCM, dried over anhydrous sodium sulfate, filtered and concentrated to obtain C016-2(2.1g, crude). ¹H NMR(CDCl₃, 400Hz): δ 8.15-8.17(m, 1H), 7.24-7.31(m, 3H), 4.43 (q, J=7.2 Hz, 2H), 3.68(s, 3H), 2.78(s, 3H), 1.49(t, J=7.2 Hz, 3H). LC-MS: m/z= 218.2[M+H]⁺

### Step 3:

C016-2(1.0g, 4.6 mmol) was dissolved in carbon tetrachloride (10 ml), NBS(0.82g, 4.6 mmol) and BPO(56 mg, 0.23 mmol) were added at room temperature, and the mixture was heated and refluxed for 2 hours. TLC showed the reaction was completed. The reaction solution was filtered, the filtrate was concentrated, and the crude was separated and purified by silica gel column chromatography to obtain C016-3(1.0g, yield for two steps: 72%) as a light yellow solid. LC-MS:m/z= 296.0 [M+H]⁺

### Step 4:

C016-3(1.5g,5.06 mmol) was dissolved in DMF(5mL), sodium azide (494 mg,7.60 mmol) was added at room temperature, and the system was heated to 60 °C for 2 hours. TLC showed the reaction was completed. The reaction solution was cooled to room temperature, quenched with water, extracted with ethyl acetate, the organic phases were combined, washed with saturated salt water, dried over anhydrous sodium sulfate, and concentrated to obtain C016-4(1.3 g, crude) as a light yellow solid. LC-MS:m/z= 281.1 [M+Na]⁺

### Step 5:

C016-4(1.5g,5.81 mmol) was dissolved in methanol (20 mL), Pd/C(150 mg) was added, and the mixture was reacted overnight at room temperature. LCMS showed the reaction was completed. The reaction liquid was filtered by diatomite, the filtrate was concentrated to obtain a light yellow solid, slurried with DCM, filtered, and the filter cake was washed and dried to obtain C016-5(1.0g, crude) as a white solid. LC-MS:m/z=216.1 [M+H]⁺

### Step 6:

C016-5(150 mg, crude) was dispersed in acetonitrile (5mL), DIPEA(336 mg,2.60 mmol) and pyrazole formamidine hydrochloride (104 mg,0.71 mmol) were added, the mixture was stirred to react at room temperature overnight, white solids were precipitated. The reaction solution was filtered, and the filter cake was washed and dried to obtain a white solid (120 mg, yield for three steps: 50%). ¹H NMR: (DMSO-*d₆*, 400MHz): δ1.39 (t, J = 7.2 Hz, 3H), 3.86 (s, 3H), 4.36 (q, J = 7.2 Hz, 2H), 4.94 (d, J = 4.8 Hz, 2H), 7.24-7.78 (m, 6H), 8.03 (d, J = 7.6Hz, 1H), 8.29-8.35 (m, 1H). LC-MS: m/z=275.2 [M+H]⁺ (99.35% purity, 220 nm)

### Example 15: Preparation of Compound SYY-C017

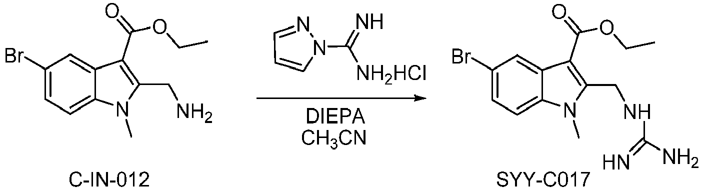

C-IN-012(300 mg, 0.96 mmol) was dispersed in acetonitrile (5 mL), DIPEA(503 mg,3.86 mmol) and pyrazole formamidine hydrochloride (155 mg,1.06 mmol) were added, the mixture was stirred to react at room temperature overnight, white solids were precipitated. The reaction solution was filtered, and the filter cake was washed and dried to obtain SYY-C017(100 mg, yield 29%) as a white solid. ¹H NMR: (DMSO-*d₆*, 400MHz): δ1.37 (t, J = 7.2 Hz, 3H), 3.85 (s, 3H), 4.37 (q, J = 7.2 Hz, 2H), 4.91 (s, 2H), 7.08-7.89 (m, 6H), 8.14 (d, J = 2.0 Hz, 1H). LC-MS: m/z = 353.1[M+H]⁺

### Example 16: Preparation of Compound SYY-C020

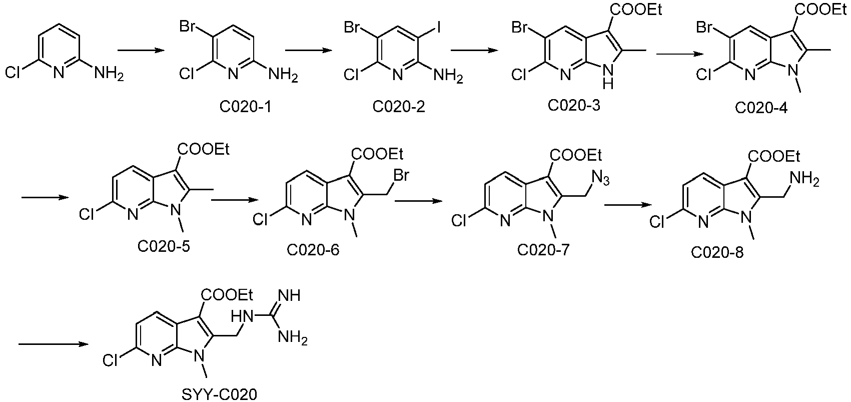

### Step 1:

Under the protection of N₂, 2-amino-6-chloropyridine (5.0g,38.89 mmol) was dissolved in DMF(50 mL), cooled to about 0°C under ice water bath, NBS(7.3g,40.78 mmol) was added in batches, and the mixture was stirred to react for 18h after addition. TLC showed the raw materials were reacted completely. The reaction solution was slowly added into water, extracted with ethyl acetate. The organic phases were combined, washed with saturated salt water, dried over anhydrous sodium sulfate, and concentrated to obtain C020-1(8.0g, crude) as an off-white solid.

### Step 2:

Under the protection of N₂, the C020-1(8.0g, crude) was dissolved in DMF(80 mL), NIS(10.5g,46.67 mmol) was added at room temperature, and the mixture was heated 90°C and stirred for 1h. TLC showed the raw materials were basically reacted completely. The reaction solution was cooled to room temperature, water was slowly added to the reaction solution, extracted with ethyl acetate, the organic phases were combined, washed with saturated salt water, dried over anhydrous sodium sulfate, and concentrated to obtain C020-2(11.4g, crude) as a dark brown solid.

### Step 3:

Under the protection of N₂, C020-2(6.2g, crude), CuI(345 mg,1.81 mmol), Cs₂CO₃(11.8g,36.22 mmol), ethyl acetoacetate (14.0 mL,110.70 mmol) and tetrazole acetic acid (463 mg,3.61 mmol) were dispersed into DMSO(80 mL), and the mixture was heated to 110°C and stirred for 2 hours. TLC showed the raw materials were basically reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C020-3(5.6g, crude) as a dark brown solid.

### Step 4:

Under the protection of N₂, C020-3(5.6g, crude) was dissolved in DMF(60 mL), NaH(60%)(1.06g,26.50 mmol) was added in batches under ice bath, after stirring for 15min, Mel(1.3 mL,20.88 mmol) was added, and the mixture was stirred for 0.5h at room temperature. TLC showed the raw materials were reacted completely. Under ice bath, the reaction solution was slowly added into saturated ammonium chloride solution, extracted with ethyl acetate, and the organic phases were combined, washed with saturated salt water, dried over anhydrous sodium sulfate, and concentrated, and purified by silica gel column chromatography to obtain C020-4(2.7g, yield for four steps: 44%) as a yellow solid. LCMS:m/z=331.0[M+H]⁺

### Step 5:

Under the protection of N₂, C020-4(620 mg ,1.87 mmol) was dissolved in THF(20 mL), cooled to -65°C, n-BuLi(2.5M)(2.3 mL,5.75mmol) was slowly added, and the mixture was stirred for 0.5h at -65°C after addition. TLC showed the raw materials were reacted completely. The reaction solution was quenched with saturated ammonium chloride solution, extracted with ethyl acetate, and the organic phases were combined, washed with saturated salt water, dried over anhydrous sodium sulfate, concentrated, and purified by silica gel column chromatography to obtain C020-5(470 mg, yield 99%) as a yellow solid. ¹H NMR (400 MHz, CDCl₃) δ 1.46 (t, *J* = 7.2 Hz, 3H),2.81 (s, 3H), 3.82 (s, 3H), 4.40 (q, *J* = 7.2 Hz, 2H), 7.18 (d, *J* = 8.2 Hz, 1H), 8.26 (d, *J* = 8.2 Hz, 1H). LCMS: m/z=253.1 [M+H]⁺

### Step 6:

Under the protection of N₂, C020-5(230 mg,0.91 mmol) was dissolved in CCl₄(6 mL), BPO(70%)(20 mg,0.058 mmol) was added at room temperature. After heating to 80°C, NBS(163 mg,0.92 mmol) was added, and the mixture was continued to react for 2 h. TLC showed the raw materials were reacted completely. The reaction solution was cooled to room temperature, and the crude product was purified by silica gel column chromatography to obtain C020-6(185 mg, yield 61%) as a light yellow solid. ¹H NMR (400 MHz, CDCl₃) δ 1.48 (t, *J=* 7.2 Hz, 3H), 3.95 (s, 3H), 4.45 (q, *J* = 7.2 Hz, 2H), 5.16 (s, 2H), 7.24 (d, *J* = 8.4 Hz, 1H), 8.34 (d, *J* = 8.4 Hz, 1H)

### Step 7:

Under the protection of N₂, C020-6(185 mg,0.56 mmol) was dissolved in DMF (10 mL), NaN₃(85 mg,1.31 mmol) was added at room temperature, and the mixture was heated to 50°C, stirred for 1 h, and stirred overnight at room temperature. TLC showed the raw materials were reacted completely. The reaction solution was cooled to room temperature, water was added to the reaction solution for quenching, extracted with ethyl acetate, the organic phases were combined, washed with saturated salt water, dried over anhydrous sodium sulfate, and concentrated to obtain C020-7(164mg, crude) which was directly used in the next step.

### Step 8:

Under the protection of N₂, C020-7 (164 mg, 0.56 mmol) was dissolved in THF/H₂O (6 mL/ 1.2 mL), PPh₃(293 mg, 1.12 mmol) was added at room temperature, and the mixture was heated to 50°C and stirred for 2 hours. TLC showed the raw materials and intermediates disappeared. The reaction solution was purified by silica gel column chromatography to obtain C020-8(130 mg, yield for two steps: 87%) as a light brown semi-solid.

### Step 9:

Under the protection of N₂, C020-8(130mg,0.49mmol) and DIEA(314mg,2.43 mmol) were dispersed into acetonitrile (8 mL), stirred to dissolve clear, 1H-1-methyl-formamidine hydrochloride (144 mg,0.98 mmol) was added, and the mixture was stirred at room temperature (solid had been precipitated) for 48h. TLC showed that the raw materials were completely consumed. The reaction solution was filtered, and the filter cake was washed with acetonitrile, and dried to obtain SYY-C020(100 mg, yield 67%) as an off-white solid. ¹H NMR (400 MHz, DMSO_d6): δ 1.38 (t, *J =* 7.2 Hz, 3H), 3.86 (s, 3H), 4.37 (q, *J=* 7.2 Hz, 2H), 4.97 (s, 2H), 7.41 (d, *J=* 8.4 Hz, 1H), 7.43-7.64 (m, 3H), 8.35 (d, *J* = 8.4 Hz, 1H), 8.38-8.41 (m, 1H). LCMS: m/z=310.1[M+H]⁺

### Example 17: Preparation of Compound SYY-C022

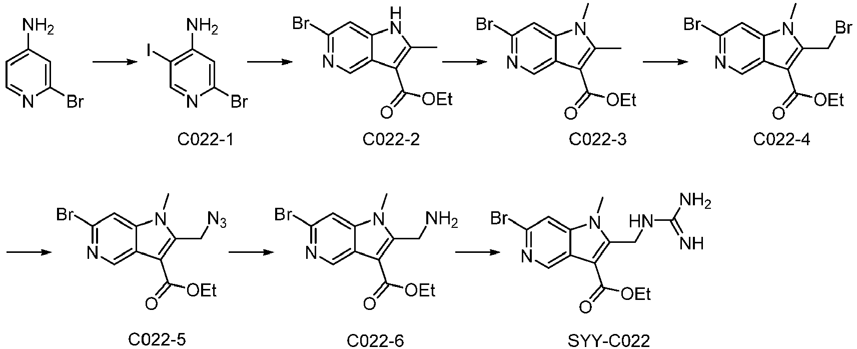

### Step 1:

4-Amino-2-bromopyridine (3.3g,19.07 mmol) and TFA(2.4g,21.05 mmol) were dissolved in DMF(30 mL), NIS(4.7g,20.89 mmol) was added at room temperature, and the mixture was heated to 50 °C to react for 2h. TLC detection showed that the raw materials were reacted completely. The reaction solution was cooled to room temperature, poured into ice water, neutralized with saturated sodium carbonate aqueous solution, extracted with ethyl acetate, the organic phases were combined, washed with saturated salt water, dried over anhydrous sodium sulfate, and concentrated, and the crude product was purified by silica gel column chromatography to obtain C022-1(2.3g) and C022-1-1(2.6g).

C022-1: ¹H NMR: (CDCl₃, 400MHz): δ4.75 (s, 2H), 6.80 (s, 1H), 8.33 (s, 1H).

C022-1-1: ¹H NMR: (CDCl₃, 400MHz): δ5.01 (s, 2H), 6.49 (d, J = 5.6 Hz, 1H), 7.88 (d, J = 5.6 Hz, 1H).

### Step 2:

C022-1(2.3g,7.69 mmol), ethyl acetoacetate (6.0g,46.10mmol), tetrazoleacetic acid (176 mg,1.37mmol), cuprous iodide (147 mg,0.77 mmol) and cesium carbonate (5.0g,15.35 mmol) were dispersed into DMSO(50mL), and the mixture was heated to 150°C to react for 7h under the protection of nitrogen. TLC detection showed that the raw materials were completely reacted. The reaction solution was cooled to room temperature, filtered with diatomite, washed, the filtrate was diluted with water, extracted with ethyl acetate, filtered with diatomite, separated, washed with water, the organic phases were combined, washed with saturated salt water, dried over anhydrous sodium sulfate, and concentrated, and the crude product was purified by silica gel column chromatography to obtain C022-2(560 mg, yield 26%) as a brown-yellow solid. LCMS:m/z=283.0/285.0 [M+H]⁺

### Step 3:

C022-2(560 mg,1.98 mmol) was dissolved in DMF(10 mL), cooled to about 0°C under the protection of nitrogen, 60% sodium hydride (95 mg,2.38 mmol) was added in batches, the mixture was returned to room temperature after addition, and stirred to react for 30 minutes, then cooled to about 0°C, a solution of methyl iodide (295 mg,2.08 mmol) in DMF(2 mL) was added dropwise, and the mixture was reacted at 0 °C for 30 minutes after addition. TLC monitored that the raw materials were reacted completely. The reaction solution was quenched with ice water, extracted with ethyl acetate, the organic phases were combined, washed with saturated salt water, dried over anhydrous sodium sulfate, and concentrated, and the crude product was purified by silica gel column chromatography to obtain C022-3(530 mg, yield 90%) as a light brown yellow solid. ¹H NMR (400 MHz, CDCl₃) : δ 1.46 (t, *J=* 7.2 Hz, 3H) , 2.79 (s, 3H) , 3.69 (s, 3H) , 4.42 (q, *J=* 7.2 Hz, 2H) , 7.41 (s, 1H), 9.05 (s, 1H). LCMS: m/z=297.0/299.0 [M+H]⁺

### Step 4:

C022-3(530 mg,1.78 mmol), NBS(317 mg,1.78 mmol) and BPO(44 mg,0.18 mmol) were dispersed in carbon tetrachloride (30 mL), and the mixture was heated until reflux to react for 2h. TLC detection showed a small amount of raw materials was remained. The reaction solution was cooled to room temperature and directly purified by silica gel column chromatography to obtain C022-4(410 mg, yield 61%) as a white solid.

### Step 5:

C022-4(410 mg,1.09 mmol) was dissolved in DMF(10 mL), sodium azide (107 mg,1.65mmol) was added, and the mixture was stirred overnight at room temperature. TLC detection showed that the raw materials were reacted completely. The reaction solution was quenched with water, extracted with ethyl acetate, and the organic phases were combined, washed with saturated salt water, dried over anhydrous sodium sulfate, and concentrated to obtain C022-5 (crude, TLC purity was good) as a yellowish brown solid which was directly used in the next reaction.

### Step 6:

C022-5 (crude product in the previous step, according to theoretical calculation, 1.09 mmol) was dissolved in THF(15 mL) and water (3 mL), triphenylphosphine (430 mg,1.64 mmol) was added at room temperature, and the reaction solution was heated to 60 °C to react for 2 hours. TLC detection showed that the raw materials were reacted completely. The reaction solution was cooled to room temperature, concentrated, dissolved with ethyl acetate, washed with water, washed with saturated salt water, dried over anhydrous sodium sulfate, and concentrated, and the crude product was purified by silica gel column chromatography to obtain C022-6(250 mg, yield for two steps: 74%) as an off-white solid. ¹H NMR (400 MHz, DMSO_d6) : δ 1.37 (t, *J=* 7.2 Hz, 3H) , 1.90 (br, 2H), 3.84 (s, 3H), 4.17 (s, 2H), 4.33 (q, *J* = 7.2 Hz, 2H), 7.94 (s, 1H), 8.88 (s, 1H). LCMS: m/z=312.1[M+H]⁺

### Step 7:

C022-6(100 mg,0.32 mmol) was dispersed in acetonitrile (3 mL), DIEA(165 mg,1.28 mmol) was added, stirred but not dissolved, methanol (1 mL) was added, the reaction solution was clarified, pyrazole formamidine hydrochloride (94 mg,0.64 mmol) was added, and the reaction solution was stirred at room temperature for the weekend. The reaction solution precipitated solid, filtered, and the filter cake was washed with acetonitrile, and dried to obtain an off-white solid (73mg, yield 65%). ¹H NMR: (DMSO-*d₆*, 400MHz): δ1.39 (t, J = 7.2 Hz, 3H), 3.86 (s, 3H), 4.38 (q, J = 7.2 Hz, 2H), 4.94 (d, J = 4.0 Hz, 2H), 7.46 (br, 3H), 8.06 (s, 1H), 8.38 (s, 1H), 8.96 (s, 1H). LCMS:m/z=354.1[M+H]⁺ (98.07% purity, 220 nm).

### Example 18: Preparation of Compound SYY-C023

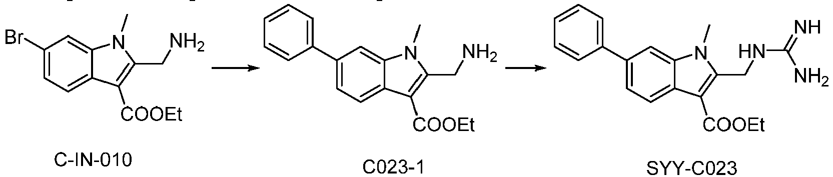

### Step 1:

C-IN-010(200 mg,0.64 mmol), phenylboronic acid (157 mg,1.29 mmol), PdCl₂(dppf) dichloromethane complex (52 mg,0.06 mmol) and sodium carbonate (266 mg,2.51 mmol) were dissolved in a mixed solvent of dioxane (20ml) and water (5ml), and the mixture was heated to 100°C to react for 2 hours. LCMS detection showed the reaction was completed. The reaction solution was cooled to room temperature, added with water, extracted with ethyl acetate, the organic phases were combined, washed with saturated salt water, dried over anhydrous sodium sulfate, concentrated and dried, and the crude product was purified by Pre-TLC (DCM/MeOH = 10/1) to obtain 220mg brown oil, 0.5ml DCM was added, 5ml PE was added dropwise, stirred for 30min, filtered, and the filter cake was washed and dried to obtain C023-1(55 mg, crude product) as a yellow solid.

### Step 2:

C023-1(55 mg,0.18 mmol), 1H-pyrazol-1-formamidine hydrochloride (40 mg,0.27 mmol), and DIEA(92 mg,0.71mmol) were dissolved in acetonitrile (4ml) and reacted at room temperature for 1 day with solid precipitation. The reaction solution was filtered and the filter cake was washed with 1ml acetonitrile to obtain white solid(54 mg, yield for two steps: 24%). ¹H NMR: (DMSO-*d₆*, 400MHz): δ1.41 (t, J = 7.2 Hz, 3H), 3.93 (s, 3H), 4.38 (q, J = 7.2 Hz, 2H), 4.96 (s, 2H), 7.22-7.71 (m, 7H), 7.76-7.82 (m, 2H), 7.94 (s, 1H), 8.09 (d, J = 8.4 Hz, 1H), 8.30 (br, 1H). LC-MS: m/z=351.2[M+H]⁺(99.66% purity, 254 nm)

### Example 19: Preparation of Compound SYY-C024 and SYY-C025

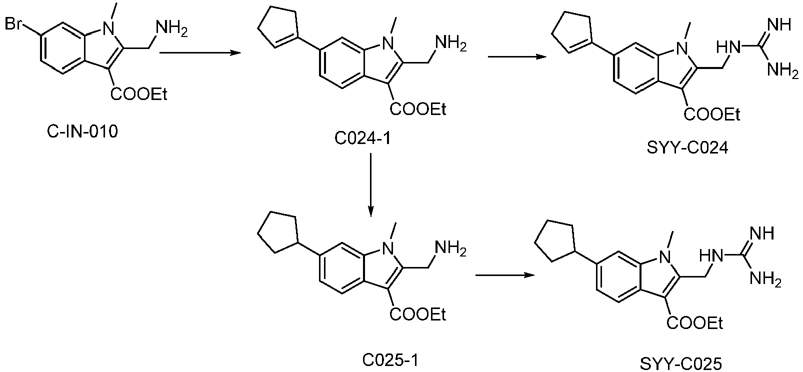

### Step 1:

C-IN-010(600 mg,1.93 mmol), cyclopenten-1-ylboronic acid (432 mg,3.86 mmol), Pd(dppf)Cl₂(157 mg,0.19 mmol) and sodium carbonate (798 mg,7.53 mmol) were dissolved in a mixed solvent of 1,4-dioxane (30ml) and water (10ml), and heated to 100°C to react for 2 hours under the protection of nitrogen. LCMS monitored the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(DCM/MeOH = 80/1) to obtain C024-1(510 mg, yield 89%) as a yellow solid. LCMS:m/z=282.2[M-NH₂]⁺

### Step 2:

C024-1(100 mg,0.34 mmol), 1H-pyrazol-1-formamidine hydrochloride (74 mg,0.50 mmol), and DIEA(173 mg,1.34 mmol) were dissolved in acetonitrile (6 ml) and reacted at room temperature for 1 day with solid precipitation. The reaction solution was filtered, and the filter cake was washed with 1ml acetonitrile, and dried to obtain SYY-C024(87 mg, yield 76%) as a light yellow solid. ¹H NMR: (DMSO-*d₆*, 400MHz): δ1.39 (t, J = 7.2 Hz, 3H), 1.94-2.06 (m, 2H), 2.52-2.56 (m, 1H), 2.72-2.82 (m, 2H), 3.87 (s, 3H), 4.36 (q, J = 7.2 Hz, 2H), 4.92 (s, 2H), 6.35 (s, 1H), 7.07-7.72 (m, 6H), 7.95 (d, J = 8.4 Hz, 1H), 8.29 (br, 1H). LCMS: m/z=341.2[M+H]⁺ (98.08% purity, 254 nm).

### Step 3:

C024-1(200 mg,0.67 mmol) was dissolved in methanol (10 ml), palladium/carbon (200 mg) was added at room temperature, replaced with hydrogen for three times, and the mixture was heated to 50°C to react for 1.5h. LCMS detection showed that the reaction was completed. The reaction solution was cooled to room temperature, filtered with diatomite, the filtrate was concentrated, and the crude product was purified by Pre-TLC (DCM/MeOH = 10/1) to obtain C025-1(140 mg, yield 70%) as a white solid. LCMS:m/z = 284.1[M-NH₂]⁺

### Step 4:

C025-1(70 mg,0.23 mmol), 1H-pyrazol-1-formamidine hydrochloride (52 mg,0.35mmol), and DIEA(121 mg,0.94 mmol) were dissolved in acetonitrile (6 ml), reacted at room temperature for 1 days, solids were precipitated, filtered, and the filter cake was washed with 1ml acetonitrile to obtain light yellow solid(44 mg, yield 55%). ¹H NMR: (DMSO-*d₆*, 400MHz): δ1.38 (t, J = 7.2 Hz, 3H), 1.56-1.88 (m, 6H), 2.00-2.12 (m, 2H), 3.07-3.15 (m, 1H), 3.83 (s, 3H), 4.35 (q, J = 7.2 Hz, 2H), 4.91 (s, 2H), 7.16-7.92 (m, 7H). LCMS: m/z=343.2[M+H]⁺ (99.39% purity, 220 nm).

### Example 20: Preparation of Compound SYY-C026

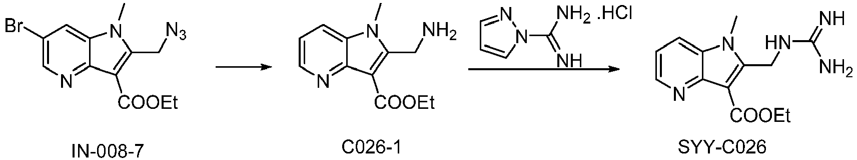

### Step 1:

IN-008-7(210 mg,0.62 mmol) was dissolved in methanol (5 ml), 10% Pd(OH)2/C(210 mg) was added at room temperature, replaced with hydrogen for three times, and the mixture was heated to 60°C to react for 1h. TLC monitored (raw materials were reacted completely). The reaction solution was cooled to room temperature, filtered with diatomite, the filtrate was concentrated, and the crude product was purified by Pre-TLC (DCM/MeOH = 7/1) to obtain C026-1(106 mg, yield 73%) as a white solid. LCMS:m/z=234.1[M +H]⁺

### Step 2:

C026-1(100 mg,0.43mmol), 1H-pyrazol-1-formamidine hydrochloride (75mg,0.51mmol), DIEA(221mg,1.71mmol) were dispersed into acetonitrile (4ml) and methanol (2mL), and the mixture was reacted at room temperature for 2 days with solid precipitation. The reaction solution was filtered, and the filter cake was washed with acetonitrile, and dried to obtain SYY-C026(36 mg, yield 31%) as a white solid. ¹H NMR: (DMSO-*d₆*, 400MHz): δ1.34 (t, J = 7.2 Hz, 3H), 3.88 (s, 3H), 4.37 (q, J = 7.2 Hz, 2H), 4.95 (s, 2H), 7.25-7.65 (m, 4H), 8.08 (d, J = 8.4 Hz, 1H), 8.31 (br, 1H), 8.55 (d, J = 4.4 Hz, 1H). LCMS:m/z=276.2[M+H]⁺ (97.63% purity, 220 nm)

### Example 21: Preparation of Compound SYY-C028

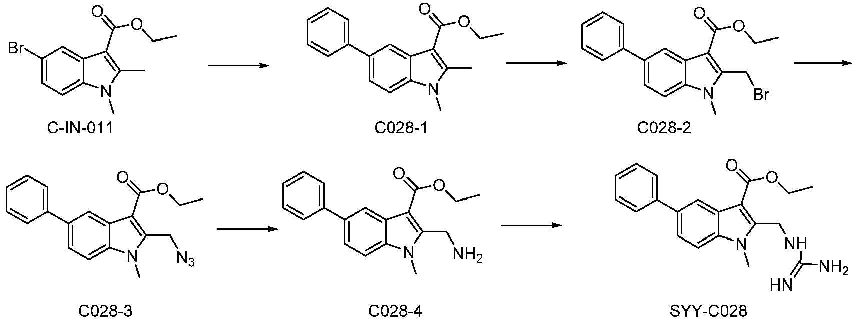

### Step 1:

C- IN-011(1.21g,4.1 mmol), phenylboronic acid (100g,8.2 mmol), Pd(dppf)Cl₂(355 mg,0.41 mmol) and sodium carbonate (870 mg,8.2 mmol) were dispersed into a mixed solvent of dioxane(100ml) and water (5ml), and the mixture was heated to 100°C to react for 2h, and the reaction was completed. The reaction solution was cooled to room temperature, added with water, extracted with ethyl acetate, the organic phases were combined, washed with saturated salt water, dried over anhydrous sodium sulfate, concentrated and dried, and the crude product was purified by pre-TLC to obtain C028-1(800 mg, yield 67%) as a yellow solid.

### Step 2:

C028-1(378 mg,1.29 mmol) was dissolved in carbon tetrachloride (100 mL), NBS(229 mg,1.28 mmol) and BPO(31 mg,0.12 mmol) were added, and the mixture was heated to 70°C to react for 3 hours. TLC showed the raw materials disappeared. The reaction solution was cooled to room temperature, filtered, the filtrate was concentrated, and purified by pre-TLC to obtain C028-2(273 mg, yield 57%) as a yellow solid.

### Step 3:

C028-2(273 mg,0.73 mmol) was dissolved in DMF(5 mL), sodium azide (72 mg,1.10 mmol) was added at room temperature, and the system was heated to 60 °C for 2 hours. TLC showed the reaction was completed. The reaction solution was cooled to room temperature, quenched with water, extracted with ethyl acetate, and the organic phases were combined, washed with saturated salt water, dried over anhydrous sodium sulfate, and concentrated to obtain C028-3(200 mg, crude) as a light yellow oil.

### Step 4:

C028-3(200 mg,0.60 mmol) was dissolved in MeOH (10 mL), Pd/C(50 mg) was added at room temperature, the temperature was raised to 40°C, and the reaction was carried out in hydrogen atmosphere for 5h. TLC showed the reaction was completed. The reaction solution was filtered with diatomite, and the filtrate was concentrated to obtain C028-4(130 mg, crude) as a light yellow oil.

### Step 5:

C028-4(130 mg, crude), 1H-pyrazol-1-formamidine hydrochloride (68 mg,0.46 mmol) and DIEA(217 mg,1.68 mmol) were dispersed into acetonitrile (5ml) and reacted at room temperature for 18h, and solids were precipitated. The reaction solution was filtered and the filter cake was washed with 1ml acetonitrile, and dried to obtain a white solid(50 mg, yield for two steps: 24%). ¹H NMR: (DMSO-*d₆*, 400MHz): δ1.40 (t, J = 7.2 Hz, 3H), 3.89 (s, 3H), 4.39 (q, J = 7.2 Hz, 2H), 4.95 (s, 2H), 7.24-7.80 (m, 10H), 8.28-8.36 (m, 2H). LC-MS: m/z = 351.2[M+H]⁺

### Example 22: Preparation of Compound SYY-C029

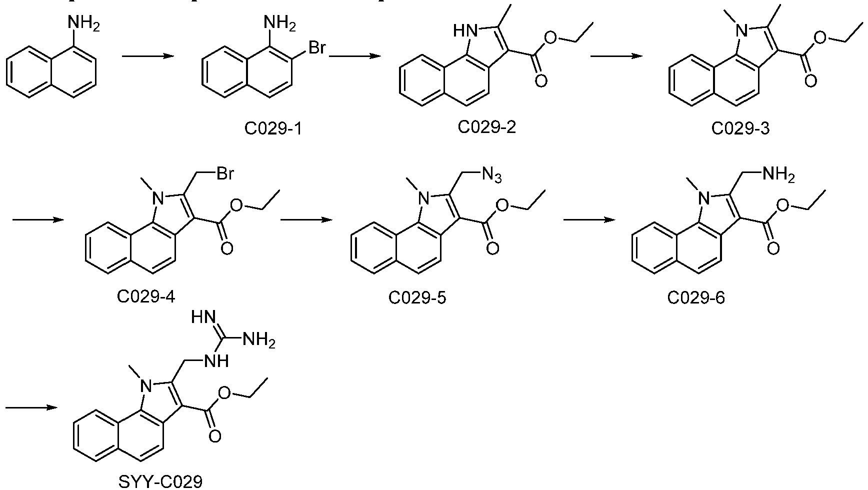

### Step 1:

1-naphthylamine (9.34g,65.23 mmol), NBS(11.61g,65.23 mmol) and acetic acid (4.11g,68.49 mmol) were dispersed into acetonitrile (200ml) and reacted at room temperature for 18h. TCL showed the raw materials disappeared and there were two new points. The reaction solution was diluted with ethyl acetate, washed with saturated NaCO₃ aqueous solution, washed with saturated salt water, dried over anhydrous sodium sulfate, and concentrated, and the crude was purified by silica gel column chromatography to obtain C029-1(7.0g, yield 48%).

### Step 2:

C029-1(7.0g,31.52 mmol), ethyl acetoacetate (24.61g,189.12 mmol), tetrazoleacetic acid(807 mg,6.30 mmol), cuprous iodide (600 mg,3.15 mmol) and cesium carbonate (20.54g,63.04 mmol) were dispersed into DMSO(100 mL), and the mixture was heated to 100 degrees Celsius to react for 3 hours under the protection of nitrogen. TCL showed the raw materials disappeared. The reaction solution was cooled to room temperature, diluted with ethyl acetate, washed with saturated salt water, dried over anhydrous sodium sulfate, and concentrated, and the crude product was separated and purified by silica gel column chromatography to obtain C029-2(1.2g, yield 15%).

### Step 3:

C029-2(1.2g,4.74 mmol) was dissolved in DMF(10 mL), 60% sodium hydride (284 mg,7.11 mmol) was added at 0°C, stirred for 30 min, methyl iodide (1.0g,7.11 mmol) was added, and the mixture was reacted at 0°C for 2h. TLC showed the raw materials disappeared. The reaction solution was quenched with ice water, extracted with ethyl acetate, the organic phases were combined, washed with saturated salt water, dried over anhydrous sodium sulfate, and concentrated, and the crude product was purified by silica gel column chromatography to obtain solid C029-3(350 mg, yield 28%).

### Step 4:

C029-3(350 mg,1.31 mmol), NBS(233 mg,1.31 mmol) and BPO(45 mg,0.13 mmol) were dissolved in carbon tetrachloride and heated to 80°C to react for 2 hours. TLC showed the reaction was completed. The reaction solution was concentrated to obtain C029-4 (crude), which was directly used in the next step.

### Step 5:

C029-4 (crude) and NaN3(128 mg,1.97 mmol) were dispersed into DMF(10 mL), and heated to 70°C to react for 2h. TLC showed the raw materials disappeared. The reaction solution was cooled to room temperature, added with water, extracted with ethyl acetate, washed with saturated salt water, dried over anhydrous sodium sulfate, and concentrated to obtain C029-5 (crude) which was directly used in the next step.

### Step 6:

The C029-5 (crude) was dissolved in methanol (10 mL), 10% Pd/C(300 mg) was added at room temperature, replaced with hydrogen for three times, and the mixture was reacted at room temperature for 48h. TLC showed the raw material disappeared. The reaction solution was filtered by diatomite, the filtrate was concentrated, and the crude silica was separated and purified by silica gel column chromatography to obtain solid C029-6(80 mg, yield for three steps: 22%)

### Step 7:

C029-6(80 mg, 0.28 mmol), 1H-pyrazol-1-formamidine hydrochloride (45 mg,0.31 mmol) and DIPEA(145 mg,1.12 mmol) were dispersed into acetonitrile (5ml) and reacted at room temperature for 18h, and solids were precipitated. The reaction solution was filtered, and the filter cake was washed and dried to obtain SYY-C029(25 mg, yield 27%) as a white solid.

¹H NMR: (DMSO-*d₆*, 400MHz): δ1.42 (t, J = 7.2 Hz, 3H), 4.29 (s, 3H), 4.41 (q, J = 7.2 Hz, 2H), 5.02 (s, 2H), 7.24-7.76 (m, 7H), 8.06 (d, J = 8.0 Hz, 1H), 8.23 (d, J = 8.8 Hz, 1H), 8.70 (d, J = 8.4 Hz, 1H). LC-MS: m/z = 325.2 [M+H]⁺

### Example 23: Preparation of Compound SYY-C030

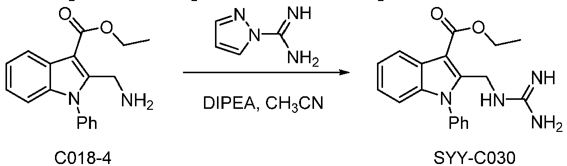

C018-4(300 mg,1.02 mmol) and 1H-pyrazol-1-formamidine hydrochloride (165 mg,1.12 mmol) were dissolved in CH₃CN(5 ml), DIPEA(516 mg,4.08 mmol) was added, and stirred overnight at room temperature. TLC showed the reaction was completed. In the process of concentration of the reaction solution, solid was precipitated, filtered and the filter cake was washed with acetonitrile to obtain SYY-C030(260 mg, yield 76%) as a white solid. ¹H NMR: (DMSO-*d₆*, 400MHz): δ1.39 (t, J = 7.2 Hz, 3H), 4.38 (q, J = 7.2 Hz, 2H), 4.55 (d, J = 4.0 Hz, 2H), 6.95-7.49 (m, 6H), 7.51-7.60 (m, 2H), 7.62-7.72 (m, 3H), 8.11-8.17 (m, 2H). LC-MS: m/z=337.1[M+H]⁺ (99.57% purity, 220 nm)

### Example 24: Preparation of Compound SYY-C031

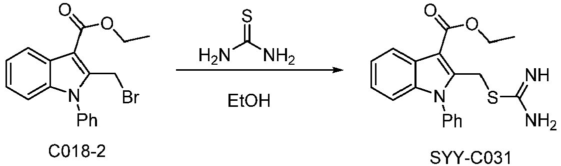

C018-2(470 mg, 1.31 mmol) was dissolved in EtOH(5 ml), thiourea (110 mg,1.45 mmol) was added at room temperature, and the mixture was heated to 80 °C and stirred for 2 hours. TLC showed the reaction was completed. The reaction solution was concentrated and the crude product was purified by pre-TLC to obtain solid (110 mg, yield 24%). ¹H NMR: (DMSO-*d₆*, 400MHz): δ 1.40 (t, J = 7.2 Hz, 3H), 4.40 (q, J = 7.2 Hz, 2H), 4.75 (s, 2H), 7.01(d, J = 8.0Hz, 1H), 7.28-7.33(m, 2H), 7,53-7.55(m, 2H), 7.68-7.71(m, 3H), 8.11(d, J = 8.0Hz, 1H), 8.98(br, 2H). LC-MS: m/z=354.1[M+H]⁺ (93.59% purity, 254 nm).

### Example 25: Preparation of Compound SYY-C032

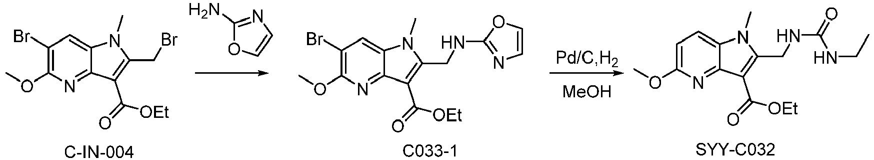

### Step 1:

C- IN-004(400 mg,0.99 mmol), 2-aminooxazole (168 mg,2.00 mmol) and DIEA(322 mg,2.49 mmol) were dissolved in dichloromethane (10 mL), and stirred for 2 days at room temperature. TLC detected the raw materials were remained, and new spots were produced. The reaction solution was purified by silica gel column chromatography to obtain C033-1(85 mg, yield 21%) as an off-white solid. LCMS:m/z=409.0/412.0[M +H]⁺

### Step 2:

C033-1(85 mg,0.21 mmol) was dissolved in methanol (10 mL), palladium carbon (85 mg) was added, replaced with hydrogen for three times, and the mixture was heated to 60 °C to react for 2 h. TLC detection showed the reaction was completed. The reaction solution was cooled to room temperature, filtered with diatomite, the filtrate was concentrated, and the crude product was neutralized with saturated sodium carbonate aqueous solution, extracted with ethyl acetate, the organic phases were combined, washed with saturated salt water, dried over anhydrous sodium sulfate, and concentrated, and the crude product was purified by Pre-TLC to obtain SYY-C032(40 mg, yield 58%) as a white solid. ¹H NMR: (CD₃OD, 400MHz): δ1.00 (t, J = 7.2 Hz, 3H), 1.46 (t, J = 7.2 Hz, 3H), 3.21 (q, J = 7.2 Hz, 2H), 4.01 (s, 3H), 3.79 (s, 3H), 4.40 (q, J = 7.2 Hz, 2H), 5.25 (s, 2H), 6.72 (d, J = 8.8 Hz, 1H), 7.81 (d, J = 8.8 Hz, 1H). LCMS: m/z=335.2[M +H]⁺ (96.30% purity, 220 nm)

### Example 26: Preparation of Compound SYY-C034

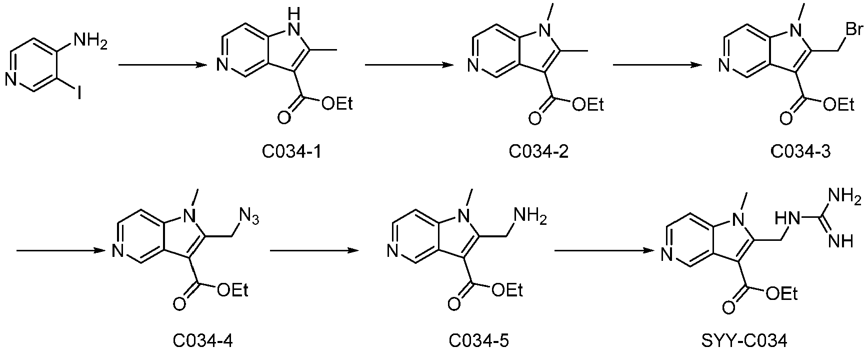

### Step 1:

Under the protection of nitrogen, 4-amino-3-iodopyridine (4.00g,18.18 mmol), ethyl acetoacetate (14.20g,109.11 mmol), tetrazoleacetic acid (415 mg,3.24 mmol), cuprous iodide (347 mg,1.82 mmol) and cesium carbonate (1.18g,3.62 mmol) were dispersed into DMSO(80mL), and the mixture was heated to 150°C to react for 7h. TLC detection showed the raw materials were reacted completely. The reaction solution was cooled to room temperature, filtered with diatomite, washed, the filtrate was diluted with water, extracted with ethyl acetate, the organic phases were combined, washed with saturated salt water, dried over anhydrous sodium sulfate, and concentrated, and the crude product was purified by silica gel column chromatography to obtain C034-1(2.1g, yield 57%) as a brown-yellow solid. LCMS:m/z=205.1 [M+H]⁺

### Step 2:

C034-1(1.0g,4.90 mmol) was dispersed in DMF(10mL), cooled to about 0°C under the protection of nitrogen, 60% sodium hydride (255 mg,6.38 mmol) was added in batches, and the mixture was returned to room temperature to react for half an hour after addition. After cooling to 0°C again, a solution of methyl iodide (765 mg,5.39 mmol) in DMF(5 ml) was added dropwise, and the reaction was continued at 0°C for half an hour after addition. TLC detection showed the raw materials were reacted completely. The reaction solution was quenched with water, extracted with ethyl acetate, the organic phases were combined, washed with saturated salt water, dried over anhydrous sodium sulfate, and concentrated, and the crude product was purified by silica gel column chromatography to obtain C034-2(630 mg, yield 59%) as a light brown solid. LCMS:m/z=219.1[M+H]⁺

### Step 3:

C034-2(510 mg,2.34 mmol) was dissolved in trifluoroacetic acid (5 mL), NBS(749 mg,4.21mmol) was added, and the reaction solution was stirred overnight at room temperature. TLC detection showed the raw materials were reacted completely. The reaction solution was concentrated at room temperature to obtain C034-3 (crude), which was directly used in the next reaction.

### Step 4:

C034-3 (crude product in the previous step, 2.34 mmol by theoretical calculation) was dissolved in DMF(5 mL), cooled to about 0°C under an ice bath, neutralized with DIEA, NaN3(183 mg,2.81 mmol) was added, and the reaction solution was heated to 40°C to react for 2h. LCMS detection showed that the reaction was completed. The reaction solution was cooled to room temperature, poured into water, extracted with ethyl acetate, organic phases were combined, washed with saturated salt water, dried over anhydrous sodium sulfate, and concentrated, and the crude product was purified by silica gel column chromatography to obtain C034-4(320 mg, yield for two steps: 53%) as a brown-yellow viscous oil. LCMS:m/z=260.1[M+H]⁺

### Step 5:

C034-4(320 mg,1.23 mmol) was dissolved in a mixed solvent of THF(15mL) and water (3mL), triphenylphosphine (486 mg,1.85 mol) was added at room temperature, and the mixture was heated to 60°C to react for 2h. TLC and LCMS detection showed that the reaction was completed. The reaction solution was cooled to room temperature, concentrated to an amount of solvent, diluted with water, extracted with ethyl acetate, the organic phases were combined, washed with saturated salt water, dried over anhydrous sodium sulfate, and concentrated, and the crude product was purified by Pre-TLC to obtain C034-5(230 mg, yield 80%) as a gray-white solid. LC-MS: Not available

### Step 6:

C034-5(55 mg,0.24 mmol) was dispersed in acetonitrile (5 mL), DIEA(122 mg,0.94 mmol) was added, after stirring to be dissolved, pyrazole formamidine hydrochloride (69 mg,0.47 mmol) was added at room temperature, the reaction solution was heated to 25°C to react for 3 days after addition, and solids were precipitated. The reaction liquid was filtered, and the filter cake was washed and dried to obtain a white solid (36mg, yield 55%). ¹H NMR: (CD₃OD, 400MHz): δ1.50 (t, J = 7.2 Hz, 3H), 3.95 (s, 3H), 4.51 (q, J = 7.2 Hz, 2H), 5.05 (s, 2H), 7.65 (d, J = 6.0 Hz, 1H), 8.38 (d, J = 6.0 Hz, 1H), 9.23 (s, 1H). LCMS: m/z=276.1[M+H]⁺ (99.12% purity, 220 nm)

### Example 27: Preparation of Compound SYY-C035

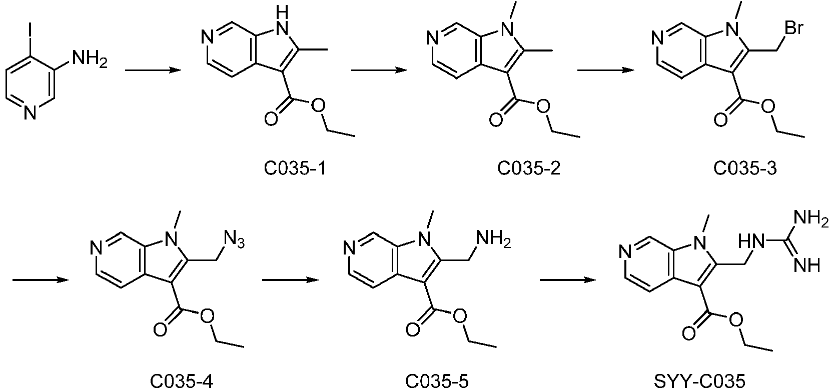

### Step 1:

Under the protection of nitrogen, 3-amino-4-iodopyridine (3.0g,13.64 mmol) was dissolved in DMSO(50 ml), ethyl acetoacetate (10.6g,81.45 mmol), tetrazoleacetic acid (349 mg,2.732mmol), cuprous iodide (260 mg,1.37 mmol) and cesium carbonate (8.9g,27.32 mmol) were added, and the mixture was heated to 150°C to react for 7 hours after the addition. TLC detection showed the raw materials were reacted completely. The reaction solution was cooled to room temperature, filtered with diatomite, the filtrate was added with water, extracted with ethyl acetate, the organic phases were combined, washed with saturated salt water, dried over anhydrous sodium sulfate, and concentrated, and the crude product was purified by silica gel column chromatography (DCM/MeOH = 15/1) to obtain C035-1(1.96g, yield 71%) as a yellow solid. LCMS:m/z=205.1[M+H]⁺

### Step 2:

Under the protection of nitrogen, C035-1(1.0g,4.90 mmol) was dissolved in dry DMF(15 ml), cooled under an ice water bath, 60% NaH(206 mg,5.15 mmol) was added in batches at 0 °C, and the mixture was stirred for 0.5h at room temperature after addition, then cooled to 0°C, methyl iodide (731 mg,5.15 mmol) was added dropwise, and the reaction was continued for 20 min after addition. TLC detection showed that the raw materials were reacted completely. The reaction solution was quenched with water, extracted with ethyl acetate, the organic phases were combined, washed with saturated salt water, dried over anhydrous sodium sulfate, and concentrated, and the crude product was purified by silica gel column chromatography (DCM/MeOH = 100/1) to obtain C035-2(485 mg, yield 45%) as a yellow solid. LCMS:m/z=219.1[M+H]⁺

### Step 3:

C035-2(436 mg,2.00 mmol) was dissolved in TFA(10 ml), NBS(374 mg,2.10 mmol) was added, and the mixture was reacted overnight at room temperature. TLC detection showed that a small amount of raw materials were not reacted, additional NBS(36 mg,0.20 mmol) was added, and the reaction was continued at room temperature for 3 hours. TLC detection showed that the raw materials were reacted completely. The reaction solution was concentrated at room temperature to obtain C035-3 (crude) which was directly used in the next step.

### Step 4:

C035-3 (crude, 2.00mmol by theory) was dissolved in DMF(10 ml), DIEA was added to adjust pH to be neutral, NaN₃(195 mg,3.00 mmol) was added, and the mixture was heated to 50°C to react for 2h. LCMS detection showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C035-4(345 mg, yield for two steps: 67%) as a yellow solid. LCMS:m/z=260.1[M+H]⁺

### Step 5:

C035-4(340 mg,1.31 mmol) and PPh₃(516 mg,1.97 mmol) were dissolved in a mixed solvent of THF(12 ml) and water (4 ml), and reacted overnight at room temperature. LCMS monitored (raw materials were reacted completely). The reaction solution was diluted with ethyl acetate, washed with water, washed with saturated salt water, dried over anhydrous sodium sulfate, and concentrated, and the crude product was purified by pre-TLC (DCM/MeOH = 15/1) to obtain C035-5(210 mg, yield 69%) as a light yellow solid. LCMS:m/z=234.1[M+H]⁺

### Step 6:

C035-5(100 mg,0.43 mmol), 1H-pyrazol-1-formamidine hydrochloride (95 mg,0.65 mmol), and DIEA(222 mg,1.72 mmol) were dissolved in acetonitrile (6 ml) and reacted at room temperature for 36h. Solid was precipitated in the reaction solution, filtered, and the filter cake was washed and dried to obtain SYY-C035(105 mg, yield 89%) as a white solid. ¹H NMR: (DMSO-*d₆*, 400MHz): δ1.39 (t, J = 7.2 Hz, 3H), 3.98 (s, 3H), 4.37 (q, J = 7.2 Hz, 2H), 4.98 (s, 2H), 7.52 (br, 3H), 7.90 (dd, J = 6.0, 1.2 Hz, 1H), 8.35 (d, J = 6.0 Hz, 1H), 8.42 (br, 1H), 9.03 (d, J = 1.2 Hz, 1H). LCMS: m/z=276.2[M+H]⁺ (95.94% purity, 220 nm)

### Example 28: Preparation of Compound SYY-C036

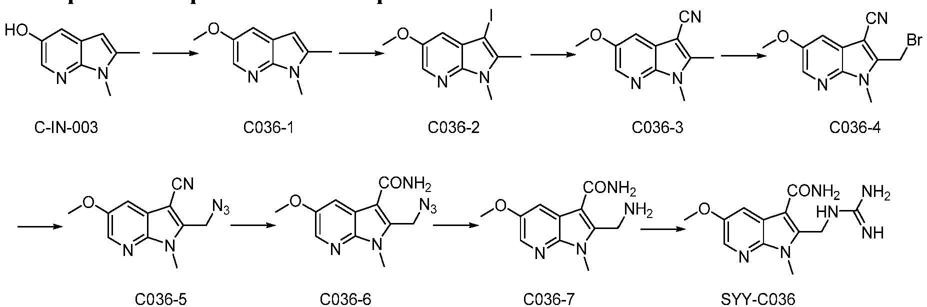

### Step 1:

Under the protection of N₂, C- IN-003(1.5g,9.25 mmol) was dissolved in DMF(20 mL), NaH(60%)(560 mg,14.00 mmol) was added under ice bath, after stirring for 15 min, MeI(0.7 mL,11.24 mmol) was added, and the mixture was stirred for 0.5h at room temperature. TLC showed the raw materials were reacted completely. Under ice bath, the reaction solution was slowly poured into water, extracted with ethyl acetate, the organic phases were combined, washed with saturated salt water, dried over anhydrous sodium sulfate, and concentrated, and the crude product was purified by silica gel column chromatography to obtain C036-1(1.0g, yield 62%) as a light yellow solid.

### Step 2:

Under the protection of N₂, C036-1(1.0g,5.67 mmol) was dissolved in DMF(20mL), NIS(1.28g,5.69 mmol) was added under an ice bath, and stirred for 2min. TLC showed the raw materials disappeared. The reaction solution was slowly added with water, extracted with ethyl acetate, the organic phases were combined, washed with saturated salt water, dried over anhydrous sodium sulfate, and concentrated to obtain C036-2(1.5g, crude) as a light yellow solid.

### Step 3:

Under the protection of N₂, C036-2(1.3g, crude) was dissolved in NMP(13 mL), CuCN(3.0g,34.42 mmol) was added at room temperature, and the mixture was heated to 110°C and stirred for 14h. TLC showed the raw materials were basically reacted completely. The reaction solution was cooled to room temperature and ethyl acetate and NH₃.H₂O/NH₄Cl aq(1:3) were added, stratified, aqueous layer was extracted with ethyl acetate, the organic phases were combined, washed with saturated salt water, dried over anhydrous sodium sulfate, and concentrated, and the crude product was purified by silica gel column chromatography to obtain C036-3(415mg, yield for two steps: 42%).

### Step 4:

Under the protection of N₂, C036-3(300 mg, 1.49 mmol) was dissolved in carbon tetrachloride (4 mL), BPO(70%)(20 mg,0.06 mmol) was added at room temperature, the temperature was raised to 80°C, NBS(300 mg, 1.49 mmol) was added, and the reaction was continued for 2 hours. TLC showed the raw materials were reacted completely. The reaction solution was cooled to room temperature, and the crude product was purified by silica gel column chromatography to obtain C036-4(376 mg, yield 90%) as a light yellow solid. ¹H NMR (400 MHz, CDCl₃) δ 3.92 (s, 3H), 3.95 (s, 3H), 4.75 (s, 2H), 7.46 (d, *J* = 2.8 Hz, 1H), 8.22 (d, *J* = 2.8 Hz, 1H)

### Step 5:

Under the protection of N₂, C036-4(376 mg,1.34 mmol) was dissolved in DMF (10 mL), NaN₃(233 mg,3.58mmol) was added at room temperature, and the mixture was heated to 50°C and stirred for 1 h, and cooled to room temperature and stirred overnight. TLC showed the raw materials were reacted completely. The reaction solution was quenched with water, extracted with ethyl acetate. The organic phases were combined, washed with saturated salt water, dried over anhydrous sodium sulfate, and concentrated to obtain C036-5(300mg, crude).

### Step 6:

Under the protection of N₂, C036-5(300 mg, crude) was dissolved in MeOH(30 mL), H₂O₂ (30%)(30 mL) and KOH solution (15mL,2M) were added at room temperature, and the mixture was heated to 50°C and stirred for 1h. TLC showed the raw materials were reacted completely. The reaction solution was cooled to room temperature, 1M hydrochloric acid was added to adjust the pH of the system to about 5, extracted with ethyl acetate. The organic phases were combined, washed with water, washed with 10% Na₂SO₃ solution, dried over anhydrous sodium sulfate, and concentrated to obtain C036-6(270 mg, yield for two steps: 77%) as a white solid. ¹H NMR (400 MHz, DMSO_d6): δ3.81 (s, 3H), 3.89 (s, 3H), 5.03 (s, 2H), 7.39 (s, 2H), 7.79 (d, *J* = 2.8 Hz, 1H), 8.13 (d, *J* = 2.8 Hz, 1H). LCMS: m/z=261.1[M+H]⁺

### Step 7:

Under the protection of N₂, C036-6(150 mg,0.58 mmol) was dissolved in mixed solvent of MeOH/THF(10 mL/10 mL), Pd/C (10%)(50 mg) was added at room temperature, and the mixture was heated to 40°C and stirred for 1h. TLC showed the raw materials were reacted completely. The reaction solution was cooled to room temperature, filtered with diatomite, the filter cake was washed with THF and MeOH, the filtrate was concentrated, and the residue was slurried with petroleum ether and ethyl acetate, and filtered to obtain C036-7(90 mg, crude) as an off-white solid. LCMS:m/z=235.1[M+H]⁺

### Step 8:

Under the protection of N₂, C036-7(90 mg, crude) and DIEA(245mg,1.90 mmol) were dispersed into acetonitrile (6 mL), stirred to dissolve clear, 1H-1-methyl-formamidine hydrochloride (113 mg,0.77 mmol) was added, and the mixture was stirred at room temperature for 18 h and a large amount of solids were precipitated. TLC showed the raw material was completely consumed. The reaction solution was filtered, and the filter cake was washed with acetonitrile, and dried to obtain SYY-C036(80 mg, yield for two steps: 50%) as a white solid. ¹H NMR ((DMSO-*d₆*, 400MHz): δ 3.89 (s, 6H), 4.84 (s, 2H), 7.28 (br, 3H), 7.75 (s, 1H), 7.85 (s, 2H), 8.12 (s, 1H), 9.0(br, 1H). LCMS: m/z=277.1[M+H]⁺

### Example 29: Preparation of Compounds SYY-C037 and SYY-C039

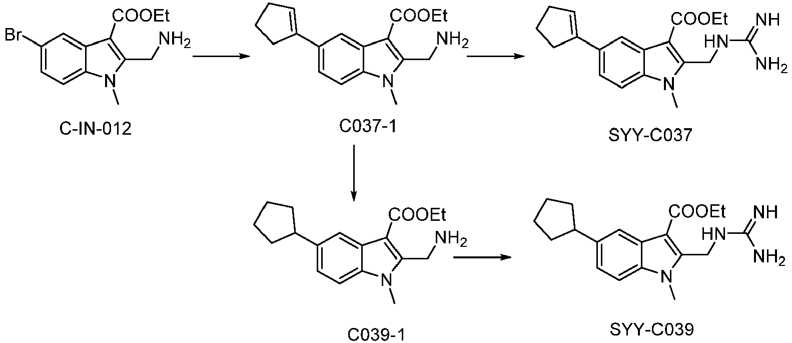

### Step 1:

Under the protection of nitrogen, C-IN-012(650 mg ,2.09 mmol) was dissolved in a mixed solvent of dioxane(13 ml) and water (4 ml), and Pd(dppf)Cl₂ dichloromethane complex (172 mg,0.21 mmol), cyclopenten-1-ylboronic acid (588 mg,5.25 mmol) and Na₂CO₃(668 mg,6.31mmol) were added at room temperature, and the mixture was heated to 100°C and stirred for 3h. TLC showed the raw materials disappeared. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C037-1(430 mg, yield 69%) as a brown solid.

### Step 2:

C037-1(150 mg ,0.50 mmol) and DIEA(323mg ,2.50 mmol) were dispersed into acetonitrile (3 mL), stirred to dissolve clear, 1H-1-methyl-formamidine hydrochloride (147 mg, 1.00 mmol) was added, and the mixture was stirred at room temperature for 20 h. The reaction solution was filtered, and the filter cake was washed with acetonitrile, and dried to obtain SYY-C037(30 mg, yield 18%) as a light brown solid. ¹H NMR (400 MHz, DMSO_d6) δ 1.40 (t, *J* = 7.2 Hz, 3H), 1.94-2.05 (m, 2H),2.53-2.55 (m, 2H), 2.70-2.77(m, 2H), 3.85 (s, 3H), 4.36 (q, *J* = 7.2Hz, 2H), 4.92 (d, *J* = 4.4 Hz, 2H), 6.26 (s, 1H), 7.42(br, 3H), 7.57-7.60 (m, 2H), 8.03 (s, 1H), 8.26-8.33 (m, 1H). LCMS: m/z=341.2[M+H]⁺ (88.84% purity, 254 nm)

### Step 3:

Under hydrogen atmosphere, C037-1(250 mg ,0.84 mmol) was dissolved in methanol (6 mL), 10% Pd/C(200 mg) was added at room temperature, and the mixture was heated to 40°C and stirred for 4 h. LCMS showed the raw materials disappeared. The reaction solution was filtered, the filter cake was washed with methanol, and the filtrate was concentrated, and purified by pre-TLC to obtain C039-1(200 mg, yield 79%) as a light pink solid. ¹H NMR (400 MHz, DMSO_d6) : δ1.40 (t, *J* = 7.2 Hz, 3H), 1.50-1.63(m, 2H), 1.64-1.74(m, 2H), 1.79 -1.82(m, 2H), 2.02-2.09(m, 2H), 3.07-3.14(m, 1H), 3.84 (s, 3H), 4.26-4.38(m, 4H), 5.68 (br, 2H), 7.18 (d, *J* = 8.5 Hz, 1H), 7.50 (d, *J* = 8.5 Hz, 1H), 7.90 (s, 1H)

### Step 4:

C039-1(100 mg ,0.33 mmol) and DIEA(213mg ,1.65 mmol) were dispersed into acetonitrile (3 mL), stirred to dissolve clear, 1H-1-methyl-formamidine hydrochloride (100 mg,0.68 mmol) was added, and the mixture was stirred at room temperature for 40 h, and a large amount of solids were precipitated. The reaction solution was filtered, and the filter cake was washed with acetonitrile, and dried to obtain SYY-C039(50 mg, yield 44%) as an off-white solid. ¹H NMR (400 MHz, DMSO_d6): δ 1.39 (t, *J* = 7.2 Hz, 3H), 1.53-1.62 (m, 2H), 1.64-1.74 (m, 2H), 1.75-1.84 (m, 2H), 2.02-2.10 (m, 2H), 3.09-3.13 (m, 1H), 3.83 (s, 3H), 4.35 (q, *J* = 7.2 Hz, 2H), 4.91 (s, 2H), 7.23 (dd, *J* = 8.8, 1.6 Hz, 1H), 7.28-7.80(m, 4H), 7.90 (s, 1H), 8.21(br, 1H). LCMS: m/z=343.2[M+H]⁺ (99.27 purity, 220 nm).

### Example 30: Preparation of Compound SYY-C040

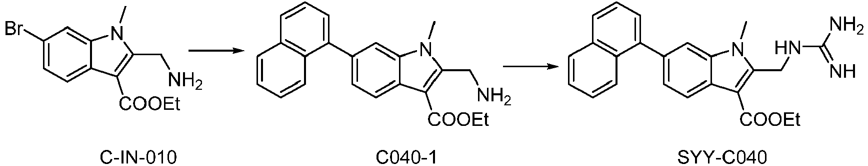

### Step 1:

Under the protection of nitrogen, C-IN-010 (280 mg ,0.90 mmol) was dissolved in a mixed solvent of 1,4-Dioxane(30 ml) and water (10 ml), and Pd(dppf)Cl₂ dichloromethane complex (74 mg,0.091 mmol), 2-naphthalene boronic acid (389 mg,2.26 mmol) and Na₂CO₃(288 mg,2.72 mmol) were sequentially added at room temperature, and the mixture was heated to 100°C and stirred for 1.5h. TLC showed the raw materials disappeared. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain dark brown solid, and then purified by Pre-TLC to obtain C040-1(220 mg, yield 68%) as a light yellow solid.

### Step 2:

Under the protection of nitrogen, C040-1(150 mg ,0.42 mmol) was dissolved in acetonitrile (3 ml), DIEA(272mg ,2.10 mmol) and 1H-1-methyl-formamidine hydrochloride (123 mg,0.84 mmol) were added, and the mixture was stirred at room temperature for 40h, and a large amount of solids were precipitated. The reaction solution was filtered, and the filter cake was washed with acetonitrile, and dried to obtain SYY-C040(120 mg, yield 72%) as an off-white solid. ¹H NMR (400 MHz, DMSO_d6): δ 1.42 (t, *J* = 7.2 Hz, 3H), 3.91 (s, 3H), 4.40 (q, *J* = 7.2Hz, 2H), 5.00 (s, 2H), 7.38 (dd, *J* = 8.0, 0.8 Hz,1H), 7.43-7.74 (m, 6H), 7.77 (s, 1H), 7.84 (d, *J* = 8.4 Hz, 1H), 8.00 (dd, *J* = 17.6, 8.4 Hz, 2H), 8.14-8.76 (m, 3H) LCMS: m/z=401.2[M+H]⁺

### Example 31: Preparation of Compound SYY-C041

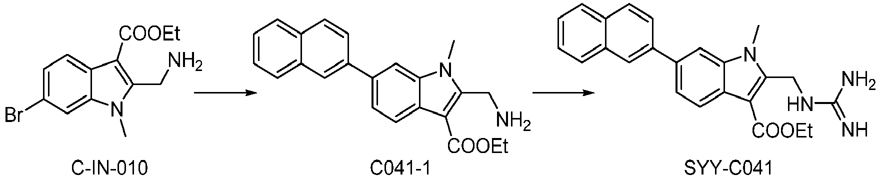

### Step 1:

Under the protection of nitrogen, C-IN-010 (280 mg ,0.90 mmol) was dissolved in a mixed solvent of 1,4-Dioxane(30 ml) and water (10 ml), and Pd(dppf)Cl₂ dichloromethane complex (74 mg,0.091 mmol), 2-naphthalene boronic acid (389 mg,2.26 mmol) and Na₂CO₃(288 mg,2.72 mmol) were sequentially added at room temperature, and the mixture was heated to 100°C and stirred for 1.5h. TLC showed the raw materials disappeared. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain dark brown solid, and then purified by Pre-TLC to obtain C041-1(220 mg, yield 68%) as a light yellow solid.

### Step 2:

Under the protection of nitrogen, C041-1(150 mg ,0.42 mmol) was dissolved in acetonitrile (3 ml), DIEA(272mg ,2.10 mmol) and 1H-1-methyl-formamidine hydrochloride (123 mg,0.84 mmol) were added, the mixture was stirred at room temperature for 40h, and a large amount of solids were precipitated. The reaction solution was filtered, and the filter cake was washed with acetonitrile, and dried to obtain SYY-C041(150 mg, yield 89%) as an off-white solid. ¹H NMR (400 MHz, DMSO_d6) δ 1.42 (t, *J* = 7.2 Hz, 3H), 3.98 (s, 3H), 4.40 (q, *J* = 7.2 Hz, 2H), 4.98 (s, 2H), 7.18-7.72 (m, 5H), 7.77 (dd, *J* = 8.4, 1.2 Hz, 1H), 7.96-8.05(m, 4H), 8.11-8.14 (m, 2H), 8.34- 8.47 (br, 2H). LCMS: m/z=401.2[M+H]⁺.

### Example 32: Preparation of Compound SYY-C042

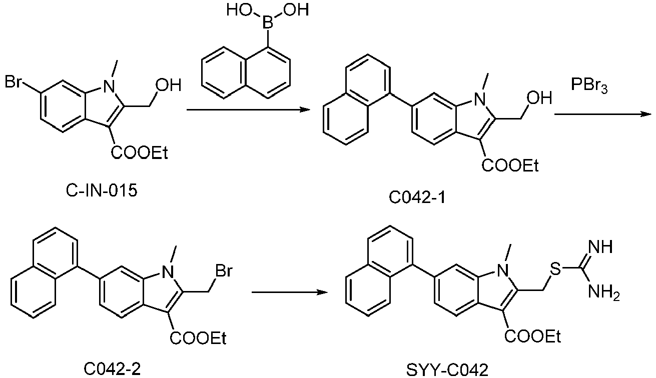

### Step 1:

C-IN-015(300 mg,0.96 mmol) was dissolved in a mixed solvent of 1.4-dioxane(30 ml) and water (10 ml), 1-naphthaleneboronic acid (331 mg,1.92 mmol), PdCl₂(dppf) dichloromethane complex (79 mg,0.10 mmol) and sodium carbonate (398 mg,3.76 mmol) were added at room temperature, replaced with nitrogen for three times, and the mixture was heated to 100°C to react for 2 hours. TLC detection showed that the raw materials were reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C042-1(183 mg, yield 53%) as a white solid.

### Step 2:

C042-1(116 mg,0.32 mmol) was dissolved in dichloromethane (3 mL), cooled to 0°C under an ice water bath, PBr₃(88 mg,0.33mmol, diluted with 0.5ml DCM) was added dropwise under the protection of nitrogen, and the mixture was reacted at room temperature for 1h after addition. TLC detection showed raw materials were reacted completely. The reaction solution was diluted by adding 3ml DCM, saturated sodium bicarbonate solution was added dropwise to be neutral, liquid separation was carried out, the organic phases were combined, washed with saturated salt water, dried over anhydrous sodium sulfate, filtered, and the filtrate was concentrated to obtain C042-2(129 mg, crude) as a pink solid.

### Step 3:

Under the protection of nitrogen, C042-2(129 mg, crude) was dissolved in ethanol (5 ml), thiourea (21 mg,0.28 mmol) was added at room temperature, and the mixture was heated to 80°C to react for 1.0 h. TLC detection showed that the raw materials were reacted completely. The reaction solution was cooled to room temperature, a large amount of solids were precipitated, stirred for 20min, filtered, and the filter cake was washed with ethanol to obtain SYY-C042(60 mg, yield for two steps: 45%) as a white solid. ¹H NMR: (DMSO-*d₆*, 400MHz): δ1.42 (t, J = 7.2 Hz, 3H), 3.92 (s, 3H), 4.40 (q, J = 7.2 Hz, 2H), 5.18 (s, 2H), 7.39 (dd, J = 8.4, 1.2 Hz, 1H), 7.45-7.66 (m, 4H), 7.76 (s, 1H), 7.86 (d, J = 8.4 Hz, 1H),7.98-8.04(m, 2H), 8.14 (d, J = 8.4 Hz, 1H), 9.25 (br, 3H). LCMS: m/z=418.1[M+H]⁺ (99.17% purity, 220 nm).

### Example 33: Preparation of Compound SYY-C043

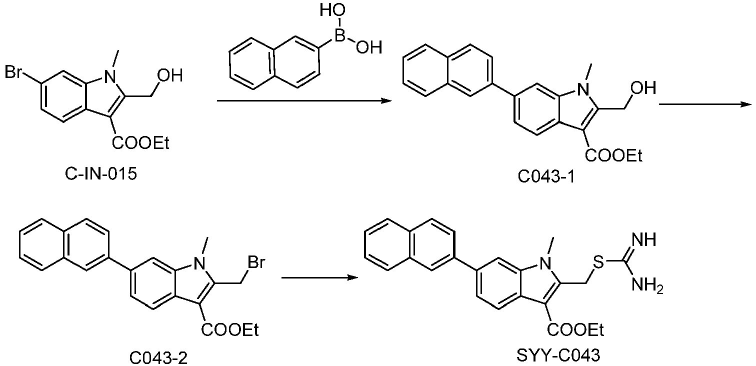

### Step 1:

C-IN-015(400 mg,1.28 mmol) was dissolved in a mixed solvent of 1.4-dioxane(30 ml) and water (10 ml), 2-naphthalene boric acid (441 mg,2.56 mmol), PdCl₂(dppf) dichloromethane complex (105 mg,0.13 mmol) and sodium carbonate (531 mg,5.01 mmol) were added at room temperature, replaced with nitrogen for three times, and the mixture was heated to 100°C to react for 2 hours. TLC showed the raw materials were reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=3/1) to obtain C043-1(361 mg, yield 79%) as a light yellow solid.

### Step 2:

C043-1(150 mg,0.42 mmol) was dissolved in dichloromethane (4 mL), cooled to 0°C under an ice water bath, PBr₃(113 mg,0.42 mmol, diluted with 0.5ml DCM) was added dropwise under the protection of nitrogen, and the mixture was reacted at room temperature for 1h after addition. TLC detection showed the raw materials were reacted completely. The reaction solution was diluted by adding 3ml DCM, saturated sodium bicarbonate solution was added dropwise to adjust pH to be neutral, liquid separation was carried out, the organic phases were washed with saturated salt water, dried over anhydrous sodium sulfate, filtered, and the filtrate was concentrated to obtain C043-2(176 mg, crude) as a yellow solid.

### Step 3:

Under the protection of nitrogen, C043-2(176 mg, crude) was dissolved in ethanol (5 ml), thiourea (32 mg,0.42mmol) was added at room temperature, and the mixture was heated to 80°C to react for 1.0 h. TLC detection showed that the raw materials were reacted completely. The reaction solution was cooled to room temperature, a large amount of solids were precipitated, stirred for 20min, filtered, and the filter cake was washed with ethanol, and dried to obtain SYY-C043(120 mg, yield for two steps: 69%) as a white solid. ¹H NMR: (DMSO-*d₆*, 400MHz): δ1.42 (t, J = 7.2 Hz, 3H), 3.99 (s, 3H), 4.38 (q, J = 7.2 Hz, 2H), 5.17 (s, 2H), 7.47-7.62 (m, 2H), 7.77 (dd, J = 8.4, 1.2 Hz, 1H), 7.93-8.15 (m, 6H), 8.34 (s, 1H), 9.26 (br, 3H). LCMS: m/z=418.2[M+H]⁺ (99.27% purity, 254 nm).

### Example 34: Preparation of Compound SYY-C045

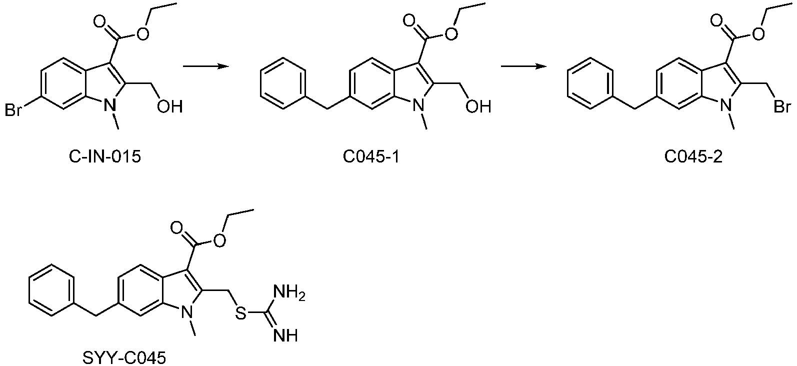

### Step 1:

C- IN-015(200 mg,0.64 mmol) and benzyl borate (210 mg,0.96 mmol) were dissolved in dioxane (10 mL), saturated sodium carbonate aqueous solution (3 mL) and PdCl2(dppf) dichloromethane complex (45 mg,0.06 mmol) were sequentially added, replaced with nitrogen for three times, and the mixture was heated to 80 °C to react for 2 hours. TLC detection showed the raw materials were reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C045-1(130 mg, yield: 63%) as a light yellow solid. LCMS:m/z=306.1[M-17]⁺

### Step 2:

C045-1(130mg,0.40 mmol) was dissolved in dichloromethane (10mL), cooled to 0°C under an ice water bath, and a solution of PBr₃(108 mg,0.40 mmol) in dichloromethane (2mL) was added dropwise under the protection of nitrogen, and the mixture was reacted at 0 °C for half an hour after addition. TLC detection showed that the reaction was completed. The reaction solution was quenched with ice water, neutralized with sodium bicarbonate, liquid separation was carried out, and the organic phases were combined, washed with saturated salt water, dried over anhydrous sodium sulfate, and concentrated to obtain C045-2(140 mg, crude) as a light yellow solid.

### Step 3:

C045-2(140 mg,0.36 mmol) and thiourea (25 mg,0.33mmol) were dispersed into ethanol (3 mL), and heated to 80 °C to react for 2 hours. TLC detection showed the reaction was completed. The reaction solution was cooled to room temperature, no solid was precipitated, concentrated to a small amount, slurried with acetonitrile, and solids were precipitated, filtered, and the filter cake was washed and dried to obtain 106 mg off-white solid. After the crude product was heated and dissolved with ethanol (1 mL), the solid was precipitated by stirring overnight, filtered, and the filter cake was washed with cold ethanol, and dried to obtain SYY-C045(60 mg, yield for two steps: 39%) as a white solid. ¹H NMR: (DMSO-*d₆*, 400MHz): 1.37 (t, J = 7.2 Hz, 3H), 3.84 (s, 3H), 4.07 (s, 2H), 4.33 (q, J = 7.28 Hz, 2H), 5.11 (s, 2H), 7.12- 7.30 (m, 6H), 7.53 (s, 1H), 7.91 (d, J = 8.4 Hz, 1H), 9.21 (br, 3H). LCMS: m/z=382.1[M +H]⁺ (94.28% purity, 220 nm)

### Example 35: Preparation of Compound SYY-C046

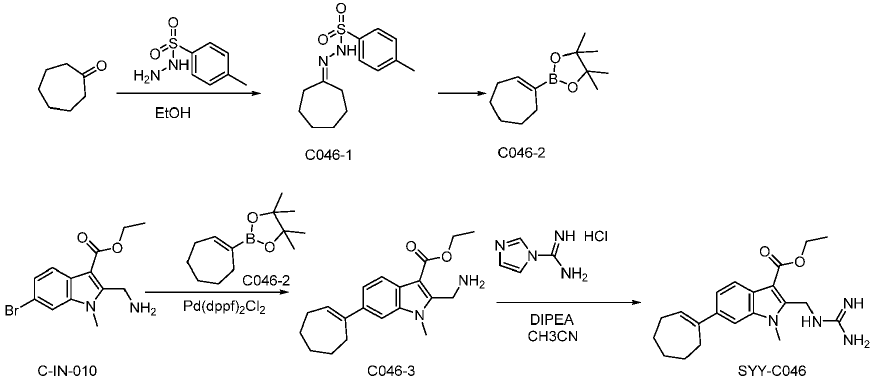

### Step 1:

Cycloheptanone (2.00g,17.83 mmol) and p-methylbenzenesulfonylhydrazide (3.32g,17.83 mmol) were dispersed in ethanol (10 mL) and heated until the reflux to react for 1h. TLC detection showed the reaction was completed. The reaction solution was concentrated to dry to obtain C046-1(5.0g, crude) as a white solid.

### Step 2:

C046-1(2.8g, crude) was dissolved in n-hexane (30 mL) and TMEDA(30 mL), cooled to -70 °C under the protection of nitrogen, n-butyllithium solution (15 mL, 37.5 mmol, 2.5M) was added dropwise, and the mixture was stirred and reacted for 1 hour at -70 °C after addition, then heated to room temperature and stirred to react for 1 hour. The reaction solution was then cooled to -70 °C, and isopropyl borate (7.06g,37.92 mmol) was added dropwise in about 15 min, it was continued to be stirred and reacted for 1h, and the mixture was heated to room temperature and stirred and reacted overnight. The reaction solution was quenched with saturated ammonium chloride aqueous solution, MTBE was added for extraction, filtered with diatomite, the organic phase was washed with saturated salt water, dried over anhydrous sodium sulfate, and concentrated, and the crude product was purified by silica gel column chromatography to obtain colorless oil (1.5g, yield for two steps: 38%). ¹H NMR (400 MHz, CDCl₃) :δ 1.27(s, 12H),1.45-1.52 (m, 4H), 1.73-1.79(m, 2H), 2.22-2.29(m, 4H), 6.79(t, J = 6.0Hz, 1H).

### Step 3:

Under the protection of nitrogen, C-IN-010(500 mg ,1.61 mmol) was dissolved in a mixed solvent of Dioxane(20 ml) and water (2 ml), and Pd(dppf)Cl₂ (130 mg,0.16 mmol), cyclopenten-1-ylboronic acid (538 mg,2.42 mmol) and Na₂CO₃(512 mg,4.83 mmol) were added at room temperature, and the mixture was heated to 100°C and stirred for 3h. LCMS showed the raw materials disappeared. The reaction solution was cooled to room temperature, added with water, extracted with ethyl acetate, the organic phases was washed with saturated salt water, dried over anhydrous sodium sulfate, and concentrated, and the crude product was purified by silica gel column chromatography to obtain C046-3(250 mg, yield 48%). LCMS:m/z=310.2[M-NH₂]⁺

### Step 4:

C046-3(100 mg,0.31 mmol) and DIEA(160 mg,1.24 mmol) were dissolved in acetonitrile (5 mL), 1H-1-methyl-formamidine hydrochloride (90 mg,0.62 mmol) was added at room temperature, and the mixture was stirred for 2 days. The reaction solution was filtered, and the filter cake was washed with acetonitrile, and dried to obtain solid (80 mg, yield 70%). ¹H NMR: (DMSO-*d₆*, 400MHz): δ1.38 (t, J = 7.2 Hz, 3H), 1.50-1.58 (m, 2H), 1.59-1.69 (m, 2H), 1.79-1.86 (m, 2H), 2.25-2.35 (m, 2H), 2.64-2.68 (m, 2H), 3.85 (s, 3H), 4.35 (q, J = 7.2 Hz, 2H), 4.92 (s, 2H), 6.19 (t, J = 6.8 Hz, 1H), 7.20-7.64 (m, 5H), 7.92 (d, J = 8.4 Hz, 1H), 8.27 (br, 1H). LC-MS: m/z = 369.2 [M+H]⁺

### Example 36: Preparation of Compound SYY-C047

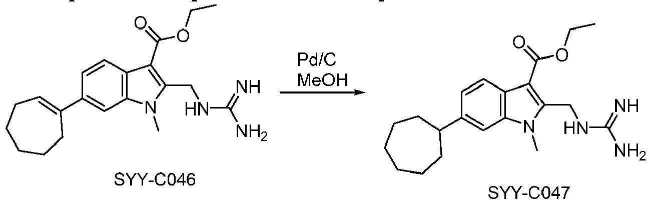

Under hydrogen atmosphere, SYY-C046(40 mg ,0.11 mmol) was dissolved in methanol (10 mL), 10% Pd/C(10 mg) was added at room temperature, and the temperature was raised to 50°C and stirred for 2 h. LCMS showed the raw materials disappeared. The reaction solution was filtered, and the filter cake was washed with methanol, and the filtrate was concentrated to obtain white solid (36 mg, yield 88%). ¹H NMR: (DMSO-*d₆*, 400MHz): δ 1.38 (t, J = 7.2 Hz, 3H), 1.51-1.92 (m, 12H), 2.73- 2.87 (m, 1H), 3.82 (s, 3H), 4.35 (q, J = 7.2 Hz, 2H), 4.91 (d, J = 1.6 Hz, 2H), 7.07-7.60 (m, 5H), 7.90 (d, J = 8.0 Hz, 1H), 8.18-8.27 (m, 1H). LC-MS: m/z = 371.2 [M+H]⁺

### Example 37: Preparation of Compound SYY-C048

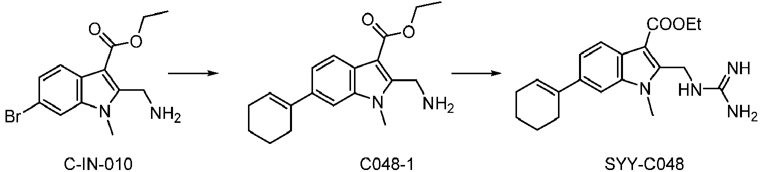

### Step 1:

Under the protection of nitrogen, C-IN-010(400 mg ,1.29 mmol) was dissolved in a mixed solvent of 1,4-Dioxane(20 ml) and water (2 ml), and Pd(dppf)Cl₂ (104 mg,0.13 mmol), cyclopenten-1-ylboronic acid (324 mg,2.58 mmol) and Na₂CO₃(410 mg,3.87 mmol) were added at room temperature, and the mixture was heated to 100°C and stirred for 3h. LCMS showed the raw materials disappeared. The reaction solution was cooled to room temperature, added with water, extracted with ethyl acetate, the organic phases was washed with saturated salt water, dried over anhydrous sodium sulfate, and concentrated, and the crude product was purified by silica gel column chromatography to obtain C048-1(350 mg, yield 87%). LCMS:m/z=296.2[M-NH₂]⁺

### Step 2:

C048-1(100 mg ,0.32 mmol) and DIEA(165 mg,1.28 mmol) were dissolved in acetonitrile (5 mL), 1H-1-methyl-formamidine hydrochloride (52 mg,0.35 mmol) was added at room temperature, and the mixture was stirred for 4 days. LCMS detection showed that the reaction was completed. The reaction solution was filtered, and the filter cake was washed with acetonitrile, and dried to obtain solid (30 mg, yield 26%). 1H NMR: (DMSO-d6, 400MHz): δ1.38 (t, J = 7.2 Hz, 3H), 1.59-1.70 (m, 2H), 1.72- 1.83 (m, 2H), 2.17-2.27 (m, 2H), 2.45-2.49 (m, 2H), 3.85 (s, 3H), 4.35 (d, J = 7.2 Hz, 2H), 4.92 (s, 2H), 6.21-6.27 (m, 1H), 7.01-7.50 (m, 4H), 7.59 (s, 1H), 7.93 (d, J = 8.4 Hz, 1H), 8.23 (br, 1H). LC-MS: m/z = 355.2 [M-NH₂]⁺

### Example 38: Preparation of Compound SYY-C049

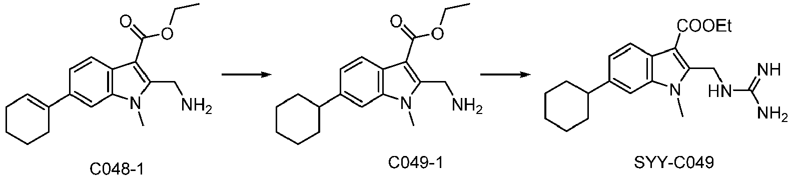

### Step 1:

Under hydrogen atmosphere, C048-1(100 mg ,0.32 mmol) was dissolved in methanol (5 mL), 10% Pd/C(20 mg) was added at room temperature, and the mixture was heated to 50°C and stirred for 2 h. LCMS showed the raw materials disappeared. The reaction solution was filtered, and the filter cake was washed with methanol, and the filtrate was concentrated to obtain C049-1 (crude), LC-MS:m/z = 298.2 [M-NH2]⁺.

### Step 2:

C049-1(100 mg,0.32 mmol) and DIEA(165 mg,1.28 mmol) were dissolved in acetonitrile (5 mL), 1H-1-methyl-formamidine hydrochloride (52 mg,0.35 mmol) was added at room temperature, and the mixture was stirred for 2 days. LCMS detection showed that the reaction was completed. The reaction solution was filtered, and the filter cake was washed with acetonitrile, and dried to obtain solid SYY-C049(43 mg, yield 38%). ¹H NMR: (DMSO-*d₆*, 400MHz): δ1.31-1.90 (m, 13H), 2.56-2.71 (m, 1H), 3.83 (s, 3H), 4.35 (q, J = 7.2 Hz, 2H), 4.91 (d, J = 4.8 Hz, 2H), 7.11-7.57 (m, 5H), 7.91 (d, J = 8.0 Hz, 1H), 8.24-8.30 (m, 1H). LC-MS: m/z =357.2 [M-NH₂]⁺

### Example 39: Preparation of Compounds SYY-C050 and SYY-C051

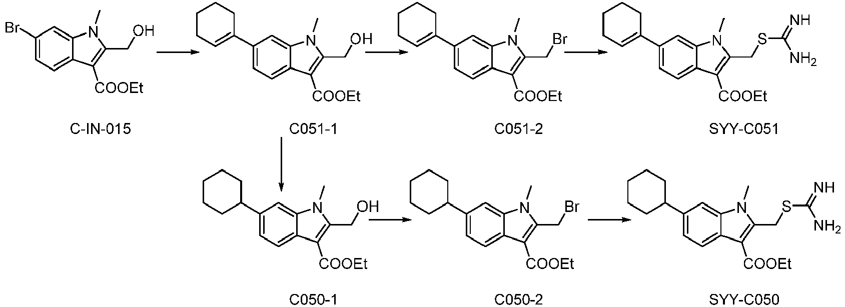

### Step 1:

C-IN-015(400 mg,1.28 mmol) was dissolved in a mixed solvent of 1,4-Dioxane(30 ml) and water (10 ml), and cyclohexene-1-ylboronic acid (323 mg,2.56 mmol), PdCl₂(dppf) dichloromethane complex (105 mg,0.13 mmol) and sodium carbonate (531 mg,5.01 mmol) were added at room temperature, replaced with nitrogen for three times, and the mixture was heated to 100°C to react for 2 hours. TLC showed the raw materials were reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=3/1) to obtain C051-1(367 mg, yield 91%) as a light yellow solid.

### Step 2:

C051-1(180 mg,0.57 mmol) was dissolved in DCM(5 ml), under the protection of nitrogen, cooled to 0°C under an ice water bath, and PBr₃(155 mg,0.57 mmol, diluted with 0.5ml DCM) was added dropwise, and the mixture was reacted at room temperature for 1h after addition. TLC monitored the reaction was completed. The reaction solution was diluted with DCM, saturated NaHCO₃ solution was added dropwise to adjust to be neutral, liquid separation was carried out, and the organic phases were washed with saturated salt water, dried over anhydrous sodium sulfate, and concentrated to obtain C051-2(188 mg, crude) as a yellow solid.

### Step 3:

Under the protection of nitrogen, C051-2(188 mg, 0.50 mmol) was dissolved in ethanol (5 ml), thiourea (26 mg,0.34 mmol) was added at room temperature, and the mixture was heated to 80°C to react for 1.0 h. TLC detection showed that the raw materials were reacted completely. The reaction solution was cooled to room temperature, no solid was precipitated, the reaction solution was concentrated, 1.0ml of ethanol was added to dissolve, 3 ml of MTBE was added dropwise, stirred for 10 min at room temperature, a large amount of solids were precipitated, stirred for 20min, filtered, and the filter cake was washed with (ethanol/MTBE = 1/3) to obtain SYY-C051(58 mg, yield for two steps: 27%) as a white solid. ¹H NMR: (DMSO-*d₆*, 400MHz): 1.38 (t, J = 7.2 Hz, 3H), 1.61-1.68 (m, 2H), 1.73-1.80 (m, 2H), 2.18-2.24 (m, 2H), 2.43-2.49 (m, 2H), 3.87 (s, 3H), 4.35 (q, J = 7.2 Hz, 2H), 5.11 (s, 2H), 6.24 (s, 1H), 7.37 (dd, J = 8.4, 1.2 Hz, 1H), 7.58 (s, 1H), 7.92 (d, J = 8.4 Hz, 1H), 9.22 (br, 4H). LCMS: m/z=372.2[M +H]⁺ (93.10% purity, 254 nm)

### Step 4:

C051-1(140 mg,0.45 mmol) was dissolved in methanol (5 ml), Pd/C(56 mg) was added, and the mixture was reacted for 1.5 h at room temperature. TLC detection showed that the raw materials were reacted completely. The reaction solution was filtered with diatomite, and the filtrate was concentrated to obtain C050-1(140 mg, crude) as a white solid.

### Step 5:

C050-1(114 mg,0.36 mmol) was dissolved in dichloromethane (5 ml), under the protection of nitrogen, cooled to 0°C under an ice water bath, and phosphine tribromide (98 mg,0.36 mmol, diluted with 0.5ml dichloromethane) was added dropwise, and the mixture was reacted for 1 h at room temperature after addition. TLC detection showed that the raw materials were reacted completely. The reaction solution was diluted by adding dichloromethane, the saturated sodium bicarbonate aqueous solution was added dropwise to adjust the pH to be neutral, liquid separation was carried out, and the organic phases were washed with saturated salt water, dried over anhydrous sodium sulfate, and concentrated to obtain C050-2(128 mg, crude) as a yellow solid.

### Step 6:

Under the protection of nitrogen, C050-2(128 mg, 0.34mmol) was dissolved in ethanol (5 ml), thiourea (23 mg,0.30 mmol) was added at room temperature, and the mixture was heated to 80°C to react for 1.0 h. TLC detection showed that the raw materials were reacted completely. The reaction solution was cooled to room temperature, no solid was precipitated, the reaction solution was concentrated, 0.5 ml of ethanol was added to dissolve, 1.5 ml of MTBE was added dropwise, stirred for 10 min at room temperature, a large amount of solids were precipitated, continued to stir for 20min, filtered, and the filter cake was washed with (ethanol/MTBE = 1/3) to obtain a white solid(119 mg, yield for three steps: 71%). ¹H NMR: (DMSO-*d₆*, 400MHz): 1.24-1.53 (m, 8H), 1.72-1.84 (m 5H), 2.59-2.66 (m, 1H), 3.85 (s, 3H), 4.34 (q, J = 7.2 Hz, 2H), 5.12 (s, 2H), 7.15 (dd, J = 8.4, 1.2 Hz, 1H), 7.44 (s, 1H), 7.90 (d, J = 8.4 Hz, 1H), 9.17(br, 2H), 9.31(br, 2H). LCMS: m/z=374.2[M +H]⁺ (99.02% purity, 220 nm)

### Example 40: Preparation of Compounds SYY-C052 and SYY-C053

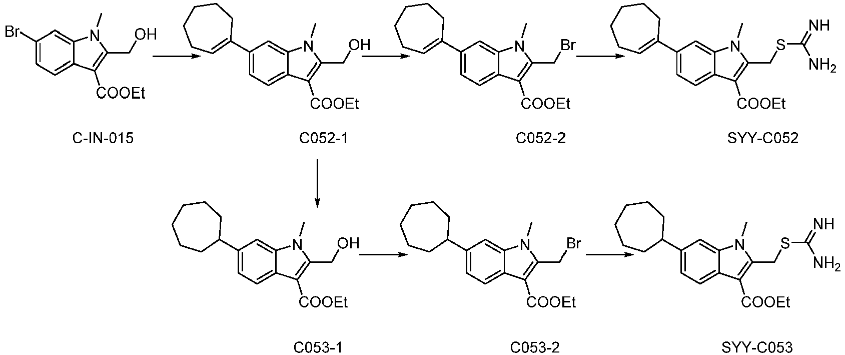

### Step 1:

C-IN-015(650 mg,2.08 mmol) was dissolved in a mixed solvent of 1,4-Dioxane(30 ml) and water (10 ml), and boric acid ester (700 mg,3.15 mmol), Pd(dppf) Cl₂ dichloromethane complex (170 mg,0.21 mmol) and sodium carbonate (862 mg,8.13 mmol) were added at room temperature, replaced with nitrogen for three times, and the mixture was heated to 100°C to react for 2 hours. TLC showed the raw materials were reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=3/1) to obtain C052-1(614 mg, yield 90%) as a yellow solid.

### Step 2:

C052-1(250 mg,0.76 mmol) was dissolved in DCM(5 ml), under the protection of nitrogen, cooled to 0°C under an ice water bath, and PBr₃(206 mg,0.76 mmol, diluted with 0.5ml DCM) was added dropwise, and the mixture was reacted at room temperature for 1h after addition. TLC showed the raw materials were reacted completely, the reaction solution was diluted by adding DCM, saturated NaHCO₃ solution was added dropwise to adjust pH to be neutral, liquid separation was carried out, and the organic phases were washed with saturated salt water, dried over anhydrous sodium sulfate, and concentrated to obtain C052-2(400 mg, crude) as a yellow oil.

### Step 3:

Under the protection of nitrogen, C052-2(400 mg, 1.02 mmol) was dissolved in ethanol (5 ml), thiourea (42 mg,0.55 mmol) was added at room temperature, and the mixture was heated to 80°C to react for 1.0 h. TLC detection showed that the raw materials were reacted completely. The reaction solution was cooled to room temperature, no solid was precipitated, the reaction solution was concentrated, 2.0 ml of ethanol was added to dissolve, 6.0 ml of methyl tert-butyl ether was added dropwise, stirred at room temperature for 10 min, a large amount of solids were precipitated, continued to stir for 20min, filtered, and the filter cake was washed with (ethanol/MTBE = 1/3), and dried to obtain SYY-C052(67 mg, yield for two steps: 23%) as a white solid. ¹H NMR: (DMSO-*d₆*, 400MHz): 1.38 (t, J = 7.2Hz, 3H), 1.49-1.57 (m, 2H), 1.59-1.68 (m, 2H), 1.80-1.86 (m, 2H), 2.26-2.33 (m, 2H), 2.64-2.67 (m, 2H), 3.87 (s, 3H), 4.34 (q, J = 7.2 Hz, 2H), 5.13 (s, 2H), 6.18 (t, J = 6.8 Hz, 1H), 7.26 (dd, J = 8.4, 1.2 Hz, 1H), 7.49 (s, 1H), 7.91 (d, J = 8.4 Hz, 1H), 9.33 (br, 3H). LC-MS: m/z=386.2 [M+H]⁺ (95.71% purity, 254 nm)

### Step 4:

C052-1(180 mg,0.55 mmol) was dissolved in methanol (5 ml), 10% Pd/C(108 mg) was added, replaced with hydrogen for three times, and the mixture was heated to 50°C to react for 1.0 h. LCMS detection showed that the raw materials were reacted completely. The reaction solution was cooled to room temperature, filtered with diatomite, and the filtrate was concentrated to obtain C053-1(172 mg, crude) as a white solid.

### Step 5:

C053-1(170 mg,0.52 mmol) was dissolved in DCM(5 ml), under the protection of nitrogen, cooled to 0°C under an ice water bath, and PBr₃(140 mg,0.52 mmol, diluted with 0.5ml DCM) was added dropwise. The mixture was reacted for 1 h at room temperature after addition. TLC detection showed that the raw materials were reacted completely. The reaction solution was diluted by adding DCM, saturated NaHCO₃ solution was added dropwise to adjust pH to be neutral, liquid separation was carried out, and the organic phase was washed with saturated salt water, dried over anhydrous sodium sulfate, and concentrated to obtain C053-2(182 mg, crude) as a white solid.

### Step 6:

Under the protection of nitrogen, C053-2(182 mg, 0.46 mmol) was dissolved in ethanol (5 ml), thiourea (28 mg,0.37 mmol) was added at room temperature, and the mixture was heated to 80°C to react for 1.0 h. TLC detection showed that the raw materials were reacted completely. The reaction liquid was cooled to room temperature, no solid was precipitated, the reaction solution was concentrated, the residue was dissolved with 1.0 ml of ethanol, 3.0 ml of MTBE was added dropwise, stirred for 10 min at room temperature, a large amount of solids were precipitated, continued to stir for 20min, filtered, and the filter cake was washed with (ethanol/MTBE = 1/2) to obtain SYY-C053(110 mg, yield for three steps: 51%) as a white solid. ¹H NMR: (DMSO-*d₆*, 400MHz): 1.37 (t, J = 7.2 Hz, 3H), 1.46-1.91 (m, 12H), 2.75-2.83 (m, 1H), 3.84 (s, 3H), 4.34 (q, J = 7.2 Hz, 2H), 5.11 (s, 2H), 7.14 (dd, J = 8.4, 1.2Hz, 1H), 7.42 (s, 1H), 7.89 (d, J = 8.4 Hz, 1H), 9.22 (br, 3H). LC-MS: m/z=388.2 [M+H]⁺ (95.32% purity, 220 nm)

### Example 41: Preparation of Compound SYY-C054

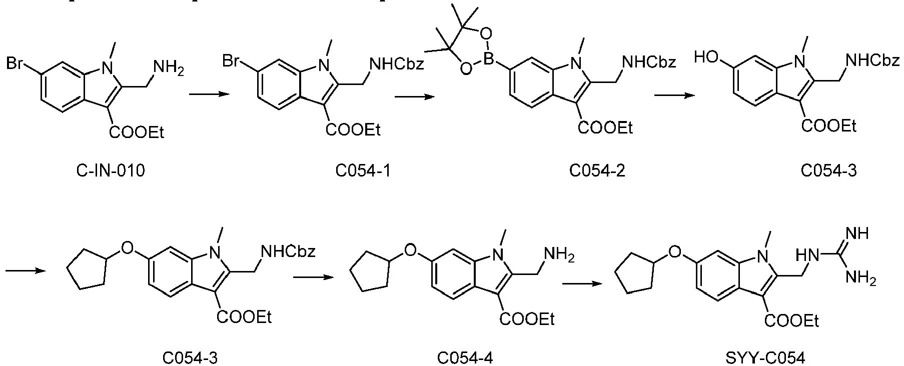

### Step 1:

Under the protection of nitrogen, C-IN-010(200 mg,0.64 mmol) and Na₂CO₃(138 mg,1.30mmol) were dissolved in a mixed solvent of DCM and H₂O(10mL/3mL), cooled to about 0°C under an ice water bath, and Cbz-Cl(132 mg,0.77 mmol) was added and stirred for 0.5h. TLC showed small amount of the raw materials was left, and additional Cbz-Cl(132 mg,0.77 mmol) was added and stirred. TLC showed the raw materials were reacted completely. The reaction solution was stratified, separated, aqueous phase was extracted with dichloromethane, the organic phases were combined, washed with saturated salt water, dried over anhydrous sodium sulfate, and concentrated, and the crude product was purified by silica gel column chromatography to obtain C054-1(270 mg, yield 94%) as a white solid. LC-MS: Not available

### Step 2:

Under the protection of nitrogen, C054-1(270 mg ,0.91 mmol) was dissolved in 1,4-Dioxane (5 mL), PdCl₂(dppf) dichloromethane complex (50 mg,0.06mmol), bis(pinacolato)diboron (235 mg,0.93 mmol) and potassium acetate (180 mg,1.83mmol) were added at room temperature, and the mixture was heated to 100°C to react for 3h. TLC showed the raw materials were basically reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C054-2(225 mg, yield 72%) as a white solid.

### Step 3:

C054-2(480 mg,1.40 mmol) was dissolved in AcOH(9 mL), 30% hydrogen peroxide (0.45 mL,4.50 mmol) was added, and a large amount of solids were precipitated after stirring at room temperature for 0.5h. TLC showed the raw materials disappeared. Water (90mL) was added to the reaction solution, the mixture was stirred for 15min, and filtered, and the filter cake was washed with a large amount of water, and dried to obtain C054-3(320 mg, yield 98%) as an off-white solid. ¹H NMR (400 MHz, DMSO_d6) : δ 1.33 (t, J = 7.2 Hz, 3H), 2.66 (s, 3H), 3.60 (s, 3H), 4.25 (q, J = 7.2 Hz, 2H), 6.68 (dd, J = 8.4, 2.0 Hz, 1H), 6.79 (d, J = 2.0 Hz, 1H), 7.73 (d, J = 8.4 Hz, 1H), 9.17 (s, 1H). LCMS: m/z=234.1 [M+H]⁺

### Step 4:

C054-3(100 mg,0.40 mmol) was dissolved in DMF(3mL), K₂CO₃(166 mg,1.20 mmol) and bromocyclopentane (0.2 mL,1.87 mmol) were added at room temperature, heated to 50°C and stirred for 10h. TLC showed the raw materials were basically reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C054-4(110mg, yield 87%).

### Step 5:

C054-4(100 mg,0.22 mmol) was dissolved in methanol (10 ml), 10% Pd/C(50 mg) was added, replaced with hydrogen for three times, and the mixture was reacted for 1.5 h at room temperature. TLC showed the raw materials were basically reacted completely. The reaction solution was filtered with diatomite, and the filtrate was concentrated to obtain C054-5(65 mg, crude) as a colorless oil.

### Step 6:

The C054-5(65 mg, crude) was dissolved in acetonitrile (3 ml), 1H-pyrazol-1-formamidine hydrochloride (45 mg,0.31 mmol) and DIEA(106 mg,0.82 mmol) were added at room temperature, the reaction solution was stirred for 2 days at room temperature after addition. TLC detection showed that the raw materials were reacted completely. The reaction solution was filtered, and the filter cake was washed and dried to obtain SYY-C054(45 mg, yield for two steps: 56%) as a white solid. ¹H NMR: (DMSO-*d₆*, 400MHz): δ 1.37 (t, J = 7.2 Hz, 3H), 1.53-1.66(m, 2H), 1.67-1.80 (m, 4H), 1.87-2.04 (m, 2H), 3.80 (s, 3H), 4.34 (q, J = 7.2 Hz, 2H), 4.87-4.96 (m, 3H), 6.88 (dd, J = 2.0, 8.4 Hz, 1H), 7.12 (d, J = 1.6 Hz, 1H), 7.38 (br, 3H), 7.86 (d, J = 8.8 Hz, 1H), 8.26 (br, 1H). LCMS: m/z =359.2 [M+H]⁺ (98.62% purity, 220 nm)

### Example 42: Preparation of Compound SYY-C055

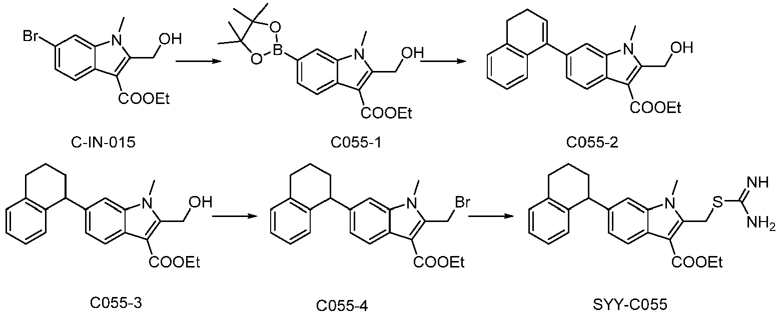

### Step 1:

C- IN-015(1.0g,3.20 mmol) was dissolved in 1,4-Dioxane(25 ml), bis(pinacolato)diboron (1.28g,5.04 mmol), Pd Cl₂(dppf) dichloromethane complex (140 mg,0.17 mmol) and potassium acetate (660 mg,6.72mmol) were added at room temperature, replaced with nitrogen for three times, and the mixture was heated to 100°C to react for 3.0 h. TLC showed the reaction was completed. The reaction solution was cooled to room temperature, added with water, extracted with ethyl acetate, the organic phase was washed with saturated salt water, dried over anhydrous sodium sulfate, and concentrated, and the crude product was purified by silica gel column chromatography(PE/EA=2/1) to obtain C055-1(1.07 g, yield 93%) as a white solid. LCMS:m/z=342.2[M-17]⁺

### Step 2:

C055-1(300 mg,0.84 mmol) was dissolved in DME(15 ml), 3, 4-dihydronaphthalen-1-yl trifluoromethanesulfonate (462 mg,1.66mmol), Pd(PPh₃)₄(96 mg,0.083 mmol), NaHCO₃(211mg,2.51 mmol) and water (3mL) were added at room temperature, replaced with nitrogen for three times, and the mixture was heated to 85°C to react for 2.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C055-2(300 mg, yield 99%) as a brown oil.

### Step 3:

C055-2(150 mg,0.42 mmol) was dissolved in methanol (10 ml), 10% Pd/C(80 mg) was added at room temperature, replaced with hydrogen for three times, and the mixture was stirred for 1.5 h at room temperature. LCMS detection showed that the raw materials were reacted completely. The reaction solution was filtered with diatomite, and the filtrate was concentrated to obtain C055-3(153 mg, crude) as a white solid. LC-MS: Not available

### Step 4:

C055-3(153 mg,0.42 mmol) was dissolved in DCM(5 ml), was cooled to 0°C in an ice water bath, and PBr₃(113 mg,0.42 mmol, diluted with 0.5ml DCM) was added dropwise under the protection of nitrogen, and the mixture was reacted for 1 h at room temperature after addition. TLC detection showed that the raw materials were reacted completely. The reaction solution was diluted with DCM, saturated NaHCO₃ solution was added dropwise to adjust pH to be neutral, liquid separation was carried out, and the organic phase was washed with saturated salt water, dried over anhydrous sodium sulfate, filtered, and the filtrate was concentrated to obtain C055-4(176 mg, crude) as a white solid.

### Step 5:

Under the protection of nitrogen, C055-4(176 mg, crude) was dissolved in ethanol (5 ml), thiourea (25 mg,0.33 mmol) was added at room temperature, and the mixture was heated to 80°C to react for 1.0 h. TLC monitored that the reaction was completed. The reaction solution was cooled to room temperature, no solid was precipitated, the reaction solution was concentrated, the residue was slurried with 2.0ml of ethanol, a large amount of solids were precipitated, continued to stir for 20min, filtered, and the filter cake was washed with ethanol to obtain white solid(75 mg, yield for three steps: 43%).

¹H NMR: (DMSO-*d₆*, 400MHz): δ 1.37 (t, J = 7.2 Hz, 3H), 1.68-1.81 (m, 1H), 1.84-1.97 (m, 2H), 2.06-2.19 (m, 1H), 2.77-2.99 (m, 2H), 3.81 (s, 3H), 4.17-4.28 (m, 1H), 4.33 (q, J = 7.2 Hz, 2H), 5.11 (s, 2H), 6.69 (d, J = 7.6 Hz, 1H), 6.93-7.02 (m, 2H), 7.06-7.17 (m, 2H), 7.40 (s, 1H), 7.91 (d, J = 8.4 Hz, 1H), 9.20 (br, 3H). LCMS: m/z=422.2[M +H]⁺ (95.92% purity, 254 nm)

### Example 43: Preparation of Compounds SYY-C056 and SYY-C060

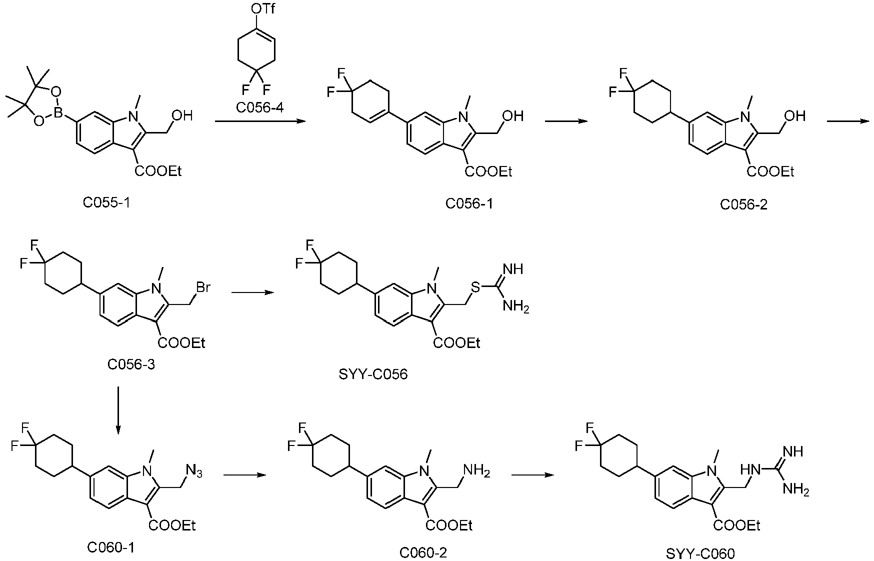

### Step 1:

4,4-Difluorocyclohexone (3.00g,22.37 mmol) was dissolved in dichloromethane (40 mL), cooled to about 0°C under the protection of nitrogen, pyridine compound (5.5g,26.78mmol) was added, stirred for 10 minutes, trifluoromethanesulfonic anhydride (7.57g,26.83 mmol) was added, the reaction solution slowly returned to room temperature to react overnight after addition. TLC detection showed that raw materials did not react completely. The reaction solution was heated to 30 °C to react for 6 h, and TLC detected that raw materials did not react completely. The reaction solution was cooled to room temperature, filtered, the filtrate was concentrated, the residue was slurred with petroleum ether, filtered, the filtrate was concentrated, and the crude was purified by silica gel column chromatography to obtain a colorless oil (1.3g, yield 22%).

### Step 2:

C055-1(360 mg,1.00 mmol) was dissolved in DME(20 ml), C056-4 (400 mg,1.50 mmol), Pd(PPh₃)₄(116 mg,0.10 mmol), NaHCO₃(250mg,3.00 mmol) and water (4mL) were added at room temperature, replaced with nitrogen for three times, and the mixture was heated to 85°C to react for 2.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=3/1) to obtain C056-1(320 mg, yield 91%) as a yellow solid. LCMS:m/z=332.2[M-17]⁺

### Step 3:

C056-1(130 mg,0.37 mmol) was dissolved in methanol (15 ml), 10% Pd/C(100 mg) was added at room temperature, replaced with hydrogen for three times, and the mixture was heated to 40°C to react for 1.0 h. TLC showed the reaction was completed. The reaction solution was filtered with diatomite, and the filtrate was concentrated to obtain C056-2(136 mg, crude) as a white solid. LCMS:m/z=334.2[M-17]⁺

### Step 3:

C056-2(136 mg, crude) was dissolved in DCM(5 ml), cooled to 0°C in an ice water bath, and PBr₃(105 mg,0.39 mmol, diluted with 0.5 ml DCM) was added dropwise under the protection of nitrogen, the mixture was reacted at room temperature for 1h after addition. TLC monitored the reaction was completed. The reaction solution was diluted with DCM, saturated NaHCO₃ solution was added dropwise to adjust pH to be neutral, liquid separation was carried out, the organic phase was washed with saturated salt water, dried over anhydrous sodium sulfate, filtered, and the filtrate was concentrated to obtain C056-3(150 mg, crude) as a light yellow solid.

### Step 4:

Under the protection of nitrogen, C056-3(150 mg, 0.36 mmol) was dissolved in ethanol (3 ml), thiourea (22 mg,0.29mmol) was added at room temperature, and the mixture was heated to 80°C to react for 1.0 h. TLC detection showed that the reaction was completed. The reaction solution was cooled to room temperature, no solid was precipitated, concentrated, 1.5 ml of ethanol was added, solids were precipitated, stirred for 20min, filtered, and the filter cake was washed with ethanol to obtain white solid SYY-C056 (60 mg, yield for three steps: 39%). ¹H NMR: (DMSO-*d₆*, 400MHz): δ 1.38 (t, J = 7.2 Hz, 3H), 1.70-2.20 (m, 8H), 2.78-2.91 (m, 1H), 3.86 (s, 3H), 4.34 (q, J = 6.8 Hz, 2H), 5.11 (s, 2H), 7.18 (dd, J = 8.4, 1.2 Hz, 1H), 7.49 (s, 1H), 7.92 (d, J = 8.0 Hz, 1H), 9.20 (br, 3H). LCMS: m/z=410.2[M+H]⁺ (99.78% purity, 220 nm)

### Step 6:

Under the protection of nitrogen, C056-3(192 mg,0.46 mmol) was added into DMF(8 ml), and then NaN₃(45 mg,0.69 mmol) was added, and the mixture was reacted overnight at room temperature. TLC detection showed the raw materials were reacted completely. The reaction solution was added with water, extracted with ethyl acetate. The organic phase was washed with saturated salt water, dried over anhydrous sodium sulfate, and concentrated to obtain C060-1(240 mg, crude) as a colorless oil, which was directly used in the next step.

### Step 7:

Under the protection of nitrogen, the previous C060-1(240 mg, crude) was dissolved in THF(12 ml) and water (4ml), PPh₃(182 mg,0.69mmol) was added at room temperature, and the mixture was heated to 50°C to react for 1.5h. TLC detection showed that the reaction was completed. The reaction solution was diluted with ethyl acetate, liquid separation was carried out, the organic phase was washed with saturated salt water, dried over anhydrous sodium sulfate and concentrated, and the residue was purified by Pre-TLC (DCM/MeOH = 10/1) to obtain C060-2(85 mg, yield for two steps: 52%) as a white solid.

### Step 8:

Under the protection of nitrogen, C060-2(85 mg,0.24 mmol) was dissolved in acetonitrile (6 ml), 1H-pyrazol-1-formamidine hydrochloride (54 mg,0.37 mmol) and DIEA(125 mg,0.97 mmol) were added, and the mixture was reacted at room temperature for 24 h. TLC detection showed that the reaction was completed. The reaction solution was filtered to obtain SYY-C060(45 mg, yield 47%) as a white solid. ¹H NMR: (DMSO-*d₆*, 400MHz): δ1.38 (t, J = 7.2 Hz, 3H), 1.72-2.21 (m, 8H), 2.77-2.90 (m, 1H), 3.85 (s, 3H), 4.35 (q, J = 7.2 Hz, 2H), 4.92 (s, 2H), 7.17 (dd, J = 8.0, 1.2 Hz, 1H), 7.22-7.71 (m, 4H), 7.93 (d, J = 8.4 Hz, 1H), 8.32 (br, 1H). LCMS: m/z=393.2[M +H]⁺ (98.99% purity, 220 nm)

### Example 44: Preparation of Compound SYY-C057

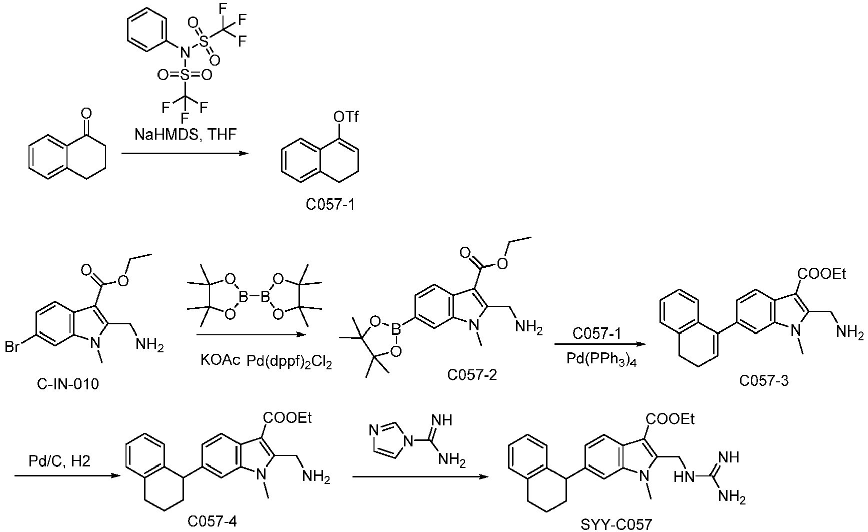

### Step 1:

1-Tetrahydronaphthone (4.0g,27.36mmol) was dissolved in THF(40mL), cooled to about -70 °C under the protection of nitrogen, a solution of NaHMDS in THF (20ml,40.00mmol,2.0M) was added dropwise, stirred at -70 °C for 1h after addition, a solution of N-phenyl bis(trifluoromethanesulphonimide) (11.6g,32.47mmol) in THF(10 mL) was added, the reaction continued for 1h at this temperature after addition, and the mixture was slowly heated to room temperature to react overnight. TLC detection showed the reaction was completed. The reaction solution was quenched with ammonium chloride aqueous solution, extracted with EA, the organic phases were combined, washed with saturated salt water, dried over anhydrous sodium sulfate, and concentrated, and the crude product was purified by column chromatography to obtain C057-1(5.2g, yield 68%) as a light yellow oil.

### Step 1

C-IN-010(1.5g,4.82 mmol) was dissolved in 1,4-Dioxane(50 ml), bis(pinacolato)diboron (1.84g,7.23 mmol), potassium acetate (946 mg,9.64 mmol) and Pd(dppf)₂Cl₂(176 mg,0.24 mmol) were added at room temperature, replaced with nitrogen for three times, and the mixture was heated to 100°C to react for 8h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain solid C057-2(0.8 g, yield 46%).

### Step 2

C057-2(300 mg,0.84 mmol) was dissolved in a mixed solvent of DME(50 ml) and water (5 ml), C057-1 (468 mg,1.68 mmol), sodium bicarbonate (212 mg,2.52 mmol) and Pd(PPh₃)₄ (93 mg,0.08 mmol) were added at room temperature, replaced with nitrogen for three times, and the mixture was heated to 80°C to react for 4 hours. TLC showed the reaction was completed. The reaction solution was cooled to room temperature, added with water, extracted with ethyl acetate, the organic phases were combined, washed with saturated salt water, dried over anhydrous sodium sulfate, and concentrated and the crude product was purified by pre-TLC to obtain C057-3(220 mg, crude). LCMS:m/z=344.2[M-NH₂]⁺

### Step 3

C057-3(220 mg, crude) was dissolved in methanol (10 ml), 10% Pd/C(22 mg) was added at room temperature, replaced with hydrogen for three times, and the mixture was heated to 50°C to react for 3h. LCMS monitored the raw materials were reacted completely. The reaction solution was cooled to room temperature, filtered with diatomite, and the filtrate was concentrated to obtain white solid C057-4(120 mg, crude), LC-MS:m/z = 346.2 [M-NH₂]⁺.

### Step 4

Under the protection of nitrogen, C057-4(120 mg, crude) was dissolved in acetonitrile (10 ml), 1H-pyrazol-1-formamidine hydrochloride (60 mg,0.40 mmol) and DIEA(170 mg,1.32 mmol) were added, and the mixture was reacted at room temperature for 24 h. TLC monitored that the reaction was completed. The reaction solution was filtered, and the filter cake was washed and dried to obtain SYY-C057(62 mg, yield for three steps: 46%) as a white solid. ¹H NMR: (DMSO-*d₆*, 400MHz): δ1.37 (t, J = 7.2 Hz, 3H), 1.72-1.76(m, 1H), 1.88-1.96(m, 2H), 2.11-2.20(m, 1H), 2.84-2.97(m, 2H), 3.79(s, 3H), 4.21-4.23(m, 1H),4.34 (q, J = 7.2 Hz, 2H), 6.68(d, J =8.0Hz,1H), 6.96-6.98(m, 2H), 7.08-7.16(m, 2H), 7.30(br, 4H), 7.41(s, 1H), 7.93(d, J =8.0Hz,1H). LC-MS: m/z = 405.2 [M+H]⁺

### Example 45: Preparation of Compound SYY-C058

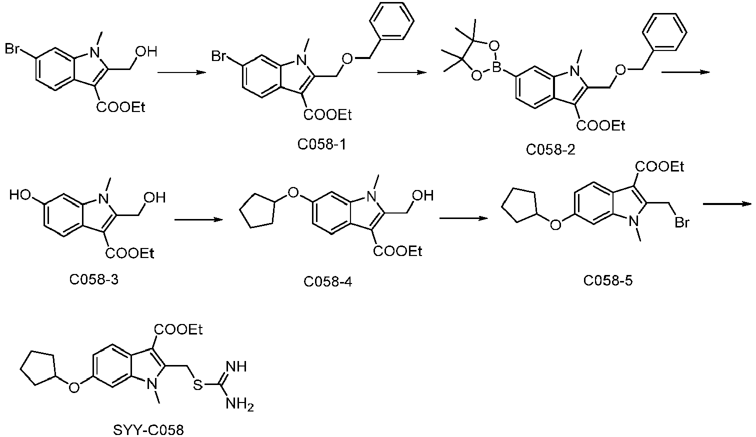

### Step 1:

Under the protection of nitrogen, C-IN-015(200 mg,0.64 mmol) was dissolved in DMF(5mL), NaH(60%)(51 mg,1.28 mmol) was added under an ice bath, benzyl bromide (132mg,0.77 mmol) was added after stirring for 15 min, continued to stir for 1h after addition. TLC showed the reaction was completed. The reaction solution was added with saturated ammonium chloride aqueous solution (20 mL), extracted with ethyl acetate, and the organic phases were combined, washed with saturated salt water, dried over anhydrous sodium sulfate, and concentrated, and the crude product was purified by silica gel column chromatography to obtain C058-1(190 mg, yield 74%) as a white solid.

### Step 2 :

Under the protection of nitrogen, C058-1(190mg ,0.47 mmol) was dissolved in dioxane (5 mL), Pd(dppf)Cl₂ dichloromethane complex (41 mg,0.05 mmol), bis(pinacolato)diboron (235 mg,0.93 mmol) and KOAc (138mg,1.41 mmol) were added at room temperature, and the mixture was heated to 100°C to stir for 3h. TLC showed the raw materials basically disappeared. The reaction solution was cooled to room temperature, added with water, extracted with ethyl acetate, the organic phases were combined, washed with saturated salt water, dried over anhydrous sodium sulfate, and concentrated, and purified by silica gel column chromatography to obtain C058-2(180 mg, yield 85%) as a colorless oil.

### Step 3:

Under the protection of nitrogen, C058-2(180mg,0.40 mmol) was dissolved in AcOH(3 mL), 30% H₂O₂(0.14 mL,1.40 mmol) was added, and the mixture was stirred at room temperature for 16h. TLC showed the raw materials basically disappeared. The reaction solution was added with water, extracted with ethyl acetate, the organic phases were combined, washed with saturated salt water, dried over anhydrous sodium sulfate, and concentrated to obtain light yellow oil, which was directly dissolved in methanol, Pd/C(80mg) was added, replaced with hydrogen balloon for 3 times, and reacted at room temperature for 2 h. TLC detection showed the reaction was completed. The reaction solution was filtered with diatomite, and the filtrate was concentrated to obtain C058-3(100 mg, crude).

### Step 4:

Under the protection of nitrogen, C058-3(100 mg,0.40 mmol) was dissolved in DMF(3 mL), K₂CO₃(166 mg,1.20mmol) and bromocyclopentane (238mg,1.60mmol) were added at room temperature, and the mixture was heated to 50°C and stirred for 10h. TLC showed the raw materials were basically reacted completely. The reaction solution was cooled to room temperature, added with water, extracted with ethyl acetate, and the organic phases were combined, washed with saturated salt water, dried over anhydrous sodium sulfate, concentrated, and purified by silica gel column chromatography to obtain C058-4(100 mg, yield for two steps: 79%) as a white solid.

### Step 5:

Under the protection of nitrogen, C058-4(100 mg,0.32 mmol) was dissolved in THF(3 mL), PBr₃(103 mg,0.38 mmol) was added under an ice bath, and continued to stir for 0.5h after addition. TLC showed the raw materials were basically reacted completely. The reaction solution was diluted by adding DCM, 10% NaHCO₃ solution was added to adjust to be neutral, extracted and stratified, and the organic phase was washed with water and saturated salt water, dried over anhydrous sodium sulfate, and concentrated to obtain C058-5(119mg, crude).

### Step 6:

Under the protection of nitrogen, C058-5(119 mg,0.31 mmol) and thiourea (22 mg,0.29 mmol) were dissolved in EtOH(4 mL), and the mixture was heated to 60°C and stirred for 0.5h. TLC showed the raw materials disappeared. The reaction solution was cooled to room temperature, and concentrated, and the crude product was purified by Pre-TLC to obtain an off-white solid (50 mg, yield for two steps: 42%). ¹H NMR (400 MHz, DMSO_d6) δ 1.37 (t, *J* = 7.2 Hz, 3H), 1.55-1.65 (m, 2H), 1.68-1.79 (m, 4H), 1.89-2.02 (m, 2H), 3.82 (s, 3H), 4.33 (q, *J* = 7.2 Hz, 2H), 4.89-4.95 (m, 1H), 5.10 (s, 2H), 6.87 (dd, *J* = 8.8, 2.0 Hz, 1H), 7.10 (d, *J* = 2.0 Hz, 1H), 7.85 (d, *J* = 8.8 Hz, 1H), 9.35 (br, 3H). LCMS: m/z=300.2[M-SC(NH)2+H]⁺

### Example 46: Preparation of Compound SYY-C059

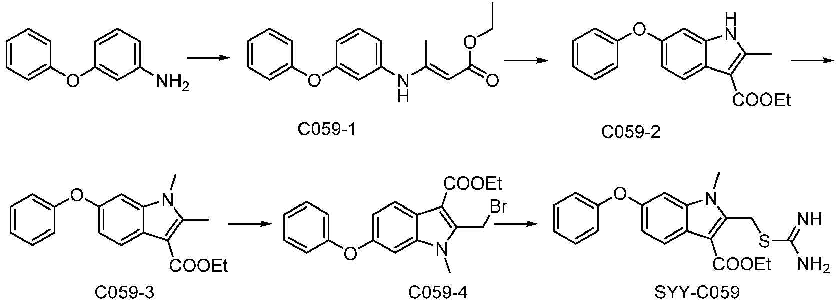

### Step 1:

Under the protection of N₂, 3-phenoxyaniline (500 mg,2.70 mmol), ethyl acetoacetate (351 mg ,2.70 mmol), AcOH (1 drop) and anhydrous magnesium sulfate (1.0g,8.31 mmol) were dissolved in DCM(5 mL) and stirred for 36 hours at room temperature. TLC showed 1/3 of the raw materials was remained. The reaction solution was filtered, and the filtrate was concentrated to obtain C059-1 (crude), which was directly used in the next step.

### Step 2:

Under the protection of N₂, C059-1 (crude, 2.70 mmol by theory), Pd(OAc)₂(61 mg ,0.27 mmol), Cu(OAc)₂ (1.53g ,8.42mmol) and K₂CO₃(1.16g ,8.39mmol) were dispersed into DMF(20 mL), and the mixture was heated to 100°C and stirred for 1h. TLC showed the raw materials disappeared. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C059-2(400 mg, yield for two steps: 50%) as a light yellow solid. ¹H NMR (400 MHz, DMSO_d6) δ 1.34 (t, *J* = 7.2 Hz, 3H), 2.64 (s, 3H), 4.27 (t, *J* = 7.2 Hz, 2H), 6.88 (dd, *J* = 8.6, 2.2 Hz, 1H), 6.96-6.98 (m,3H), 7.09 (t, *J* = 7.4 Hz, 1H), 7.30-7.39 (m, 2H), 7.91 (d, *J* = 8.6 Hz, 1H), 11.77 (s, 1H). LCMS:m/z=296.2[M+H]⁺

### Step 3:

Under the protection of N₂, C059-2(400 mg ,1.35 mmol) was dissolved in DMF(10mL), NaH(60%)(81 mg ,2.02 mmol) was added under an ice bath, continued to stir for 15min after the addition, methyl iodide (0.1mL ,1.61 mmol) was added, continued to react for 1h. TLC showed the raw materials disappeared. The reaction solution was quenched with saturated ammonium chloride aqueous solution, extracted with ethyl acetate, and the organic phases were combined, washed with saturated salt water, dried over anhydrous sodium sulfate, and concentrated, and the crude product was subjected to silica gel column chromatography to obtain C059-3(300 mg, yield 72%) as a light yellow solid. H NMR (400 MHz, CDCl₃) δ 1.47 (t, *J* = 7.2 Hz, 3H), 2.79 (s, 3H), 3.64 (s, 3H), 4.42 (q, *J* = 7.2 Hz, 2H), 6.97-7.04 (m, 4H), 7.09 (d, *J* = 7.4 Hz, 1H), 7.31-7.35 (m, 2H), 8.10 (d, *J* = 9.2 Hz, 1H)

### Step 4:

Under the protection of N₂, C059-3(300mg,0.97 mmol) was dissolved in CCl₄(5 mL), BPO(70%)(20 mg,0.058mmol) was added at room temperature. After heating to 80°C, NBS(173 mg,0.97 mmol) was added, and the mixture was continued to react for 1.5 h. TLC showed the reaction was completed. The reaction solution was cooled, and the crude product was separated and purified by silica gel column chromatography to obtain 130mg of yellow semi-solid crude product which was purified by pre-TLC to obtain C059-4(90 mg, yield 24%) as a light yellow solid.

### Step 5:

Under the protection of N₂, C059-4(90 mg,0.23 mmol) and thiourea (18 mg,0.24 mmol) were dissolved in EtOH(4 mL), and the mixture was heated to 60°C and stirred for 1h. TLC showed the reaction was completed. The reaction solution was cooled to room temperature, and concentrated, and the crude product was purified by Pre-TLC to obtain a white solid (60 mg, yield 67%). ¹H NMR (400 MHz, dmso) δ1.38 (t, *J* = 7.2 Hz, 3H), 3.81 (s, 3H), 4.35 (q, *J* = 7.2 Hz, 2H), 5.11 (s, 2H), 6.96-7.02(m, 3H), 7.08-7.13 (m, 1H), 7.35-7.39 (m, 3H), 8.01 (d, *J* = 8.8 Hz, 1H), 9.25 (br, 3H). LCMS: m/z=308.1[M-SC(NH)2+H]⁺

### Example 47: Preparation of Compound SYY-C061

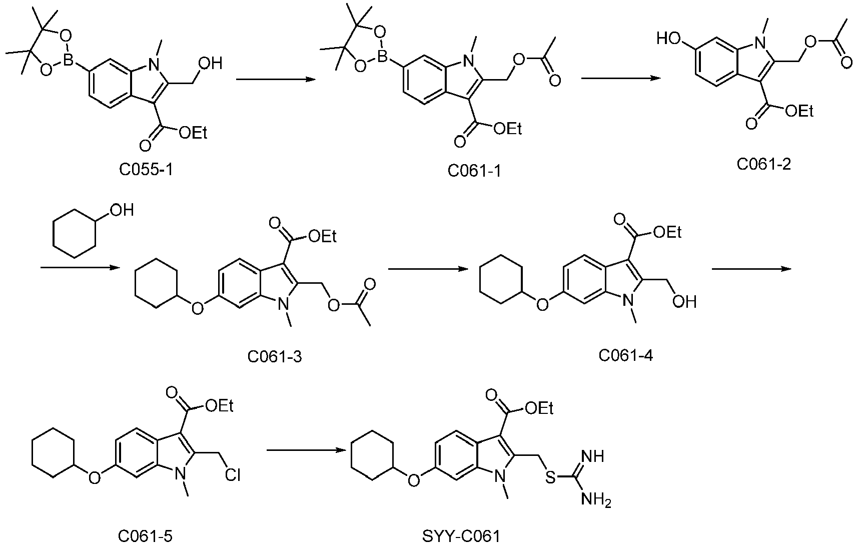

### Step 1:

C055-1(300 mg,0.84 mmol) was dissolved in pyridine (5 mL), DMAP(10 mg,0.08 mmol) and acetic anhydride (103 mg,1.01 mmol) were added in sequence, and the mixture was reacted for 2 h at room temperature. TLC detection showed that the reaction was completed. The reaction solution was poured into water, extracted with ethyl acetate. The organic phases were combined, washed with saturated salt water, dried over anhydrous sodium sulfate, and concentrated to obtain C061-1(310 mg, crude) as a brown-yellow solid.

### Step 2:

C061-1(310 mg,0.77 mmol) was dissolved in acetic acid (5 mL), 30% hydrogen peroxide (0.4 mL,3.99 mmol) was added dropwise, and the mixture was stirred and reacted at room temperature for 1h after the addition. TLC detection showed that the reaction was completed. The reaction solution was added with water, extracted with EA, and the organic phases were combined, washed with saturated salt water, dried over anhydrous sodium sulfate, and concentrated to obtain C061-2(220 mg, yield 90%) as a yellow-brown solid.

### Step 3:

C061-2(180 mg,0.62 mmol) and cyclohexanol (98 mg,0.98 mmol) were dissolved in THF(8 mL), triphenylphosphine (257mg,0.98 mmol) was added, cooled to about 0°C, DEAD(171mg,0.98 mmol) was added dropwise under the protection of nitrogen, after addition, the reaction solution was slowly raised to room temperature, and stirred to react overnight. TLC detection showed the reaction was completed. The reaction solution was quenched with water, extracted with EA, the organic phases were combined, washed with saturated salt water, dried over anhydrous sodium sulfate, and concentrated, and the crude product was purified by silica gel column chromatography to obtain C061-3(180 mg, yield 78%) as a colorless oil. ¹H NMR: (CDCl₃, 400MHz): δ1.32-1.42(m, 2H), 1.45 (t, J = 7.2 Hz, 3H), 1.56-1.62(m, 4H), 1.79-1.88(m, 2H), 1.96-2.06 (m, 2H), 2.10 (s, 3H), 3.74 (s, 3H), 4.29-4.35 (m, 1H), 4.39 (q, J = 7.2 Hz, 2H), 5.74 (s, 2H), 6.86 (d, J = 2.4 Hz, 1H), 6.94 (dd, J = 8.8, 2.0 Hz, 1H), 8.06 (d, J = 8.4 Hz, 1H).

### Step 4:

C061-3(180 mg,0.48 mmol) was dissolved in ethanol (10 mL), saturated sodium carbonate aqueous solution (5 mL) was added, the reaction solution was heated to 80°C to react for 1h. TLC detection showed the reaction was completed. The reaction solution was cooled to room temperature, added with water, extracted with EA, the organic phases were combined, washed with saturated salt water, dried over anhydrous sodium sulfate, and concentrated to obtain C061-4(110 mg, crude) as a gray-white solid. LCMS:m/z=314.1[M-17]⁺

### Step 5:

C061-4(110 mg,0.33 mmol) was dissolved in dichloromethane (5 mL), DMF(1 drop) was added, cooled to about 0°C under the protection of nitrogen, oxalyl chloride (0.17mL,0.34mmol,2.0mol/L in DCM) was added dropwise, after the addition, the mixture was reacted at 0°C for 2 h. TLC detection showed the reaction was completed. The reaction solution was concentrated at room temperature, and the residue was carried three times with dichloromethane to obtain C061-5 (crude), which was directly used in the next step.

### Step 6:

C061-5 (crude, 0.33 mmol by theory) and thiourea (13mg,0.17mmol) were dispersed into acetonitrile (3 mL), and heated to 75°C to react for 1h. TLC detection showed the reaction was completed. The reaction solution was cooled to room temperature, solids were precipitated, filtered, and the filter cake was washed and dried to obtain SYY-C061(65 mg, yield for three steps: 35%) as a light yellow solid. ¹H NMR: (DMSO-*d₆*, 400MHz): δ1.27-1.34 (m, 1H), 1.37 (t, J = 7.2 Hz, 3H), 1.35-1.38 (m, 1H), 1.41-1.48 (m, 3H), 1.52-1.61 (m, 1H), 1.69-1.79 (m, 2H), 1.90-2.02 (m, 2H), 3.82 (s, 3H), 4.33 (q, J = 7.2 Hz, 2H), 4.39-4.49 (m, 1H), 5.11 (s, 2H), 6.90 (dd, J = 8.4, 2.0 Hz, 1H), 7.16 (d, J = 2.0 Hz, 1H), 7.85 (d, J = 8.8 Hz, 1H), 9.38 (br, 3H). LCMS:m/z=314.2[M-SC(NH₂)₂ +H]⁺ (99.51% purity, 220 nm)

### Example 48: Preparation of Compound SYY-C062

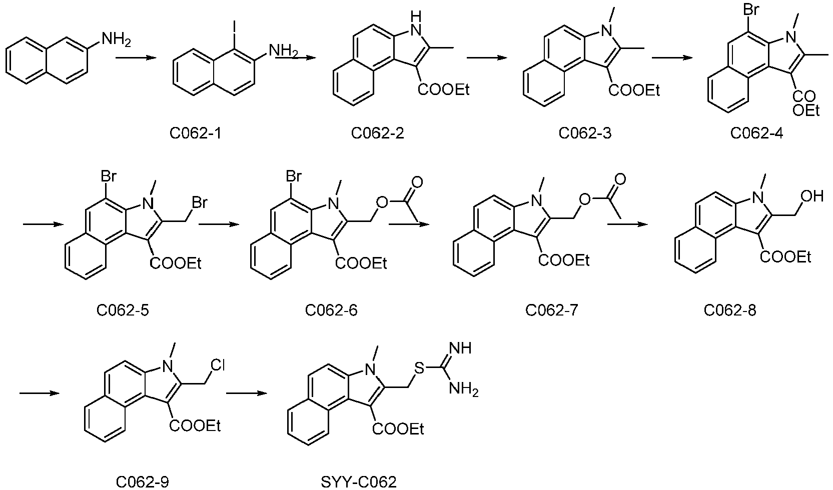

### Step 1:

Under the protection of nitrogen, 2-naphthylamine (5.0g,34.92 mmol) was dissolved in DMSO(100 ml), NIS(7.5g,33.33mmol) was added at room temperature, and the mixture was reacted at room temperature for 2.0 h. TLC showed the raw materials were reacted completely. The reaction solution was added with water, extracted with ethyl acetate, and the organic phases were combined, washed with saturated salt water, dried over anhydrous sodium sulfate, and concentrated to obtain C062-1(8.7g, yield 93%) as a brown oil. ¹H NMR (400 MHz, DMSO_d6) δ5.73 (s, 2H), 7.13 (d, *J* = 8.8 Hz, 1H), 7.20 (t, *J* = 7.4 Hz, 1H), 7.45 (t, *J* = 7.6 Hz, 1H), 7.65 (t, *J* = 7.5 Hz, 2H), 7.78 (d, *J* = 8.5 Hz, 1H). LCMS:m/z=270.1[M +H]⁺

### Step 2:

Under the protection of nitrogen, C062-1(8.7g,32.33 mmol) was dissolved in DMSO(170 ml), ethyl acetoacetate (25.2g,193.64.0 mmol), tetrazoleacetic acid (828 mg,6.46 mmol), cuprous iodide (615mg,3.23 mmol) and cesium carbonate (21.0g,64.45 mmol) were added at room temperature, and the mixture was heated to 100°C to react for 4h. TLC detection showed the reaction was completed. The reaction solution was cooled to room temperature, added with water, a large amount of solids were precipitated, filtered, the filter cake was washed with water, the filter cake was dissolved with ethyl acetate, washed with saturated salt water, dried over anhydrous sodium sulfate, and concentrated, and the crude product was purified by silica gel column chromatography to obtain C062-1(3.2g, yield 39%) as a yellow solid. LCMS:m/z=254.1[M +H]⁺

### Step 3:

Under the protection of nitrogen, C062-2(3.2g,12.63 mmol) was dissolved in DMF(30 ml), cooled to 0°C under an ice bath, NaH(586 mg,14.65 mmol) was added in batches, and the mixture was heated to room temperature to react for 0.5 h after addition, cooled to 0°C under an ice bath, and CH₃I solution (2.1g,14.79mmol,1 ml, diluted with DMF) was added dropwise, reacted at 0°C for 20min after addition. TLC monitored the reaction was completed. The reaction solution was quenched with water, a large amount of solids were precipitated, filtered, the filter cake was washed with water, the filter cake was dissolved with ethyl acetate, washed with saturated salt water, dried over anhydrous sodium sulfate, and concentrated, and the crude product was purified by silica gel column chromatography (PE/EA = 5/1) to obtain C062-3(3.0g, yield 88%) as a gray-white solid. LCMS:m/z=268.1[M +H]⁺

### Step 4:

Under the protection of nitrogen, C062-3(3.0g,11.22 mmol) was dissolved in carbon tetrachloride (60 ml), NBS(2.0g,11.24 mmol) and BPO(272 mg,1.12 mmol) were added at room temperature, and the mixture was heated to 80°C to react for 1.5h. TLC monitored that the reaction was completed. The reaction solution was cooled to room temperature, and the crude product was purified by silica gel column chromatography (DCM/PE = 1/2) to obtain C062-4(2.8g, yield 72%) as a yellow solid.

### Step 5:

Under the protection of nitrogen, C062-4(2.6 g,7.51 mmol) was dissolved in carbon tetrachloride (60 ml), NBS(1.34g,7.53 mmol) and BPO(110 mg,0.45 mmol) were added at room temperature, and the mixture was heated to 80°C to react for 1.5h. TLC monitored that the reaction was completed. The reaction solution was cooled to room temperature and concentrated to obtain C062-5 (crude), which was directly used in the next step.

### Step 6:

Under the protection of nitrogen, the previous C062-5 (crude) was dissolved in DMF(20 ml), DIEA was added to adjust to be neutral, potassium acetate (1.5g,15.28mmol) was added, and the mixture was heated to 30°C to react for 1.0 h. TLC showed the reaction was completed. The reaction solution was added with water, a large amount of solids were precipitated, filtered, the filter cake was dissolved with DCM, washed with saturated salt water, dried over anhydrous sodium sulfate, and concentrated, and the crude product was purified by silica gel column chromatography to obtain C062-6(965 mg, yield for two steps: 32%) as a light yellow solid.

### Step 7:

Under the protection of nitrogen, C062-6 (250 mg,62 mmol) was dissolved in methanol (20 ml), Pd/C(120 mg) was added at room temperature, and the mixture was heated to 30°C to react for 1.0 h. TLC detection showed the raw materials were reacted completely. The reaction solution was cooled to room temperature, filtered with diatomite, and the filtrate C062-7 (crude) was directly used for the next reaction.

### Step 8:

Saturated sodium carbonate solution (5 ml) was added to the reaction solution 062-7 (crude) in the previous step, and heated to 80 °C to react for 1 hour. TLC showed the reaction was completed. The reaction solution was concentrated, extracted with ethyl acetate, washed with saturated salt water, dried over anhydrous sodium sulfate, and concentrated, and the crude product was separated and purified by silica gel column chromatography to obtain C062-8(1 10 mg, yield for two steps: 63%) as a white solid.

### Step 9:

C062-8(110 mg,0.39 mmol) was dissolved in DCM(10 ml), DMF(1 drop) was added at room temperature, cooled under an ice bath, a solution of oxalyl chloride (51mg,0.40mmol) in DCM(1mL) was added, and stirred for 1 hour under an ice water bath after addition. TLC showed reaction was completed. The reaction solution was concentrated at low temperature to obtain C062-9 (crude), which was directly used in the next step.

### Step 10:

C062-9 (crude) and thiourea (19mg,0.25 mmol) were dissolved in acetonitrile (3mL), heated to 75 °C to react for 1h. TLC detection showed the reaction was completed. The reaction solution was cooled to room temperature, filtered, and the filter cake was washed and dried to obtain SYY-C062(65 mg, yield for two steps: 49%) as a yellowish solid. ¹H NMR (400 MHz, DMSO_d6) δ 1.42 (t, *J* = 7.2 Hz, 3H), 3.99 (s, 3H), 4.45 (q, *J* = 7.2 Hz, 2H), 5.13 (s, 2H), 7.46-7.50 (m, 1H), 7.54-7.58 (m, 1H), 7.82 (s, 2H), 7.99 (dd, *J* = 8.0, 0.8 Hz, 1H), 9.03 (d, *J* = 8.4 Hz, 1H), 9.52 (br, 3H). LCMS: m/z=266.1 [M-SC(NH)₂+H]⁺

### Example 49: Preparation of Compound SYY-C063

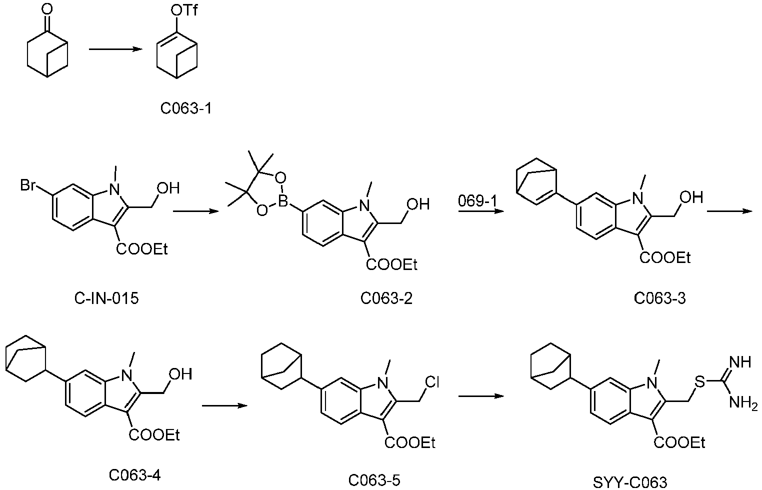

### Step 1:

Under the protection of nitrogen, a solution of bicyclo [2.2.1] heptanone (3.0g,27.23 mmol) in tetrahydrofuran (30mL) was added dropwise to a solution of LDA(14.3mL,28.60mmol,2mol/L) cooled to about -70°C for about half an hour, the reaction solution was continued to react at -70°C for 30 minutes, and then heated to 0°C and stirred for 30 minutes, then cooled to about -70°C, N-phenyl-bis(trifluoromethanesulfonimide) (10.70g,29.95 mmol) was added dropwise, continued to stir for 1h after addition, and the mixture was heated to 0°C and stirred to reacted for 3h. TLC detection showed raw materials remained. The reaction solution was quenched with saturated ammonium chloride aqueous solution, extracted with methyl tert-butyl ether , the organic phases were combined, washed with saturated salt water, dried over anhydrous sodium sulfate, and concentrated, and the crude product was purified by silica gel column chromatography to obtain C063-1(5.7g, yield 86%) as a light yellow oil.

### Step 2:

Under the protection of nitrogen, the C-IN-015(2.0g,6.41 mmol) was dissolved in 1,4-Dioxane(20 ml), bis(pinacolato)diboron (2.4 g,9.45mmol), Pd(dppf)Cl₂ dichloromethane complex (468 mg,0.57 mmol) and potassium acetate (1.3 g,13.25 mmol) were added at room temperature, replaced with nitrogen for three times, and the mixture was heated to 100°C to react for 4.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=2/1) to obtain C063-2(2.3 g, yield 100%) as a light yellow solid.

### Step 3:

Under the protection of nitrogen, C063-2(1.12g,3.12 mmol) was dissolved in DMF(20 ml), C063-1(1.66g,6.85 mmol), Pd(dppf)Cl₂ dichloromethane complex (523 mg,0.64 mmol) and Cs₂CO₃(2.0g,6.14 mmol) were added at room temperature, replaced with nitrogen for three times, and the mixture was heated to 85°C to react for 2.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=4/1) to obtain C063-3(560 mg, yield 55%) as a yellow oil.

### Step 4:

C063-3(560 mg,1.72 mmol) was dissolved in methanol (30 ml) and 10% Pd/C(90 mg) was added, replaced with hydrogen for 3 times and the mixture was reacted for 1.0 h at room temperature. TLC showed the raw materials were completely reacted, filtered with diatomite, the filtrate was concentrated, and the residue was purified by Pre-TLC to obtain C063-4(375 mg, yield 67%) as a light yellow solid.
LCMS:m/z=310.2[M-17]⁺

### Step 5:

Under the protection of nitrogen, C063-4(65 mg,0.20 mmol) was dissolved in DCM(3 ml), DMF(2 drops) was added, cooled under an ice water bath, oxalyl chloride (26 mg,0.20 mmol, diluted with 0.5ml DCM) was added dropwise at 0°C, and the mixture was reacted at 0°C for 0.5h after addition. TLC showed the reaction was completed. The reaction solution was concentrated, and the residue was carried three times with DCM to obtain C063-5 (crude), which was directly used in the next step.

### Step 6:

Under the protection of nitrogen, C063-5 (crude product) from the previous step was dissolved in acetonitrile (3 ml), thiourea (12 mg,0.16 mmol) was added at room temperature, and the mixture was heated to 80°C to react for 1.0 h. TLC monitored the reaction was completed. The reaction solution was cooled to room temperature, solids were precipitated, filtered, and the filter cake was washed and dried to obtain a white solid (24 mg, yield for two steps: 31%). ¹H NMR: (DMSO-*d₆*, 400MHz): δ 1.12-1.27 (m, 3H), 1.36 (t, J = 7.2 Hz, 3H), 1.41-1.59 (m, 4H), 1.90-1.98 (m, 1H), 2.28-2.33 (m, 1H), 2.41-2.46 (m, 1H), 3.29-3.33 (m, 1H), 3.86 (s, 3H), 4.32 (q, J = 7.2 Hz, 2H), 5.14 (s, 2H), 7.14 (d, J = 8.4 Hz, 1H), 7.40 (s, 1H), 7.90 (d, J = 8.4 Hz, 1H), 9.44 (br, 3H). LCMS:m/z=386.2[M +H]⁺ (98.07% purity, 254 nm)

### Example 50: Preparation of Compound SYY-C065

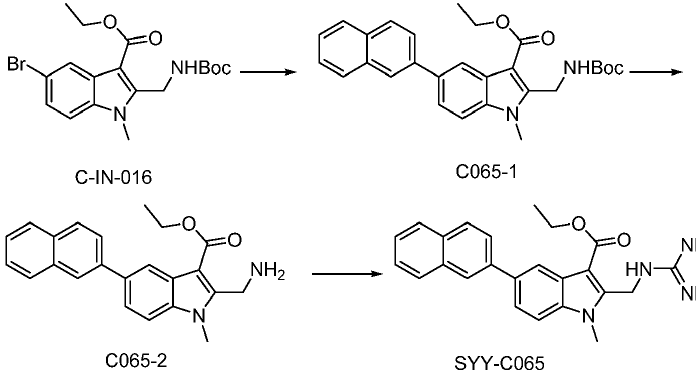

### Step 1:

C- IN-016(300mg,0.73mmol) and 2-naphthalene boric acid (188mg,1.09mmol) were dissolved in DMF(6mL), cesium carbonate (476mg,1.46mmol) and PdCl2(dppf) dichloromethane complex (60mg,0.07mmol) were added, replaced with nitrogen for three times, and the mixture was heated to 80°C to react for 2 h. TLC detection showed the reaction was completed. The reaction solution was cooled to room temperature, added with water, extracted with EA, the organic phases were combined, washed with saturated salt water, dried over anhydrous sodium sulfate, and concentrated, and the crude product was purified by silica gel column chromatography to obtain C065-1(250mg, yield 67%) as a white solid.

### Step 2:

C065-1(240mg,0.52mmol) was dissolved in dioxane hydrochloride solution (10mL, 40mmol,4mol/L), stirred at room temperature for 2 h, and white solid was precipitated. TLC detection showed that a small amount of raw materials did not react completely. The reaction solution was added with ethanol (5mL) to help dissolve, and heated to 40°C to react for 2h. TLC detection showed the reaction was completed. The reaction solution was cooled to room temperature, concentrated to a small amount, neutralized with saturated sodium carbonate aqueous solution, filtered, the filter cake was dissolved with dichloromethane and methanol (10V/1V), dried over anhydrous sodium sulfate, and concentrated, and the crude product was purified by Pre-TLC to obtain C065-2(160mg, yield 85%) as an off-white solid. ¹H NMR (400 MHz, DMSO_d6) δ 1.43 (t, *J* = 7.2 Hz, 3H), 3.42 (br, 2H), 3.96 (s, 3H), 4.39 (q, *J* = 7.2 Hz, 2H), 4.46 (s, 2H), 7.50-7.57 (m, 2H), 7.77 (s, 2H), 7.89 (dd, *J* = 8.5, 1.6 Hz, 1H), 7.95(d, *J* = 7.6 Hz, 1H), 8.03 (d, *J* = 8.4 Hz, 2H), 8.22 (s, 1H), 8.45 (s, 1H). LCMS:m/z=342.1[M-NH₂]⁺

### Step 3:

C065-2(160mg,0.45mmol) was dispersed in acetonitrile (3mL), DIEA(232mg,1.80mmol) was added, stirred for 5 minutes, but it was not completely dissolved, pyrazole formamidine hydrochloride (98mg,0.67mmol) was added, the reaction solution was stirred for 2 days at room temperature, and LCMS showed about 1/3 of the raw materials were not completely reacted. The reaction solution was filtered to obtain 130 mg of filter cake, re-charged, and the reaction was stirred at room temperature for 2d. The reaction solution was filtered, and the filter cake was washed and dried to obtain SYY-C065(110mg, yield 61%). ¹H NMR: (DMSO-*d₆*, 400MHz): δ1.42 (t, J = 7.2 Hz, 3H), 3.92 (s, 3H), 4.41 (q, J = 7.2 Hz, 2H), 4.98 (s, 2H), 7.31-7.67 (m, 5H), 7.79 (s, 2H), 7.88 (dd, J = 8.4, 1.6 Hz, 1H), 7.95 (d, J = 7.6 Hz, 1H), 7.99-8.08 (m, 2H), 8.22 (s, 1H), 8.27-8.59 (m, 2H). LCMS:m/z=401.2[M +H]⁺(99.65% purity, 254 nm)

### Example 51: Preparation of Compound SYY-C066

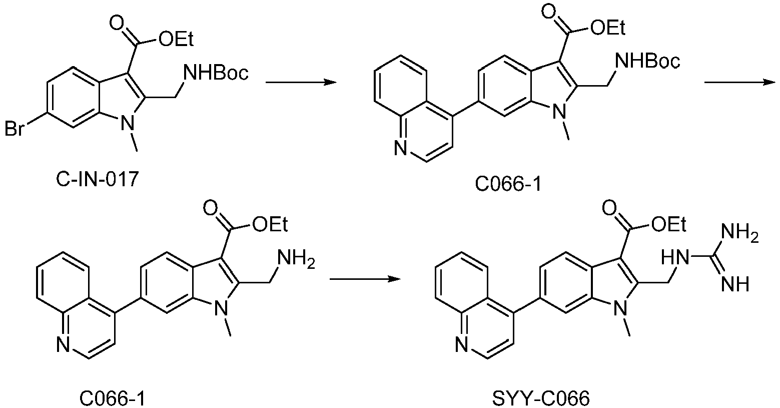

### Step 1:

C-IN-017(500mg,1.22mmol) and 4-quinoline boric acid (421mg,2.43mmol) were dissolved in DMF(10mL), cesium carbonate (795mg,2.44mmol) and PdCl2(dppf) dichloromethane complex (50mg,0.06mmol) were added, replaced with nitrogen for three times, and the mixture was heated to 80 °C for 3 h. TLC detection showed the reaction was completed. The reaction solution was cooled to room temperature, added with water, extracted with EA, the organic phases were combined, washed with saturated salt water, dried over anhydrous sodium sulfate, and concentrated, and the crude product was purified by silica gel column chromatography to obtain C066-1(530mg, yield 95%) an off-white solid.

### Step 2:

C066-1(530mg,1.15mmol) was dissolved in dichloromethane (6mL), trifluoroacetic acid (2mL) was added, and the mixture was stirred and reacted for 1h at room temperature. TLC detection showed the reaction was completed. The reaction solution was neutralized with saturated sodium carbonate aqueous solution, extracted with EA, the organic phases were combined, washed with saturated salt water, dried over anhydrous sodium sulfate, and concentrated, and the crude product was purified by silica gel column chromatography to obtain C066-2(330mg, yield 80%) as a yellowish brown solid. ¹H NMR (400 MHz, DMSO_d6) : 61.40(t, *J* = 7.2 Hz, 3H) , 1.99 (br, 2H), 3.90 (s, 3H), 4.24 (s, 2H), 4.35 (q, *J* = 7.2 Hz, 2H), 7.38 (dd, *J* = 8.4, 1.6 Hz, 1H), 7.53 (d, *J* = 4.4 Hz, 1H), 7.59 (t, *J* = 7.6 Hz, 1H), 7.83-7.73 (m, 2H), 7.98 (d, *J* = 8.4 Hz, 1H), 8.12 (d, *J* = 8.4 Hz, 1H), 8.17 (d, *J* = 8.4 Hz, 1H), 8.97 (d, *J* = 4.4 Hz, 1H). LCMS:m/z=360.2[M +H]⁺

### Step 3:

C066-2(150mg,0.42mmol) and DIEA(217mg,1.68mmol) were dissolved in acetonitrile (5mL), stirred for 5 minutes, pyrazole formamidine hydrochloride (92mg,0.63mmol) was added, and the mixture was stirred to react overnight at room temperature. Solid was precipitated in the reaction solution , filtered, and the filter cake was washed and dried to obtain SYY-C066(140mg, yield 83%) as a white solid. ¹H NMR: (DMSO-*d₆*, 400MHz): δ1.42 (t, J = 7.2 Hz, 3H), 3.93 (s, 3H), 4.41 (q, J = 7.2 Hz, 2H), 5.00 (s, 2H), 7.44-7.62 (m, 7H), 7.79-7.83 (m, 1H), 7.88(s, 1H), 7.95(d, J = 8.8Hz, 1H), 8.13(d, J = 8.4Hz, 1H), 8.20(d, J = 8.4Hz, 1H), 8.98(d, J = 8.4Hz, 1H). LCMS:m/z=402.2[M +H]⁺ (98.95% purity, 254 nm)

### Example 52: Preparation of Compound SYY-C067

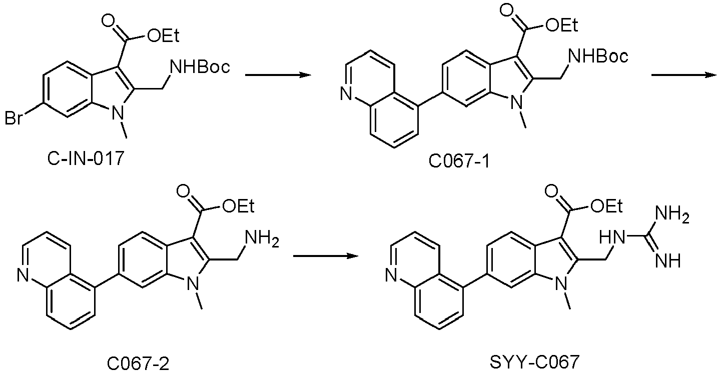

### Step 1:

C- IN-017(300mg,0.73mmol) and 5-quinoline boric acid (253mg,1.46mmol) were dissolved in dioxane (15mL) and water (5mL), sodium carbonate (155mg,1.46mmol) and PdCl2(dppf) dichloromethane complex (60mg,0.073mmol) were added, replaced with nitrogen for three times, and the mixture was heated to 100 °C to react for 3 h. TLC detection showed the reaction was completed. The reaction solution was cooled to room temperature, added with water, extracted with EA, the organic phases were combined, washed with saturated salt water, dried over anhydrous sodium sulfate, and concentrated, and the crude product was purified by silica gel column chromatography to obtain C067-1(330mg, yield 99%) as an off-white solid.

### Step 2:

C067-1(330mg,0.72mmol) was dissolved in dichloromethane (3mL), trifluoroacetic acid (1mL) was added, and the mixture was stirred and reacted for 1h at room temperature. TLC detection showed the reaction was completed. The reaction solution was neutralized with saturated sodium carbonate aqueous solution, extracted with EA, the organic phases were combined, washed with saturated salt water, dried over anhydrous sodium sulfate, and concentrated, and the crude product was purified by silica gel column chromatography to obtain C067-2(200mg, yield 78%) as a light yellow solid. ¹H NMR (400 MHz, DMSO_d6) δ 1.43 (t, *J* = 7.2 Hz, 3H), 3.96 (s, 3H), 4.39 (q, *J* = 7.2 Hz, 2H), 4.49 (s, 2H), 7.01(br, 2H), 7.37 (d, *J* = 8.4 Hz, 1H), 7.50-7.54 (m, 1H), 7.64 (d, *J* = 7.2 Hz, 1H), 7.79 (s, 1H), 7.84-7.88 (m, 1H), 8.09 (d, *J* = 8.4 Hz, 1H), 8.18 (d, *J* = 8.4 Hz, 1H), 8.24 (d, *J* = 8.4 Hz, 1H), 8.97-8.91 (m, 1H). LCMS:m/z=360.2[M+H]⁺

### Step 3:

C067-2(190mg,0.53mmol) and DIEA(274mg,2.12mmol) were dissolved in acetonitrile (5mL), stirred for 5 minutes, pyrazole formamidine hydrochloride (116mg,0.79mmol) was added, and the mixture was stirred to react overnight at room temperature. The reaction solution was filtered, and the filter cake was washed, and dried to obtain SYY-C067(190mg, yield 90%) as a white solid. ¹H NMR (400 MHz, DMSO_d6) δ 1.42 (t, *J* = 7.2 Hz, 3H), 3.92 (s, 3H), 4.41 (q, *J* = 7.2 Hz, 2H), 5.00(s, 2H), 7.37-7.65(m, 6H), 7.80(s, 1H), 7.84-7.88(m, 1H), 8.08(d, J =8.4Hz, 1H), 8.17(d, J =8.0Hz, 1H), 8.25(d, J =8.8Hz, 1H), 8.44(br, 1H), 8.95(dd, J1 = 4.0Hz, J2= 1.6).LCMS:m/z=402.2[M+H]⁺ (97.81% purity, 254 nm)

### Example 53: Preparation of Compound SYY-C068

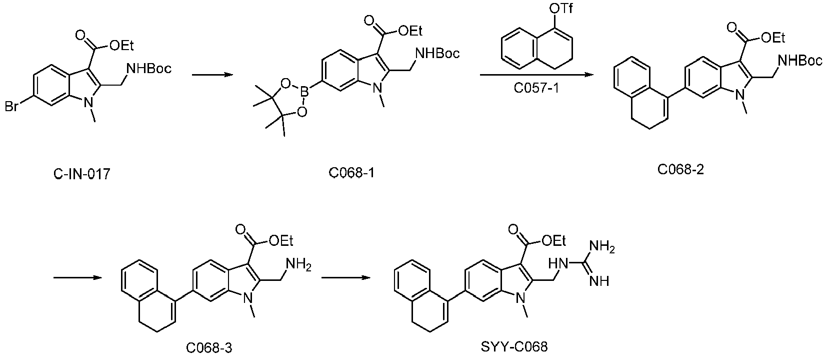

### Step 1:

C-IN-017(7.0g,17.02mmol) and bis(pinacolato)diboron (6.5g,25.60mmol) were dissolved in dioxane (200mL), potassium acetate (3.34g,34.03mmol) and PdCl2(dppf) dichloromethane complex (139mg,0.17mmol) were added, replaced with nitrogen for three times, and the mixture was heated to 100 °C to react for 3 hours. TLC detection showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain a light yellow solid(7.5g, yield 96%).

### Step 2:

C068-1(400mg,0.87mmol) and C057-1(726mg,2.59mmol) were dissolved in DMF(10mL) and water (0.5mL), cesium carbonate (567mg,1.74mmol) and PdCl₂(dppf) dichloromethane complex (70mg,0.086mmol) were added, replaced with nitrogen for three times, and the mixture was heated to 100 °C to react for 3 h. TLC detection showed the reaction was completed. The reaction solution was cooled to room temperature, added with water, extracted with EA, the organic phases were combined, washed with saturated salt water, dried over anhydrous sodium sulfate, and concentrated, and the crude product was purified by column chromatography to obtain C068-2(320mg, yield 80%) as an off-white solid.

### Step 3:

C068-2(320mg,0.69mmol) was dissolved in dichloromethane (3mL), trifluoroacetic acid (1mL) was added, and the mixture was stirred and reacted for 1h at room temperature. TLC detection showed the reaction was completed. The reaction solution was neutralized with saturated sodium carbonate aqueous solution, extracted with EA, the organic phases were combined, washed with saturated salt water, dried over anhydrous sodium sulfate, and concentrated to obtain C068-3(250mg, crude) as a yellowish brown solid with good TLC purity which was directly used in the next reaction. LCMS:m/z=344.1[M -NH₂]⁺

### Step 4:

C068-3(250mg, crude) and DIEA(358mg,2.77mmol) were dispersed into acetonitrile (5mL), stirred for 5 minutes, pyrazole formamidine hydrochloride (152mg,1.04mmol) was added, and the mixture was stirred to react overnight at room temperature. The reaction solution was filtered, and the filter cake was washed and dried to obtain SYY-C068(192mg, yield for two steps: 69%) as a white solid. ¹H NMR: (DMSO-*d₆*, 400MHz): δ1.40 (t, J = 7.2 Hz, 3H), 2.39-2.41 (m, 2H), 2.83 (t, J = 7.6 Hz, 2H), 3.86 (s, 3H), 4.38 (q, J = 7.2 Hz, 2H), 4.96 (s, 2H), 6.18 (t, J = 4.4 Hz, 1H), 6.89 (d, J = 7.2 Hz, 1H), 7.11-7.13 (m 1H), 7.15-7.23 (m, 2H), 7.25-7.59 (m, 5H), 8.03 (d, J = 8.4 Hz, 1H), 8.21-8.48 (m, 1H). LCMS:m/z=403.2[M+H]⁺

### Example 54: Preparation of Compound SYY-C069

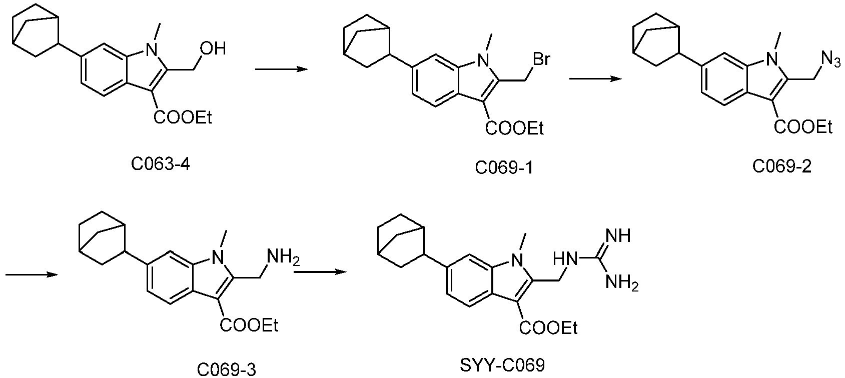

### Step 1:

Under the protection of nitrogen, C063-4(185 mg,0.57 mmol) was dissolved in DCM(8 ml), cooled under an ice water bath, PBr₃(154mg,0.57 mmol, diluted with 0.6 ml DCM) was added dropwise at 0°C, and the mixture was reacted for 0.5h at 0°C after addition. TLC showed the reaction was completed. 8ml DCM was added to the reaction solution, saturated NaHCO₃ solution was added dropwise to adjust to be neutral, extracted and separated, the organic phases were combined, washed with saturated salt water, dried over anhydrous sodium sulfate, and concentrated to obtain C069-1(212 mg, crude) as a light yellow oil.

### Step 2:

Under the protection of nitrogen, C069-1(212 mg, crude) was dissolved in DMF(5 ml), NaN₃(53 mg,0.82 mmol) was added at room temperature, and the reaction solution was heated to 50 °C to react for 1.0 h after the addition. TLC monitored that the reaction was completed. The reaction solution was cooled to room temperature, added with water, extracted with ethyl acetate, the organic phases were combined, washed with saturated salt water, dried over anhydrous sodium sulfate, and concentrated to obtain C069-2(232 mg, crude) as a light yellow oil.

### Step 3:

Under the protection of nitrogen, C069-2(232 mg, crude) was dissolved in THF(9 ml) and water (3 ml), triphenylphosphine (259mg,0.99 mmol) was added at room temperature, and the mixture was heated to 50 °C to react for 2.0 h. TLC monitored that the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by Pre-TLC (DCM/MeOH = 10/1) to obtain C069-3(140 mg, yield for three steps: 76%) as a light yellow solid. LCMS:m/z=310.2[M-NH₂]⁺

### Step 4:

Under the protection of nitrogen, C069-3(140 mg,0.43 mmol) was dissolved in acetonitrile (8 ml), DIEA(221 mg,1.71 mmol) and 1H-pyrazol-1-formamidine hydrochloride (94 mg,0.64 mmol) were added at room temperature, and the mixture was reacted at room temperature for 24 h. LCMS monitored that the reaction was completed. The reaction solution was filtered, and the filter cake was washed, and dried to obtain SYY-C069(120 mg, yield 76%) as a white solid. ¹H NMR: (DMSO-*d₆*, 400MHz): δ1.09-1.31 (m, 3H), 1.38 (t, J = 7.2 Hz, 3H), 1.43-1.61 (m, 4H), 1.91-2.02 (m, 1H), 2.30-2.35 (m, 1H), 2.43-2.48 (m, 1H), 3.36-3.39 (m, 1H), 3.85 (s, 3H), 4.35 (q, J = 7.2 Hz, 2H), 4.92 (s, 2H), 7.15 (d, J = 8.4 Hz, 1H), 7.24-7.70 (m, 4H), 7.93 (d, J = 8.4 Hz, 1H), 8.36 (br, 1H). LCMS:m/z=369.2[M+H]⁺ (99.43% purity, 220 nm)

### Example 55: Preparation of Compound SYY-C070

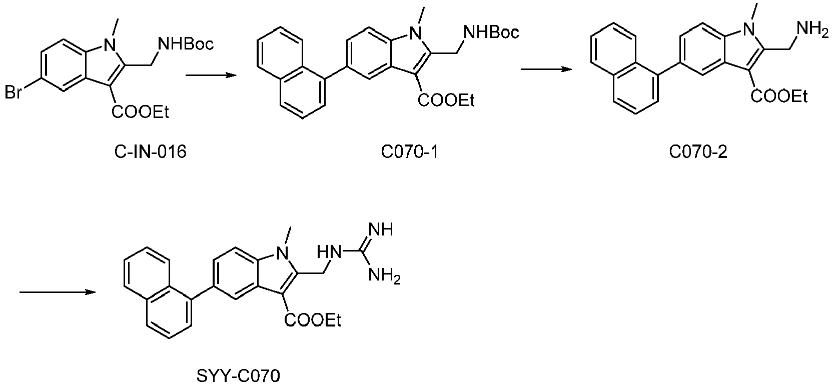

### Step 1:

Under the protection of nitrogen, C-IN-016(300 mg,0.73 mmol) was dissolved in a mixed solvent of DMF(15ml) and water (5 ml), 1-naphthalene boronic acid (251 mg,1.46 mmol), Pd(dppf)Cl₂ dichloromethane complex (60 mg,0.073mmol) and Na₂CO₃(302 mg,2.85 mmol) were added at room temperature, replaced with nitrogen for three times, and the mixture was heated to 100°C to react for 2.0 h. TLC showed the reaction was completed. The reaction solution was cooled to room temperature, added with water, extracted with ethyl acetate, the organic phases were combined, washed with saturated salt water, dried over anhydrous sodium sulfate and concentrated, and the residue was purified by silica gel column chromatography (PE/EA = 10/1) to obtain C070-1(296 mg, yield 88%) as a white solid.

### Step 2:

C070-1(296 mg,0.65 mmol) was dissolved in DCM(3 ml), cooled to 0°C under an ice water bath, trifluoroacetic acid (1.0 ml) was added dropwise, and the mixture was reacted for 1.0 h at room temperature after addition. TLC showed the reaction was completed. The reaction solution was concentrated at low temperature, the crude product was diluted with 1.0 ml of ethyl acetate, saturated sodium bicarbonate aqueous solution was added dropwise to adjust to be weak alkalinity, a large amount of solid were precipitated, stirred for 20min, filtered, and the filter cake was dissolved with dichloromethane, washed with saturated salt water, dried over anhydrous sodium sulfate, and concentrated to obtain C070-2(140 mg, crude) as a light yellow solid. ¹H NMR (400 MHz, DMSO_d6) δ 1.27 (t, *J* = 7.2 Hz, 3H), 2.07(br, 1H), 3.92 (s, 3H), 4.24 (s, 2H), 4.28 (q, *J* = 7.2 Hz, 2H), 7.34 (dd, *J* = 8.4, 1.6 Hz, 1H), 7.46-7.49 (m, 2H), 7.54 (m, 1H), 7.60(m, 1H), 7.71 (d, *J* = 8.4 Hz, 1H), 7.87 (d, *J* = 8.4 Hz, 1H), 7.95 (d, *J* = 8.4 Hz, 1H), 8.01 (d, *J* = 8.4 Hz, 1H), 8.11 (d, *J* = 1.6 Hz, 1H). LCMS:m/z=342.1[M-NH₂]⁺

### Step 3:

C070-2(140 mg,0.39 mmol) was dissolved in acetonitrile (5 ml), DIPEA(202 mg,1.56 mmol) and 1H-pyrazol-1-formamidine hydrochloride (86 mg,0.59 mmol) were added, and the mixture was reacted at room temperature for 24 h. LCMS monitored that the reaction was completed. The reaction solution was filtered, and the filter cake was washed and dried to obtain SYY-C070(110 mg, yield for two steps: 43%) as a white solid. ¹H NMR: (DMSO-*d₆*, 400MHz): δ1.27 (t, J = 7.2 Hz, 3H), 3.95 (s, 3H), 4.32 (q, J = 7.2 Hz, 2H), 5.00 (s, 2H), 7.29-7.71- (m, 8H), 7.80 (d, J = 8.4 Hz, 1H), 7.84 (d, J = 8.4 Hz, 1H), 7.97 (d, J = 8.0 Hz, 1H), 8.02 (d, J = 8.0 Hz, 1H), 8.13-8.78 (m, 2H). LCMS:m/z=401.1[M+H]⁺ (98.53% purity, 220 nm)

### Example 56: Preparation of Compound SYY-C071

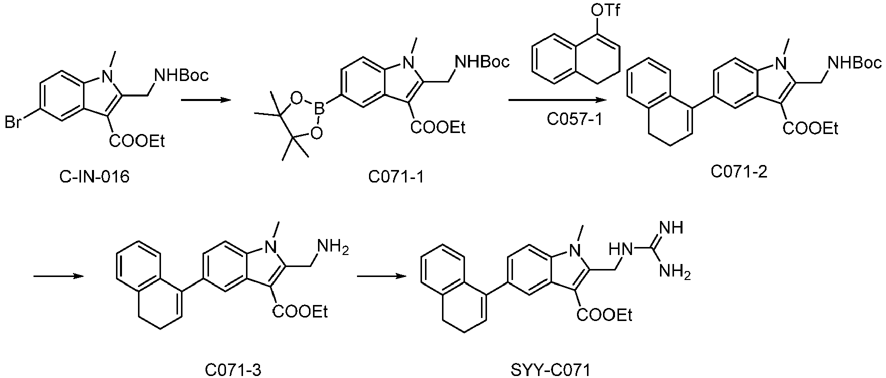

### Step 1:

Under the protection of nitrogen, the C-IN-016(500 mg,1.22 mmol) was dissolved in 1, 4-dioxane (15 ml), and bis(pinacolato)diboron (464 mg,1.83 mmol), Pd(dppf)Cl₂ dichloromethane complex (50 mg,0.061mmol) and potassium acetate (238 mg,2.43 mmol) were added at room temperature, replaced with nitrogen for three times, and the mixture was heated to 100°C to react for 3 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=2/1) to obtain C071-1(509 mg, yield 91%) as a light yellow oil.

### Step 2:

Under the protection of nitrogen, C071-1(488 mg,1.06 mmol) was dissolved in DME(27 ml), C057-1(1.15g,4.14 mmol), Pd(PPh₃)₄(123 mg,0.11 mmol) and saturated NaHCO₃ solution (2.7 ml) were added at room temperature, replaced with nitrogen for three times, and the mixture was heated to 85°C to react for 2 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=10/1) to obtain C071-2(490 mg, yield 100%) as a brown yellow oil.

### Step 3:

C071-2(490 mg,1.06 mmol) was dissolved in DCM(6 ml), cooled to 0°C under an ice water bath, trifluoroacetic acid (2.0 ml) was added dropwise, and the mixture was reacted for 1.0 h at room temperature after addition. TLC showed the reaction was completed. The reaction solution was added with saturated sodium bicarbonate aqueous solution to adjusted to be weak alkalinity, stirred for 20min, extracted with dichloromethane, washed with saturated salt water, dried over anhydrous sodium sulfate, and concentrated, and the crude product was purified by Pre-TLC (DCM/MeOH = 10/1) to obtain C071-3(210 mg, yield 55%) as a white solid. ¹H NMR (400 MHz, DMSO_d6) : δ 1.32 (t, *J=* 7.2 Hz, 3H), 2.39 (m, 2H), 2.83 (t, *J* = 7.8 Hz, 2H), 3.94 (s, 3H), 4.31 (q, *J* = 7.2 Hz, 2H), 4.48 (s, 2H), 6.13 (t, *J* = 4.7 Hz, 1H), 6.88 (d, *J* = 7.6 Hz, 1H), 7.10 (t, *J* = 7.2 Hz, 1H), 7.18 (t, *J* = 7.6 Hz, 1H), 7.28 - 7.23 (m, 2H), 7.66 (d, *J* = 8.4 Hz, 1H), 8.01 (s, 1H). LCMS:m/z=344.1[M-NH₂]⁺

### Step 4:

C071-3(200 mg,0.56 mmol) was dissolved in acetonitrile (10 ml), DIPEA(286 mg,2.21 mmol) and 1H-pyrazol-1-formamidine hydrochloride (122 mg,0.83 mmol) were added, and the mixture was reacted at room temperature for 24 h. LCMS monitored that the reaction was completed. The reaction solution was filtered, and the filter cake was washed, and dried to obtain SYY-C071(151mg, yield 68%) as a white solid. ¹H NMR: (DMSO-*d₆*, 400MHz): δ1.29 (t, J = 7.2 Hz, 3H), 2.34-2.44 (m, 2H), 2.82 (t, J = 7.6 Hz, 2H), 3.89 (s, 3H), 4.32 (q, J = 7.2 Hz, 2H), 4.95 (s, 2H), 6.13 (t, J = 4.0 Hz, 1H), 6.89 (d, J = 7.6 Hz, 1H), 7.10 (t, J = 7.2 Hz, 1H), 7.17 (t, J = 7.2 Hz, 1H), 7.25 (d, J = 4.0 Hz, 2H), 7.31-7.90 (m, 5H), 8.00 (s, 1H). LCMS:m/z=403.2[M+H]⁺ (94.83% purity, 254 nm)

### Example 57: Preparation of Compound SYY-C072

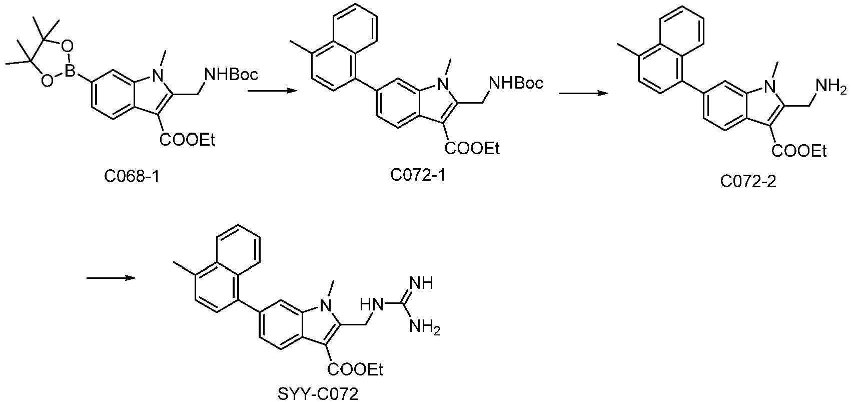

### Step 1:

Under the protection of nitrogen, C068-1(500 mg,1.09 mmol) was dissolved in DMF(10 ml), 1-bromo-4-methylnaphthalene (485 mg,2.20mmol), Pd(dppf)Cl₂ dichloromethane complex (80 mg,0.10 mmol) and Cs₂CO₃(712 mg,2.19 mmol) were added at room temperature, replaced with nitrogen for three times, and the mixture was heated to 85°C to react for 2.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=20/1) to obtain C072-1(324 mg, yield 63%) as a light yellow oil.

### Step 2:

C072-1(324 mg,0.69 mmol) was dissolved in DCM(6 ml), cooled under an ice water bath, trifluoroacetic acid (2.0 ml) was added dropwise at 0°C , and the mixture was reacted for 1.0 h at room temperature after addition. TLC showed the reaction was completed. The reaction solution was concentrated to dryness at low temperature, the crude product was diluted by adding 1.0 ml EA, saturated sodium bicarbonate aqueous solution was added dropwise to adjust to be weak alkalinity, a large amount of solid were precipitated, stirred for 20min, filtered, the filter cake was dissolved with dichloromethane, washed with saturated salt water, dried over anhydrous sodium sulfate, concentrated, the crude product was added with 2.0 ml ethyl acetate, stirred for 30min, and filtered by suction to obtain C072-2(140 mg, yield 62%) as a light yellow solid. LCMS:m/z=356.2[M-NH₂]⁺

### Step 3:

C072-2(140 mg,0.38 mmol) was dissolved in acetonitrile (15 ml), DIEA(195 mg,1.51 mmol) and 1H-pyrazol-1-formamidine hydrochloride (83 mg,0.57 mmol) were added, and the mixture was reacted at room temperature for 24 h. The reaction solution was filtered to obtain a solid (95 mg, yield 61%). ¹H NMR: (DMSO-*d₆*, 400MHz): δ1.42 (t, J = 7.2 Hz, 3H), 2.72 (s, 3H), 3.90 (s, 3H), 4.41 (q, J = 7.2 Hz, 2H), 5.00 (s, 2H), 7.29-7.66 (m, 8H), 7.72 (s, 1H), 7.85 (d, J = 8.8 Hz, 1H), 8.10-8.61 (m, 3H). LCMS:m/z=415.2[M +H]⁺ (96.38 % purity, 254 nm)

### Example 58: Preparation of Compound SYY-C073

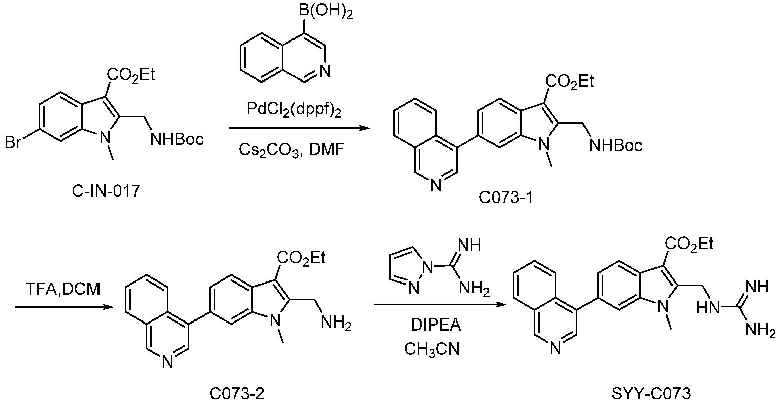

### Step 1:

C- IN-017(300 mg, 0.73mmol) and 4-isoquinoline boric acid (260 mg, 1.50mmol) were dissolved in DMF(10 ml), water (0.5ml) was added, Cs₂CO₃(714 mg, 2.19mmol) and PdCl₂(dppf) dichloromethane complex (50 mg, 0.061mmol) were added, and replaced with N₂ for three times, and the mixture was reacted at 100 °C for 2 h under the protection of N₂. TLC showed the reaction was completed. The reaction solution was washed with water, extracted with EA, washed with saturated salt water three times, dried, concentrated, and purified by silica gel column chromatography to obtain product C073-1(330 mg, yield 99%).

### Step 2:

C073-1(330 mg,0.72mmol) was dissolved in DCM(6 ml), then TFA(2 ml) was added, and the mixture was reacted at room temperature for 2 h. TLC showed the reaction was completed. The reaction solution was concentrated, neutralized with NaHCO₃ solution, solid was precipitated, filtered, and the filter cake was dissolved with DCM, dried over anhydrous Na₂SO₄, filtered, spin-dried, and purified by silica gel column chromatography to obtain product C073-2(250 mg, yield 97%).

### Step 3:

C073-2(150 mg, 0.42mmol) was dissolved in CH₃CN(5 ml), DIPEA(220 mg,1.70mmol) was added, stirred at room temperature for 20min, then pyrazole formamidine hydrochloride (92 mg,0.63 mmol) was added, and the mixture was stirred overnight at room temperature. LC-MS showed the raw materials were reacted completely. The reaction solution was filtered, and the filter cake was washed and dried to obtain C073-3 (102 mg, yield 61%) as a white solid. ¹H NMR: (DMSO-*d₆*, 400MHz): δ1.42 (t, J = 7.2 Hz, 3H), 3.93 (s, 3H), 4.41 (q, J = 7.2 Hz, 2H), 5.02 (s, 2H), 7.40-7.82 (m, 6H), 7.86 (s, 1H), 7.91 (d, J = 8.4 Hz, 1H), 8.19 (d, J = 8.4 Hz, 1H), 8.24 (d, J = 8.4 Hz, 1H), 8.52 (s, 1H), 8.57 (br, 1H), 9.37 (s, 1H). LC-MS: m/z=402.2 (98.02% purity, 254 nm)

### Example 59: Preparation of Compound SYY-C074

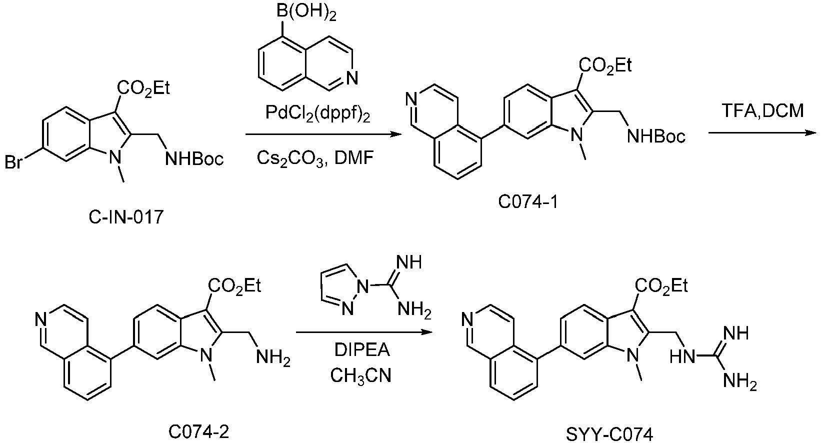

### Step 1:

C- IN-017(400 mg, 0.97mmol) and 5-isoquinoline boric acid (340 mg, 1.97mmol) were dissolved in DMF(10 ml), water (0.5ml), Cs₂CO₃(980 mg, 3.01mmol) and PdCl₂(dppf) dichloromethane complex (50 mg, 0.061 mmol) were added, replaced with N₂ for three times, and the mixture was heated to 100 °C to react for 2 h. TLC showed the reaction was completed. The reaction solution was added with water, extracted with EA, the organic phases were combined, washed with saturated salt water, dried over anhydrous sodium sulfate, and concentrated, and the crude product was purified by silica gel column chromatography to obtain C074-1(370 mg, yield 83%).

### Step 2:

C074-1(340 mg,0.74mmol) was dissolved in DCM(6 ml), TFA(2 ml) was added, and the mixture was reacted at room temperature for 2 h. TLC showed the reaction was completed. The reaction solution was concentrated, neutralized with saturated NaHCO₃ solution, a solid was precipitated, filtered, and the filter cake was dissolved with dichloromethane, dried over anhydrous Na₂SO₄, and concentrated, and crude product was purified by silica gel column chromatography to obtain C074-1(250 mg, yield 78%).

### Step 3:

C074-1(150 mg, 0.42mmol) was dissolved in CH₃CN(5 ml), DIPEA(220 mg,1.70mmol) was added, and the mixture was stirred at room temperature for 20min, then pyrazole formamidine hydrochloride (92 mg,0.63mmol) was added, stirred overnight at room temperature. LC-MS showed the raw materials were reacted completely. The reaction solution was filtered, and the filter cake was washed and dried to obtain SYY-C074(134mg, yield 80%). ¹H NMR: (DMSO-*d₆*, 400MHz): δ1.42 (t, J = 7.2 Hz, 3H), 3.92 (s, 3H), 4.41 (q, J = 7.2 Hz, 2H), 5.00 (s, 2H), 6.96-8.03 (m, 9H), 8.15-8.24 (m, 2H), 8.49 (d, J = 6.0 Hz, 1H), 9.42 (s, 1H). LCMS:m/z=402.2[M+H]⁺ (93.05% purity, 220 nm)

### Example 60: Preparation of Compound SYY-C075

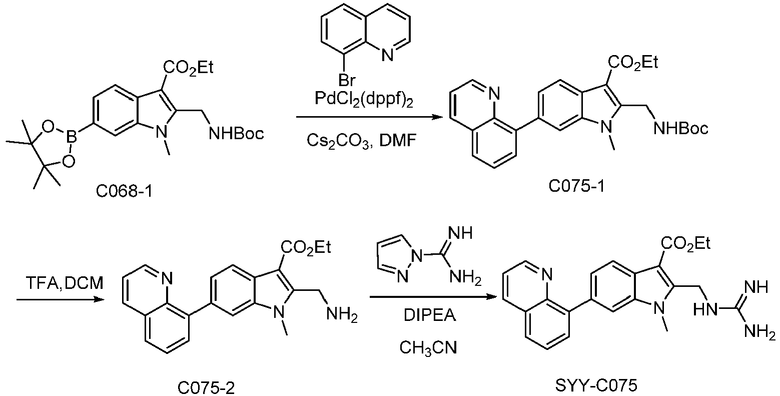

### Step 1:

C068-1(400 mg, 0.87mmol) and 8-bromoisoquinoline (340 mg, 1.63mmol) were dissolved in DMF(10 ml), water (0.5ml), Cs₂CO₃(980 mg, 3.01mmol) and PdCl₂(dppf) dichloromethane complex (50 mg, 0.061mmol) were added at room temperature, replaced with nitrogen for three times, and the mixture was heated to 100 °C to react for 2 h. TLC showed the reaction was completed. The reaction solution was washed, extracted with ethyl acetate, the organic phases were combined, washed with saturated salt water, dried over anhydrous sodium sulfate, and concentrated, and the crude product was purified by silica gel column chromatography to obtain C075-1(340 mg, yield 85%).

### Step 2:

C075-1(340 mg,0.74mmol) was dissolved in DCM(6 ml), TFA(2 ml) was added, and the mixture was reacted at room temperature for 2 h. TLC showed the reaction was completed. The reaction solution was concentrated, neutralized with saturated NaHCO₃ solution, a solid was precipitated, filtered, and the filter cake was dissolved with dichloromethane, dried over anhydrous Na₂SO₄, and concentrated, and the crude product was purified by silica gel column chromatography to obtain C075-2(240 mg, yield 90%).

### Step 3:

C075-2(150 mg, 0.42mmol) was dissolved in CH₃CN(5 ml), DIPEA(220 mg,1.70mmol) was added, and the mixture was stirred at room temperature for 20min, then pyrazole formamidine hydrochloride (92 mg,0.63 mmol) was added, and the mixture was stirred overnight at room temperature. The reaction solution was filtered, and the filter cake was washed, and dried to obtain SYY-C075(130mg, yield 77%) as a white solid. ¹H NMR: (DMSO-*d₆*, 400MHz): δ1.41 (t, J = 7.2 Hz, 3H), 3.89 (s, 3H), 4.39 (q, J = 7.2 Hz, 2H), 4.99 (s, 2H), 7.31-7.72 (m, 6H), 7.85 (dd, J = 7.2, 1.2 Hz, 1H), 7.88 (s, 1H), 8.01 (dd, J = 8.0, 1.2 Hz, 1H), 8.08 (d, J = 8.4 Hz, 1H), 8.36-8.48 (m, 2H), 8.90 (dd, J = 4.4, 2.0 Hz, 1H). LCMS:m/z=402.2[M+H]⁺ (95.38% purity, 254 nm)

### Example 61: Preparation of Compound SYY-C076

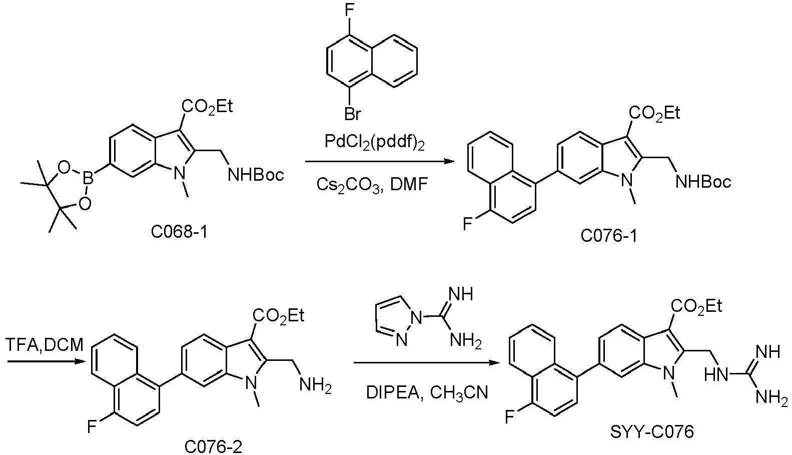

### Step 1:

C068-1(460 mg, 1.00 mmol) and 1-bromo-4-fluoronaphthalene (450 mg,2.01 mmol) were dissolved in DMF(10 ml), water (0.5ml), Cs₂CO₃(660 mg, 2.03 mmol) and PdCl₂(dppf) dichloromethane complex (90 mg, 0.11mmol) were added at room temperature, replaced with N₂ for three times, and the mixture was heated to 100 °C to react for 2 h. TLC showed the reaction was completed. The reaction solution was cooled to room temperature, extracted with ethyl acetate, the organic phases were combined, washed with saturated salt water, dried over anhydrous sodium sulfate, and concentrated, and the crude product was purified by silica gel column chromatography to obtain C076-1(420 mg, yield 88%).

### Step 2:

C076-1(420 mg,0.88 mmol) was dissolved in DCM(6 ml), TFA(3 ml) was added, and the mixture was reacted at room temperature for 2 h. TLC showed the reaction was completed. The reaction solution was concentrated, neutralized with saturated NaHCO₃ solution, the solid was precipitated, filtered, the filter cake was dissolved with dichloromethane, dried over anhydrous sodium sulfate, filtered, and concentrated, and the crude product was purified by silica gel column chromatography to obtain C076-2(240 mg, yield 72%).

### Step 3:

C076-2(150 mg, 0.40 mmol) was dissolved in CH₃CN(5 ml), DIPEA(220 mg,1.70 mmol) was added, stirred at room temperature for 20min, then pyrazole formamidine hydrochloride (92 mg,0.63 mmol) was added, and the mixture was stirred overnight at room temperature. LC-MS showed the raw materials were reacted completely. The reaction solution was filtered, and the filter cake was washed and dried to obtain SYY-C076(113 mg, yield 68%). ¹H NMR: (DMSO-*d₆*, 400MHz): δ1.41 (t, J = 7.2 Hz, 3H), 3.89 (s, 3H), 4.40 (q, J = 7.2 Hz, 2H), 4.99 (s, 2H), 7.18-7.67 (m, 8H), 7.76 (s, 1H), 7.86 (d, J = 8.8 Hz, 1H), 8.13-8.16 (m, 2H), 8.39 (br, 1H). LCMS:m/z=419.2[M+H]⁺ (97.63% purity, 254 nm)

### Example 62: Preparation of Compound SYY-C077

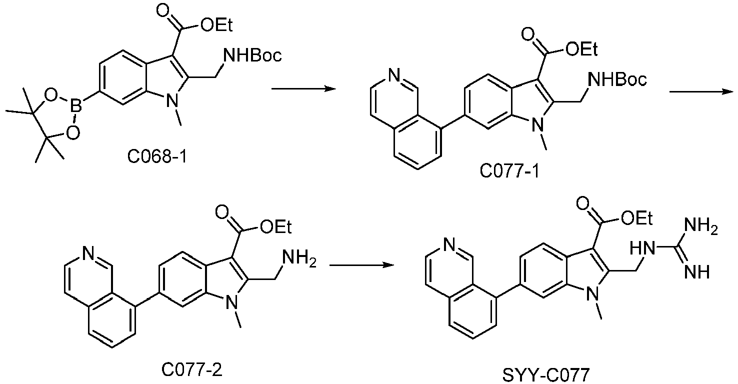

### Step 1:

C068-1(400mg,0.87mmol) and 8-bromoisoquinoline (271mg,1.29mmol) were dissolved in DMF(10mL) and water (1mL), cesium carbonate (567mg,1.74mmol) and PdCl2(dppf) dichloromethane complex (70mg,0.086mmol) were added, replaced with nitrogen for 3 times, and the mixture was heated to 100 °C to react for 3 h. TLC detection showed the reaction was completed. The reaction solution was cooled to room temperature, added with water, extracted with EA, the organic phases were combined, washed with saturated salt water, dried over anhydrous sodium sulfate, and concentrated, and the crude product was purified by silica gel column chromatography to obtain C077-1(300mg, yield 75%) as an off-white solid.

### Step 2:

C077-1(300mg,0.65mmol) was dissolved in dichloromethane (3mL), cooled to about 0 °C, a solution of trifluoroacetic acid (1mL) in dichloromethane (1mL) was added, and the mixture was stirred to react at 0 °C for 1h after addition. TLC detection showed the raw materials were reacted completely. The reaction solution was neutralized with saturated sodium carbonate aqueous solution, extracted with EA, the organic phases were combined, washed with saturated salt water, dried over anhydrous sodium sulfate, and concentrated to obtain C077-2(230mg, yield 98%) as a yellow solid. ¹H NMR (400 MHz, DMSO_d6) : δ 1.39(t, *J* = 7.2 Hz, 3H) , 3.08 (br, 2H), 3.88 (s, 3H) , 4.24 (s, 2H), 4.5 (q, *J* = 7.2 Hz, 2H) , 7.36 (dd, *J* = 8.2, 1.3 Hz, 1H) , 7.64 (d, *J* = 7.0 Hz, 1H) ,7.76 (s, 1H) , 7.82-7.85(m, 1H), 7.89 (d, *J* = 5.6 Hz, 1H), 7.98 (d, *J* = 8.2 Hz, 1H), 8.16 (d, *J* = 8.2 Hz, 1H), 8.53 (d, *J* = 5.7 Hz, 1H), 9.24 (s, 1H). LCMS:m/z=360.2[M +H]⁺

### Step 3:

C077-2(220mg,0.61mmol) and DIEA(315mg,2.44mmol) were dispersed in acetonitrile (5mL), pyrazole formamidine hydrochloride (135mg,0.92mmol) was added, and the mixture was stirred overnight at room temperature. TLC detection showed the raw materials did not completely react. An off-white solid (145mg,0.40mmol) was obtained by filtration of the reaction solution, and DIEA(208mg,1.61mmol) were dispersed in acetonitrile (5mL) and methanol (1mL), and stirred at room temperature for weekend. LCMS detection showed the reaction was completed. The reaction solution was filtered, and the filter cake was washed, and dried to obtain SYY-C077(100mg, yield 41%) as a white solid. ¹H NMR: (DMSO-*d₆*, 400MHz): δ1.42 (t, J = 7.2 Hz, 3H), 4.40 (q, J = 7.2 Hz, 2H), 3.91 (s, 3H), 4.99 (s, 2H), 7.02-7.91 (m, 8H), 7.93 (d, J = 6.0 Hz, 1H), 8.03 (d, J = 8.4 Hz, 1H), 8.18 (d, J = 8.4 Hz, 1H), 8.55 (d, J = 5.6 Hz, 1H), 9.21 (s, 1H). LCMS:m/z=402.2[M+H]⁺ (99.71% purity, 254 nm)

### Example 63: Preparation of Compound SYY-C078

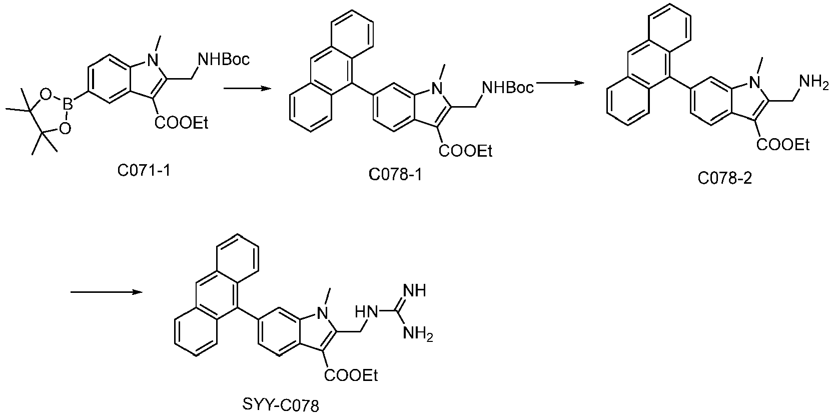

### Step 1:

Under the protection of nitrogen, C071-1(500 mg,1.09 mmol) was dissolved in DMF(10 ml), 9-bromoanthracene(561 mg,2.19 mmol), Pd(dppf)Cl₂ dichloromethane complex (80 mg,0.10 mmol) and Cs₂CO₃(712 mg,2.19 mmol) were added at room temperature, replaced with nitrogen for 3 times, and the mixture was heated to 85°C to react for 2.0 h. TLC showed the raw materials were reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=20/1) to obtain C078-1(366 mg, yield 66%) as a light yellow oil.

### Step 2:

Under the protection of nitrogen, the C078-1(366 mg,0.72 mmol) was dissolved in DCM(4.5 ml), cooled to 0°C under an ice water bath, trifluoroacetic acid (1.5 ml) was added dropwise, and the mixture was reacted for 1.0 h at room temperature after addition. TLC showed the reaction was completed. The reaction solution was concentrated at low temperature, the crude product was diluted with ethyl acetate (1.0 ml), saturated sodium bicarbonate aqueous solution was added dropwise to adjust to be weak alkalinity, a large amount of solids were precipitated, stirred for 20min, filtered, the filter cake was dissolved with dichloromethane, washed with saturated salt water, dried over anhydrous sodium sulfate, and concentrated, and the crude product was added with 2.0 ml ethyl acetate, stirred for 30min, filtered, and the filter cake was washed and dried to obtain C078-2(153 mg, yield 52%) as a light yellow solid.
LCMS: m/z=392.1[M-NH₂]⁺

### Step 3:

Under the protection of nitrogen, C078-2(105 mg,0.26 mmol) was dissolved in acetonitrile (10 ml), DIEA(133 mg,1.03 mmol) and 1H-pyrazol-1-formamidine hydrochloride (56 mg,0.38mmol) were added, and the mixture was reacted at room temperature for 48 h. LCMS detection showed that the raw materials were reacted completely. The reaction solution was filtered, and the filter cake was washed and dried to obtain SYY-C078(70 mg, yield 60 %). ¹H NMR: (DMSO-*d₆*, 400MHz): δ1.43 (t, J = 7.2 Hz, 3H), 3.87 (s, 3H), 4.42 (q, J = 7.2 Hz, 2H), 5.01 (s, 2H), 7.17-7.99 (m, 12H), 8.16 (d, J = 8.4 Hz, 2H), 8.25 (d, J = 8.0 Hz, 1H), 8.69 (s, 1H). LCMS:m/z=451.2[M+H]⁺ (96.72% purity, 220 nm)

### Example 64: Preparation of Compound SYY-C079

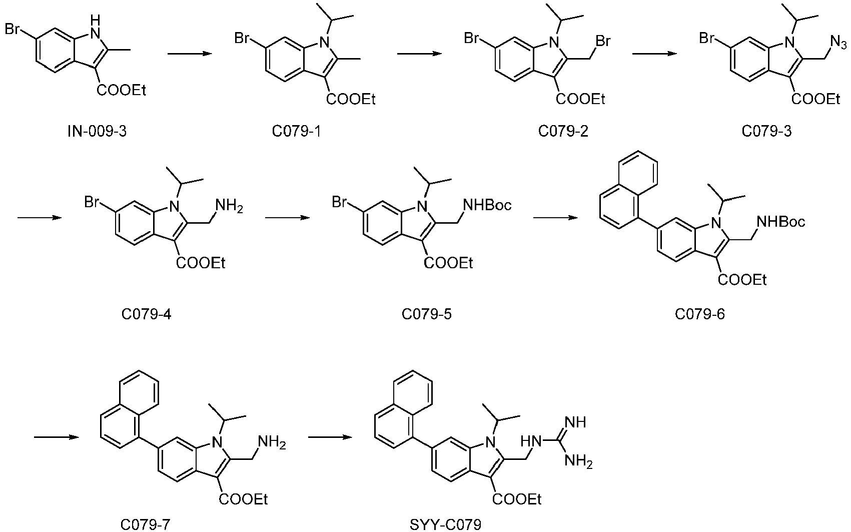

### Step 1:

Under the protection of nitrogen, IN-009-3(2.0g,7.12 mmol) was dissolved in DMF(20 ml), cooled to 0°C under an ice water bath, 60% NaH(850 mg,21.25 mmol) was added in batches, and the mixture was reacted at room temperature for 30min after addition. 2-Iodopropane (12g,70.60 mmol) was added dropwise at 0°C, and the mixture was reacted overnight at 40°C after addition. TLC monitored the reaction was completed. The reaction solution was quenched with water, a large amount of solid were precipitated, stirred for 20min, filtered, the filter cake was dissolved with ethyl acetate, dried over anhydrous sodium sulfate, and concentrated, and the crude product was purified by silica gel column chromatography to obtain C079-1(1.0g, yield 43%) as a brown-yellow solid. LCMS:m/z=324.0[M+H]⁺

### Step 2:

Under the protection of nitrogen, C079-1(874 mg,2.71 mmol) was dissolved in CCl₄ (20 ml), BPO(65 mg,0.27mmol) and NBS(576 mg,3.24 mmol) were added at room temperature, and the mixture was heated to 80°C to react for 1.5h. TLC monitored that the reaction was completed. The reaction was cooled to room temperature, and the crude product was purified by silica gel column chromatography (PE/EA = 10/1~5/1) to obtain C079-2(1.45g, crude) as a yellow oil.

### Step 3:

Under the protection of nitrogen, C079-2(1.45g, crude) was dissolved in DMF(20 ml), NaN₃(352 mg,5.41 mmol) was added, and the mixture was reacted overnight at room temperature. TLC monitored that the reaction was completed. The reaction solution was quenched with water, extracted with ethyl acetate. The organic phases were combined, washed with saturated salt water, dried over anhydrous sodium sulfate, and concentrated to obtain C079-3(1.1g, crude) as a brown-yellow solid.

### Step 4:

Under the protection of nitrogen, C079-3(1.1 g, crude) was dissolved in THF(21 ml) and water (7 ml), triphenylphosphine (1.18 g,4.50 mmol) was added at room temperature, and the mixture was heated to 50 °C to react for 2.0 h. TLC monitored that the reaction was completed. The reaction solution was cooled to room temperature, added with water, extracted with ethyl acetate, the organic phase was washed with saturated salt water, dried over anhydrous sodium sulfate, and concentrated, and the crude product was purified by silica gel column chromatography (DCM/MeOH = 60/1) to obtain C079-4(590 mg, yield for three steps: 64%) as a light yellow-brown oil. LCMS:m/z=339.1/341.1 [M +H]⁺

### Step 5:

Under the protection of nitrogen, C079-4(590mg,1.75mmol) was dissolved in DCM(10 ml), DIPEA(449 mg,3.47 mmol) was added, cooled to 0°C under an ice water bath, di-tert-butyl dicarbonate (417 mg,1.91 mmol) was added in batches, and the mixture was reacted at room temperature for 1.0 h after addition. TLC monitored that the raw materials were reacted completely. 1N diluted hydrochloric acid (10mL) was added to the reaction solution, extracted with dichloromethane, the organic phases were combined, washed with saturated salt water, dried over anhydrous sodium sulfate, and concentrated to obtain C079-5(843 mg, crude) as a yellow-brown oil.

### Step 6:

Under the protection of nitrogen, C079-5(843 mg,1.92 mmol) was dissolved in 1,4-dioxane (30 ml) and water (10 ml), boric acid (660 mg,3.84 mmol), Pd(dppf)Cl₂ dichloromethane complex (156 mg,0.19 mmol) and Na₂CO₃(610 mg,5.76 mmol) were added at room temperature, replaced with nitrogen for three times, and the mixture was heated to 95°C to react for 2.0 h. TLC showed the reaction was completed. The reaction solution was cooled to room temperature, added with water, extracted with ethyl acetate, the organic phases were combined, washed with saturated salt water, dried over anhydrous sodium sulfate and concentrated, and the residue was purified by silica gel column chromatography (PE/EA = 10/1) to obtain C079-6(800 mg, crude) as a yellow oil.

### Step 7:

Under the protection of nitrogen, the C079-6(800mg,1.65mmol) was dissolved in DCM(9 ml), cooled to 0°C under an ice water bath, trifluoroacetic acid (3.0 ml) was added dropwise, and the mixture was reacted for 1.0 h at room temperature after addition. TLC showed the raw materials were reacted completely. The reaction solution was added with saturated sodium bicarbonate aqueous solution to adjust to be weak alkalinity, stirred for 20min, extracted with dichloromethane, and the organic phases were combined, washed with saturated salt water, dried over anhydrous sodium sulfate, and concentrated to obtain C079-7(590 mg, crude) as a brownish yellow oil. LCMS:m/z=370.1[M-NH₂]⁺

### Step 8:

Under the protection of nitrogen, C079-7(360 mg,0.93 mmol) was dissolved in acetonitrile (10 ml), DIEA(481 mg,3.72 mmol) and 1H-pyrazol-1-formamidine hydrochloride (205 mg,1.40 mmol) were added, and the mixture was reacted at room temperature for 24 h. LCMS monitored that the reaction was completed. The reaction solution was filtered, and the filter cake was washed and dried to obtain SYY-C079(270 mg, yield four steps: 59%) as a light yellow solid. 1H NMR: (DMSO-d6, 400MHz): δ1.41 (t, J = 9.2 Hz, 3H), 1.62 (d, J = 7.2 Hz, 6H), 4.40 (q, J = 7.2 Hz, 2H), 4.84-4.96 (m, 1H), 5.02 (s, 2H), 7.27-7.75 (m, 8H), 7.80 (s, 1H), 7.86 (d, J = 8.8 Hz, 1H), 7.97 (d, J = 8.0 Hz, 1H), 8.02 (d, J = 8.0 Hz, 1H), 8.21 (d, J = 8.4 Hz, 1H), 8.34 (m, 1H). LCMS:m/z=429.2[M+H]⁺ (97.19% purity, 220 nm).

### Example 65: Preparation of Compound SYY-C080

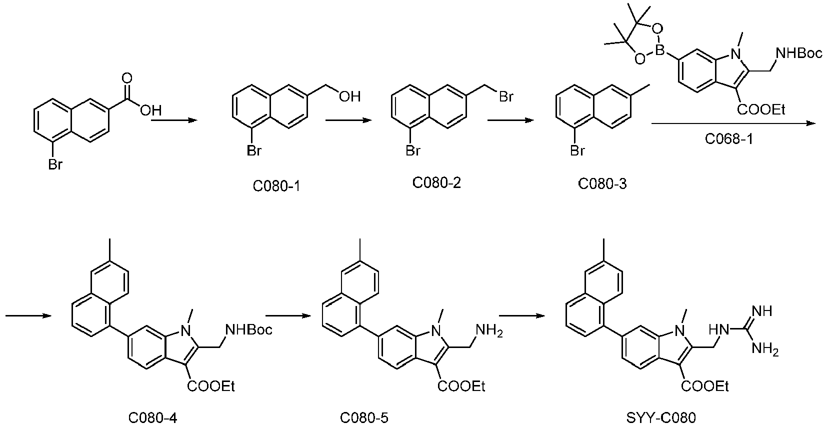

### Step 1:

5-Bromo-2-naphthoic acid (2.0g,8.00 mmol) was dispersed in THF(25 mL), cooled to about 0 °C, borane tetrahydrofuran complex (20 mL,20.00 mmol,1mol/L) was added dropwise under the protection of nitrogen, continued to react for half an hour at 0 °C after addition, and the mixture was returned to room temperature to react for 1h. TLC detection showed the amount of reaction was small, and heated to 75 °C to react for 1h. TLC detection showed that the reaction was completed. The reaction solution was cooled to 0 °C, methanol was added dropwise to quench the reaction, added with water, extracted with ethyl acetate, the organic phases were combined, washed with saturated salt water, dried over anhydrous sodium sulfate, and concentrated to obtain C080-1(1.5g, crude) as a white solid.

### Step 2:

Under the protection of nitrogen, the C080-1(1.5g, crude) was dissolved in DCM(60 ml), cooled under an ice water bath, phosphorus tribromide (2.28g,8.42 mmol, 5.0 ml, diluted with DCM) was added dropwise at 0°C, and the mixture was reacted for 0.5h at 0°C after addition. TLC showed the reaction was completed. The reaction solution was adjusted to be neutral by adding saturated sodium bicarbonate aqueous solution, liquid separation was carried out, the organic phases were washed with saturated salt water, dried over anhydrous sodium sulfate, and concentrated to obtain C080-2(1.6g, crude) as a white solid.

### Step 3:

Under the protection of nitrogen, C080-2(1.6g, crude) was dissolved in DMSO(20 ml). Sodium borohydride (407 mg,10.76 mmol) was added in batches at room temperature, and the mixture was reacted at room temperature for 0.5 h after addition. TLC showed the reaction was completed. The reaction solution was quenched with water, stirred at room temperature for 1h, and extracted with ethyl acetate. The organic phases were combined, washed with saturated salt water, dried over anhydrous sodium sulfate, and concentrated to obtain C080-3(1.14g, crude) as a light yellow solid. ¹H NMR (400 MHz, DMSO_d6) δ 2.48 (s, 3H), 7.37 (t, J = 8.0Hz, 1H), 7.49 (dd, *J* = 8.8, 1.6 Hz, 1H), 7.73 (s, 1H), 7.77 (d, *J* = 6.4 Hz, 1H), 7.85 (d, *J* = 8.0 Hz, 1H), 8.00 (d, *J* = 8.8 Hz, 1H)

### Step 4:

Under the protection of nitrogen, C068-1(300 mg,0.65mmol) was dissolved in DMF(10 ml), C080-3(290 mg,1.32 mmol), Pd(dppf)Cl₂ dichloromethane complex (54 mg,0.066 mmol) and Cs₂CO₃(427 mg,1.31 mmol) were added at room temperature, replaced with nitrogen for 3 times, and the mixture was heated to 80°C to react for 3.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=10/1) to obtain C080-4(203 mg, yield for four steps: 43%) as a light yellow oil.

### Step 5:

Under the protection of nitrogen, C080-4(200 mg,0.42 mmol) was dissolved in DCM(3 ml), cooled under an ice water bath, trifluoroacetic acid (1.0 ml) was added dropwise at 0°C , and the mixture was reacted for 1.0 h at room temperature after addition. TLC showed the reaction was completed. The reaction solution was added with sodium bicarbonate aqueous solution to adjust to be weak alkalinity, stirred for 20min, extracted with DCM, and the organic phases were combined, washed with saturated salt water, dried over anhydrous sodium sulfate, and concentrated to obtain C080-5(147 mg, crude) as a light yellow oil. ¹H NMR (400 MHz, DMSO_d6) δ 1.40 (t, *J* = 7.2 Hz,3H), 2.04 (br, 2H),2.48 (s, 3H), 3.85 (s, 3H), 4.23 (s, 2H), 4.34 (q, *J* = 7.2 Hz, 2H), 7.28-7.32 (m, 2H), 7.42 (d, *J* = 7.0 Hz, 1H), 7.57-7.51 (m, 1H), 7.64 (s, 1H), 7.76-7.78 (m, 2H), 7.85 (d, *J* = 8.1 Hz, 1H), 8.11 (d, *J* = 8.2 Hz, 1H). LCMS:m/z=356.1[M-NH₂]⁺

### Step 6:

Under the protection of nitrogen, C080-5(147 mg,0.39 mmol) was dissolved in acetonitrile (10 ml), DIEA(204mg,1.58 mmol) and 1H-pyrazol-1-formamidine hydrochloride (87 mg,0.59mmol) were added, and the mixture was reacted at room temperature for 48 h. LCMS monitored that the reaction was completed. The reaction solution was filtered, and the filter cake was washed and dried to obtain SYY-C080(120 mg, yield for two steps: 68%) as a light yellow solid. ¹H NMR: (DMSO-*d₆*, 400MHz): δ1.42 (t, J = 7.2 Hz, 3H), 2.49 (s, 3H), 3.90 (s, 3H), 4.40 (q, J = 7.2 Hz, 2H), 5.00 (s, 2H), 7.31-7.78 (m, 11H), 7.87 (d, J = 8.4 Hz, 1H), 8.14 (d, J = 8.8 Hz, 1H). LCMS:m/z=415.2[M+H]⁺ (95.40% purity, 220 nm)

### Example 66: Preparation of Compound SYY-C081

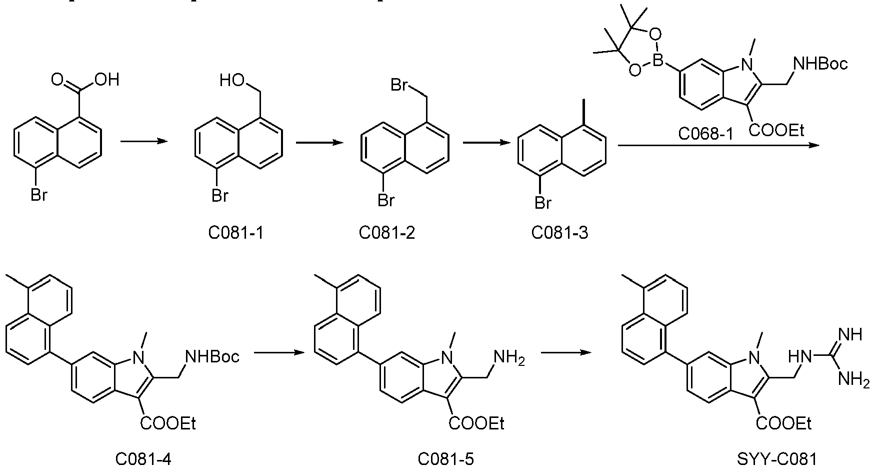

### Step 1:

Under the protection of nitrogen, 5-bromo-1-naphthoic acid (2.0g,7.97 mmol) was dissolved in THF(25 ml), replaced with nitrogen for three times, cooled under an ice water bath, a solution of borane in THF (20 ml,20.00mmol,1.0 M) was added dropwise at 0°C, and the mixture was reacted overnight at room temperature after the addition. TLC showed the reaction was completed. The reaction solution was quenched by adding methanol at low temperature, stirred for 20min, the reaction solution was concentrated to dryness, added with water, extracted with ethyl acetate, and the organic phases were combined, washed with saturated salt water, dried over anhydrous sodium sulfate, and concentrated to obtain C081-1(1.9g, crude) as a white solid. ¹H NMR (400 MHz, CDCl₃): δ1.88 (s, 1H), 5.17 (d, *J* = 2.8 Hz, 2H), 7.39-7.43 (m, 1H), 7.56 - 7.61 (m, 2H), 7.85 (dd, *J* = 7.6, 0.8 Hz, 1H), 8.13 (d, *J* = 8.4 Hz, 1H), 8.27 (dd, *J* = 7.6, 1.2 Hz, 1H)

### Step 2:

Under the protection of nitrogen, C081-1(1.9g,8.01mmol) was dissolved in dichloromethane (60 ml), cooled under an ice water bath, phosphorus tribromide (2.16g,7.98mmol, diluted with 5.0 ml DCM) was added dropwise at 0°C, and the mixture was reacted for 0.5h at 0°C after addition. TLC showed the reaction was completed. The reaction solution was adjusted to be neutral by adding saturated sodium bicarbonate aqueous solution, extracted with dichloromethane, the organic phases were combined, washed with saturated salt water, dried over anhydrous sodium sulfate, and concentrated to obtain C081-2(1.55g, crude) as a white solid.

### Step 3:

Under the protection of nitrogen, C081-2(1.55 g, 5.17mmol) was dissolved in DMSO(20 ml). Sodium borohydride (394 mg,10.42 mmol) was added in batches at room temperature, and the mixture was reacted at room temperature for 0.5 h after addition. TLC showed the reaction was completed. The reaction solution was quenched with water, stirred at room temperature for 1h, and extracted with ethyl acetate. The organic phases were combined, washed with saturated salt water, dried over anhydrous sodium sulfate, and concentrated to obtain C081-3(1.0 g, crude) as a light yellow solid. ¹H NMR (400 MHz, CDCl₃) δ 2.74 (s, 3H), 7.36-7.42 (m, 2H), 7.49-7.53 (m, 1H), 7.83 (dd, *J* = 7.2, 0.8 Hz, 1H), 8.01 (d, *J* = 8.4 Hz, 1H), 8.18 (d, *J* = 8.4Hz, 1H)

### Step 4:

Under the protection of nitrogen, C068-1(400 mg,0.87 mmol) was dissolved in DMF(10 ml), C081-3(386 mg,1.75 mmol), Pd(dppf)Cl₂ dichloromethane complex (72 mg,0.09 mmol) and Cs₂CO₃(569 mg,1.75 mmol) were added, replaced with nitrogen for three times, and the mixture was heated to 80°C to react for 3.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA= 10/1) to obtain C081-4(281 mg, yield for four steps: 68%) as a white solid.

### Step 5:

Under the protection of nitrogen, C081-4(281 mg,0.59 mmol) was dissolved in DCM(3 ml), cooled under an ice water bath, trifluoroacetic acid (1.0 ml) was added dropwise at 0°C, and the mixture was reacted for 1.0 h at room temperature after addition. TLC showed the reaction was completed. The reaction solution was added with saturated sodium bicarbonate aqueous solution to adjust to be weak alkalinity, stirred for 20min, extracted with dichloromethane, and the organic phases were combined, washed with saturated salt water, dried over anhydrous sodium sulfate, and concentrated to obtain C081-5(220 mg, crude) as a light yellow oil. ¹H NMR (400 MHz, DMSO_d6) δ 1.40 (t, *J* = 7.2 Hz, 3H), 1.92 (br, 2H), 2.71 (s, 3H), 3.86 (s, 3H), 4.22 (s, 2H), 4.34 (q, *J* = 7.2 Hz, 2H), 7.27 (dd, *J* = 8.0, 12 Hz, 1H), 7.32 - 7.39 (m, 2H), 7.50 (d, *J* = 6.0 Hz, 1H), 7.61-7.65 (m, 2H), 7.70 (d, *J* = 8.4 Hz, 1H), 8.06 (d, *J* = 8.4 Hz, 1H), 8.11 (d, *J* = 8.0 Hz, 1H) LCMS:m/z=356.2[M-NH₂]⁺

### Step 6:

Under the protection of nitrogen, C081-5(220 mg,0.59 mmol) was dissolved in acetonitrile (10 ml), DIEA(381mg,2.95 mmol) and 1H-pyrazol-1-formamidine hydrochloride (174 mg,1.19 mmol) were added, and the mixture was reacted at room temperature for 24 h. LCMS monitored that the raw materials were reacted completely. The reaction solution was filtered, and the filter cake was washed and dried to obtain SYY-C081(170 mg, yield for two steps: 69%) as a light yellow solid. ¹H NMR: (DMSO-*d₆*, 400MHz): δ1.42 (t, J = 7.2 Hz, 3H), 2.72 (s, 3H), 3.90 (s, 3H), 4.40 (q, J = 7.2 Hz, 2H), 5.00 (s, 2H), 7.17-7.98 (m, 11H), 8.08 (d, J = 8.4 Hz, 1H), 8.14 (d, J = 8.0 Hz, 1H). LCMS:m/z=415.2[M+H]⁺ (98.55% purity, 220 nm)

### Example 67: Preparation of Compound SYY-C082

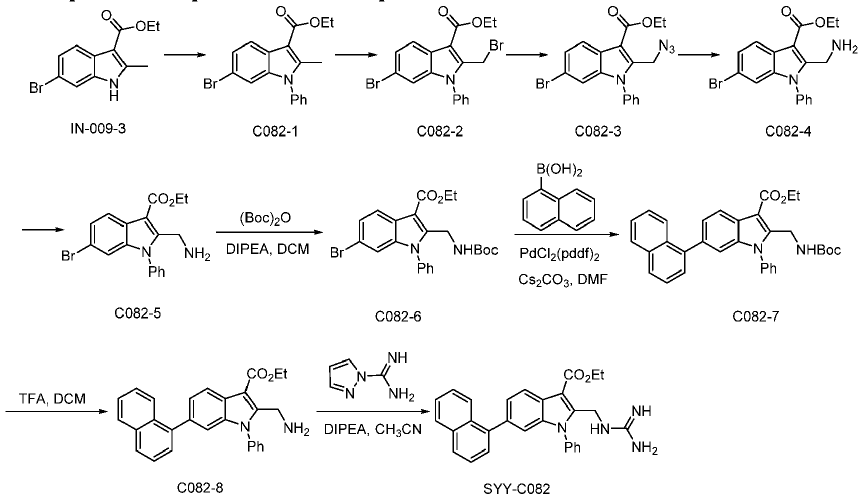

### Step 1:

IN-009-3(6.0g,21.27 mmol) and phenylboric acid (3.0g,25.52mmol) were dissolved in dichloromethane (60 mL), copper acetate (5.5g,31.91mmol), DMAP(3.7g,31.91mmol) and triethylamine (3.1g,31.91 mmol) were added in sequence, replaced with nitrogen for 3 times and the mixture was reacted overnight at room temperature. New spots were produced in TLC detection, and a lot of raw materials were not completely reacted. The reaction solution was quenched with 1N hydrochloric acid solution, extracted with dichloromethane, the organic phases were combined, washed with saturated salt water, dried over anhydrous sodium sulfate, and concentrated, and the crude product was purified by silica gel column chromatography to obtain C082-1(1.5g, crude) as a brown-yellow oil.

### Step 2:

C082-1(1.5g, crude), NBS(754mg,4.23mmol) and BPO(50mg,0.21 mmol) were dispersed into carbon tetrachloride (30 mL) and heated to 80 °C to react for 2 h. The reaction solution was cooled to room temperature and directly purified by silica gel column chromatography to obtain C082-2(1.6g, crude) as a brown-yellow oil.

### Step 3:

C082-2(1.6g, crude) was dissolved in DMF(15mL), sodium azide (277mg,4.27mmol) was added at room temperature, the reaction solution was heated to 60 °C to react for 2 h. TLC detected the reaction was completed. The reaction solution was cooled to room temperature, added with water, extracted with ethyl acetate, the organic phases were combined, washed with saturated salt water, dried over anhydrous sodium sulfate, and concentrated to obtain C082-3(1.4 g, crude) as a brownish-yellow oil.

### Step 4:

C082-3(1.4g, crude) and triphenylphosphine (1.85g,7.0 mmol) were dispersed into a mixed solvent of THF(50mL) and water (5mL), and heated to 80 °C to react for 3 h. TLC showed the reaction was completed. The reaction solution was cooled to room temperature, 1N diluted hydrochloric acid/EA was added, extracted and separated, the organic layer was discarded, sodium hydroxide was added to adjust the water layer to be alkaline, extracted with EA, the organic phases were combined, washed with saturated salt water, dried over anhydrous sodium sulfate, and concentrated to obtain C082-4(400 mg, yield for four steps: 5%) as a light yellow solid.

### Step 5:

C082-4(400 mg, 1.07 mmol) was dissolved in DCM(10 ml), DIPEA(420 mg, 3.21 mmol) was added, stirred under an ice bath for 20min, then (Boc)₂O(281 mg,1.28 mmol) was slowly added dropwise, and the mixture was stirred for 2 h at room temperature. TLC showed the reaction was completed. The reaction solution was added with 1N diluted hydrochloric acid, extracted with dichloromethane, the organic phases were combined, washed with saturated salt water, dried over anhydrous sodium sulfate, and concentrated to obtain C082-5(380 mg, crude), which was directly used in the next step.

### Step 6:

C082-5(380 mg, crude) and boric acid (210 mg,1.20 mmol) were dissolved in DMF(10 ml), water (0.5ml), Cs₂CO₃(530 mg,1.60 mmol) and PdCl₂(dppf)(70 mg, 0.08 mmol) were added, replaced with N₂ for three times, and the mixture was heated to 100 °C to reflux for 2 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C082-6(460 mg, yield for two steps: 91%).

### Step 7:

C082-6(460 mg,0.88 mmol) was dissolved in DCM(6 ml), TFA(3 ml) was added, and the mixture was reacted at room temperature for 2 h. TLC showed the reaction was completed. The reaction solution was concentrated, saturated NaHCO₃ solution was added for neutralization, extracted with ethyl acetate, the organic phases were combined, washed with saturated salt water, dried over anhydrous sodium sulfate, and concentrated, and the crude product was purified by silica gel column chromatography to obtain C082-7(210 mg, yield 56%).

### Step 8:

C082-7(200 mg, 0.476 mmol) was dissolved in CH₃CN(5 ml), DIPEA(250 mg,1.904 mmol) was added, stirred at room temperature for 20min, then 1H-pyrazol-1-formamidine hydrochloride (105 mg,0.714 mmol) was added, stirred overnight at room temperature. LCMS showed the raw materials were reacted completely. The reaction solution was filtered, and the filter cake was washed and dried to obtain SYY-C082(160 mg, yield 73%). LC-MS:m/z= 463.2 [M+H]⁺,1H NMR: (DMSO-d6, 400MHz): δ1.42 (t, J = 9.2 Hz, 3H), 4.42 (q, J = 7.2 Hz, 2H), 4.60 (s, 2H), 7.01(s, 1H), 7.40-7.64 (m, 13H), 7.78 (d, J = 8.0Hz, 1H), 7.92 (d, J = 8.8 Hz, 1H), 7.97 (d, J = 8.0 Hz, 1H), 8.28 (d, J = 8.4 Hz, 1H).

### Example 68: Preparation of Compound SYY-C083

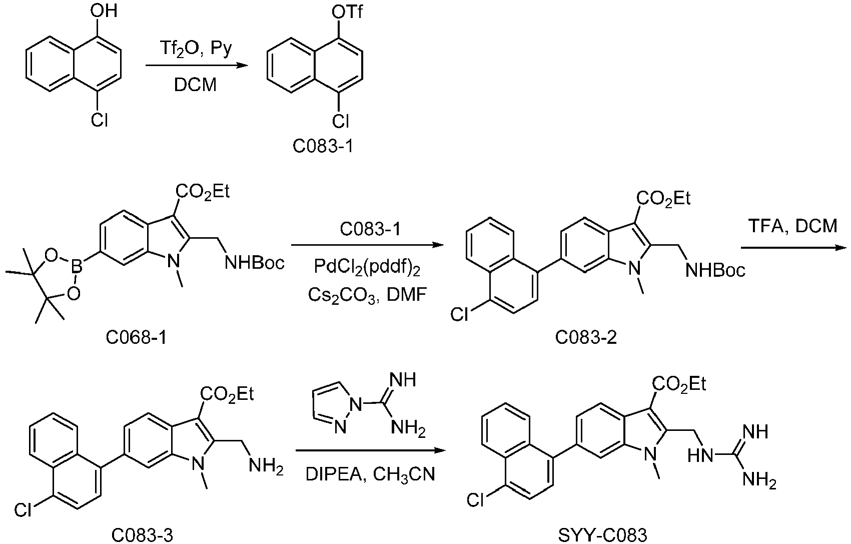

### Step 1:

4-Chloro-1-naphthol (1.0g,5.6 mmol) was dissolved in DCM(15 ml), pyridine (1.3g, 16.8 mmol) was added at room temperature, a solution of Tf₂O(1.9g,6.7 mmol) in DCM(5 ml) was added dropwise under an ice bath, and the mixture was reacted for 1h under an ice bath after addition. TLC showed the reaction was completed. The reaction solution was neutralized with 1M HCl solution, extracted with dichloromethane, the organic phases were combined, washed with saturated salt water, dried over anhydrous sodium sulfate, and concentrated to obtain C083-1(1.8g, crude), which was directly used in the next step.

### Step 2:

C068-1(300 mg,0.65 mmol) and C083-1(400 mg, crude) were dissolved in DMF(10 ml), H₂O(1 ml), Cs₂CO₃(427 mg,1.30 mmol) and PdCl₂(dppf)(60 mg,0.065 mmol) were added, replaced with N₂ for three times, and the mixture was heated to 100°C to react for 2 h under the protection of N₂. TLC showed the reaction was completed. The reaction solution was cooled to room temperature, quenched with water, extracted with ethyl acetate, the organic phases were combined, washed with water, and washed with saturated salt water, dried over anhydrous sodium sulfate, and concentrated, and the crude product was separated and purified by silica gel column chromatography to obtain C083-3(240 mg, yield 75%).

### Step 3:

C083-2(240 mg,0.49 mmol) was dissolved in DCM(5 ml), TFA(2 ml) was added, and the mixture was reacted at room temperature for 2 h. TLC showed the reaction was completed. The reaction solution was concentrated, neutralized with saturated NaHCO₃ solution, extracted with dichloromethane, the organic phases were combined, washed with saturated salt water, dried over anhydrous sodium sulfate, and concentrated to obtain C083-4(170 mg, crude).

### Step 4:

C083-3(170 mg, crude) was dissolved in CH₃CN(5 ml), DIPEA(200 mg,1.72 mmol) was added, stirred at room temperature for 20min, then 1H-pyrazol-1-formamidine hydrochloride (85 mg,0.65 mmol) was added, and the mixture was stirred overnight at room temperature. LC-MS showed the raw materials were reacted completely. The reaction solution was filtered, and the filter cake was washed and dried to obtain SYY-C083(116 mg, yield for two steps: 55%). ¹H NMR: (DMSO-*d₆*, 400MHz): 1.41 (t, J = 7.2 Hz, 3H), 3.90 (s, 3H), 4.40 (d, J = 7.2 Hz, 2H), 5.00 (s, 2H), 7.17-8.00 (m, 11H), 8.15 (d, J = 8.4 Hz, 1H), 8.29 (d, J = 8.4 Hz, 1H). LC-MS:m/z= 435.1 [M+H]⁺ (95.72% purity, 254 nm)

### Example 69: Preparation of Compound SYY-C084

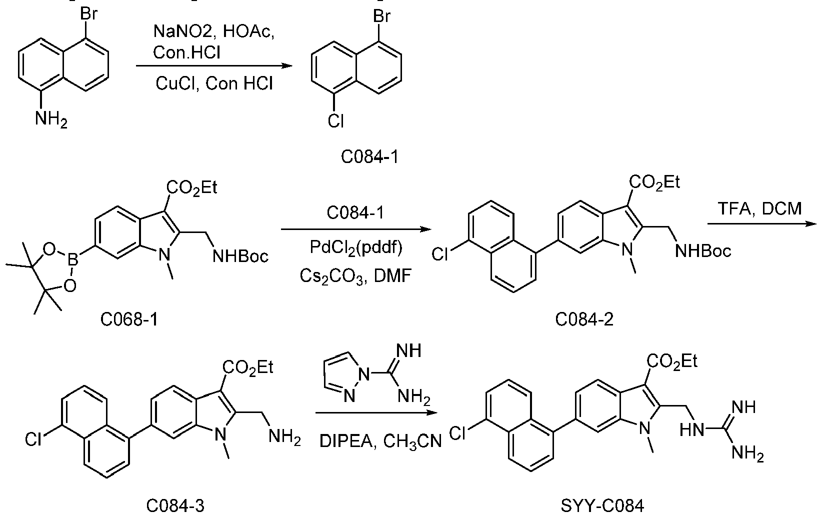

### Step 1:

1-Amino-5-bromnaphthalene (500mg,2.24mmol) was dispersed in acetic acid (5 mL), water (2 mL) and hydrochloric acid (1 mL), cooled to 0 °C, a solution of sodium nitrite(185 mg,2.69 mmol) in water (2 mL) was added dropwise under stirring, and stirred at 0 °C for half an hour after addition. 1/3 volume of diazonium salt solution was taken, treated with cuprous chloride (488mg, 4.92mmol) and concentrated hydrochloric acid with heating to 70 °C. TLC detection showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C084-1(150 mg) as a white solid. HNMR(CDCl₃, 400Hz): δ8.28 (d, J=8.4 Hz, 1H), 8.21 (d, J=8.8 Hz, 1H), 7.85 (dd, J1=0.8, J2=7.6 Hz 1H), 7.64 (dd, J1=0.8, J2=7.6 Hz 1H), 7.50 (d, J=7.6 Hz, 1H), 7.43 (d, J=7.6 Hz, 1H).

### Step 2:

C068-1(484 mg, 1.06 mmol) and C084-1(260 mg, 1.06 mmol) were dissolved in DMF(10 ml), water (1 ml), Cs₂CO₃(690 mg, 2.12 mmol) and PdCl₂(dppf)(90 mg,0.1 mmol) were added, replaced with N₂ for three times, and the mixture was heated to 100 °C to reflux for 2 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C084-2(420 mg, yield 81%).

### Step 3:

C084-2(420 mg,0.85 mmol) was dissolved in DCM(6 ml), TFA(3 ml) was added, and the mixture was reacted at room temperature for 2 h. TLC showed the reaction was completed. The reaction solution was concentrated, neutralized with saturated NaHCO₃ solution, a solid was precipitated, and the filter cake was dissolved with dichloromethane, dried over anhydrous sodium sulfate, filtered, and concentrated to obtain C084-3(240 mg, crude). HNMR(DMSO-d6, 400Hz):68.26 (d, J=8.4 Hz, 1H), 8.12 (d, J=8.4 Hz, 1H), 7.83 (d, J=8.4 Hz 1H), 7.73-7.79 (br, 2H), 7.69 (s, 1H), 7.62 (d, J=6.8 Hz, 1H) , 7.45 (t, J=8.0 Hz, 1H), 7.29 (d, J=6.8 Hz, 1H), 4.35 (q, J=7.2 Hz, 2H), 4.26 (s, 2H), 3.87 (s, 3H), 1.40 (t, J=7.2 Hz, 3H). LC-MS:m/z= 376.1 [M-NH₂]⁺

### Step 4:

C084-3(240 mg, 0.61 mmol) was dissolved in CH₃CN(5 ml), DIPEA(320 mg,2.44 mmol) was added, and the mixture was stirred at room temperature for 20min, then 1H-pyrazol-1-formamidine hydrochloride (135 mg,0.92 mmol) was added, and the mixture was stirred overnight at room temperature. LC-MS showed the raw materials were reacted completely. The reaction solution was filtered, and the filter cake was washed and dried to obtain SYY-C084(198 mg, yield for two steps: 54%). ¹H NMR: (DMSO-*d₆*, 400MHz): δ1.42 (t, J = 7.2 Hz, 3H), 3.91 (s, 3H), 4.41 (q, J = 7.2 Hz, 2H), 5.00 (br, 2H), 7.37 (dd, J = 8.0, 1.2 Hz, 1H), 7.45-7.83 (m, 9H), 8.16 (d, J = 8.4 Hz, 1H), 8.28 (d, J = 8.8 Hz, 1H), 8.44 (br, 1H). LC-MS:m/z= 435.1 [M+H]⁺ (92.03% purity, 254 nm)

### Example 70: Preparation of Compound SYY-C085

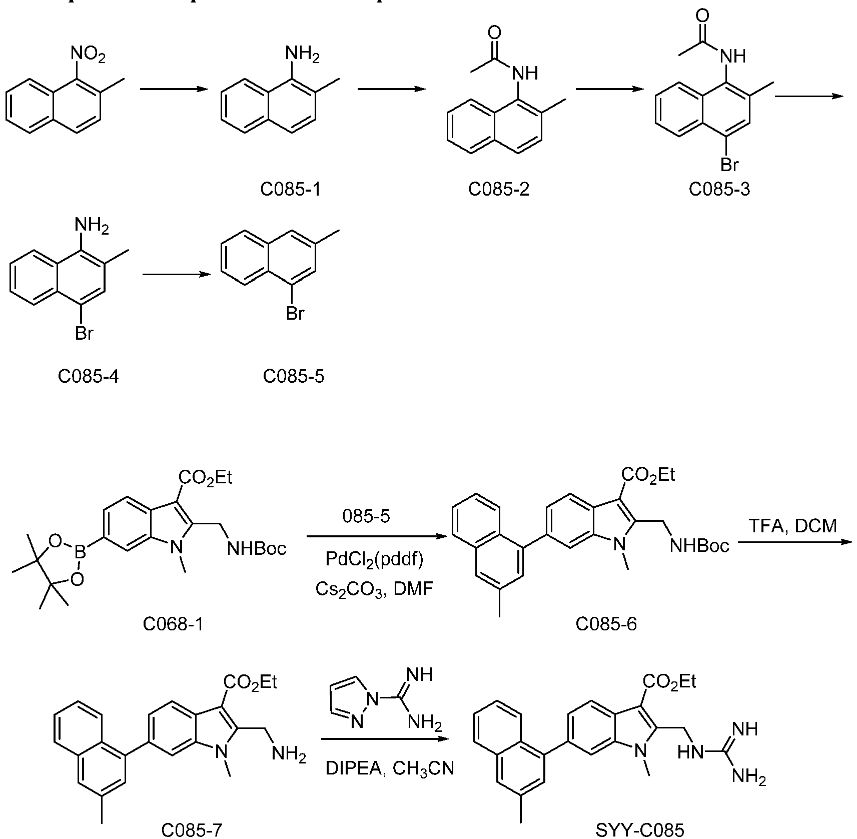

### Step 1:

2-Methyl-dinitronaphthol (8.0g,42.78 mmol) was dispersed in a mixed solvent of ethanol (210 mL) and water (50 mL), ammonium chloride solid (11.5g,213.90 mmol) was added, the system was heated to about 80 °C, iron powder (12.0g,213.9 mmol) was added in batches, and the mixture was refluxed to react for 2 h. TLC detection showed the reaction was completed. The reaction solution was filtered while hot, the filtrate was concentrated to a small amount, added with water, extracted with ethyl acetate, and the organic phases were combined, washed with saturated salt water, dried over anhydrous sodium sulfate, and concentrated to obtain C085-1(7.5g, crude) as a brown solid.

### Step 2:

C085-1(7.5g, crude) was dissolved in dichloromethane (100mL), pyridine (10.0g,128.34 mmol) was added, cooled to about 0 °C under the protection of nitrogen, acetic anhydride (6.5g,64.17 mmol) was added dropwise under stirring, returned to room temperature and stirred overnight after addition. TLC detection showed the reaction was completed. Solid in the reaction solution was precipitated, filtered, and the filter cake was washed and dried to obtain white solid (2.2g). The mother liquor was slurried with a mixture of MTBE and DCM to obtain C085-2(3.2g, crude) as a white solid. HNMR, (DMSO-d6, 400Hz):9.70 (s, 1H), 7.88 (t, J=7.8 Hz, 1H), 7.77 (d, J=8.4 Hz, 1H), 7.44-7.53 (br, 2H), 7.42 (d, J=8.4 Hz, 1H), 2.31 (s, 3H) , 2.18 (s, 3H).

### Step 3:

C085-2(1.0g, crude) was dissolved in acetic acid (15 mL), a solution of bromine (0.27 mL,5.33 mmol) in acetic acid (3 mL) was added dropwise at room temperature, and the mixture was stirred at room temperature for 16 h after addition. TLC detection showed that the raw material was completely reacted. The reaction solution was added with water, neutralized with saturated sodium carbonate solution, extracted with ethyl acetate. The organic phases were combined, washed with saturated salt water, dried over anhydrous sodium sulfate, and concentrated to obtain C085-3(1.4g, crude) as an off-white solid. HNMR(DMSO-d6, 400Hz):δ 9.80 (s, 1H), 8.08-8.11 (m, 1H), 7.93-7.96 (m, 1H), 7.85 (s, 1H), 7.62-7.65 (m, 2H), 2.31 (s, 3H) , 2.18 (s, 3H).

### Step 4:

C085-3(2.8g, crude) was dissolved in ethylene glycol (30 mL), potassium hydroxide (1.7g,30.55 mmol) was added, and the reaction solution was heated to reflux to react for 7h. TLC detection showed that some raw materials were remained. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C085-4(700 mg, yield for four steps: 19%) as a light red brown solid. HNMR(CDCl3, 400Hz):δ 8.15-8.18 (m, 1H), 7.77-7.80 (m, 1H), 7.46-7.54 (m, 3H), 4.12 (br, 2H) , 2.32 (s, 3H).

### Step 5:

C085-4(100 mg,0.43mmol) was dispersed in acetic acid (2 mL) and hydrochloric acid (0.2 mL), cooled to about 0 °C, sodium nitrite (32 mg,0.47 mmol) aqueous solution was added dropwise, and the mixture was reacted at 0 °C for 1h after addition. Diazonium salt solution was poured into hypophosphorous acid aqueous solution (5mL,50%), and reacted at room temperature for 18 h. TLC detection showed the reaction was completed. The reaction solution was poured into ice water, extracted with PE/EA, the organic phases were combined, washed with saturated salt water, dried over anhydrous sodium sulfate, and concentrated, and the crude product was purified by silica gel column chromatography to obtain C085-5(85 mg, yield 91%) as a colorless oil. HNMR, (CDCl3, 400Hz):δ8.21 (d, J=8.0 Hz, 1H), 7.76-7.78 (br, 1H), 7.68 (s, 1H), 7.61 (s, 1H) , 7.50-7.58(br, 2H), 2.52 (s, 3H).

### Step 6:

C068-1(300 mg, 0.654 mmol) and C085-5(180 mg,0.785 mmol) were dissolved in DMF(10 ml), water (1 ml), Cs₂CO₃(256 mg, 1.308 mmol) and PdCl₂(dppf)(60 mg,0.065 mmol) were added, replaced with N₂ for three times, and the mixture was heated to 100 °C to reflux for 2 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was separated and purified by silica gel column chromatography to obtain C085-6(270 mg, yield 87%) as a white solid.

### Step 7:

C085-6(270 mg,0.57 mmol) was dissolved in DCM(5 ml), TFA(3 ml) was added, and the mixture was reacted at room temperature for 2 h. TLC showed the reaction was completed. The reaction solution was concentrated, neutralized with saturated NaHCO₃ solution, a solid was precipitated, filtered, and the filter cake was dissolved with dichloromethane, dried over anhydrous sodium sulfate, and concentrated to obtain C085-7(260 mg, crude). HNMR,(DMSO-d6, 400Hz):δ 8.33 (s, 3H), 8.17 (d, J=8.0 Hz, 1H), 7.92 (d, J=8.0 Hz, 1H), 7.73-7.77 (br, 3H), 7.50 (t, J=8.0 Hz, 1H), 7.37-7.41 (br, 3H), 4.64 (s, 2H), 4.41 (q, J=7.2 Hz, 2H), 3.93 (s, 3H) , 2.54 (s, 3H), 1.43 (t, J=7.2 Hz, 3H). LC-MS:m/z= 357.2 [M-NH₂]⁺

### Step 8:

C085-7(100 mg, crude) was dissolved in CH₃CN(5 ml), DIPEA(320 mg,0.805 mmol) was added, and the mixture was stirred at room temperature for 20min, then 1H-pyrazol-1-formamidine hydrochloride (135 mg,0.402 mmol) was added, and the mixture was stirred overnight at room temperature. LC-MS showed the raw materials were reacted completely. The reaction solution was filtered, and the filter cake was washed and dried to obtain SYY-C085(62 mg, yield for two steps: 56%). ¹H NMR: (DMSO-*d₆*, 400MHz): δ1.42 (t, J = 7.2 Hz, 3H), 2.53 (s, 3H), 3.92 (s, 3H), 4.40 (q, J = 7.2 Hz, 2H), 5.03 (s, 2H), 7.34-7.39 (m, 3H), 7.47-7.78 (m, 7H), 7.90 (d, J = 8.0 Hz, 1H), 8.14 (d, J = 8.0 Hz, 1H), 8.65 (br, 1H). LC-MS:m/z= 415.2 [M+H]⁺ (95.36% purity, 254 nm).

### Example 71: Preparation of Compound SYY-C086

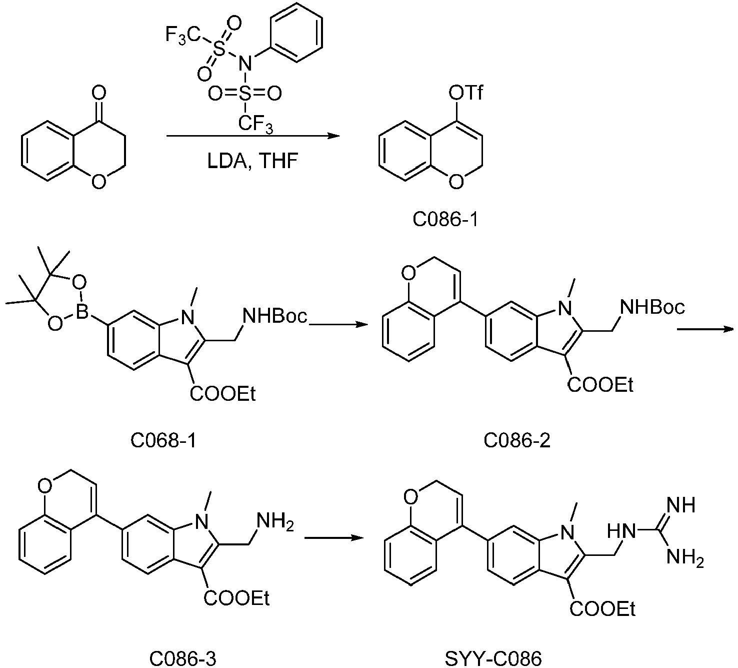

### Step 1:

4-Chromanone (2.0g,13.50 mmol) was dissolved in THF(40 mL), cooled to -70 °C under the protection of nitrogen, and a solution of LDA in THF (10 mL, 20.0 mmol, 2M) was added dropwise in about half an hour, and the mixture was continued to react at -70 °C for 1 h. N-Phenyl-bis(trifluoromethanesulfonimide) (7.2g,20.15 mmol) was added dropwise, and the reaction solution continued to react at -70 °C for 1h after addition and the mixture was slowly heated to room temperature to react for 16 h. The reaction solution was quenched with saturated ammonium chloride aqueous solution, concentrated to a small amount, extracted with ethyl acetate, the organic phases were combined, washed with saturated salt water, dried over anhydrous sodium sulfate, and concentrated, and the crude product was purified by silica gel column chromatography to obtain C086-1(1.5g, yield 40%) as a light yellow oil.

### Step 2:

Under the protection of nitrogen, C068-1(500 mg,1.09 mmol) was dissolved in DMF(10 ml), C086-1(610 mg,2.18 mmol), Pd(PPh₃)₄(115 mg,0.10 mmol) and cesium carbonate (710 mg,2.18 mmol) were added at room temperature, replaced with nitrogen for 3 times, and the mixture was heated to 85°C to react for 2.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography (PE/EA = 20/1) to obtain C086-2(290 mg, yield 57%) as a light yellow solid.

### Step 3:

Under the protection of nitrogen, the C086-2(240 mg,0.52 mmol) was dissolved in DCM(5ml), cooled to 0°C under an ice water bath, trifluoroacetic acid (1.0 ml, diluted with 1.0 ml DCM) was added dropwise, and the mixture was reacted at 0°C for 1.0 h after addition. TLC showed the reaction was completed. The reaction solution was added with saturated sodium bicarbonate aqueous solution to adjust to be weak alkalinity, stirred for 20min, extracted with dichloromethane, the organic phases were combined, washed with saturated salt water, dried over anhydrous sodium sulfate, and concentrated, and the crude product was purified by Pre-TLC (DCM/MeOH = 12/1) to obtain C086-3(69 mg, yield 37%) as a light yellow oil. LCMS:m/z=346.1[M-NH₂]⁺

### Step 4:

Under the protection of nitrogen, C086-3(69 mg,0.19 mmol) was dissolved in acetonitrile (5 ml), DIEA(123 mg,0.95 mmol) and 1H-pyrazol-1-formamidine hydrochloride (56 mg,0.38 mmol) were added, and the mixture was reacted at room temperature for 48 h. LCMS monitored the reaction was completed. The reaction solution was filtered, and the filter cake was washed and dried to obtain SYY-C086(57 mg, yield 74%) as a light yellow solid. ¹H NMR: (DMSO-*d₆*, 400MHz): δ1.40 (t, J = 7.2 Hz, 3H), 3.87 (s, 3H), 4.38 (q, J = 7.2 Hz, 2H), 4.86 (d, J = 4.0 Hz, 2H), 4.96 (s, 2H), 5.99 (t, J = 4.0 Hz, 1H), 6.86- 6.98 (m, 3H), 7.17-7.22 (m, 2H), 7.34-7.68 (m, 4H), 8.06 (d, J = 8.0 Hz, 1H), 8.42 (br, 1H). LCMS:m/z=405.2[M+H]⁺ (90.01% purity, 254 nm)

### Example 72: Preparation of Compound SYY-C087

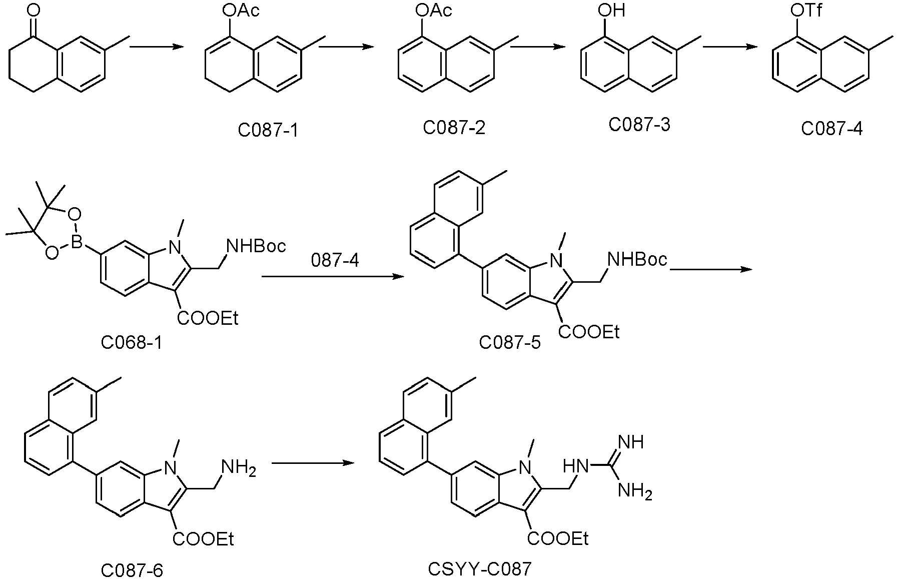

### Step 1:

Under the protection of nitrogen, 7-methyl -3, 4-dihydro-2H-1-naphthalenone (2.0g,12.48mmol) was dissolved in isopropenyl acetate(19.5ml,179.18 mmol), p-toluenesulfonic acid (40 mg,0.23 mmol) was added at room temperature, and the mixture was heated to 100°C to reflux for 20 h. TLC showed the raw materials were reacted completely. The reaction liquid was cooled to room temperature to obtain C087-1 (crude), which was directly used in the next step.

### Step 2:

Under the protection of nitrogen, the reaction solution of C087-1 (crude) from the previous step was cooled to 0°C under an ice water bath, DDQ(5.68g,25.02 mmol) was added in batches, and the mixture was heated to 85°C to react for 20 h. TLC showed the reaction was completed. The reaction solution was concentrated, added with water, extracted with ethyl acetate, the organic phases were combined, washed with saturated salt water, dried over anhydrous sodium sulfate, and concentrated, and the crude product was purified by silica gel column chromatography(PE/EA=40/1) to obtain C087-2(2.0g, yield for two steps: 80%) as a light yellow oil.

### Step 3:

Under the protection of nitrogen, C087-2(1.7g,8.49 mmol) was dissolved in ethanol (34 ml) and water (17 ml), sodium carbonate (4.5g,42.4 mmol) was added at room temperature, and the mixture was heated to 85°C to react for 2 h. TLC showed the reaction was completed. The reaction solution was cooled to room temperature, filtered, the filtrate was concentrated, added with water, extracted with ethyl acetate, the organic phases were combined, washed with saturated salt water, dried over anhydrous sodium sulfate, and concentrated, and the crude product was purified by silica gel column chromatography(PE/EA=60/1) to obtain the product, and the product was slurried with PE(40ml), filtered, and the filter cake was washed and dried to obtain C087-3(984 mg, yield 73%) as a white solid. ¹H NMR (400 MHz, cdcl₃) δ 2.57 (s, 3H), 5.23 (d, *J* = 4.0 Hz, 1H), 6.82 (d, *J=* 7.6 Hz, 1H), 7.27 (t, *J=* 8.0Hz ,1H), 7.37 (dd, *J=* 8.4, 1.6 Hz, 1H), 7.44 (d, *J=* 8.0 Hz, 1H), 7.75 (d, *J=* 8.4 Hz, 1H), 7.98 (s, 1H). LCMS:m/z=159.1[M+H]⁺

### Step 4:

Under the protection of nitrogen, C087-3(984 mg,6.22 mmol) was dissolved in DCM(20ml), pyridine (1.48g,18.71mmol) was added at room temperature, cooled to 0°C under an ice water bath, trifluoromesylate anhydride (1.96g,6.95mmol, diluted with 2.0 ml DCM) was added dropwise, and the mixture was reacted at 0°C for 1h after addition. TLC showed the reaction was completed. The reaction solution was washed with 1N hydrochloric acid (20mL), washed with saturated salt water, dried over anhydrous sodium sulfate, and concentrated to obtain C087-4(2.2g, crude) as a green oil.

### Step 5:

Under the protection of nitrogen, C068-1(300 mg,0.65 mmol) was dissolved in DMF(10 ml), C087-4(568 mg,1.96 mmol), Pd(dppf)Cl₂ dichloromethane complex (54 mg,0.066 mmol) and Cs₂CO₃(427 mg,1.31 mmol) were added, replaced with nitrogen for 3 times, and the mixture was heated to 80°C to react for 3.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=10/1) to obtain C087-5(258 mg, yield 83%) as a light yellow oil.

### Step 6:

Under the protection of nitrogen, the C087-5(258 mg,0.55 mmol) was dissolved in DCM(5 ml), cooled to 0°C under an ice water bath, trifluoroacetic acid (1.5 ml, diluted with 1.5 ml DCM) was added dropwise, and the mixture was reacted at room temperature for 0.5 h after addition. TLC showed the reaction was completed. The reaction solution was added with saturated sodium bicarbonate aqueous solution to adjust to be weak alkalinity, stirred for 20min, extracted with dichloromethane, and the organic phases were combined, washed with saturated salt water, dried over anhydrous sodium sulfate, and concentrated to obtain C087-6(200mg, yield 99%) as a light brown yellow oil. ¹H NMR (400 MHz, DMSO_d6): δ 1.41 (t, *J* = 7.2 Hz, 3H), 2.38 (s, 3H), 3.88 (s, 3H), 4.24 (s, 2H), 4.36 (q, *J* = 7.2 Hz, 2H), 7.29 (d, *J* = 9.4 Hz, 1H), 7.38 (d, *J* = 8.6 Hz, 1H), 7.45 (d, *J* = 6.0 Hz, 1H), 7.55-7.48 (m, 1H), 7.62 (s, 1H), 7.64 (s, 1H), 7.90 (d, *J* = 8.0 Hz, 1H),7.92 (d, *J=* 8.4 Hz, 1H), 8.12 (d, *J=* 8.2 Hz, 1H). LCMS:m/z=356.1[M-NH₂]⁺

### Step 7:

Under the protection of nitrogen, C087-6(150 mg,0.40 mmol) was dissolved in acetonitrile (5 ml), DIEA(260 mg,2.01mmol) and 1H-pyrazol-1-formamidine hydrochloride (118 mg,0.80mmol) were added, and the mixture was reacted at room temperature for 48 h. LCMS monitored that the reaction was completed. The reaction solution was filtered, and the filter cake was washed and dried to obtain SYY-C087(130 mg, yield 78%) as a light yellow solid. ¹H NMR: (DMSO-*d₆*, 400MHz): δ1.42 (t, J =7.2 Hz, 3H), 2.37 (s, 3H), 3.90 (s, 3H), 4.41 (q, J = 7.2 Hz, 2H), 5.00 (s, 2H), 7.33-7.70 (m, 8H), 7.73 (s, 1H), 7.92 (d, J = 8.0 Hz, 2H), 8.15 (d, J = 8.0 Hz, 1H), 8.46 (br, 1H). LCMS:m/z=415.2[M+H]⁺ (96.22% purity, 254 nm)

### Example 73: Preparation of Compound SYY-C088

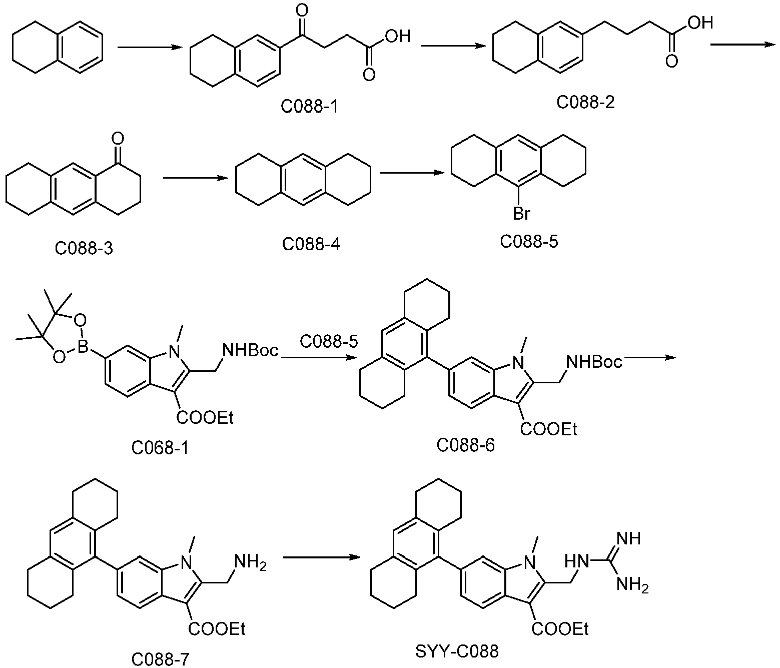

### Step 1:

Aluminum trichloride (6.4g,48.00mmol) was suspended in dichloromethane (20 mL), cooled to about 0 °C, a suspension of succinic anhydride (5.8g,57.96mmol) in dichloromethane (20 mL) was added dropwise under the protection of nitrogen, reacted at 0 °C for 1h after addition, then a solution of tetrahydronaphthalene (3.0g,22.69 mmol) in dichloromethane (15mL) was added, and the mixture was returned to room temperature to react for 2h. TLC detection showed the reaction was completed. The reaction solution was quenched by adding ice hydrochloric acid solution (1M), extracted and separated, and the organic phases were combined, washed with saturated salt water, dried over anhydrous sodium sulfate, and concentrated to obtain C088-1(4.6g, yield 87%) as an off-white solid. ¹H NMR (400 MHz, CDCl₃) δ1.84 (dd, *J* = 6.6, 3.5 Hz, 4H) 2.8-2.84 (m, 6H), 3.31 (t, *J* = 6.6 Hz, 2H), 7.17 (d, *J* = 8.5 Hz, 1H), 7.71 (m, 2H).

### Step 2:

C088-1(3.7g,15.93mmol) and 80% hydrazine hydrate (5 mL,82.30 mmol) were dissolved in diethylene glycol (15mL), and the mixture was heated to 90 °C to react for half an hour, cooled to 60 °C, potassium hydroxide solid (4.5g,80.20mmol) was added, and the mixture was heated to 130°C to react for 1 h, then heated to 195 °C to react for 3 h. TLC detection showed that the reaction was basically completed. The reaction solution was cooled to room temperature, added with water, concentrated hydrochloric acid was added to adjust the solution to be acidic, extracted with ethyl acetate, washed with saturated salt water, dried over anhydrous sodium sulfate, and concentrated to obtain C088-2(4.0g, crude) as a yellow oil.

### Step 3:

C088-2(4.0g, crude) was dissolved in dichloromethane (80 mL), DMF(2d) was added, oxalyl chloride (2.6g,20.48 mmol) was added at 0 °C, and the mixture was slowly heated to room temperature to react for half an hour after the addition. TLC detection showed the reaction was completed. The reaction solution was concentrated to dryness at room temperature (carried with dichloromethane three times), dissolved with dichloromethane (40 mL), the mixture was added dropwise to a suspension of aluminum trichloride (3.7g,27.75 mmol) in dichloromethane (15mL) at 0 °C, and the mixture was reacted at room temperature for 1h after addition. TLC detection showed the reaction was completed. The reaction solution was quenched with ice hydrochloric acid solution (1M), extracted with ethyl acetate, the organic phases were combined, washed with saturated salt water, dried over anhydrous sodium sulfate, and concentrated, and the crude product was purified by silica gel column chromatography to obtain C088-3(3.0g, yield for two steps: 81%) as a light yellow oil. ¹H NMR (400 MHz, CDCl₃): δ1.77-1.88 (m, 4H), 2.09-2.16 (m, 2H), 2.62-2.66 (m, 2H), 2.79-2.82 (m, 4H), 2.91 (t, *J=* 6.0 Hz, 2H), 6.97 (s, 1H), 7.78 (s, 1H).

### Step 4:

C088-3(1.0g,4.99 mmol) and 85% hydrazine hydrate (1.2 mL,19.75 mmol) were dissolved in diethylene glycol (10 mL), heated to 90 °C to react for half an hour, cooled to about 60 °C, potassium hydroxide (1.1g, 19.60 mmol) was added, and the mixture was heated to 130°C to react for 1 h, then heated to 195 °C to react for 3 h. TLC detection showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C088-4(520 mg, yield 56%) as a white solid. ¹H NMR (400 MHz, CDCl₃) δ 1.78-1.81 (m, 8H), 2.73 (s, 8H), 6.81 (s, 2H)

### Step 5:

C088-4(400 mg,2.15 mmol) was dissolved in acetic acid (10 mL), a solution of bromine (344mg,2.15 mmol) in acetic acid (2 mL) was added dropwise at room temperature, and the mixture was reacted overnight at room temperature after addition. The reaction solution was poured into ice water, neutralized with saturated sodium carbonate aqueous solution, extracted with ethyl acetate. The organic phases were combined, washed with saturated salt water, dried over anhydrous sodium sulfate, and concentrated to obtain C088-5(570 mg, crude) as an off-white solid. ¹H NMR (400 MHz, CDCl₃): δ 1.69-1.89 (m, 8H), 2.73-2.78 (m, 8H), 6.81 (s, 1H).

### Step 6:

Under the protection of nitrogen, C068-1(350 mg, crude) was dissolved in DMF(10 ml), C088-5(403 mg,1.52 mmol), Pd(dppf)Cl₂ dichloromethane complex (62 mg,0.076 mmol) and Cs₂CO₃(500 mg,1.53 mmol) were added at room temperature, replaced with nitrogen for 3 times, and the mixture was heated to 100°C to react for 3.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=40/1) to obtain C088-6(258 mg, yield for two steps: 65%) as a white solid.

### Step 7:

Under the protection of nitrogen, the C088-6(250mg,0.48 mmol) was dissolved in DCM(5 ml), then cooled to 0°C under an ice water bath, trifluoroacetic acid (1.5 ml, diluted with 1.5 ml DCM) was added dropwise, and the mixture was reacted at room temperature for 0.5 h after addition. TLC showed the reaction was completed. The reaction solution was added with saturated sodium bicarbonate aqueous solution to adjust to be weak alkalinity, stirred for 20min, extracted with dichloromethane, and the organic phases were combined, washed with saturated salt water, dried over anhydrous sodium sulfate, and concentrated to obtain C088-7(180 mg, crude) as a white solid.

### Step 8:

Under the protection of nitrogen, C088-7(180mg, crude) was dissolved in acetonitrile (5 ml), then DIEA(280 mg, 2.17mmol) and 1H-pyrazol-1-formamidine hydrochloride (126 mg,0.86 mmol) were added, and the mixture was reacted at room temperature overnight. The reaction solution was filtered, and the filter cake was washed and dried to obtain solid SYY-C088(76 mg, yield for two steps: 34%). ¹H NMR: (DMSO-*d₆*, 400MHz): δ1.40 (t, J = 7.2 Hz, 3H), 1.52-1.61 (m, 4H), 1.62-1.71 (m, 4H), 2.10-2.27 (m, 4H), 2.71 (t, J = 6.0 Hz, 4H), 3.84 (s, 3H), 4.37 (t, J = 7.2 Hz, 2H), 4.95 (s, 2H), 6.81 (s, 1H), 6.95 (dd, J = 8.0, 1.2 Hz, 1H), 7.14-7.67 (m, 4H), 8.06 (dd, J = 8.4, 0.8 Hz, 1H), 8.34 (br, 1H). LCMS:m/z=459.3[M+H]⁺

### Example 74: Preparation of Compound SYY-C089

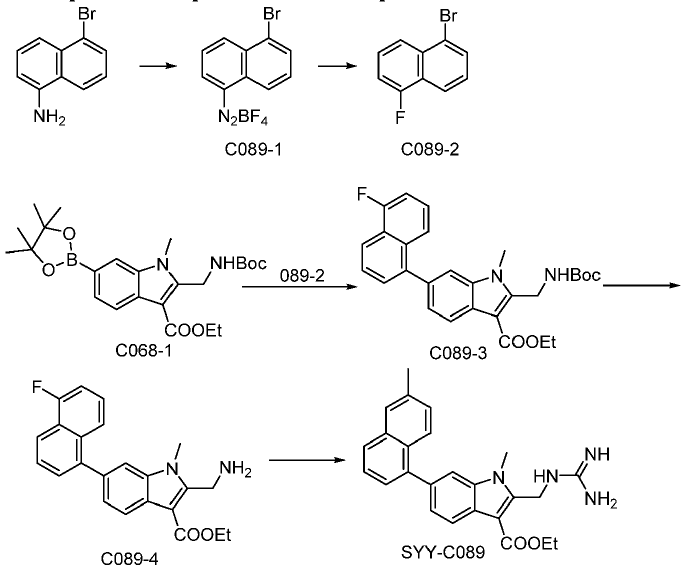

### Step 1:

Under the protection of nitrogen, 1-amino-5-bromnaphthalene (2.0g,9.01 mmol) was dissolved in fluoroboric acid (20 ml), heated to 50°C and stirred for 30min, then cooled to 0°C under an ice salt bath, sodium nitrite (681 mg,9.87 mmol, 1.5 ml, dissolved with water) was added dropwise, reacted at 0°C for 1.5 h after addition, and 20 ml of water was added dropwise, stirred for 20min, filtered, and the filter cake was washed with water for 3 times, and washed with ice methanol for 2 times, slurried with MTBE for 3 times (red brown pigment was removed, until the filter cake was washed to light yellow brown), and dried to obtain light yellow brown solid (2.15g, crude).

### Step 2:

Under the protection of nitrogen, C089-1(2.15g,6.70 mmol) was dissolved in xylene (30 ml) and the mixture was heated to 140 °C to react for 1.0 h. TLC showed the reaction was completed. The reaction solution was concentrated, and the crude product was purified by silica gel column chromatography (PE) to obtain C089-2(1.29g, yield for two steps: 64%) as a light green oil .

### Step 3:

Under the protection of nitrogen, C068-1(300 mg,0.65 mmol) was dissolved in DMF(10 ml) and water(0.5ml), C089-2(442 mg,1.96 mmol), Pd(dppf)Cl₂ dichloromethane complex (53 mg,0.065 mmol) and Cs₂CO₃(427 mg,1.31 mmol) were added at room temperature, replaced with nitrogen for 3 times, and the mixture was heated to 100°C to react for 3.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography(PE/EA=10/1) to obtain C089-3(237 mg, yield 76%) as a white solid.

### Step 4:

Under the protection of nitrogen, the C089-3(237 mg,0.50 mmol) was dissolved in DCM(10 ml), then cooled to 0°C under an ice water bath, trifluoroacetic acid (2.0 ml, diluted with 2.0 ml DCM) was added dropwise, and the mixture was reacted at room temperature for 0.5 h after addition. TLC showed the reaction was completed. The reaction solution was added with saturated sodium bicarbonate aqueous solution to adjust to be weak alkalinity, stirred for 20min, extracted with DCM, and the organic phases were combined, washed with saturated salt water, dried over anhydrous sodium sulfate, and concentrated to obtain C089-4(110 mg, yield 59%) as a light brown yellow oil. ¹H NMR (400 MHz, DMSO_d6) : δ 1.40 (t, *J=* 7.2 Hz, 3H), 3.88 (s, 3H), 4.25 (s, 2H), 4.35 (q, *J=* 7.2 Hz, 2H), 7.31 (dd, *J=* 8.0, 1.2 Hz, 1H), 7.35-7.41 (m, 1H), 7.44-7.50 (m, 1H), 7.61 (d, *J=* 6.0 Hz, 1H), 7.75-7.65 (m, 3H), 8.15- 8.09 (m, 2H). LCMS:m/z=360.1[M-NH₂]⁺

### Step 5:

Under the protection of nitrogen, C089-4(100 mg, 0.27 mmol) was dissolved in acetonitrile (5 ml), then DIPEA(172 mg, 1.33 mmol) and 1H-pyrazol-1-formamidine hydrochloride (78 mg,0.53 mmol) were added, and the mixture was reacted at room temperature overnight. The reaction solution was filtered, and the filter cake was washed and dried to obtain solid SYY-C089(98 mg, yield 88%). ¹H NMR: (DMSO-*d₆*, 400MHz): δ1.42 (t, J = 7.2 Hz, 3H), 3.91 (s, 3H), 4.41 (q, J = 7.2 Hz, 2H), 5.00 (s, 2H), 7.78 (s, 1H), 7.18-7.77 (m, 10H), 8.12-8.17 (m, 2H). LCMS:m/z=419.2[M+H]⁺ (90.04% purity, 254 nm)

### Example 75: Preparation of Compound SYY-C090

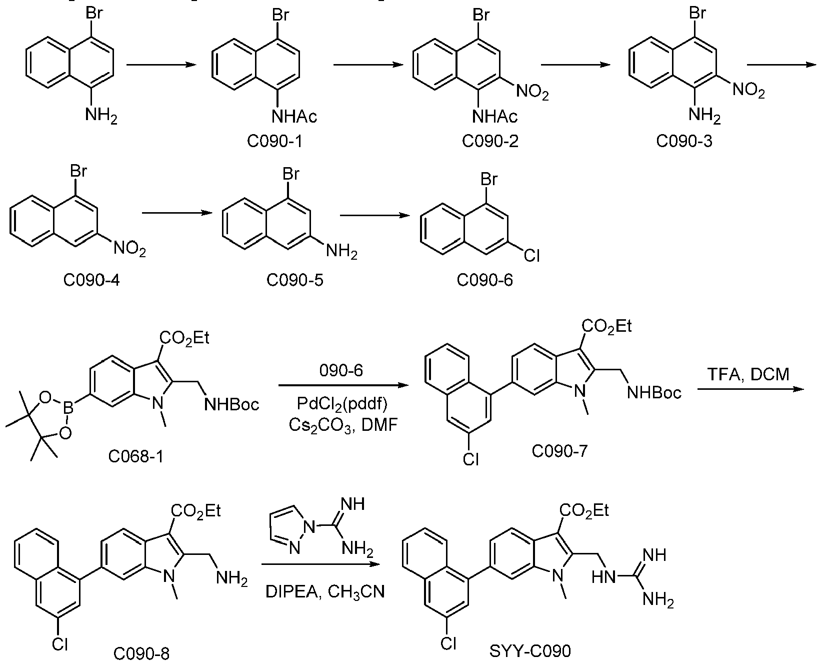

### Step 1:

4-Bromo-1-naphthylamine (4.0g,18.00 mmol) was dissolved in dichloromethane (80 mL), pyridine (4.3g,54.00 mmol) and DMAP(220 mg,1.8 mmol) were added, cooled to about 0°C, a solution of acetic anhydride (2.2g,21.6 mmol) in dichloromethane (20 mL) was added dropwise in about 20 minutes under the protection of nitrogen, and the reaction solution was heated to room temperature and stirred for 3h. TLC detection showed the reaction was completed. The reaction liquid was concentrated, the residue was slurried with a small amount of dichloromethane, filtered, and the filter cake was washed and dried to obtain C090-1(3.8g, crude) as an off-white solid.

### Step 2:

C090-1(2.8g, crude product) was dispersed in acetic anhydride (30 mL). At 0°C, a mixed solution of concentrated nitric acid (2.5mL,26.61mmol) and acetic anhydride (5 mL)was added dropwise and the mixture was reacted at 0°C for half an hour after addition, then heated to room temperature and stirred for 1 hour. It was difficult to stir. Additional acetic anhydride (10mL) was added and continued to react for 1 h. TLC detection showed the reaction was basically completed. The reaction solution was added with ice water, stirred for about 30 minutes, filtered, and the filter cake was washed to obtain a light yellow solid (6.0g, wet weight crude).

### Step 3:

C090-2(1.1g, wet weight crude) was dispersed in ethanol (20 mL), concentrated hydrochloric acid (15 mL) was added, and the system was heated to reflux to react for 7h. TLC detection showed the reaction was completed. The reaction solution was cooled to room temperature, filtered, and the filter cake was washed, and dried to obtain C090-3 (400 mg, crude) as a yellow solid.

### Step 4:

C090-3(1.85g, crude) was dispersed in 50% sulfuric acid solution (20 mL), acetic acid (6 mL) was added, the system was cooled to 0°C, sodium nitrite (491 mg,0.24 mmol) aqueous solution (2 mL) was added dropwise, the mixture was stirred and reacted at 0-5 °C for half an hour after addition, and the system was gradually clarified. TLC detection showed the raw materials were reacted completely. Diazonium salt solution was added to hypophosphoric acid (10mL), and the mixture was stirred overnight at room temperature. TLC detection showed the reaction was completed. The reaction solution was quenched with ice water, extracted with ethyl acetate, the organic phases were combined, washed with saturated salt water, dried over anhydrous sodium sulfate, and concentrated, and the crude product was purified by column chromatography to obtain C090-4(1.5g, yield for four steps: 53%) as an off-white solid.

### Step 5:

C090-4(1.8g,7.11 mmol) was dispersed in ethanol (40mL) and water (10mL), ammonium chloride solid (1.9g,35.57mmol) was added, heated to 50°C, reduced iron powder (2.0g,35.57mmol) was added, and the mixture was reacted for half an hour, then heated to reflux to react for 2 h. TLC detection showed the reaction was completed. The reaction solution was thermally filtered with diatomite, concentrated to remove ethanol, added with ethyl acetate for extraction, the organic phases were combined, washed with saturated salt water, dried over anhydrous sodium sulfate, and concentrated, and the crude product was purified by column chromatography to obtain C090-6(1.26g, yield 80%) as an off-white solid.

### Step 6:

C090-5(200 mg,0.90 mmol) was dispersed in acetic acid (8mL) and concentrated hydrochloric acid (1.5mL), cooled to 0-5 °C, sodium nitrite (68 mg,0.99 mmol) aqueous solution (1 mL) was added dropwise, and the mixture was reacted at 0 °C for half an hour after addition. Diazonium salt solution was added dropwise to a mixture of cuprous chloride (179 mg, 1.8 mmol) and concentrated hydrochloric acid (4mL) heated to 65°C, and the mixture was reacted at 65°C for half an hour. TLC detection showed that the reaction was completed. The reaction solution was cooled to room temperature, quenched with water, extracted with ethyl acetate, the organic phases were combined, washed with saturated salt water, dried over anhydrous sodium sulfate, and concentrated, and the crude product was purified by column chromatography to obtain C090-6(180mg, yield 83%) as an off-white solid. HNMR,(CDCl3, 400Hz): δ 8.20 (d, J=8.4 Hz, 1H), 7.81 (m, 1H), 7.74-7.77 (m, 2H), 7.53-7.61 (m, 2H).

### Step 7:

C068-1(200 mg, 0.44 mmol) and C090-6(180 mg,0.75 mmol) were dissolved in DMF(10 ml), water (1 ml), Cs₂CO₃(300 mg, 0.88 mmol) and PdCl₂(dppf)(40 mg,0.044 mmol) were added at room temperature, replaced with N₂ for three times, and the mixture was heated to 100 °C to reflux for 2 h. TLC showed the reaction was completed. The reaction solution was cooled to room temperature, added with water, extracted with ethyl acetate, and the organic phases were combined, washed with saturated salt water, dried over anhydrous sodium sulfate, concentrated, separated and purified by silica gel column chromatography to obtain C090-7(180 mg, yield 83%) as a white solid.

### Step 8:

C090-7(200 mg,0.41 mmol) was dissolved in DCM(5 ml), TFA(3 ml) was added, and the reaction was carried out at room temperature for 1 h. TLC showed the reaction was completed. The reaction solution was concentrated, neutralized with saturated NaHCO₃ solution, solids were precipitated, filtered, and the filter cake was dissolved with dichloromethane, dried over anhydrous Na₂SO₄, filtered, and the filtrate was concentrated to obtain C090-8(150 mg, crude). HNMR(DMSO-d6, 400Hz): δ8.12 (d, J=8.4 Hz, 1H), 8.08 (d, J=2.0 Hz, 1H), 8.01 (d, J=8.4 Hz, 1H), 7.71 (s, 1H), 7.59 (t, J=8.0 Hz, 1H), 7.48-7.52 (m, 2H), 7.30 (dd, J1=1.6, J2=8.0 Hz, 2H), 4.34 (q, J=7.2 Hz, 2H), 4.22 (s, 2H), 3.88 (s, 3H), 1.39 (t, J=7.2 Hz, 3H). LC-MS:m/z=393.1 [M+H]⁺

### Step 9:

C090-8(150 mg, 0.38 mmol) was dissolved in acetonitrile(5 ml), DIPEA(190 mg,1.52 mmol) was added, stirred at room temperature for 20min, then 1H-pyrazol-1-formamidine hydrochloride (80 mg,0.57 mmol) was added, and the mixture was stirred for weekend at room temperature. The reaction solution was filtered, and the filter cake was washed and dried to obtain SYY-C090(107 mg, yield 65%). ¹H NMR: (DMSO-*d₆*, 400MHz): δ1.32-1.48 (m, 3H), 3.91 (s, 3H), 4.24-4.51 (m, 2H), 5.01 (s, 2H), 6.99-7.96 (m, 10H), 7.97-8.05 (m, 1H), 8.05-8.18 (m, 2H), 8.64 (br, 1H). (95.90% purity, 254 nm) LC-MS:m/z=435.1 [M+H]⁺

### Example 76: Preparation of Compound SYY-C091

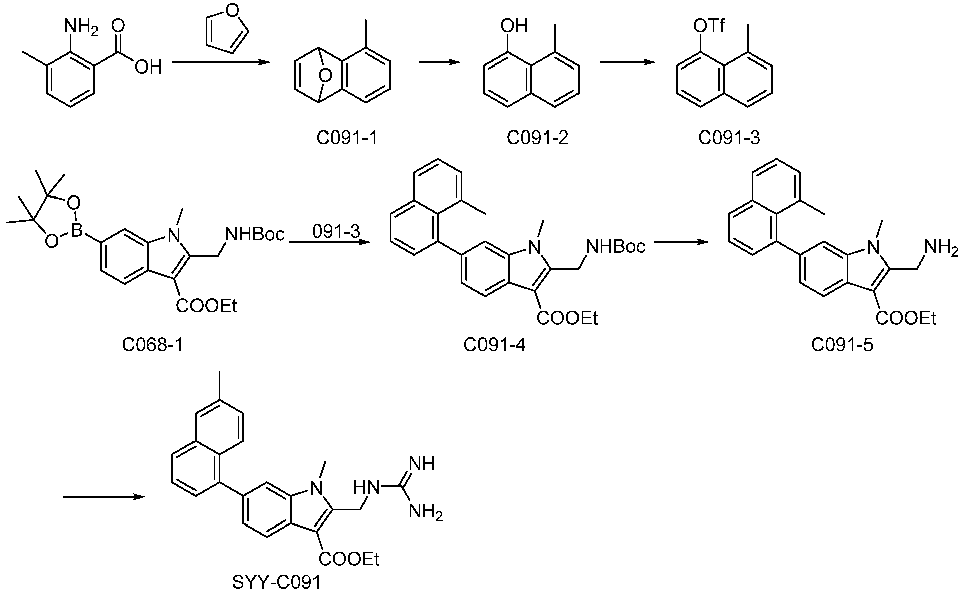

### Step 1:

Under the protection of nitrogen, furan (5.41g,79.4 mmol) was dissolved in DCM (80 ml), isoamyl nitrite (11.75g,100 mmol) was added at room temperature, and the mixture was heated to reflux, and then 2-amino-3-methylbenzoic acid (10.0g,66.2 mmol,80 ml, dissolved with THF) was added dropwise, and the mixture was continued to reflux for 2 h after addition. TLC showed the reaction was completed. The reaction solution was cooled to room temperature, concentrated and dried, added with water, extracted with ethyl acetate, washed with saturated sodium bicarbonate aqueous solution, and washed with saturated salt water, dried over anhydrous sodium sulfate, and concentrated, and the crude product was separated and purified by silica gel column chromatography to obtain C091-1(6.15g, yield 59%) as a light yellow oil.

### Step 2:

Under the protection of nitrogen, C091-1(6.15g,38.92 mmol) was dissolved in methanol (125 ml), concentrated hydrochloric acid (12.5 ml) was added at room temperature, and the mixture was heated to reflux to react for 4.0 h. TLC showed (the raw materials were reacted completely, two new points were generated). The reaction solution was concentrated, added with water, saturated sodium bicarbonate aqueous solution was added dropwise to adjust PH to be neutral, extracted with EA, the organic phases were combined, washed with saturated salt water, dried over anhydrous sodium sulfate, and concentrated, and the crude product was separated and purified by silica gel column chromatography to obtain C091-1(3.1g, yield 50%) as a brown oil. HNMR(CDCl3, 400Hz):δ7.66 (d, J=8.0 Hz, 1H), 7.43 (d, J=8.0 Hz, 1H), 7.35 (t, J=8.0 Hz, 1H), 7.22-7.39 (br, 2H), 6.74 (dd, J1=1.2, J2=8.4 Hz, 2H), 3.00 (s, 3H).

### Step 3:

C091-2(400 mg,1.26 mmol) was dissolved in dichloromethane (5 mL), cooled to 0 °C, 60% sodium hydride (112 mg,2.8 mmol) was added under the protection of nitrogen, and the mixture was reacted at 0 °C for half an hour, then a solution of trifluoromethyl sulfonic anhydride (852 mg,3.04 mmol) in dichloromethane (2 mL) was added, and the mixture was reacted at 0 °C for half an hour. TLC detection showed the reaction was completed. The reaction solution was quenched with ice water, extracted with dichloromethane, the organic phases were combined, washed with saturated salt water, dried over anhydrous sodium sulfate, and concentrated, and the crude product was separated and purified by silica gel column chromatography to obtain C091-3(430mg, yield 58%) as a colorless oil.

### Step 4:

C068-1(200 mg,0.44 mmol), C091-3(153 mg,0.53mmol) and cesium carbonate (287 mg,0.88 mmol) were dissolved in DMF(8mL) and water (5 mL), PdCl₂(dppf)(50mg,0.068 mmol) was added, replaced with nitrogen for 3 times, and the mixture was heated to 100 °C to react for 3 h. TLC detection showed the raw materials were reacted completely. The reaction solution was cooled to room temperature, quenched with water, extracted with ethyl acetate, the organic phases were combined, washed with saturated salt water, dried over anhydrous sodium sulfate, and concentrated, and the crude product was purified by silica gel column chromatography to obtain C091-4(150 mg, yield 73%) as a white solid.

### Step 5:

C091-4(150 mg,0.32 mmol) was dissolved in DCM(5 ml), TFA(3 ml) was added, and the mixture was reacted at room temperature for 1 h. TLC showed the reaction was completed. The reaction solution was concentrated, neutralized with saturated NaHCO₃ solution, solids were precipitated, filtered, and the filter cake was dissolved with DCM, dried over anhydrous Na₂SO₄, and concentrated to obtain C091-5(85 mg, crude). LC-MS:m/z=356.1 [M-NH₂]⁺.

### Step 6:

C091-5(85 mg, crude) was dissolved in CH₃CN(5 ml), DIPEA(120 mg,0.93 mmol) was added, stirred at room temperature for 20min, then 1H-pyrazol-1-formamidine hydrochloride (50 mg,0.34 mmol) was added, and the mixture was stirred for a weekend at room temperature. The reaction solution was filtered, and the filter cake was washed and dried to obtain solid SYY-C091(38 mg, yield for two steps: 29%). ¹H NMR: (DMSO-*d₆*, 400MHz): δ1.41 (t, J = 7.2 Hz, 3H), 1.90 (s, 3H), 3.89 (s, 3H), 4.38 (q, J = 7.2 Hz, 2H), 5.01 (s, 2H), 7.19 (dd, J = 8.4, 1.2 Hz, 1H), 7.25 (d, J = 6.8 Hz, 1H), 7.34 (dd, J = 8.4, 1.2 Hz, 1H), 7.41 (t, J = 7.6 Hz, 1H), 7.51 ((t, J = 7.6 Hz, 1H), 7.55-8.28 (m, 8H). LC-MS:m/z=415.2 [M+H]⁺ (94.13% purity, 254 nm)

### Example 77: Preparation of Compound SYY-C092

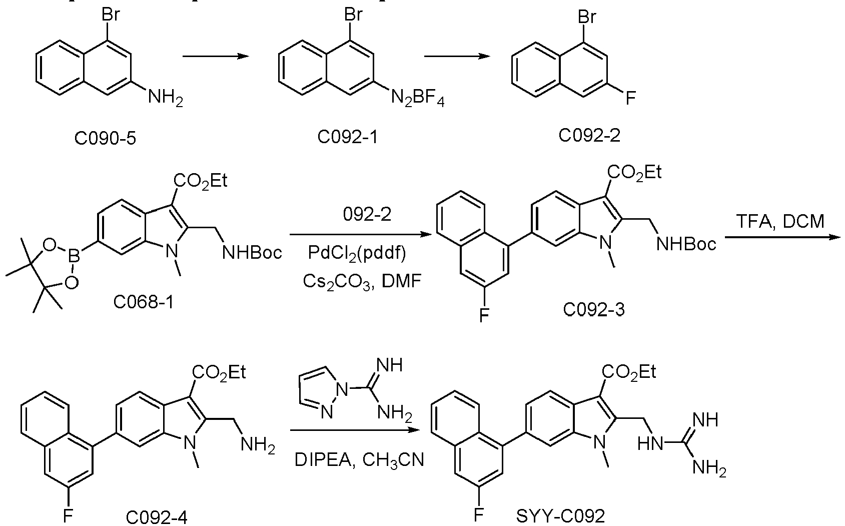

### Step 1:

C090-5(1.02g,2.57mmol) was dispersed in tetrafluoroboric acid (20mL), cooled to about 0 °C, sodium nitrite (347 mg,5.04 mmol) aqueous solution (2 mL) was added dropwise, and continued to react at this temperature for half an hour, filtered, and the filter cake was washed with cold water, and washed with cold methanol, then washed to be yellow with MTBE, and the filter cake was dried to obtain C092-1(910 mg, crude) as a yellow solid.

### Step 2:

C092-1(910 mg, crude) was dispersed in xylene (30mL) and heated reflux to react for 2h. TLC detection showed the reaction was completed. The reaction solution was cooled to room temperature, and concentrated, and the crude product was purified by silica gel column chromatography to obtain C092-2(500 mg, yield for two steps: 48%) as a colorless oil.

### Step 3:

C068-1(300 mg, 0.65 mmol) and C092-2(230 mg,1.03 mmol) were dissolved in DMF(10 ml), water (1 ml), Cs₂CO₃(430 mg, 1.30 mmol) and PdCl₂(dppf)(60 mg,0.065 mmol) were added at room temperature, replaced with N₂ for three times, and the mixture was heated to 100 °C to reflux to react for 2 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C092-3(280 mg, yield 90%).

### Step 4:

C092-3(230 mg,0.483 mmol) was dissolved in DCM(6 ml), TFA(3 ml) was added, and the reaction was carried out at room temperature for 1 h. TLC showed the reaction was completed. The reaction solution was concentrated, neutralized with saturated NaHCO₃ solution, solids were precipitated, filtered, and the filter cake was dissolved with dichloromethane, dried over anhydrous Na₂SO₄, and concentrated to obtain C092-4(170 mg, crude). HNMR(DMSO-d6, 400Hz): 8.12 (d, J=8.0 Hz, 1H), 8.00 (d, J=8.0 Hz, 1H), 7.86 (d, J=8.4 Hz, 1H), 7.75 (dd, J1=2.8, J1=10 Hz, 2H), 7.71 (s, 1H), 7.58 (t, J=8.0 Hz, 1H), 7.39-7.47 (m, 2H), 7.31 (dd, J1=1.6, J1=8.4 Hz, 2H), 4.34 (q, J=7.2 Hz, 2H), 4.22 (s, 2H), 3.88 (s, 3H), 1.39 (t, J=7.2 Hz, 3H). LC-MS:m/z=377.1 [M+H]⁺

### Step 5:

C092-4(170 mg, crude) was dissolved in acetonitrile(5 ml), DIPEA(175 mg,1.36 mmol) was added, stirred at room temperature for 20min, then 1H-pyrazol-1-formamidine hydrochloride (100 mg,0.68 mmol) was added, and the mixture was stirred overnight at room temperature. The reaction solution was filtered, and the filter cake was washed and dried to obtain SYY-C092(110 mg, yield for two steps: 58%). ¹H NMR: (DMSO-*d₆*, 400MHz): δ1.41 (t, J = 7.2 Hz, 3H), 3.93 (s, 3H), 4.40 (q, J = 7.2 Hz, 2H), 5.03 (s, 2H), 7.36-7.86 (m, 10H), 8.01 (d, J = 8.0 Hz, 1H), 8.16 (d, J = 8.0 Hz, 1H), 8.66 (br, 1H). LC-MS:m/z=419.1 [M+H]⁺ (93.75% purity, 254 nm)

### Example 78: Preparation of Compound SYY-C093

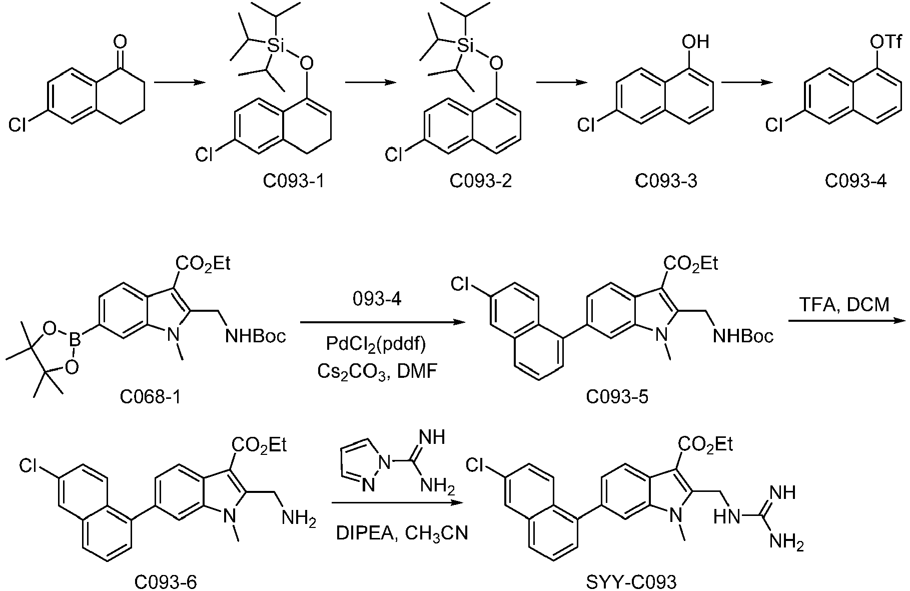

### Step 1:

6-Chloro-3,4-dihydro-2H-1-naphthalenone (1.0g,5.54mmol) was dissolved in dichloromethane (15mL), triethylamine (949 mg,9.42 mmol) was added, triisopropylsilyl trifluoromethanesulfonate (1.9g,6.09 mmol) was added after replacement with nitrogen for three times, and the reaction solution was stirred at room temperature for 2 h. TLC detection showed that the reaction was completed. The reaction solution was quenched with ice water, washed with saturated sodium bicarbonate aqueous solution, dried over anhydrous sodium sulfate, and concentrated to obtain C093-1(2.1g, crude) as a light yellow oil which was directly used in the next step.

### Step 2:

C093-1(2.1g, crude, 5.54mmol by theoretical calculation) was dissolved in acetonitrile (40mL), DDQ(2.5g,11.08 mmol) was added, and the mixture was heated to 80 °C to react for 3 h. TLC detection showed that the mixture was inseparable. The reaction solution was concentrated, and the crude product was purified by silica gel column chromatography to obtain C093-2(1.4g, yield for two steps: 76%) as a colorless oil. HNMR(CDCl3, 400Hz): 8.18(d, J=8.8 Hz, 1H)7.81 (d, J=2.0 Hz, 1H), 7.44 (dd, J1=2.0, J2=8.8 Hz, 1H), 7.33-7.38 (br, 2H), 6.83 (dd, J1=1.8, J2=6.0 Hz, 2H), 1.09 (s, 18H).

### Step 3:

C093-3(1.5g,4.5 mmol) was dissolved in NMP(2mL), methanol (3 mL), water (1 mL) and sodium hydroxide (1.08g,27 mmol) were added, and the reaction solution was heated to 60 °C to react for 2 h. TLC detection showed the reaction was completed. The reaction solution was cooled to room temperature, quenched with water, pH was adjusted to 5 with 1M hydrochloric acid, extracted with ethyl acetate, the organic phases were combined, washed with saturated salt water, dried over anhydrous sodium sulfate, and concentrated, and the crude product was purified by silica gel column chromatography to obtain C093-4(760 mg, yield 95%) as a colorless oil.

### Step 4:

C093-4(1.1g,4.48 mmol) was dissolved in dichloromethane (20mL), pyridine (1.0g,13.45mmol) was added, cooled to about 0 °C, a solution of trifluoromethanesulfonic anhydride (1.4g,4.93 mmol) in dichloromethane (5 mL) was added under the protection of nitrogen, and the mixture was returned to room temperature to react for 1h after addition. TLC detection showed the reaction was completed. The reaction solution was washed with 1M ice hydrochloric acid, dried over anhydrous sodium sulfate, and concentrated to obtain C093-5(1.8g, crude) as a yellow oil which was directly used in the next step.

### Step 5:

C068-1(300 mg, 0.65 mmol) and C093-5(230 mg, crude) were dissolved in DMF(10 ml), water (1 ml), Cs₂CO₃(430 mg, 1.30 mmol) and PdCl₂(dppf)(60 mg,0.065 mmol) were added at room temperature, replaced with N₂ for three times, and the mixture was heated to 100 °C to reflux to react for 2 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C093-6(320 mg, yield 99%).

### Step 6:

C093-6(230 mg,0.47 mmol) was dissolved in DCM(6 ml), TFA(3 ml) was added, and the mixture was reacted at room temperature for 1 h. TLC showed the reaction was completed. The reaction solution was concentrated, neutralized with saturated NaHCO₃ solution, solids were precipitated, filtered, and the filter cake was dissolved with DCM, dried over anhydrous Na₂SO₄, and concentrated to obtain C093-7(270 mg, crude). LC-MS:m/z=376.1 [M-NH₂]⁺.

### Step 7:

C093-7(170 mg, crude) was dissolved in CH₃CN(5 ml), DIPEA(222 mg,1.72 mmol) was added, stirred at room temperature for 20min, then 1H-pyrazol-1-formamidine hydrochloride (100 mg,0.65 mmol) was added, and the mixture was stirred for a weekend at room temperature. The reaction solution was filtered, and the filter cake was washed and dried to obtain a white solid (110 mg, yield for two steps: 59%). ¹H NMR: (DMSO-*d₆*, 400MHz): δ1.37-1.45 (m, 3H), 3.91 (s, 3H), 4.34-4.47 (m, 2H), 5.01 (s, 2H), 7.29-7.38 (m, 1H), 7.43- 8.00 (m, 10H) 8.09-8.18 (m, 2H). LC-MS:m/z=435.1 [M+H]⁺ (94.06% purity, 254 nm)

### Example 79: Preparation of Compound SYY-C094

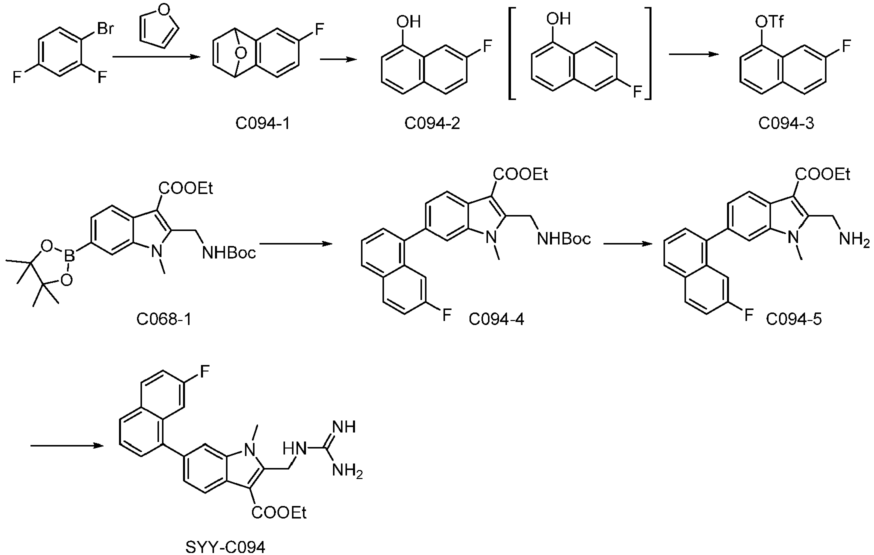

### Step 1:

Under the protection of nitrogen, 500 ml of three-neck flask, replaced with nitrogen for 3 times, 1-bromo-2, 4-difluorobenzene (10.0g,51.8 mmol) and ether (130 ml) were added, n-butyl lithium (24 ml,60 mmol,2.5M) was added dropwise at -65 °C, and the mixture was reacted at -65 °C for 30min after addition, then furan (40 ml,518 mmol) was added dropwise at -65 °C, and the mixture was reacted at -65 °C for 30min after the addition, naturally heated to room temperature, and the mixture was reacted overnight. TLC showed the reaction was completed. The reaction solution was quenched with water, filtered, the filtrate was stratified, the aqueous phase was extracted with ethyl acetate, the organic phases were combined, washed with saturated salt water, dried over anhydrous sodium sulfate, and concentrated, and the crude product was separated and purified by silica gel column chromatography to obtain C094-1(4.6g, yield 55%) as a light yellow oil, HNMR(DMSO-d6, 400Hz):δ7.16-7.19 (br, 1H), 7.01-7.09 (br, 3H), 6.64-6.69 (br, 1H), 5.72 (t, J=4.0 Hz, 2H).

### Step 2:

Under the protection of nitrogen, C094-1(4.6g,28.36 mmol) was dissolved in methanol (90 ml), concentrated hydrochloric acid (9 ml) was added, and the mixture was heated to reflux to react for 4.0 h. TLC showed the reaction was completed (two new points were generated, and the new points cannot be separated). The reaction solution was concentrated, added with water, added dropwise with saturated sodium bicarbonate aqueous solution to adjust PH to be neutral, extracted with ethyl acetate, the organic phases were combined, washed with saturated salt water, dried over anhydrous sodium sulfate, concentrated, added with 30ml PE, stirred for 30min, filtered, the filtrate was concentrated, and the crude product was purified by silica gel column chromatography to obtain light yellow solid mixture (1.5g, crude) (preparation chromatography cannot separate by-products). The filter cake was washed and dried to obtain a white solid, added with MIBE (3ml) to dissolve until be clear, added with PE(20ml) dropwise under ultrasound, stirred at room temperature, solids were precipitated, filtered, and the filter cake was washed and dried to obtain C094-2(300 mg) as a white solid. HNMR(DMSO-d6, 400Hz):δ 7.81-7.85 (m, 1H), 7.47 (d, J= 7.6 Hz, 1H), 7.28-7.33 (m, 2H), 6.86 (d, J=7.6 Hz, 2H), 5.19 (s, 1H).

### Step 3:

Under the protection of nitrogen, C094-2(300 mg,1.85 mmol) was dissolved in DCM(6 ml), pyridine (439 mg,5.56 mmol) was added, cooled to 0°C under an ice bath, trifluoromethanesulfonic anhydride (585 mg,2.07 mmol, diluted with 0.5 ml DCM) was added dropwise, and the mixture was reacted at 0°C for 1h after addition. TLC monitored the reaction was completed. The reaction solution was washed with 1N hydrochloric acid, washed with saturated salt water, dried over anhydrous sodium sulfate, and concentrated to obtain C094-3(650 mg, crude) as a green oil.

### Step 4:

Under the protection of nitrogen, C068-1(300 mg,0.655 mmol) was dissolved in DMF(10 ml) and water (0.5ml), C094-3(650 mg, crude), Pd(dppf)Cl₂ (54 mg,0.065mmol) and Cs₂CO₃(428 mg,1.31 mmol) were added at room temperature, replaced with nitrogen for 3 times, and the mixture was heated to 100°C to react for 2.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated and the crude product was purified by pre-TLC to obtain C094-4(240 mg, yield for two steps: 77%) as a light yellow oil .

### Step 5:

C094-4(240 mg,0.50 mmol) was dissolved in DCM(6 ml), TFA(3 ml) was added, and the mixture was reacted at room temperature for 1 h. TLC showed the reaction was completed. The reaction solution was concentrated, neutralized with saturated NaHCO₃ solution, solids were precipitated, filtered, and the filter cake was dissolved with dichloromethane, dried over anhydrous Na₂SO₄, and concentrated to obtain C094-5(170 mg, yield 90%). LC-MS:m/z= 377.2 [M+H]⁺

### Step 6:

C094-5(170 mg, 0.45 mmol) was dissolved in CH₃CN(5 ml), DIPEA(233 mg,1.80 mmol) was added, stirred at room temperature for 20min, then 1H-pyrazol-1-formamidine hydrochloride (100 mg,0.68 mmol) was added, and the mixture was stirred for a weekend at room temperature. The reaction solution was filtered, and the filter cake was washed and dried to obtain solid SYY-C094(90 mg, yield 48%)¹H NMR: (DMSO-*d₆*, 400MHz): δ1.34-1.46 (m, 3H), 3.91 (s, 3H), 4.31-4.47 (m, 2H), 5.01 (s, 2H), 7.30-7.92 (m, 10H), 7.98-8.07 (m, 1H), 8.10-8.18 (m, 2H). LC-MS:m/z=419.2 [M+H]⁺ (96.56% purity, 220 nm)

### Example 80: Preparation of Compound SYY-C095

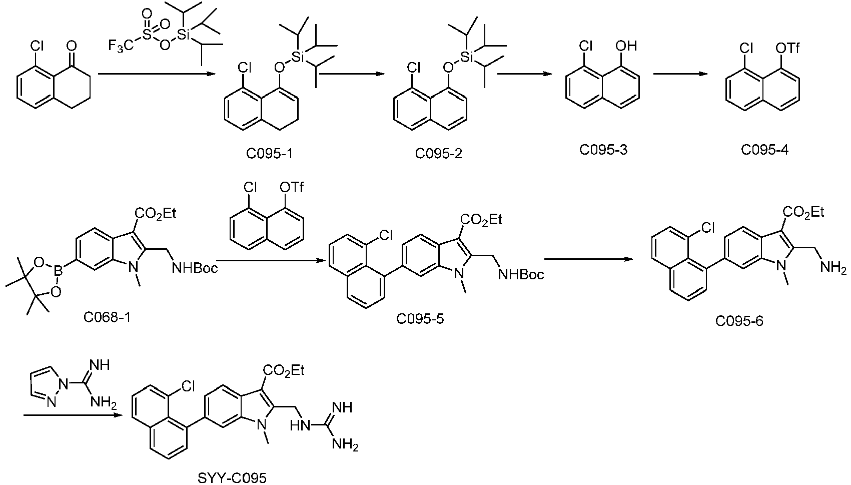

### Step 1:

8-Chloro-A-tetralone (1.29g,7.14 mmol) was dissolved in DCM(40 ml), TEA(1.56g,12.13 mmol) was added, replaced with N₂ for three times, a solution of triisopropylsilyl trifluoromethanesulfonate (2.4g,7.85 mmol) in DCM(5 ml) was added dropwise, and the mixture was stirred overnight at room temperature after addition. TLC showed the reaction was completed. The reaction solution was washed with water, washed with saturated NaHCO₃ solution, dried over anhydrous sodium sulfate, and concentrated to obtain 095-1(2.4g, crude).

### Step 2:

C095-1(2.4g, crude) was dissolved in CH3CN(30 ml), DDQ(3.25g,14.24 mmol) was added at room temperature, and heated to 80°C to react for 5 h. The reaction solution was concentrated, and the crude product was separated and purified by silica gel column chromatography to obtain C095-2(2.1g, yield for two steps: 88%) as a white solid.

### Step 3:

C095-2(2.4g,7.16 mmol) was dissolved in NMP(4 ml), MeOH(5 ml), H₂O(2 ml) and NaOH(1.7g,43.00mmol) were added at room temperature, and the mixture was heated 60°C to react for 1 h. TLC showed the reaction was completed. The reaction solution was cooled to room temperature, washed with 1 M HCl solution, extracted with ethyl acetate, the organic phases were combined, washed with saturated salt water, dried over anhydrous sodium sulfate, and concentrated, and the crude product was purified by silica gel column chromatography to obtain C095-3(1.2g, yield 94%).

### Step 4:

C095-3(1.2g,6.72 mmol) was dissolved in DCM(15 ml), pyridine (1.6g,20.16 mmol) was added at room temperature, a solution of Tf₂O(2.3g,8.06 mmol) in DCM(2 ml) was added under an ice bath, and the mixture was reacted under an ice bath for 1 h. TLC showed the reaction was completed. The reaction solution was slowly added with 1 M HCl solution, extracted with dichloromethane, and the organic phases were combined, washed with saturated salt water, dried over anhydrous sodium sulfate, and concentrated to obtain C095-4(1.1g, crude).

### Step 5:

C068-1(300 mg, 0.65 mmol) and C095-4(410 mg, crude) were dissolved in DMF(10 ml), water (1 ml), Cs₂CO₃(430 mg, 1.30 mmol) and PdCl₂(dppf)(60 mg,0.065 mmol) were added, replaced with N₂ for three times, and the mixture was heated to 100 °C to reflux for 2 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was separated and purified by silica gel column chromatography to obtain C095-5(290 mg, yield 90%).

### Step 6:

C095-5(290 mg,) was dissolved in DCM(6 ml), TFA(3 ml) was added, and the mixture was reacted at room temperature for 1 h. TLC showed the reaction was completed. The reaction solution was concentrated, neutralized with saturated NaHCO₃ solution, solids were precipitated, filtered, and the filter cake was dissolved with DCM, dried over anhydrous Na₂SO₄, and concentrated to obtain C095-6(270 mg, crude). LC-MS:m/z = 376.1[M-NH₂]⁺.

### Step 7:

C095-6(270 mg, crude) was dissolved in CH₃CN(8 ml), DIPEA(360 mg,2.76 mmol) was added, stirred at room temperature for 20min, then 1H-pyrazol-1-formamidine hydrochloride (160 mg,1.04 mmol) was added, and the mixture was stirred for a weekend at room temperature. The reaction solution was filtered, and the filter cake was washed and dried to obtain a white solid (160 mg, yield for two steps: 53%) . ¹H NMR: (DMSO-*d₆*, 400MHz): δ1.38 (t, J = 6.4 Hz, 3H), 3.87 (s, 3H), 4.37 (d, J = 6.8 Hz, 2H), 4.99 (s, 2H), 7.17 (d, J = 8.0 Hz, 1H), 7.42-8.15 (m, 12H). LC-MS: m/z=435.1 [M+H]⁺ (98.26% purity, 254 nm)

### Example 81: Preparation of Compound SYY-C096

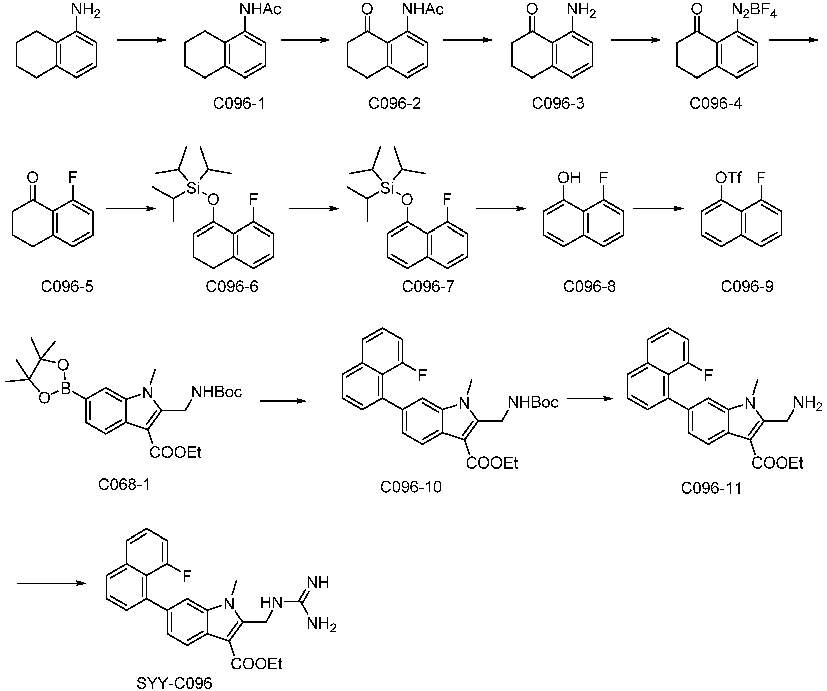

### Step 1:

Tetrahydronaphthalen-1-amine (3.0g,20.38 mmol) was dissolved in dichloromethane (50 mL), DMAP(342 mg,2.80 mmol) and pyridine (6.9g,87.23 mmol) were added, the reaction solution was cooled to about 0 °C, acetic anhydride (3.5g,34.28 mmol) was added dropwise, and the mixture was reacted at room temperature for 18 h after addition. TLC detection showed the reaction was completed. The reaction solution was quenched with water, washed with citric acid, dried over anhydrous sodium sulfate, and concentrated to obtain C096-1(3.8g, crude) as a gray solid.

### Step 2:

C096-1(8.0g,42.27 mmol) was dissolved in acetone (300 mL), 15% magnesium sulfate (899 mg,7.47 mmol) aqueous solution (5 mL) was added at room temperature, potassium permanganate solid (2.0g,12.66 mmol) was added in batches in about half an hour, and the mixture was stirred overnight at room temperature. TLC detection showed the raw materials were reacted completely. The reaction solution was added with ethyl acetate, stirred vigorously, filtered with diatomite, washed, the filtrate was stratified, washed with water, and washed with saturated salt water, dried over anhydrous sodium sulfate, and concentrated, and the crude product was purified by silica gel column chromatography to obtain C096-2(6.5g, yield 76%) as a yellow solid. ¹H NMR (400 MHz, cdcl₃) δ2.05-2.17 (m, 2H), 2.25 (s, 3H), 2.65-2.76 (m, 2H), 2.99 (t, *J* = 6.0 Hz, 2H), 6.94 (dd, *J* = 7.6, 0.8 Hz, 1H), 7.46 (t, *J* = 8.0 Hz,1H), 8.61 (d, *J* = 8.4 Hz, 1H), 12.16 (s, 1H). LCMS:m/z=204.2[M+H]⁺

### Step 3:

C096-2(6.5g,31.98mmol) was dissolved in ethanol (50 mL), concentrated hydrochloric acid (5 mL) was added, and the mixture was heated to 90 °C to react for 2 h. TLC detection showed the reaction was completed. The reaction solution was cooled to 0 °C, no solid was precipitated, and directly concentrated to obtain C096-3(6.2g, crude), which was directly used in the reaction.

### Step 4:

C096-3(5.2g, crude product) was dispersed in fluoroboric acid (50 mL), heated to 60 °C and then completely dissolved, cooled to 0-5 °C, a solution of sodium nitrite (2.0g,28.99mmol) in water (2mL) was added dropwise, and continued to stir and react at 0-5 °C for 30 minutes after the addition. No solid was precipitated from the reaction system, and the reaction solution was cooled to about -60 °C, solids were precipitated, and filtered quickly, washed with tetrahydrofuran and MTBE and thoroughly dried to obtain C096-4(3.1g, crude) as a light yellow solid, which turned brown solid when placed.

### Step 5:

C096-4(3.1g, crude) was dispersed in toluene (20 mL) and heated to reflux to react for 1h. The reaction solution was cooled to room temperature, and concentrated, and the crude product was purified by column chromatography to obtain C096-5(880 mg, yield for three steps: 17%) as a light green oil. ¹H NMR (400 MHz, cdcl₃) δ2.16-2.06 (m, 2H), 2.63-2.67 (m, 2H), 2.97 (t, *J =* 6.1 Hz, 2H), 6.97 (dd, *J =* 11.3, 8.3 Hz, 1H), 7.05 (d, *J* = 7.6 Hz, 1H), 7.41 (td, *J* = 8.0, 5.2 Hz, 1H)

### Step 6:

C096-5(880 mg,5.36 mmol) and triethylamine (920 mg,9.09 mmol) were dissolved in dichloromethane (15 mL), triisopropylsilyl trifluoromethanesulfonate (1.8g,5.87 mmol) was added under the protection of nitrogen, and the mixture was stirred and reacted at room temperature for 3h. TLC detection showed the reaction was completed. The reaction solution was quenched with ice water, washed with saturated sodium bicarbonate, the liquid was separated, dried over anhydrous sodium sulfate, and concentrated to obtain C096-6(1.7g, crude) as a yellow oil.

### Step 7:

C096-6(1.7g, crude) was dissolved in acetonitrile (30mL), DDQ(2.4g,10.57mmol) was added at room temperature, and the reaction solution was heated to reflux to react for 3h. The reaction solution was concentrated and directly purified by silica gel column chromatography to obtain C096-7(920 mg, yield for two steps: 53%) as a colorless oil.

### Step 8:

C096-7(920 mg,4.05mmol) was dissolved in a mixed solvent of NMP(2 ml), methanol (4 ml) and water (1.6 ml), sodium hydroxide (615 mg,15.38 mmol) was added at room temperature, heated to 60°C to react for 2.0 h. TLC monitored that the reaction was completed. The reaction solution was cooled to room temperature, added with 2N hydrochloric acid dropwise to adjust PH to be neutral, extracted with ethyl acetate, the organic phases were combined, washed with saturated salt water, dried over anhydrous sodium sulfate, and concentrated, and the crude product was purified by silica gel column chromatography (PE) to obtain C096-8(400 mg, yield 61%) as a light yellow solid.

### Step 9:

Under the protection of nitrogen, C096-8(400 mg,2.47 mmol) was dissolved in DCM(20 ml), cooled to 0°C under an ice water bath, 60% NaH(90 mg,2.25 mmol) was added, reacted for 20 min after addition, trifluoromethanesulfonic anhydride (626 mg,2.22mmol, diluted with 1.0 ml DCM) was added dropwise at 0°C, and the mixture was reacted at 0°C for 20 min after addition. TLC monitored the raw materials were reacted completely. The reaction solution was quenched with ice water, and the liquid was separated, and the organic phases were combined, washed with saturated salt water, dried over anhydrous sodium sulfate, and concentrated to obtain C096-9(1.3g, crude) as a light brown oil.

### Step 10:

Under the protection of nitrogen, C068-1(250 mg,0.55 mmol) was added into DMF(10 ml), C096-9(730 mg, crude), Pd(dppf)Cl₂ dichloromethane complex (45 mg,0.055 mmol) and Cs₂CO₃(356 mg,1.09 mmol) were added at room temperature, replaced with nitrogen for 3 times, and the mixture was heated to 100°C to react for 2.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by Pre-TLC (PE/EA = 10/1) to obtain C096-10(235 mg, yield 90%) as a light yellow oil.

### Step 11:

Under the protection of nitrogen, C096-10(235 mg,0.49 mmol) was dissolved in DCM(10 ml), cooled to 0°C under an ice bath, trifluoroacetic acid (2.0 ml, diluted with 2.0 ml DCM) was added dropwise, and the mixture was reacted at room temperature for 0.5 h after addition. TLC showed the raw materials were reacted completely. The reaction solution was added with saturated sodium bicarbonate aqueous solution to adjust to be weak alkalinity, stirred for 20min, extracted with dichloromethane, washed with saturated salt water, dried over anhydrous sodium sulfate, and concentrated to obtain C096-11(178mg, crude) as a light brown yellow oil. LCMS:m/z=360.1[M-NH₂]⁺

### Step 12:

Under the protection of nitrogen, C096-11(178 mg, crude) was dissolved in acetonitrile (10ml), and DIEA(305 mg,2.36 mmol) and 1H-pyrazol-1-formamidine hydrochloride (139 mg,0.95 mmol) were added at room temperature and the mixture was reacted at room temperature for 48h. The reaction solution was filtered, and the filter cake was washed and dried to obtain SYY-C096(140 mg, yield for two steps: 68%) as a white solid. ¹H NMR: (DMSO-*d₆*, 400MHz): δ1.41 (t, J = 7.2 Hz, 3H), 3.87 (s, 3H), 4.39 (q, J = 7.2 Hz, 2H), 4.97 (s, 2H), 7.21-7.69 (m, 9H), 7.89 (d, J = 8.0 Hz, 1H), 8.03-8.07 (m, 2H), 8.33 (m, 1H). (90.60% purity, 254 nm) LCMS:m/z=419.2[M+H]⁺

### Example 82: Preparation of Compound SYY-C097

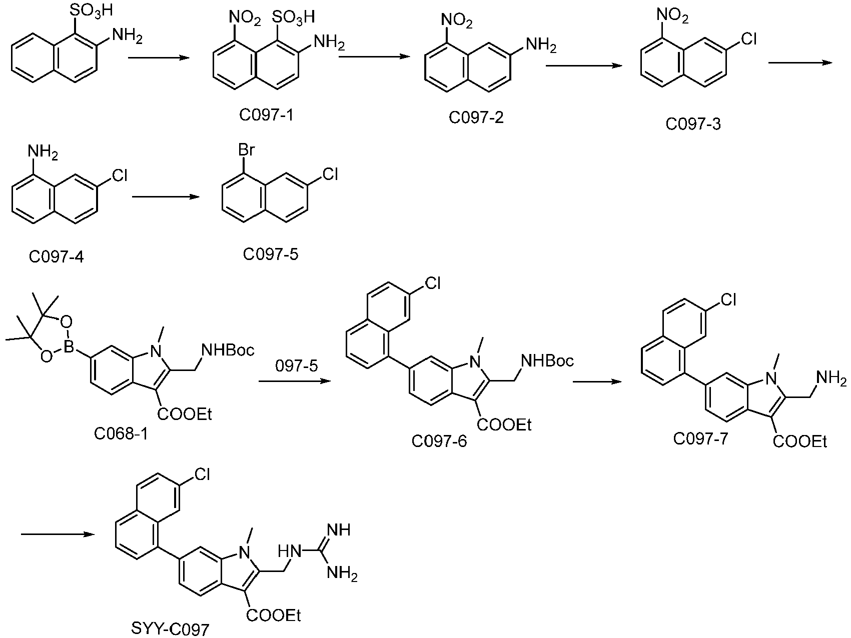

### Step 1:

2-Naphthylamine-1-sulfonic acid (20.0g,89.59 mmol) was dissolved in concentrated sulfuric acid (50 mL) cooled under an ice bath, cooled to about -15 °C, potassium nitrate (13.6g,134.52mmol) was added in batches, and the mixture was reacted at -10 °C for 2 h after addition. The reaction solution was poured into ice water, solids were precipitated, filtered, and the filter cake was washed to obtain C097-1 (crude) as a brown solid mixture.

### Step 2:

C097-1 (crude) was dissolved in 50% sulfuric acid (50 mL), heated to 100 °C to react for 1h. TLC detection showed that there was a new point. The reaction solution was cooled to room temperature, poured into ice water, neutralized with saturated sodium carbonate, extracted with ethyl acetate, the organic phases were combined, washed with saturated salt water, dried over anhydrous sodium sulfate, and concentrated, and the crude product was purified by silica gel column chromatography to obtain C097-2(3.7g, yield for two steps: 22%) as a red solid. ¹H NMR (400 MHz, DMSO_D6) δ 6.18 (s, 2H), 7.09 (dd, *J* = 8.8, 2.2 Hz, 1H), 7.19 (t, *J* = 7.8 Hz, 1H), 7.54 (d, *J* = 2.1 Hz, 1H), 7.81 (d, *J* = 8.9 Hz, 1H), 8.04 (d, *J* = 7.9 Hz, 1H), 8.18 (dd, *J=* 7.8, 1.2 Hz, 1H).

### Step 3:

C097-2(3.6g,19.13 mmol) was dispersed in acetic acid (35 mL) and hydrochloric acid (10 mL), cooled to 0-5 °C, a solution of sodium nitrite (1.32g,19.13 mmol) in water (5 mL) was added dropwise, and the reaction solution became clarified after addition. Diazonium salt solution was slowly added to a solution of cuprous chloride (3.8g,38.38mmol) and concentrated hydrochloric acid (40 mL) which was heated to 65°C, continued to react for half an hour after addition. TLC detection showed that the reaction was completed. The reaction solution was cooled to room temperature, added with water, extracted with ethyl acetate, and the organic phases were combined, washed with saturated salt water, dried over anhydrous sodium sulfate, and concentrated to obtain C097-3(4.5g, crude) which was directly used in the next step.

### Step 4:

C097-3(4.5g, crude) was dissolved in ethanol (50 mL) and water (10 mL), ammonium chloride solid (5.1g,95.34 mmol) was added, and the mixture was heated to 50 °C, iron powder (5.3g,94.90mmol) was added in batches, and the reaction mixture was heated to 80 °C to react for 2 h. TLC detection showed the reaction was completed. The reaction solution was filtered while hot, the filtrate was concentrated to a small amount, added with water, extracted with ethyl acetate, the organic phases were combined, washed with saturated salt water, dried over anhydrous sodium sulfate, and concentrated, and the crude product was purified by silica gel column chromatography to obtain C097-4(3.2g, yield for two steps: 80%) as a red oil. ¹H NMR (400 MHz,DMSO_D6) δ 5.82 (s, 2H), 6.73 (dd, *J=* 7.5, 1.0 Hz, 1H), 7.10 (d, *J* = 8.1 Hz, 1H), 7.19 - 7.26 (m, 1H), 7.39 (dd, *J* = 8.8, 2.1 Hz, 1H), 7.77 (d, *J=* 8.8 Hz, 1H), 8.19 (d, *J=* 2.0 Hz, 1H).

### Step 5:

C097-4(1.0g,5.63 mmol) was dissolved in acetic acid (50 ml) and water (10 ml), 48% hydrobromic acid (5 ml) was added dropwise at room temperature, heated to 60°C for 1h after addition, cooled to 0°C under an ice bath, NaNO₂(428 mg,6.20mmol, dissolved with 2.0 ml water) was added dropwise, and the mixture was reacted at 0°C for 1.5 h after addition. TLC monitored the raw materials were reacted completely. Diazonium salt solution was slowly dropped into a solution of CuBr(1.77g,12.34 mmol) in 48% hydrobromic acid (20mL) which was heated to 60 °C, and the mixture was reacted at 60°C for 0.5h after addition. TLC monitored the reaction was completed. The reaction solution was cooled to room temperature, added with water, extracted with ethyl acetate, the organic phases were combined, washed with saturated sodium bicarbonate solution, and washed with saturated salt water, dried over anhydrous sodium sulfate, and concentrated, and the crude product was purified by silica gel column chromatography to obtain C097-5(1.3g, yield 97%) as a white solid.

### Step 6:

Under the protection of nitrogen, C068-1(250 mg, 0.55 mmol) was dissolved in DMF(10 ml), C097-5(264 mg,1.09 mmol), Pd(dppf)Cl₂ dichloromethane complex (45 mg,0.055 mmol) and Cs₂CO₃(356 mg,1.09 mmol) were added at room temperature, replaced with nitrogen for 3 times, and the mixture was heated to 100°C to react for 2.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by Pre-TLC (PE/EA = 10/1) to obtain C097-6(215 mg, yield 80%) as a light yellow oil.

### Step 7:

C097-6(215mg,0.44mmol) was dissolved in DCM(10 ml), cooled to 0°C under an ice bath, trifluoroacetic acid (2.0 ml, diluted with 2.0 ml DCM) was added dropwise, and the mixture was reacted at room temperature for 0.5 h after addition. TLC showed the raw materials were reacted completely. The reaction solution was added with saturated sodium bicarbonate aqueous solution to adjust to be weak alkalinity, stirred for 20min, extracted with dichloromethane, and the organic phases were combined, washed with saturated salt water, dried over anhydrous sodium sulfate, and concentrated to obtain C097-7(120 mg, crude) as a light yellow oil. LCMS:m/z=376.1[M-NH₂]⁺

### Step 8:

Under the protection of nitrogen, C097-7(120 mg,0.31 mmol) was dissolved in acetonitrile (5 ml), and DIEA(188 mg,1.45 mmol) and 1H-pyrazol-1-formamidine hydrochloride (86 mg,0.59mmol) were added at room temperature, and the mixture was reacted at room temperature for 24h. The reaction solution was filtered, and the filter cake was washed and dried to obtain a white solid (70 mg, yield for two steps: 37%) . ¹H NMR: (DMSO-*d₆*, 400MHz): δ1.42 (t, J = 7.2 Hz, 3H), 3.91 (s, 3H), 4.41 (q, J = 7.2 Hz, 2H), 5.00 (s, 2H), 7.35-7.68 (m, 7H), 7.75 (s, 1H), 7.78 (s, 1H), 8.04 (d, J = 8.0 Hz, 1H), 8.10 (d, J = 8.8 Hz, 1H), 8.17 (d, J = 8.4 Hz, 1H), 8.42 (br, 1H). LCMS:m/z=435.1[M+H]⁺ (97.73% purity, 254 nm)

### Example 83: Preparation of Compound SYY-C098

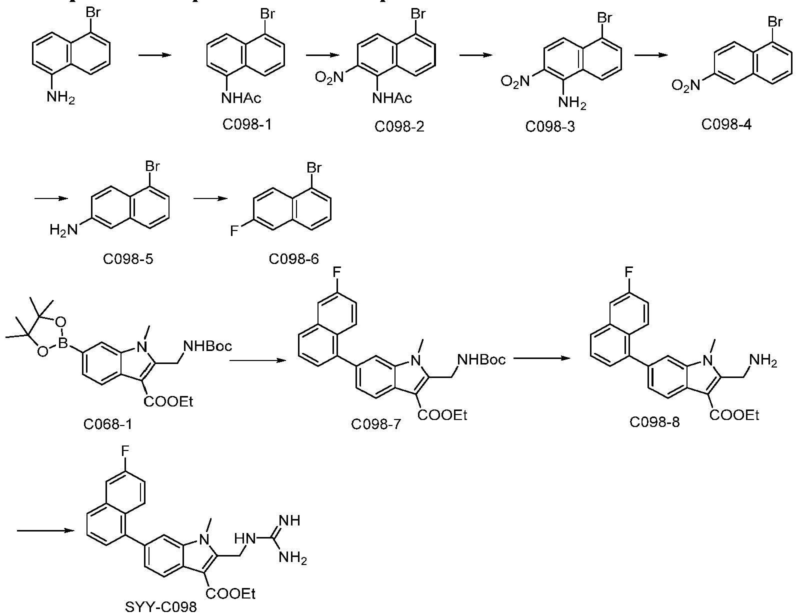

### Step 1:

Under the protection of nitrogen, 1-amino-5-bromonaphthalene(10.0g,45.03 mmol) was dissolved in dichloromethane (140 ml), pyridine (10.8g,136.54 mmol) and DMAP(55 mg,0.45 mmol) were added at room temperature, acetic anhydride (6.0g,58.77 mmol, diluted with 6 ml DCM) was added dropwise, and the mixture was reacted at room temperature for 4.0h after addition. TLC monitored the reaction was completed. The reaction solution was concentrated, 40ml of dichloromethane was added, stirred for 30min, filtered, and the filter cake was washed and dried to obtain C098-1(10.8g, crude) as a white solid.

### Step 2:

Under the protection of nitrogen, C098-1(10.4g, crude) was dissolved in acetic anhydride (200 ml), cooled to 0°C under an ice water bath, 65% nitric acid solution (9.4 ml,137.70 mmol) was added dropwise (preparation method: 9.4 ml nitric acid was added dropwise into 25 ml acetic anhydride at 0°C, and stirred for half an hour), stirred at room temperature for 20min, and the mixture was reacted at 0°C for 1.5 h after addition. TLC monitored the reaction was completed. The reaction solution was added with 200 ml of water, stirred for 30min, filtered, and the filter cake was washed and dried to obtain C098-2(13.6g, crude) as a light yellow solid.

### Step 3:

C098-2(13.6g, crude) was dissolved in ethanol (100 mL), concentrated hydrochloric acid (40 mL) was added at room temperature, and the mixture was heated to 90 °C to react for 3 h. TLC detection showed that the raw materials were basically reacted completely. After the reaction solution was concentrated to 1/2 volume, cooled to 0 °C and stirred for 1h, solids were precipitated, filtered, the filter cake was neutralized with saturated sodium carbonate aqueous solution, extracted with ethyl acetate, the organic phases were combined, washed with saturated salt water, dried over anhydrous sodium sulfate, and concentrated, and the crude product was purified by silica gel column chromatography to obtain C098-3(3.2g, yield for three steps: 27%) as a yellow-brown solid.

### Step 4:

Sodium nitrite solid (566 mg,8.20 mmol) was dissolved in concentrated sulfuric acid (20 mL), heated to 70 °C to react for 15 minutes, then cooled to room temperature, a solution of C098-3(2.0g,7.49 mmol) in acetic acid (50 mL) was added dropwise, and the mixture was reacted at room temperature for half an hour after addition. TLC detection showed the diazotization was completed. The diazonium salt solution was poured into hypophosphoric acid solution (80 mL), and the mixture was reacted for 3h at room temperature. TLC detection showed that the reaction was completed. The reaction solution was poured into water, solids were precipitated, filtered, and the filter cake was washed and dried to obtain C098-4(2.0g, wet weight) as a yellow-brown solid. TLC purity was good and did not need purification.

### Step 5:

C098-4(2.0g,7.93 mmol) was dissolved in ethanol (40 mL) and water (8 mL), ammonium chloride (2.1g,39.26 mmol) was added, heated to 50°C, iron powder (2.2g,39.39 mmol) was added in batches, and the mixture was heated to 80 °C to react for 2 h. TLC detection showed the reaction was completed. The reaction solution was filtered while hot, washed, the filtrate was concentrated, added with water, extracted with ethyl acetate, the organic phases were combined, washed with saturated salt water, dried over anhydrous sodium sulfate, and concentrated, and the crude product was purified by silica gel column chromatography to obtain C098-5(1.5g, yield for two steps: 90%) as a red-brown solid. ¹H NMR (400 MHz, CDCl₃): δ 3.95 (br, 2H), 6.98 (d, *J =* 2.4 Hz, 1H), 7.06 (dd, *J=* 9.2, 2.4 Hz, 1H), 7.18- 7.25 (m, 1H), 7.50 - 7.58 (m, 2H), 8.07 (d, *J=* 9.2 Hz, 1H)

### Step 6:

C098-5(1.5g,6.75mmol) was dispersed in fluoroboric acid (15 mL), heated to 80 °C to make the dispersion uniform, cooled to 0-5 °C, aqueous solution of sodium nitrite (510 mg,7.39 mmol) was added dropwise, and the mixture was stirred at 0-5 °C for 1 h after addition. TLC detection showed that the raw materials were basically reacted completely. The reaction solution was filtered, washed with cold water, methanol and MTBE in turn until a yellow solid was obtained and dried to obtain 2.4g of yellow solid. 1.2g of the above yellow solid was dispersed in xylene (20mL) and heated to reflux to react for 1h. The reaction solution was cooled and concentrated, and the crude product was purified by silica gel column chromatography to obtain colorless oil, which was placed and solidified into white solid C098-6(380 mg, yield 50%).

### Step 7:

C068-1(300 mg,0.65 mmol) and C098-6(380 mg,1.69 mmol) were dissolved in DMF(10 mL) and water (0.5 mL), cesium carbonate solid (398 mg,1.22 mmol) and PdCl₂(dppf) dichloromethane complex (50 mg,0.061 mmol) were added, replaced with nitrogen for three times, and the mixture was heated to 100 °C for 3 h. TLC detection showted the raw materials were reacted completely. The reaction solution was cooled to room temperature, poured into water, extracted with ethyl acetate, the organic phases were combined, washed with saturated salt water, dried over anhydrous sodium sulfate, and concentrated, and the crude product was purified by silica gel column chromatography to obtain C098-7(310mg, yield 99%) as a white solid.

### Step 8:

Under the protection of nitrogen, C098-7(310 mg,0.65 mmol) was dissolved in DCM(10 ml), cooled to 0°C under an ice bath, trifluoroacetic acid (2.0 ml, diluted with 2.0 ml DCM) was added dropwise, and the mixture was reacted at room temperature for 0.5 h after addition. TLC showed the raw materials were reacted completely. The reaction solution was added with saturated sodium bicarbonate aqueous solution to adjust to be weak alkalinity, stirred for 20min, extracted with DCM, and the organic phases were combined, washed with saturated salt water, dried over anhydrous sodium sulfate, and concentrated to obtain C098-8(230mg, crude) as a light yellow oil. LCMS:m/z=360.1[M-NH₂]⁺

### Step 9:

Under the protection of nitrogen, C098-8(230 mg, 0.61 mmol) was dissolved in acetonitrile (10 ml), then DIEA(395 mg, 3.06mmol) and 1H-pyrazol-1-formamidine hydrochloride (180 mg,1.23mmol) were added, and the mixture was reacted for a weekend at room temperature. A white solid was precipitated in the reaction solution, filtered, and the filter cake was washed and dried to obtain SYY-C098(154 mg, yield 57%) as a white solid. ¹H NMR: (DMSO-*d₆*, 400MHz): δ1.42 (t, J = 7.2 Hz, 3H), 3.92 (s, 3H), 4.41 (q, J = 7.2 Hz, 2H), 5.00 (s, 2H), 7.24-7.71 (m, 7H), 7.77 (s, 1H), 7.82 (dd, J = 10.0, 2.8 Hz, 1H), 7.88 (dd, J = 9.6, 2.0 Hz, 1H), 7.98 (d, J = 8.4 Hz, 1H), 8.16 (d, J = 8.4 Hz, 1H), 8.42 (br, 1H). LCMS:m/z=419.2[M+H]⁺ (91.62% purity, 254 nm)

### Example 84: Preparation of Compound SYY-C099

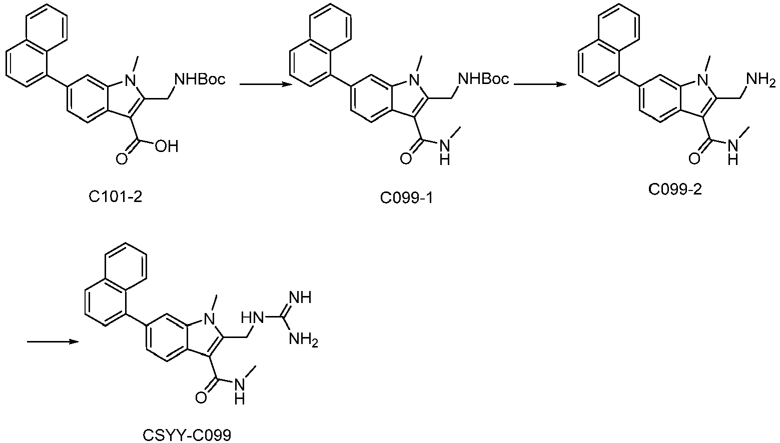

### Step 1:

C101-2(300 mg,0.70 mmol) was dissolved in DMF(15 mL), methylamine hydrochloride (95 mg,1.4 mmol), HATU(533 mg,1.4 mmol) and DIPEA(271mg,2.1 mmol) were added at room temperature, the mixture was reacted at room temperature for 16 h. TLC showed the reaction was completed. The reaction solution was quenched with water, extracted with ethyl acetate, the organic phases were combined, washed with water, and washed with saturated salt water, dried over anhydrous sodium sulfate, and concentrated, and the crude product was purified by silica gel column chromatography to obtain C099-1(260 mg, yield 84%) as a withe solid.

### Steps 2,3:

C099-1(260 mg, 0.59 mmol) and TFA(5 mL) were dissolved in dichloromethane (5 mL) and stirred at room temperature for 1 h. TLC showed the reaction was completed. The reaction solution was concentrated, the crude product was dissolved in acetonitrile (10 mL), DIPEA(227 mg,1.76 mmol) and1H-pyrazol-1-formamidine hydrochloride(103 mg,0.70 mmol) were added, and the mixture was stirred at room temperature overnight. White solids were precipitated in the reaction solution, filtered, and the filter cake was washed and dried to obtain SYY-C099(80 mg, yield for two steps: 35%) as a white solid. ¹H NMR: (DMSO-*d₆*, 400MHz): δ2.91 (d, J = 4.4 Hz, 3H), 3.90 (s, 3H), 4.83-4.84 (m, 2H), 7.08-7.33 (m, 3H), 7.43-7.62 (m, 4H), 7.72 (s, 1H), 7.86-8.02 (m, 4H), 8.29-8.37 (m, 1H), 8.99 (br, 1H). LC-MS: m/z=386.2 [M+H]⁺

### Example 85: Preparation of Compound SYY-C100

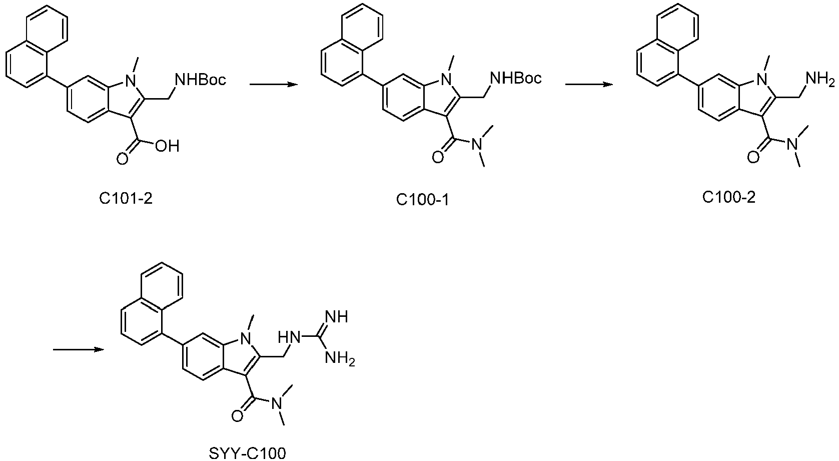

### Step 1:

C101-2(300 mg,0.70 mmol) was dissolved in DMF(15 mL), dimethylamine hydrochloride (114 mg,1.4 mmol), HATU(533 mg,1.4 mmol) and DIPEA(271mg,2.1 mmol) were added at room temperature, and the mixture was reacted at room temperature for 16 h. TLC showed the reaction was completed. The reaction solution was quenched with water, extracted with ethyl acetate, the organic phases were combined, washed with water, and washed with saturated salt water, dried over anhydrous sodium sulfate, and concentrated, and the crude product was purified by silica gel column chromatography to obtain C 1 00-1 (280 mg, yield 88%) as a withe solid.

### Step 2:

C100-1(280 mg, 0.612 mmol) and TFA(5 mL) were dissolved in dichloromethane (5 mL) and stirred at room temperature for 1 h. TLC showed the reaction was completed. The reaction solution was concentrated, the crude product was dissolved in acetonitrile (10 mL), DIPEA(237 mg,1.836 mmol) and1H-pyrazol-1-formamidine hydrochloride(108 mg,0.734 mmol) were added, and the mixture was stirred at room temperature overnight. White solids were precipitated in the reaction solution, filtered, and the filter cake was washed and dried to obtain white solid (100 mg, yield 41%). ¹H NMR: (DMSO-*d₆*, 400MHz): δ3.10 (s, 6H), 3.87 (s, 3H), 4.67 (s, 2H), 7.00-7.30 (m, 3H), 7.43-7.76 (m, 7H), 7.83- 8.06 (m, 3H), 8.73-8.80 (m, 1H). LC-MS: m/z=400.2[M+H]⁺(99.21% purity, 220 nm)

### Example 86: Preparation of Compound SYY-C101

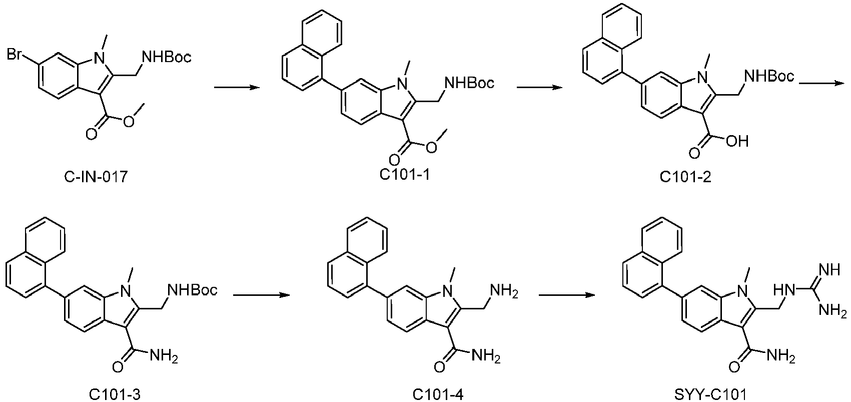

### Step 1:

C- IN-017(1.5g,3.78 mmol) was dissolved in a mixed solvent of 1,4-dioxane (30 mL) and water (15 mL), boric acid (975 mg,5.67 mmol), Pd(dppf)Cl₂(139 mg,0.17 mmol) and sodium carbonate (801 mg,7.56 mmol) were added at room temperature, and the mixture was heated to reflux to react for 2 hours under the protection of nitrogen. TLC showed the reaction was completed. The reaction solution was cooled to room temperature, quenched with water, extracted with ethyl acetate, the organic phases were combined, washed with water, and washed with saturated salt water, dried over anhydrous sodium sulfate, and concentrated, and the crude product was purified by silica gel column chromatography to obtain C101-1(1.61g, yield 96%) as a white solid.

### Step 2:

C101-1(1.61g,3.62 mmol) and potassium hydroxide (2.10g, 37.4 mmol) were dispersed into a mixed solvent of ethanol (20 mL) and water (20 mL), and the mixture was heated to reflux to react for 2 hours. TLC showed the reaction was completed. The reaction solution was cooled to room temperature, added with water, extracted with ethyl acetate, and the organic phases were combined, washed with water, washed with saturated salt water, dried over anhydrous sodium sulfate, and concentrated to obtain C101-2(1.2 g, crude) as a light yellow solid.

### Step 3:

C101-2(300 mg, crude) was dissolved in DMF(15 mL), ammonium chloride (75 mg,1.40 mmol), HATU(533 mg,1.40 mmol) and DIPEA(271mg,2.10 mmol) were added at room temperature, and the mixture was reacted at room temperature for 16 h. TLC showed the reaction was completed. The reaction solution was quenched with water, extracted with ethyl acetate, the organic phases were combined, washed with water, and washed with saturated salt water, dried over anhydrous sodium sulfate, and concentrated, and the crude product was purified by silica gel column chromatography to obtain 101-3(220 mg, yield for two steps: 74%) as a withe solid.

### Steps 4 and 5:

C101-3(220 mg, 0.51 mmol) and TFA(5 mL) were dissolved in dichloromethane (5 mL) and stirred at room temperature for 1 h. TLC showed the reaction was completed. The reaction solution was concentrated, the solid was dissolved in acetonitrile (10 mL), DIPEA(198 mg,1.53 mmol) and 1H-pyrazol-1-formamidine hydrochloride(90 mg,0.61 mmol) were added, and the mixture was stirred at room temperature overnight. White solids were precipitated in the reaction solution, filtered, and the filter cake was washed and dried to obtain SYY-C101(70 mg, yield 37%) as a white solid. ¹H NMR: (DMSO-*d₆*, 400MHz): δ3.89 (s, 3H), 4.86 (s, 2H), 6.99-7.33 (m, 3H), 7.40-8.09 (m, 12H), 8.81 (br, 1H). LC-MS:m/z=372.2[M+H]⁺

### Example 87: Preparation of Compound SYY-C102

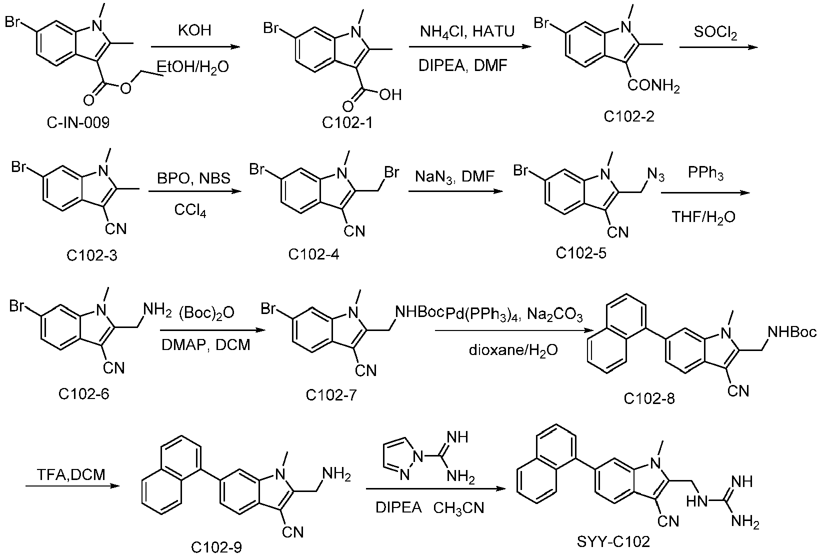

### Step 1:

C- IN-009(11.2g,40 mmol) was dissolved in ethanol (100 ml), water (20 ml) and potassium hydroxide (11.2g,200 mmol) were added at room temperature, and the mixture was heated to 100 °C to react for 4 h. TLC showed the reaction was completed. The reaction solution was concentrated, the pH was adjusted to weak acidity with 1N diluted hydrochloric acid, solids were precipitated, filtered, and the filter cake was washed and dried to obtain C 1 02-1 (5 .4g, crude).

### Step 2:

C102-1(3.6g, crude) and ammonium chloride (1.6g,29.9 mmol) were dissolved in DMF(30 ml), HATU(10.4g,27.4 mmol) and DIPEA(5.2g,40.2 mmol) were added at room temperature, and the mixture was reacted at room temperature for 16 hours. TLC showed the reaction was completed. The reaction solution was added with water, extracted with ethyl acetate, and the organic phases were combined, washed with saturated salt water, dried over anhydrous sodium sulfate, and slurried with dichloromethane after concentration to obtain C102-2(2.5g, crude).

### Step 3:

C102-2(2.0g, crude) was dissolved in SOCl₂(15 ml) and heated to 80 °C to react for 2 h. TLC showed the reaction was completed. The reaction solution was concentrated to obtain C102-3 (900 mg, crude).

### Step 4:

C102-3(900 mg, crude) was dissolved in carbon tetrachloride (15 ml), NBS(720 mg,4.04 mmol) and BPO(180 mg,0.74 mmol) were added at room temperature, and the mixture was heated to 70 °C to react for 2 h. TLC showed the reaction was completed. The reaction solution was cooled to room temperature, added with water, extracted with dichloromethane, and the organic phases were combined, dried over anhydrous sodium sulfate, and concentrated to obtain C102-4(600 mg, crude).

### Step 5:

C102-4(600 mg, crude) was dissolved in DMF(20 ml), sodium azide (131 mg,2.0 mmol) was added at room temperature, and the mixture was heated to 70 °C to react for 2 h. TLC showed the reaction was completed. The reaction solution was cooled to room temperature, added with water, extracted with ethyl acetate, and the organic phases were combined, dried over anhydrous sodium sulfate, and concentrated to obtain C102-5 (crude), which was directly used in the next step.

### Step 6:

The C102-5 (crude) was dissolved in THF(10 ml), water (2 ml) and PPh₃(720 mg, 2.74 mmol) were added, and the mixture was reacted at room temperature for 16 hours. TLC showed the reaction was completed. The reaction solution was concentrated, 1M hydrochloric acid was added to form salt, extracted with ethyl acetate, the organic phase was discarded, and the aqueous phase was adjusted to be weak alkalinity with saturated sodium bicarbonate solution, extracted with ethyl acetate, and the organic phases were combined, dried over anhydrous sodium sulfate, and concentrated to obtain C102-6(320 mg, crude).

### Step 7:

C102-6(320 mg, crude) was dissolved in DCM(10 ml), DMAP(15 mg, 0.12 mmol) was added at room temperature, then (Boc)₂O(320 mg,1.47 mmol) was slowly added, and the mixture was reacted at room temperature for 1h after the addition. TLC showed the reaction was completed. The reaction solution was directly purified by silica gel column chromatography to obtain C102-7(170 mg, yield for 7 steps: 2%).

### Step 8:

C102-7(170 mg,0.47mmol) was dissolved in 1, 4-dioxane (10 ml), 1-naphthaleneboronic acid (100 mg,0.56 mmol), sodium carbonate (100 mg,0.94 mmol) and Pd(PPh₃)₄(50 mg,0.04 mmol) were added at room temperature, replaced with nitrogen for three times, and the mixture was heated to 100°C to react for 2 h. TLC showed the reaction was completed. The reaction solution was cooled to room temperature, added with water, extracted with ethyl acetate, the organic phases were combined, dried over anhydrous sodium sulfate, and concentrated, and the crude product was separated and purified by pre-TLC to obtain C102-8(160 mg, yield 83%).

### Step 9:

C102-8(160 mg,0.39mmol) was dissolved in DCM(6 ml), TFA(3 ml) was added at room temperature, and the mixture was reacted at room temperature for 30 min. TLC showed the reaction was completed. The reaction solution was concentrated to obtain C102-9 (crude), which was directly used in the next step.

### Step 10:

C102-9 (crude) was dissolved in acetonitrile (3 ml), 1H-pyrazol-1-formamidine hydrochloride (100 mg,0.68 mmol) and DIPEA(100 mg,0.77 mmol) were added, the mixture was stirred at room temperature for a weekend, and solids were precipitated. The reaction solution was filtered, and the filter cake was washed and dried to obtain a solid(76 mg, yield for two steps: 55%). ¹H NMR (400 MHz, DMSO-d6) δ3.85 (s, 3H), 4.88 (s, 2H), 7.38 (dd, J = 8.0, 0.8 Hz, 1H), 7.46-7.66 (m, 7H), 7.74 (d, J = 8. 0 Hz, 1H), 7.78-7.81 (m, 2H), 7.98 (d, J = 8.0 Hz, 1H), 8.02 (d, J = 8.0 Hz, 1H), 8.47 (br, 3H). LC-MS:m/z=354.2 [M+H]⁺

### Example 88: Preparation of Compound SYY-C103

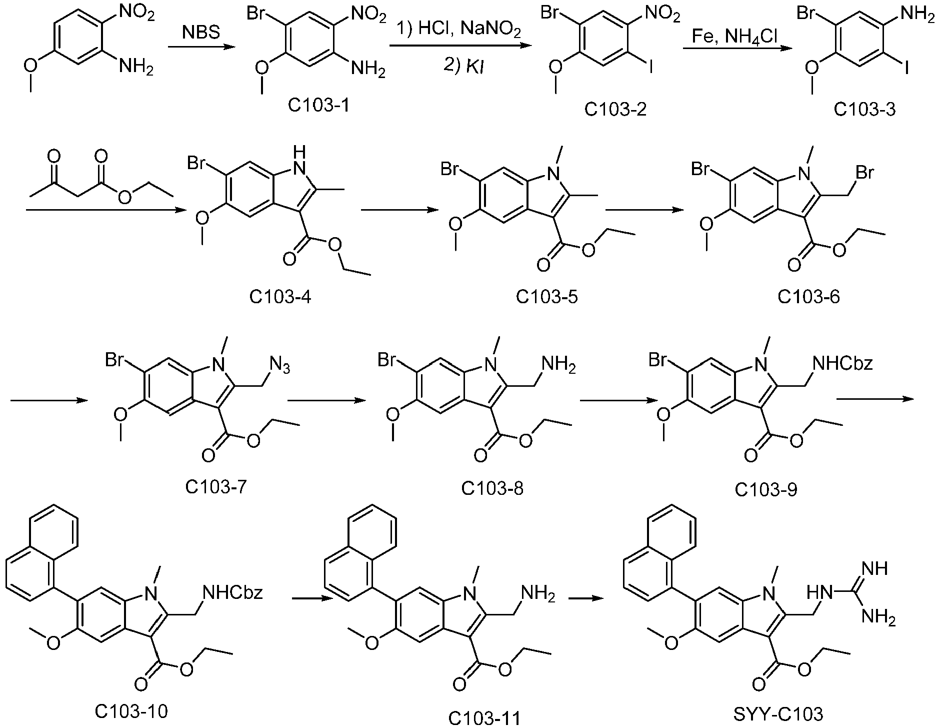

### Step 1:

Under the protection of nitrogen, 5-methoxy-2-nitroaniline (20.0g,118.94mmol) was dissolved in DMF(200 ml), NBS(21.2g,119.11 mmol) was added in batches at room temperature, and the mixture was reacted at room temperature for 0.5 h after the addition. TLC showed the raw materials were reacted completely. Water was added to the reaction solution, and a large amount of solid was precipitated, filtered, and the filter cake was washed and dried to obtain yellow-brown solid crude product. The crude product was dissolved in 120 mL mixed solution of PE/EA (5/1), stirred for 30 minutes, filtered again, and the filtrate was concentrated to obtain C103-1(14.7g, crude product) as a brown solid, which was directly used in the next step. ¹H NMR (400 MHz, DMSO_d6): δ3.85 (s, 3H), 6.59 (s, 1H), 7.59 (br, 2H), 8.11 (s, 1H).

### Step 2:

C103-1(14.7g, crude) was dispersed in hydrochloric acid (200 mL, 6 N), cooled to 0 °C, sodium nitrite (4.3g, 62.33 mmol, dissolved in 20 mL of water) was added dropwise, reacted under an ice water bath for 30 minutes after addition, then potassium iodide (28.2g, 169.88 mmol, dissolved in 50 mL of water) was slowly added dropwise, and the mixture was reacted for 1 hour at 0 °C. The reaction solution was diluted with water (300 mL), extracted with ethyl acetate, and the organic phases were combined, washed with saturated sodium sulfite solution (500 mL * 3), dried over anhydrous sodium sulfate, and concentrated to obtain C103-2(15.8g, crude) as a brown solid, which was directly used in the next step.

### Step 3:

C103-2(15.8g, crude) was dispersed in EtOH (150 mL)/H₂O (75 mL), ammonium chloride (9.7g, 181.34 mmol) and reduced iron powder (10g, 179.05mmol) were sequentially added, and the mixture was heated to 80 °C to react for 30 minutes. TLC detection showed the reaction was completed. The reaction solution was cooled to room temperature, diluted with ethyl acetate, filtered with diatomite to remove iron powder, the filtrate was concentrated and diluted with water, extracted with ethyl acetate, and the organic phases were combined, washed with saturated salt water, dried over anhydrous sodium sulfate, and concentrated to obtain crude product as a brown solid which was purified by silica gel column chromatography (eluent from pure PE to PE/EA = 20/1) to obtain C103-3 (9.5g, yield for three steps: 24%) as a yellow solid. LCMS:m/z=327.9/329.9 [M+H]⁺

### Step 4:

C103-3(9.5g, 28.97 mmol), ethyl acetoacetate (20.3g, 155.99 mmol), tetrazoleacetic acid (670 mg, 5.23 mmol), cesium carbonate (16.9g, 51.87 mmol) and cuprous iodide (500 mg, 2.63mmol) were dispersed in DMSO(150 mL), replaced with nitrogen for more than 3 times, and the mixture was heated to 100 °C to react for 3 h. TLC detection showed the reaction was completed. The reaction solution was cooled to room temperature, added with water (200 mL) and stirred for 30 minutes, a large amount of solid was precipitated, filtered, and the filter cake was dissolved with ethyl acetate, dried over anhydrous sodium sulfate, and concentrated to obtain C103-4(5.4g, yield 60%) as a gray-white solid. ¹H NMR (400 MHz, DMSO_d6): δ 1.35 (t, *J* = 7.2 Hz, 3H), 2.61 (s, 3H), 3.84 (s, 3H), 4.26 (d, *J* = 7.2 Hz, 2H), 7.54 (s, 2H), 11.77 (s, 1H). LCMS:m/z=312.1/314.1 [M+H]⁺

### Step 5:

Under the protection of nitrogen, C103-4(5.4g,17.30 mmol) was dissolved in DMF(50 ml), cooled to 0 °C under an ice bath, NaH(1.3g,32.50 mmol) was added in batches, and the mixture was reacted at room temperature for 0.5 h after the addition. Methyl iodide (3.0g,21.14 mmol, diluted with 2.0 ml DMF) was added dropwise at 0 °C, and the mixture was reacted at 0°C for 20 min after addition. TLC showed the raw materials were reacted completely. The reaction solution was added with water, and a large amount of solid was precipitated, filtered, and the filter cake was dissolved with ethyl acetate, dried over anhydrous sodium sulfate and concentrated to obtain C103-5(4.7g, crude) as a yellow solid.

### Step 6:

Under the protection of nitrogen, C103-5(4.7g,14.41 mmol) was dissolved in carbon tetrachloride (100 ml), NBS(2.6g, 14.61 mmol) and BPO(347 mg,1.43 mmol) were added, and the mixture was heated to 80°C to react for 1.5 h. TLC showed the raw materials were reacted completely. The reaction solution was cooled to room temperature, and the crude product was purified by silica gel column chromatography (PE/EA = 5/1) to obtain C103-6(5.7g, two-step yield 81%) as a yellow solid.

### Step 7:

Under the protection of nitrogen, C103-6(5.7g,14.07 mmol) was dissolved in DMF(60 ml), NaN₃(1.37g,21.07mmol) was added at room temperature, and the mixture was heated to 50°C to react for 1.0 h. TLC showed the raw materials were reacted completely. The reaction solution was cooled to room temperature, added with water, solids were precipitated, stirred at room temperature for 20min, filtered, and the filter cake was washed and dried to obtain C103-7(4.0g, crude) as a yellow solid.

### Step 8:

C103-7(4.0g, crude) was dissolved in THF(60 ml) and water (20 ml), PPh₃(3.53g,13.46 mmol) was added at room temperature, and the mixture was heated to 50°C to react for 2.0 h. TLC showed the reaction was completed. The reaction solution was cooled to room temperature, extracted with ethyl acetate, the organic phases were combined, washed with saturated salt water, dried over anhydrous sodium sulfate, and concentrated, and the crude product was purified by silica gel column chromatography (DCM/MeOH = 20/1) to obtain C103-8(3.0g, yield for two steps: 63%) as a yellow solid. ¹H NMR (400 MHz, DMSO_d6) δ 1.37 (t, *J=* 7.2 Hz, 3H), 3.77 (s, 3H), 3.85 (s, 3H), 4.10 (s, 2H), 4.29 (q, *J* = 7.2 Hz, 2H), 7.58 (s, 1H), 7.83 (s, 1H). LCMS:m/z=324.0/326.0 [M-NH₂]⁺

### Step 9:

Under the protection of nitrogen, C103-8(1.5g,4.40 mmol) was dissolved in DCM(30 ml), DIEA(2.27g,17.56 mmol) was added at room temperature, cooled to 0°C under an ice bath, CbzCl(1.5g,8.79 mmol, diluted with 3.0 ml DCM) was added dropwise, and the mixture was reacted at 0°C for 0.5 h after addition. TLC showed the raw materials were reacted completely. The reaction liquid was washed with saturated salt water, dried over anhydrous sodium sulfate and concentrated, and the crude solid was slurried with a mixed solution of petroleum ether and ethyl acetate (40ml,PE/EA = 10/1), filtered, and the filter cake was washed and dried to obtain C103-9(2.4g, crude) as a white solid.

### Step 10:

Under the protection of nitrogen, C103-9(2.4g, crude) was dissolved in DMF(60 ml) and water (3 ml), boric acid (1.71g,9.94 mmol), cesium carbonate (4.07g,12.49 mmol) and palladium tetratriphenylphosphine (300 mg,0.26mmol) were added, replaced with nitrogen for 3 times, and the mixture was heated to 80°C to react for 3.0 h. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography (DCM/MeOH = 20/1) to obtain C103-10(2.2 g, yield for two steps: 96%) as a white solid.

### Step 11:

C103-10(240 mg,0.46mmol) was dissolved in methanol (10 ml) and dichloromethane (10 ml), 10% Pd/C(50 mg) was added, replaced with hydrogen for 3 times, and the mixture was reacted at room temperature for 1.0 h. TLC showed the raw materials were reacted completely. The reaction solution was filtered with diatomite, and the filtrate was concentrated to obtain C103-11(134 mg, crude) as a white solid.

### Step 12:

Under the protection of nitrogen, C103-11(134 mg, crude) was dissolved in acetonitrile (10 ml), DIEA(223 mg,1.73 mmol) and 1H-pyrazol-1-formamidine hydrochloride(102 mg,0.70 mmol) were added, and the mixture was reacted at room temperature for 48 hours, and solids were precipitated. The reaction solution was filtered, and the filter cake was washed and dried to obtain SYY-C103(88mg, yield for two steps: 44%) as a white solid. ¹H NMR (400 MHz, DMSO-*d₆*): δ1.44 (t, J = 7.2Hz, 3H), 3.67 (s, 3H), 3.84 (s, 3H), 4.40 (q, J = 7.2 Hz, 2H), 4.96 (d, J = 3.6Hz, 2H), 7.33- 7.68 (m, 9H), 7.71 (s, 1H), 7.96 (t, J = 8.4 Hz, 2H), 8.39 (br, 1H). LCMS:m/z=431.2[M+H]⁺

### Example 89: Preparation of Compound SYY-C104

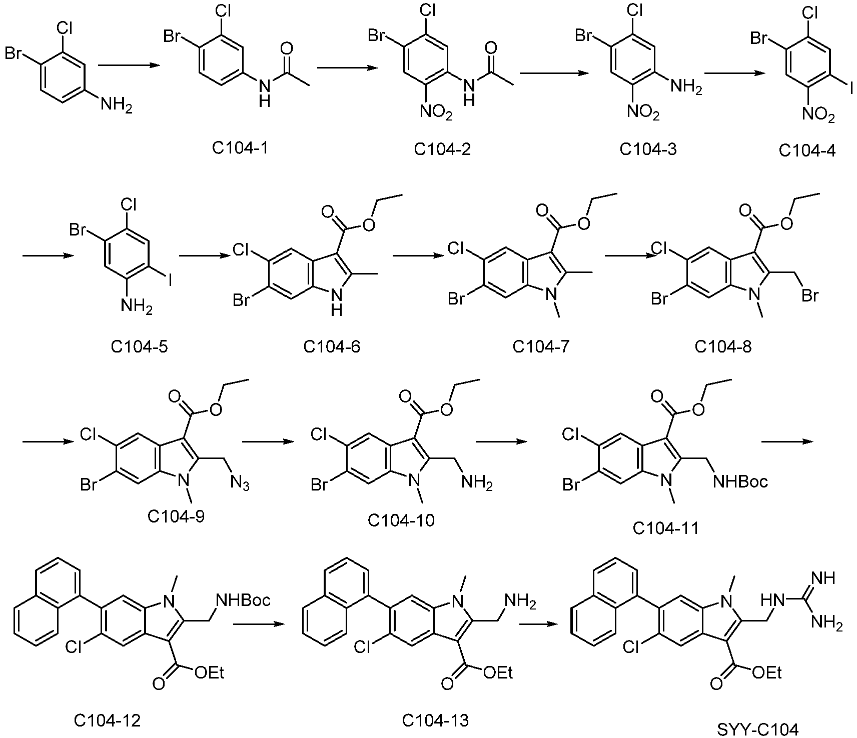

### Step 1:

4-Bromo-3-chloroaniline (20.0g,97 mmol) and triethylamine (14.7g,145 mmol) were dissolved in DCM(100 mL), acetyl chloride (9.13g,116 mmol) was added at 0°C, and the mixture was stirred at 0°C for 30min after addition. TLC showed the reaction was completed. The reaction solution was added with water, extracted with dichloromethane, and the organic phases were combined, washed with saturated salt water, dried over anhydrous sodium sulfate, and concentrated to obtain C104-1(22.0g, crude) as a white solid. LC-MS: M/z = 248.0/250.0 [M + H]⁺.

### Step 2:

KNO₃(13.4g,132 mmol) was dissolved in concentrated H₂SO₄(150 mL) at 0°C, stirred for 30min, C104-1(22.0g, crude) was added, and the mixture was stirred for 2h at 0°C. TLC showed the reaction was completed. The reaction solution was poured into ice water, and extracted with ethyl acetate. The organic phase was combined, washed with saturated salt water, dried over anhydrous sodium sulfate, and concentrated to obtain a yellow solid, slurried with 100mL ethyl acetate, filtered, and the filter cake was washed and dried to obtain C104-2(15.0g, crude) as a yellow solid. ¹H NMR (400 MHz, DMSO-d6) δ2.09 (s, 3H), 7.97 (s, 1H), 8.34 (s, 1H), 10.36 (s, 1H). LC-MS:m/z =292.9 [M+H]⁺.

### Step 3:

C104-2(7.5g, crude) was dissolved in methanol (100mL) and HCl(30mL,6M), and heated to 75°C to react for 4 h. TLC showed the reaction was completed. The reaction solution was cooled to room temperature, and was adjusted to be alkaline with 1N NaOH aqueous solution, extracted with ethyl acetate, and the organic phases were combined, washed with saturated salt water, dried over anhydrous sodium sulfate, and concentrated to obtain C104-3(6.0g, crude) as a red solid. LC-MS:m/z =248.9 [M-H]⁻

### Step 4:

NaNO2(2.0g,28.9mmol) was dissolved in concentrated sulfuric acid (100 mL), heated to 70°C and stirred for 30min, cooled to 0°C, a solution of C104-3(6.0g, crude) in acetic acid (15 mL) was added, stirred for 30min at room temperature, and then the reaction solution was added into KI(12.0g,72 mmol) aqueous solution (15 mL), and the mixture was heated to 70°C to react for 1h. TLC showed the reaction was completed. The reaction solution was cooled to room temperature, added with saturated Na2SO3 aqueous solution, extracted with ethyl acetate, and the organic phases were combined, washed with saturated salt water, dried over anhydrous sodium sulfate, and concentrated to obtain C104-4(3.0g, crude) as a red solid.

### Step 5:

C104-4(8.0g, crude) was dissolved in a mixed solvent of ethanol (100mL) and water (100mL), iron powder (6.2g,110 mmol) and ammonium chloride (5.9g,110 mmol) were added at room temperature, and the mixture was heated to 75°C to react for 3h. TLC showed the reaction was completed. The reaction solution was filtered while hot, the filtrate was concentrated to remove ethanol, added with water, extracted with ethyl acetate, and the organic phases were combined, washed with saturated salt water, dried over anhydrous sodium sulfate, and concentrated to obtain solid C104-5(6.6 g, crude), LCMS:m/z=331.9/333.9 [M +H]^{+.}

### Step 6:

C104-5(6.6g, crude) was dissolved in DMSO (200mL), ethyl acetoacetate (15.5g,119 mmol), tetrazoleacetic acid (0.5 g,3.9 mmol), cuprous iodide (0.4 g,1.9 mmol) and cesium carbonate (12.9 g,39.9mmol) were added at room temperature, and the mixture was heated to 100°C to react for 3 h under the protection of nitrogen. TLC showed the reaction was completed. The reaction solution was cooled to room temperature, added with saturated salt water, extracted with ethyl acetate, and the organic phases were combined, dried over anhydrous sodium sulfate, and concentrated to obtain C104-6(6.2g, crude) as a gray solid. LC-MS:m/z =315.9 [M+H]⁺

### Step 7:

The C104-6(6.2g, crude) was dissolved in DMF(100 mL), cooled to 0°C under an ice bath, 60% sodium hydride (1.2g,29mmol) was added in batches, reacted at this temperature for 30min after the addition, and methyl iodide (4.2g,29mmol) was added, and the mixture was reacted at this temperature for 1h. TLC showed the reaction was completed. The reaction solution was slowly quenched with ice water, extracted with ethyl acetate, the organic phases were combined, washed with saturated salt water, dried over anhydrous sodium sulfate, and concentrated, and the crude product was purified by silica gel column chromatography to obtain solid C104-7(3.4g, yield for 7 steps: 8%).

### Step 8:

C104-7(3.4 g, 10.3 mmol) was dissolved in carbon tetrachloride (100mL), NBS(1.93 g,10.8 mmol) and BPO(250 mg,1.03mmol) were added at room temperature, and the mixture was heated to 80 °C to react for 2 h. TLC showed the reaction was completed. The reaction solution was concentrated to obtain 104-8 (crude), which was directly used in the next step.

### Step 9:

C104-8 (crude) was dissolved in DMF(50 mL), NaN₃(1.0g,15.3 mmol) was added at room temperature, and reacted for half an hour. TLC showed the reaction was completed. The reaction solution was added with water, extracted with ethyl acetate, the organic phases were combined, washed with saturated salt water, dried over anhydrous sodium sulfate, and concentrated to obtain 104-9 (crude) which was directly used in the next step.

### Step 10:

C104-9 (crude) was dissolved in a mixed solvent of THF(50mL) and water (5mL), triphenylphosphine (4.0g,15.2mmol) was added at room temperature, and the mixture was heated to 75°C to react for 3 h. TLC showed the reaction was completed. The reaction solution was cooled to room temperature, adjusted to be weak acidity by slowly adding with 1N diluted hydrochloric acid, extracted with ethyl acetate, the organic layer was discarded, the water layer was added with 1N sodium hydroxide to adjust to be alkaline, extracted with ethyl acetate, the organic phases were combined, washed with saturated salt water, dried over anhydrous sodium sulfate, and concentrated to obtain solid C104-10(3.1g, crude). LC-MS:m/z = 345.0 /347.0[M + H]⁺.

### Step 11:

C104-10(3.1g, crude) was dissolved in DCM (50mL), BOC anhydride (2.3 g, 10.8mmol) was added at room temperature, stirred at room temperature for 1h, and the system changed from turbid to clear. The reaction solution was concentrated and the crude product was purified by silica gel column chromatography to obtain C104-11(2.3g, yield for four steps: 50%) as a white solid. LC-MS:m/z =467.0 [M+Na]⁺

### Step 12:

C104-11(400mg,0.9mmol) was dissolved in a mixed solvent of dioxane (50mL) and water (5mL), naphthaleneboronic acid (232mg,1.35mmol), palladium tetratriphenylphosphine (104mg , 0.09mmol) and sodium carbonate (372mg,2.7mmol) were added at room temperature, replaced with nitrogen for 3 times, and the mixture was heated to 100°C to react for 4h. TLC showed the reaction was completed. The reaction solution was cooled to room temperature, added with water, extracted with ethyl acetate, the organic phases were combined, washed with saturated salt water, dried over anhydrous sodium sulfate, and concentrated to obtain 104-12 (crude) which was directly used in the next step.

### Step 13:

The C104-12 (crude) was dissolved in DCM (10mL), TFA(SmL) was added at room temperature, and the mixture was reacted at room temperature for 30min. The reaction solution was concentrated and the crude product was purified by pre-TLC to obtain C104-13(150mg, yield for two steps: 43%).

### Step 14:

C104-12(150mg,0.38mmol) was dissolved in acetonitrile (10mL), 1H-pyrazol-1-formamidine hydrochloride (67.2mmol,0.45mmol) and DIPEA(197mg,1.53mmol) were added at room temperature, reacted at room temperature for 12h, and solids were precipitated. The reaction solution was filtered, and the filter cake was washed and dried to obtain solid SYY-C104(100mg, yield 60%). ¹H NMR (400 MHz, DMSO-d6) δ1.42 (t, J = 7.2 Hz, 3H), 3.87 (s, 3H), 4.39-4.45 (m, 1H), 4.98 (s, 3H), 7.33-7.64 (m, 8H), 7.79 (s, 1H), 8.02 (d, J = 8.0 Hz, 2H), 8.19 (s, 1H), 8.45 (br, 1H). LC-MS:m/z =435.1 [M+H]⁺

### Example 90: Preparation of Compound SYY-C105

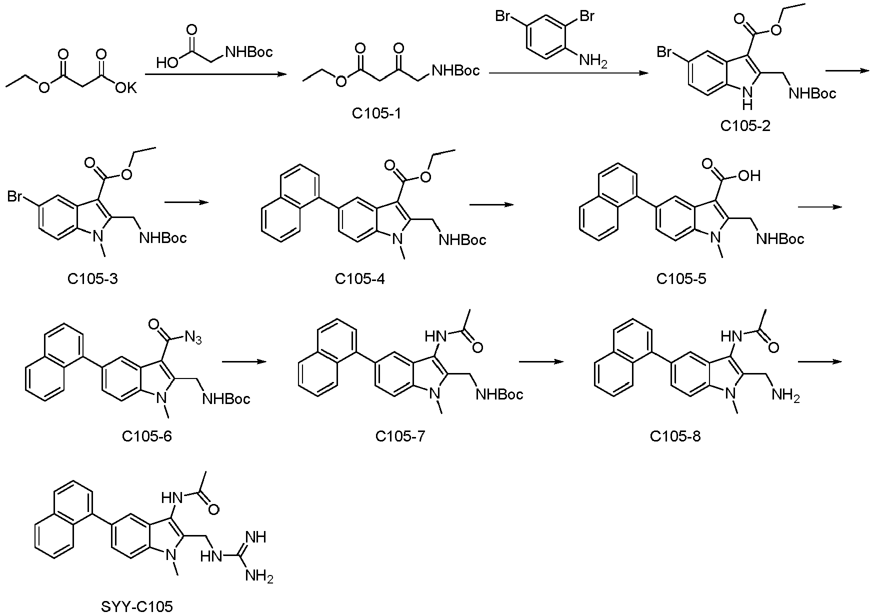

### Step 1:

BOC-glycine (50.0g, 285.4 mmol) was dispersed in THF (500 mL), CDI(50.9g,313.9 mmol) was added, stirred at room temperature for 2 h, anhydrous MgCl₂(27.2g,285.7mmol) and potassium 3-ethoxy-3-oxopropanate (48.6g, 285.5 mmol) were added, and the mixture was heated to 60 °C to react for 3 hours. The reaction solution was cooled to room temperature, filtered, the filtrate was concentrated, ethyl acetate (700 mL) was added, washed with 1N hydrochloric acid solution, the organic phases were combined, washed with water, and washed with saturated NaHCO₃ solution, dried over anhydrous sodium sulfate, and concentrated, and the crude product was purified by silica gel column chromatography to obtain C105-1(48.1g, yield 69%) as a light yellow oil.

### Step 2:

Under the protection of nitrogen, C105-1(10.0g, 40.77 mmol), 2, 4-dibromoaniline (2.6g,10.36 mmol), tetrazoleacetic acid (261 mg, 2.04 mmol), cesium carbonate (6.6g, 20.26 mmol) and cuprous iodide (194 mg,1.02 mmol) were dispersed into DMSO(30 mL), and the mixture was heated to 100 °C to react overnight. TLC showed the reaction was completed. The reaction solution was cooled to room temperature, added with water, extracted with ethyl acetate, the organic phases were combined, washed with 1N hydrochloric acid, and washed with saturated salt water, dried over anhydrous sodium sulfate, and concentrated, and the crude product was purified by silica gel column chromatography to obtain C105-2(1.1g, yield 27%, 2,4-dibromoaniline was recovered to obtain 1.2g) as a yellow solid. LC-MS:m/z= 421.0 [M+Na]⁺

### Step 3:

C105-2(1.1g, 2.77 mmol) and cesium carbonate (1.8g, 5.52 mmol) were dispersed into DMF(10 mL), methyl iodide (589 mg, 4.15 mmol) was added, and the mixture was stirred at room temperature for 2 hours. TLC showed the reaction was completed. The reaction solution was added with water, extracted with ethyl acetate, the organic phases were combined, washed with water, and washed with saturated salt water, dried over anhydrous sodium sulfate, and concentrated, and the crude product was slurried with PE/EA to obtain C105-3(720 mg, yield 63%) as a white solid. LC-MS:m/z= 433.1 [M+Na]⁺

### Step 4:

C105-3(680 mg,1.65 mmol), 1-naphthaleneboronic acid (427 mg, 2.48 mmol), sodium carbonate (350 mg, 3.30 mmol) and PdCl₂(dppf)(120 mg, 0.15 mmol) were dispersed into a mixed solvent of 1,4-dioxane (10 mL) and H₂O(4 mL), and the mixture was heated to 85 °C to react for 2 hours under the protection of nitrogen. LCMS showed the reaction was completed. The reaction solution was cooled to room temperature, added with water, extracted with ethyl acetate, the organic phases were combined, washed with water, and washed with saturated salt water, dried over anhydrous sodium sulfate, and concentrated, and the crude product was purified by silica gel column chromatography to obtain C105-4(690 mg, yield 91%) as a colorless oil. LC-MS:m/z = 481.3 [M + Na]⁺.

### Step 5:

C105-4(690 mg,1.50 mmol) and sodium hydroxide (318 mg, 7.95 mmol) were dissolved in a mixed solvent of ethanol (8 mL), tetrahydrofuran (6 mL) and water (8 mL), and the mixture was heated to 100 °C to react overnight. TLC showed the reaction was completed. The reaction solution was concentrated to remove organic solvent, 1N hydrochloric acid solution was added to adjust pH = 1, extracted with ethyl acetate, the organic phases were combined, washed with water, and washed with saturated salt water, dried over anhydrous sodium sulfate, and concentrated to obtain C105-5(670 mg, crude) as a light yellow solid which was directly used in the next step. LC-MS:m/z= 429.2 [M-H]⁻

### Step 6:

C105-5(670 mg, 1.56 mmol), DPPA(428 mg, 1.56 mmol) and triethylamine (8 mL) were dissolved in DCM(16 mL) and reacted for 1 hour at room temperature. TLC showed the reaction was completed. The reaction solution was concentrated, and the crude product was purified by silica gel column chromatography to obtain C105-6 (440 mg, yield 62%) as a white solid.

### Step 7:

C105-6(240 mg,0.53 mmol) was dissolved in toluene (8 mL), acetic acid (63 mg,1.05 mmol) and DMAP (6 mg, 0.05 mmol) were added at room temperature, and the mixture was heated to 110 °C to react for 1 hour. TLC showed the reaction was completed. The reaction solution was cooled to room temperature, and concentrated to obtain a light yellow solid (210 mg, crude), and purified by pre-HPLC to obtain C105-7(110 mg, yield 47%) as a white solid. LC-MS:m/z= 466.2 [M+Na]⁺

### Step 8:

C105-7(110 mg,0.25 mmol) was dissolved in DCM/TFA(3/1,4 mL) and reacted at room temperature for 1 hour. TLC showed the reaction was completed. The reaction solution was concentrated to obtain C105-8(160 mg, crude) which was directly used in the next step. LC-MS:m/z= 327.2 [M-NH₂]⁺

### Step 9:

C105-8(160 mg, crude), 1H-pyrazol-1-formamidine hydrochloride (61.6 mg, 0.42 mmol) and DIPEA(135 mg, 1.05 mmol) were dissolved in acetonitrile (4 mL), the mixture was stirred at room temperature for 2 days, and solids were precipitated. The reaction solution was filtered, and the filter cake was washed with acetonitrile, and dried to obtain a white solid (40 mg, yield for two steps: 42%).

¹H NMR: (DMSO-*d₆*, 400MHz): δ2.14 (s, 3H), 3.75 (s, 3H), 4.53 (br, 2H), 7.15 (dd, J = 8.0, 0.8 Hz, 1H), 7.26-7.72 (m, 9H), 7.84 (d, J = 8.4 Hz, 1H), 7.89 (br, 1H), 7.94 (d, J = 8.4 Hz, 1H), 7.99 (d, J = 8.4 Hz, 1H), 9.74 (s, 1H). LC-MS:m/z= 386.2 [M+H]⁺ (99.63% purity, 220 nm)

### Example 91: Preparation of Compound SYY-C106

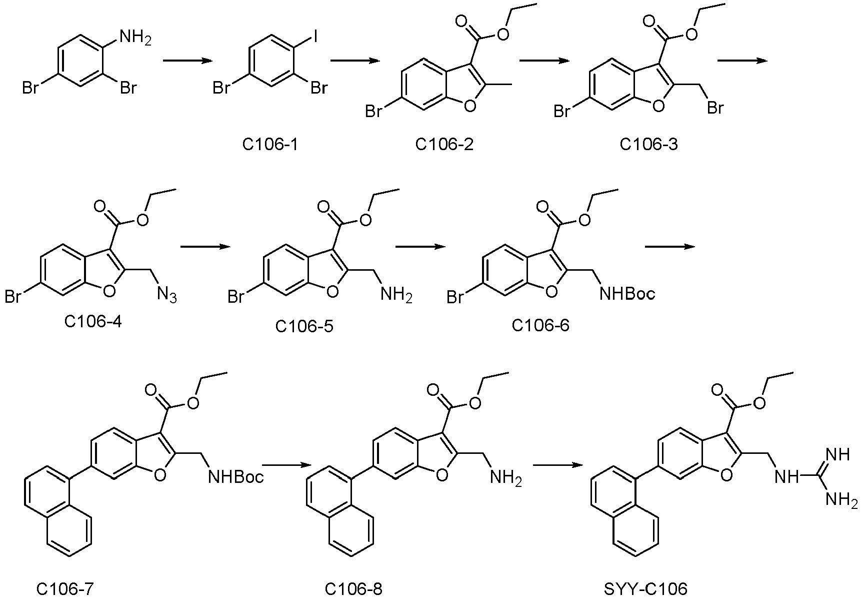

### Step 1

At 0 °C, 2,4-dibromoaniline (15.0g, 59.8 mmol) was suspended in HCl(150 mL,6N) aqueous solution, sodium nitrite (4.54g,65.8 mmol) aqueous solution (10 mL) was dropwise added, and the mixture was stirred for 10 minutes after addition. KI(19.85g,119.6 mmol) aqueous solution (10 mL) was added dropwise, and the mixture was stirred for 30 minutes after addition. TLC showed the reaction was completed. The reaction solution was extracted with EA, and the organic phases were combined, washed with saturated salt water, dried over anhydrous Na2SO4, and concentrated to obtain C106-1(19.0g, crude) as a red-brown solid.

### Step 2:

C106-1(19.0g, crude), ethyl acetoacetate (20.50g,157.5 mmol), potassium carbonate (21.77g,157.5 mmol) and cuprous iodide (1.0g,5.25 mmol) were dispersed into THF(150 mL), and the mixture was refluxed overnight under the protection of nitrogen. TLC showed the reaction was completed. The reaction solution was cooled to room temperature, added with water, extracted with EA, the organic phases were combined, washed with saturated salt water, dried over anhydrous sodium sulfate, and concentrated, and the crude product was separated and purified by silica gel column chromatography to obtain C106-2(9.2g, yield for two steps: 54%) as a light yellow solid. HNMR(CDCl₃, 400Hz):7.83 (d, J=8.0 Hz, 1H), 7.61 (d, J=1.6 Hz, 1H), 7.43 (dd, J1=1.2 Hz, J2=8.4 Hz, 1H), 4.44 (q, J=7.2 Hz, 2H), 2.77 (s, 3H), 1.46(t, J = 7.2Hz, 3H). LC-MS:m/z= 282.1 [M+H]⁺

### Step 3:

C106-2 (2.0g,7.06 mmol), NBS(1.38g,7.77 mmol) and BPO(170 mg,0.7 mmol) were dispersed into carbon tetrachloride (80mL), and the mixture was heated to 80°C to react for 5h, and the reaction was completed. The reaction solution was concentrated to obtain C106-3 (crude), which was directly used in the next step.

### Step 4:

C106-3 (crude) was dissolved in DMF(20mL), sodium azide (688 mg,10.59 mmol) was added, and the mixture was stirred at room temperature for 30min. TLC showed the reaction was completed. The reaction solution was added with water, extracted with ethyl acetate, the organic phases were combined, washed with saturated salt water, dried over anhydrous sodium sulfate, and concentrated to obtain C106-4 (crude) which was directly used in the next step.

### Step 5:

C106-4 (crude) was dissolved in THF(50mL) and water (5mL), triphenylphosphine (2.0g,7.62 mmol) was added at room temperature, and the mixture was heated to 80 °C to react for 3 h. TLC showed the reaction was completed. The reaction solution was cooled to room temperature, diluted with 1N dilute hydrochloric acid, extracted with ethyl acetate, the organic layer was discarded, sodium hydroxide was added to adjust the water layer to be alkaline, extracted with EA, and the organic phases were combined, washed with saturated salt water, dried over anhydrous sodium sulfate, and concentrated to obtain C106-5(1.3g, yield for three steps: 62%) as a light yellow solid. LC-MS:m/z = 300.0 [M + H]+.

### Step 6:

C106-5(1.3g,4.36 mmol) and BOC anhydride (1.17g,5.32 mmol) were dissolved in DCM (20 mL) and stirred for 1h at room temperature. TLC showed the reaction was completed. The reaction solution was concentrated, and the crude was separated and purified by silica gel column chromatography to obtain C106-6(1.2g, yield 69%) as a light yellow solid. HNMR:(CDCl3, 400Hz):7.82 (d, J=8.4 Hz, 1H), 7.65 (d, J=1.6 Hz, 1H), 7.43 (dd, J1=1.6 Hz, J2=8.4 Hz, 1H), 5.31-5.53 (m, 1H), 4.78 (d, J = 5.6 Hz, 2H), 4.43 (q, J=7.2 Hz, 2H), 1.44-1.48(m, 12H). LC-MS:m/z= 390.0 [M+H]⁺

### Step 7:

C106-6(400 mg,1.0 mmol), naphthaleneboronic acid (258 mg,1.50 mmol), Pd(dppf)Cl₂(73 mg,0.1 mmol) and sodium carbonate (212 mg,2.0 mmol) were dispersed into a mixed solvent of dioxane (15 mL) and water (5 mL). Under the protection of nitrogen, and the mixture was heated to 100°C to react for 4 h under the protection of nitrogen. LCMS showed the raw materials disappeared. The reaction solution was cooled to room temperature, added with water, extracted with EA, the organic phases were combined, washed with saturated salt water, dried over anhydrous sodium sulfate, and concentrated, and the crude product was separated and purified by silica gel column chromatography to obtain C106-7(420 mg, yield 94%) as a light yellow solid. LC-MS:m/z= 390.1[M-55]⁺

### Step 8:

C106-7(420 mg, 0.94 mmol) and TFA(6 mL) were dissolved in DCM (6 mL) and stirred at room temperature for 1 h. TLC showed the reaction was completed. The reaction solution was concentrated to obtain C106-8 (crude), which was directly used for the next reaction.

### Step 9:

C106-8 (crude) was dissolved in acetonitrile (10 mL), DIPEA(364 mg,2.82 mmol) and 1H-pyrazol-1-formamidine hydrochloride(166 mg,1.13 mmol) were added, and the mixture was stirred at room temperature overnight. The reaction solution was concentrated to obtain crude product and purified by pre-HPLC to obtain SYY-C106(90 mg, yield for two steps: 25%) as a yellow solid. ¹H NMR: (DMSO-*d₆*, 400MHz): δ1.42 (t, J = 7.2 Hz, 3H), 4.43 (q, J = 7.2 Hz, 2H), 4.98 (d, J = 6.0 Hz, 2H), 7.46-7.69 (m, 8H), 7.78-7.81 (m, 2H), 7.98-8.04 (m, 2H), 8.08 (d, J = 8.0 Hz, 1H), 8.34 (t, J = 6.0 Hz, 1H). LC-MS:m/z= 388.1[M+H]⁺ (96.64% purity, 220 nm)

### Example 92: Preparation of Compound SYY-C107

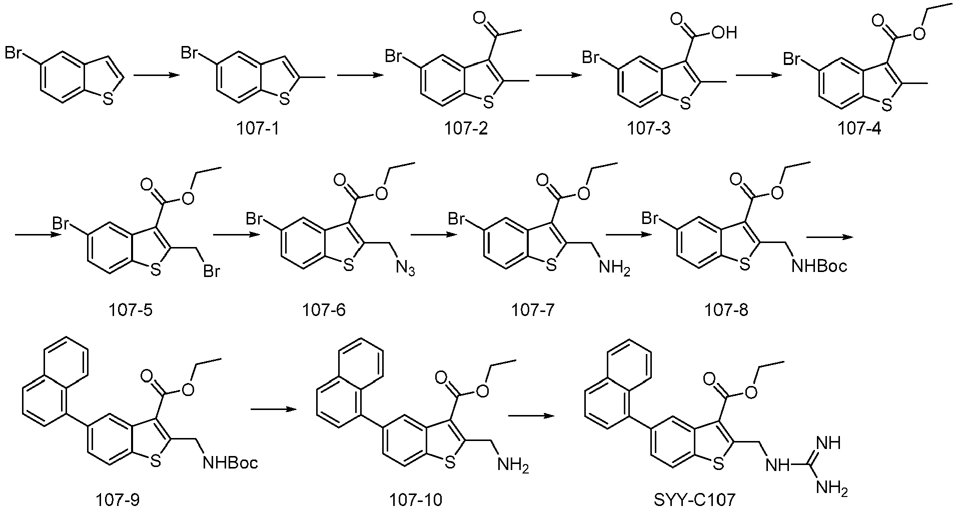

### Step 1:

5-Bromobenzothiophene (5.0g,23.5 mmol) was dissolved in THF(30 mL), LDA(3.0g,28.2 mmol) was added dropwise at -60 °C, stirred for 1 hour, methyl iodide (5.0g,35.2mmol) was added dropwise, and the mixture was heated to room temperature and stirred overnight after the addition. lcms confirmed the reaction was completed. The reaction solution was quenched with ice water, extracted with EA, and the organic phases were combined, washed with saturated salt water, dried over anhydrous Na2SO4, and concentrated to obtain C107-1(5.3g, crude) as a light yellow solid. HNMR(CDCl₃, 400Hz):δ7.78 (d, J=2.0 Hz, 1H), 7.59 (d, J=8.4 Hz, 1H), 7.34 (dd, 11=8.8, 2.0 Hz, 1H), 6.90 (s, 1H), 2.59 (m, 3H).

### Step 2:

At 0 °C, a solution of AlCl3(3.5g, 26.4 mmol) suspended in DCM(30 mL) was stirred for 10 minutes, added with a solution of acetyl chloride (1.0g, 12.7 mmol) in DCM(10 mL), continued to stir for 10 minutes, added with a solution of C107-1(3.0g, crude) in DCM(10 mL), and the mixture was stirred for 1 hour. TLC showed the reaction was completed. The reaction solution was quenched with ice water, extracted with ethyl acetate, and the organic phases were combined, washed with saturated salt water, dried over anhydrous Na2SO4, and concentrated to obtain C107-2(3.6g, crude) as a light yellow solid.

### Step 3:

At 0 °C, Br2(7.5g, 46.9 mmol) was added dropwise into a solution of NaOH(5.7g,142.5 mmol) in water (50 mL), stirred for 20 minutes, a solution of C107-2(3.6g, crude) in 1,4-dioxane (50 mL) was added dropwise, and the mixture was heated to room temperature and stirred overnight. TLC showed the reaction was completed. The reaction solution was added with concentrated HCl to adjust pH = 2, extracted with EA, and the organic phases were combined, washed with saturated salt water, dried over anhydrous Na2SO4, and concentrated to obtain C107-3(3.0g, crude), which was directly used in the next step.

### Step 4:

C107-3(3.0g, crude) and potassium carbonate (2.0g,14.7 mmol) were dispersed into DMF(20 mL), iodide ethane (2.3g,14.7 mmol) was added, and the mixture was stirred at room temperature for 2 hours. TLC showed the reaction was completed. The reaction was added with concentrated HCl to adjust pH = 2, extracted with EA, and the organic phases were combined, washed with saturated salt water, dried over anhydrous Na2SO4, and concentrated, and the crude product was separated and purified by silica gel column chromatography to obtain C107-4(2.1g, yield for three steps 46%) as a light yellow solid. HNMR(CDCl3, 400Hz):8.85 (d, J=1.6 Hz, 1H), 7.56 (d, J=8.8 Hz, 1H), 7.41 (dd, J=8.8, 2.0 Hz, 1H), 4.44 (q, J=7.2 Hz, 2H), 2.83 (s, 3H), 1.46 (t, J=7.2 Hz, 3H). LC-MS:m/z= 299.0 [M+H]⁺

### Step 5:

C107-4(2.1 g, 7.0 mmol) was dissolved in carbon tetrachloride (80mL), NBS(1.31 g,7.0 mmol) and BPO(169 mg,0.7 mmol) were added at room temperature, and the mixture was heated to 80 °C to react for 2 h. TLC showed the reaction was completed. The reaction solution was concentrated to obtain C107-5(2.4g, crude), which was directly used in the next step.

### Step 6:

C107-5 (2.18g,crude) was dissolved in DMF(20mL), sodium azide (400 mg,6.2 mmol) was added, and stirred at room temperature for 30min. TLC showed the reaction was completed. The reaction solution was added with EA/water, extracted and separated, and the organic phases were combined, washed with saturated salt water, dried over anhydrous sodium sulfate, and concentrated to obtain C107-6(2.1g, crude), which was directly used in the next step.

### Step 7:

C107-6 (2.1 g, crude) was dissolved in THF(50mL) and water (5mL), triphenylphosphine (2.0 g,7.6 mmol) was added at room temperature, and the mixture was heated to 80°C to react for 3 h. TLC showed the reaction was completed. The reaction solution was added with 1N dilute hydrochloric acid and ethyl acetate, extracted and separated, the organic layer was discarded, the water layer was added with sodium hydroxide to adjust to be alkaline, extracted with EA, the organic phases were combined, washed with water, and washed with saturated salt water, dried over anhydrous sodium sulfate, and concentrated to obtain C107-7(1.0g, crude). LC-MS:m/z= 314.0[M+H]⁺

### Step 8:

C107-7(1.0g, crude) was dissolved in DCM (20 mL), BOC anhydride (800 mg,3.66 mmol) was added, and the mixture was stirred at room temperature for 1h. TLC showed the reaction was completed. The reaction solution was concentrated, and the crude was separated and purified by silica gel column chromatography to obtain C107-8(850 mg, yield for four steps: 29%) as a light yellow solid.

### Step 9:

C107-8(400 mg,0.96 mmol), naphthaleneboronic acid (250mg), Pd(dppf)Cl₂(66 mg,0.09 mmol) and sodium carbonate (310 mg,2.9 mmol) were dispersed into a mixed solvent of dioxane (15 mL) and water (5 mL), and the mixture was heated to 100°C to react for 4 h under the protection of nitrogen. LCMS showed the raw materials disappeared. The reaction solution was cooled to room temperature, added with EA/water, extracted and separated, the organic phases were combined, washed with saturated salt water, dried over anhydrous sodium sulfate, and concentrated, and the crude product was separated and purified by silica gel column chromatography to obtain C107-9(420 mg, yield 94%) as a light yellow solid. LC-MS:m/z = 406.1 [M-55]⁺.

### Steps 10, 11:

C107-9(420 mg, 0.91 mmol) and TFA(6 mL) were dissolved in DCM (6 mL) and stirred at room temperature for 1 h. TLC showed the reaction was completed. The reaction solution was concentrated, and the crude product was dissolved in CH3CN(10 mL), DIPEA(353 mg,2.73 mmol) and 1H-pyrazol-1-formamidine hydrochloride(160 mg,1.09 mmol) were added, and the mixture was stirred at room temperature overnight, and concentrated to obtain the crude product which was purified by pre-HPLC to obtain SYY-C107(52 mg, yield 14%) as a white solid. ¹H NMR: (DMSO-*d₆*, 400MHz): δ1.32 (t, J = 7.2 Hz, 3H), 4.35 (q, J = 7.2 Hz, 2H), 5.05 (d, J = 6.4 Hz, 2H), 7.48-7.65 (m, 8H), 7.81 (d, J = 8.40 Hz, 1H), 8.00-8.55 (m, 2H), 8.22 (d, J = 8.29 Hz, 1H), 8.37 (br, 1H),8.49 (d, J = 1.2 Hz, 1H). LC-MS: m/z= 404.1 [M+H]⁺ (95.42% purity, 220 nm)

### Example 93: Preparation of Compound SYY-C108

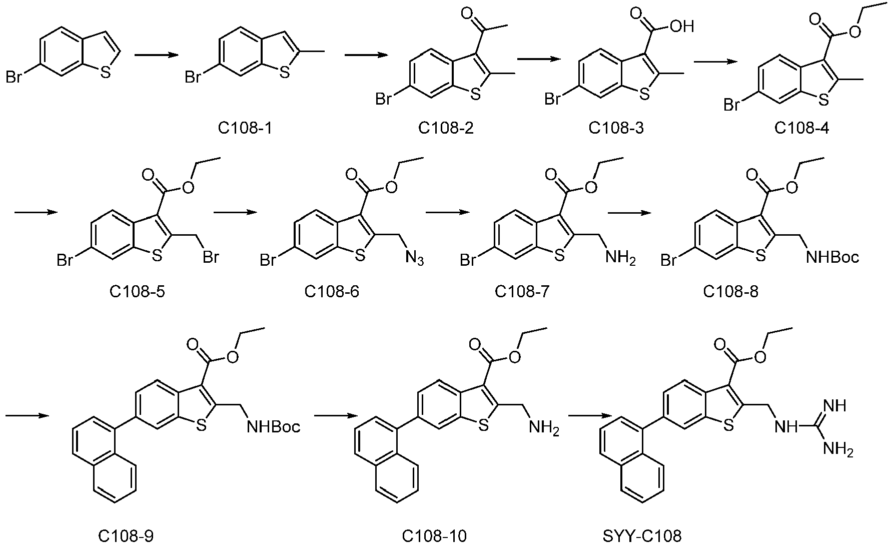

### Step 1:

6-Bromobenzothiophene (5.0g,23.5 mmol) was dissolved in THF(30 mL), LDA(3.0g,28.2 mmol) was added dropwise at -60 °C, stirred at 60 °C for 1 hour after addition, methyl iodide (5.0g,35.2 mmol) was added dropwise, and the mixture was heated to room temperature and stirred overnight after addition. lcms confirmed the reaction was completed. The reaction solution was quenched with ice water, extracted with EA, the organic phases were combined, dried over anhydrous sodium sulfate, and concentrated to obtain C108-1(5.1g, crude) as a light yellow solid. LC-MS: Not available

### Step 2:

At 0 °C, AlCl₃(5.9 g, 44.2 mmol) was suspended in DCM(30 mL) and stirred for 10 minutes, added with a solution of acetyl chloride (1.73 g,22.1 mmol) in DCM(10 mL), continued to stir for 10 minutes, added with a solution of C108-1(5.1 g, crude) in DCM(10 mL), and the mixture was stirred for 1 hour. TLC showed the reaction was completed. The reaction solution was quenched with ice water, extracted with EA, the organic phases were combined, dried over anhydrous Na2SO4, and concentrated to obtain C108-2(2.1 g, crude) as a light yellow solid. LC-MS:m/z = 269.0 [M + H]⁺.

### Step 3:

At 0 °C, Br2(3.74 g, 23.4 mmol) was added dropwise into a solution of NaOH(2.81 g,70.3 mmol) in water (30 mL), stirred for 20 minutes after addition, added dropwise with a solution of C108-2(2.1 g, crude) in 1,4-dioxane (30 mL), and the mixture was heated to room temperature and stirred overnight after addition. TLC showed the reaction was completed. The reaction solution was added with concentrated HCl to adjust pH = 2, extracted with EA, and the organic phases were combined, dried over anhydrous Na2SO4, and concentrated to obtain C108-3(2.1g, crude) which was directly used for the next reaction. LC-MS:m/z = 269.0 [M-H]⁻.

### Step 4:

C108-3(2.1 g, crude) and K2CO3 (2.16 g,15.6 mmol) were dispersed into DMF(20 mL), iodide ethane (1.46 g, 9.4 mmol) was added, and the mixture was stirred at room temperature for 2 hours. TLC showed the reaction was completed. The reaction solution was added with concentrated HCl to adjust pH = 2, extracted with ethyl acetate, the organic phase were combined, dried over anhydrous sodium sulfate, and concentrated, and the crude product was separated and purified by silica gel column chromatography to obtain C108-4(1.7g, yield for four steps: 24%) as a light yellow solid. ¹H NMR,(CDCl₃, 400Hz): 8.28 (d, J=8.8 Hz, 1H), 7.86 (d, J=1.6 Hz, 1H), 7.51 (dd, J=8.8, 2.0 Hz, 1H), 4.45 (q, J=7.2 Hz, 2H), 2.83 (s, 3H), 1.47 (t, J=7.2 Hz, 3H). LC-MS:m/z= 302.1 [M+H]⁺

### Step 5:

C108-4 (1.7g,5.7 mmol) was dissolved in carbon tetrachloride (20mL), NBS(1.05g,5.9 mmol) and BPO(138 mg,0.57 mmol) were added at room temperature, and the mixture was heated to 80°C to react for 2 h. TLC showed the reaction was completed. The reaction solution was concentrated to obtain C108-5(2.3 g, crude), which was directly used in the next step.

### Step 6:

C108-5 (2.3 g,crude) was dissolved in DMF(20mL), sodium azide (480 mg,7.38 mmol) was added, and the mixture was stirred at room temperature for 30min. TLC showed the reaction was completed. The reaction solution was added with EA and water, extracted and separated, and the organic phases were combined, washed with saturated salt water, dried over anhydrous sodium sulfate, and concentrated to obtain C108-6(2.0 g, crude), which was directly used in the next step.

### Step 7:

C108-6 (2.0 g, crude) was dissolved in THF(50mL) and water (5mL), triphenylphosphine (1.54 g,5.88 mmol) was added at room temperature, and the mixture was heated to 80°C to react for 3 h. TLC showed the reaction was completed. The reaction solution was cooled to room temperature, diluted with 1N dilute hydrochloric acid and EA, extracted and separated, and the organic layer was discarded, sodium hydroxide was added to adjust the water layer to be alkaline, extracted with EA, the organic phases were combined, washed with water, and washed with saturated salt water, dried over anhydrous sodium sulfate, and concentrated to obtain C108-7(1.2g, crude) as a light yellow solid, which was directly used in the next step. LC-MS:m/z = 314.0 [M + H]⁺.

### Step 8:

C108-7(1.2 g, crude) was dissolved in DCM (20 mL), BOC anhydride (1.0 g,4.58 mmol) was added, and the mixture was stirred at room temperature for 1h. TLC showed the reaction was completed. The reaction solution was concentrated, separated and purified by silica gel column chromatography to obtain C108-8(720 mg, yield for four steps: 31%) as a light yellow solid. LC-MS:m/z = 358.0 [M-55]⁺.

### Step 9:

C108-8(300 mg,0.73 mmol), naphthaleneboronic acid (156 mg,0.91 mmol), Pd(dppf)Cl2(51 mg,0.06 mmol) and sodium carbonate (153 mg,1.44 mmol) were dispersed into a mixed solvent of dioxane (15 mL) and water (5 mL), and the mixture was heated to 100°C to react for 4 h under the protection of nitrogen. LCMS showed the raw materials disappeared. The reaction solution was cooled to room temperature, added with water and EA, extracted and separated, the organic phases were combined, washed with saturated salt water, dried over anhydrous sodium sulfate, and concentrated, and the crude product was separated and purified by silica gel column chromatography to obtain C108-9(310 mg, yield 93%) as an off-white solid.

### Step 10:

C108-9(310 mg, 0.67 mmol) and TFA(6 mL) were dissolved in DCM (6 mL) and stirred at room temperature for 1 h. TLC showed the reaction was completed. The reaction solution was concentrated to obtain C108-10(243 mg, crude), which was directly used in the next step.

### Step 11:

C108-10(243 mg, crude) was dissolved in acetonitrile (10 mL), DIPEA(272 mg, 2.10 mmol) and 1H-pyrazol-1-formamidine hydrochloride(117mg, 0.80 mmol) were added, and stirred at room temperature overnight. White solids were precipitated in the reaction solution, filtered, and the filter cake was washed and dried to obtain a white solid (185 mg, yield for two steps: 68%). ¹H NMR: (DMSO-*d₆*, 400MHz): δ1.44 (t, J = 7.2 Hz, 3H), 4.44 (q, J = 7.2 Hz, 2H), 5.08 (s, 2H), 7.48-7.63 (m, 8H), 7.81 (d, J = 8.4 Hz, 1H), 7.98-8.04 (m, 2H), 8.20 (s, 1H), 8.48-8.60 (m, 2H). LC-MS:m/z= 404.1 [M+H]⁺ (99.66% purity, 254 nm).

### Example 94: Preparation of Compound SYY-C109

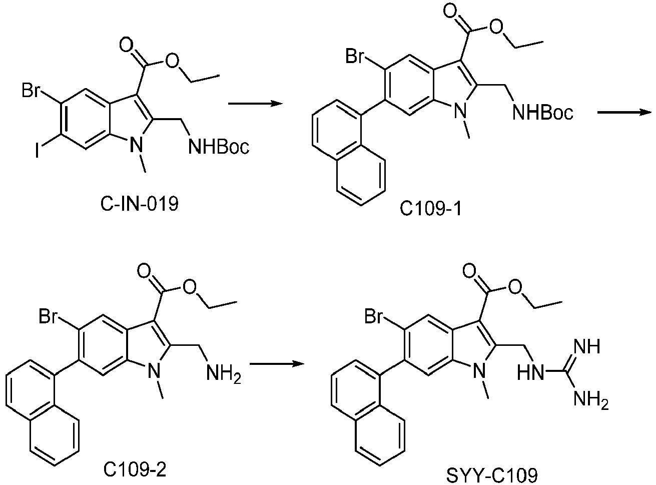

### Step 1:

C-IN-019(620 mg,1.15 mmol) was dissolved in a mixed solvent of dioxane (15 mL) and water (5 mL), naphthylboronic acid(238 mg,1.38 mmol), Pd(dppf)Cl₂(80 mg,0.11 mmol) and sodium carbonate (246 mg,2.32 mmol) were added at room temperature, replaced with nitrogen for 3 times, and the mixture was heated to 80 °C to react for 1 hour under the protection of nitrogen. LCMS showed the reaction was completed. The reaction solution was cooled to room temperature, added with water, extracted with ethyl acetate, and the organic phases were combined, washed with saturated salt water, dried over anhydrous sodium sulfate, and concentrated, and the crude product was separated and purified by silica gel column chromatography to obtain C109-1(560 mg, yield 90%) as a light yellow solid. LC-MS:m/z = 483.0[M-55]⁺.

### Step 2:

C109-1(220 mg,0.41 mmol) was dissolved in dichloromethane (6 mL), TFA(6 mL) was added, and the mixture was reacted at room temperature for 30 min. TLC showed the reaction was completed. The reaction solution was concentrated and purified by pre-TLC to obtain C109-2 (92 mg, yield 51%) as a white solid.

### Step 3:

C109-2(92 mg,0.21 mmol) was dissolved in acetonitrile (10 mL), DIPEA(82 mg,0.63 mmol) and 1H-pyrazol-1-formamidine hydrochloride(37 mg,0.25 mmol) were added at room temperature, and the mixture was reacted at room temperature for 16 hours, and white solids were precipitated. The reaction solution was filtered, and the filter cake was washed and dried to obtain SYY-C109(56 mg, yield 56%). ¹H NMR: (DMSO-*d₆*, 400MHz): δ1.41 (t, J = 7.2 Hz, 3H), 3.86 (s, 3H), 4.42(q, J = 7.2 Hz, 2H), 4.99 (s, 2H), 7.30-7.64 (m, 8H), 7.78 (s, 1H), 8.02 (d, J = 8.4 Hz, 2H), 8.37-8.55 (m, 2H). LC-MS:m/z= 479.1 [M+H]⁺ (95.01% purity, 220 nm)

### Example 95: Preparation of Compound SYY-C110

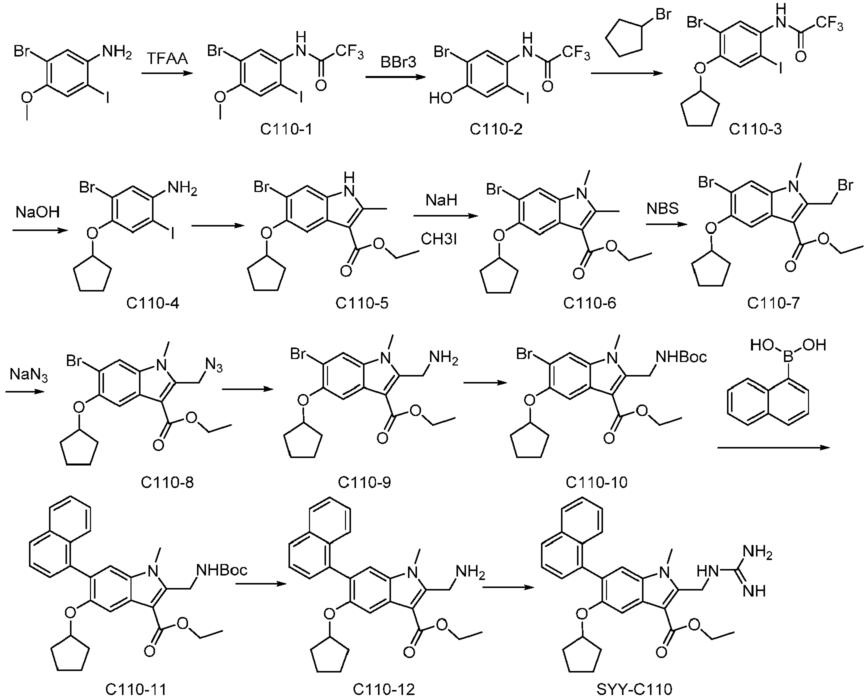

### Step 1:

5-Bromo-2-iodo-4-methoxyaniline (11.7g,35.8 mmol) was dissolved in DCM(150 mL), pyridine (2.8g,35.8 mmol) was added at 0°C, and stirred for 10min, then TFAA(8.3g,39.5 mmol) was added dropwise, and the mixture was stirred at 0°C for 1h after addition. TLC showed the raw materials disappeared. The reaction solution was added with 1N dilute HCl and extracted with DCM, and the organic phases were combined, washed with saturated salt water, dried over anhydrous sodium sulfate, and concentrated to obtain crude product, which was added with 100mL PE:EA = 5:1, slurried, filtered, and the filter cake was washed and dried to obtain C110-1(11.5g, yield 76%). LC-MS:m/z = 421.9 [M-H]⁺

### Step 2:

C110-1(11.5g,27 mmol) was dissolved in DCM(100 mL), BBr₃(1M/L,100 mL) was added, and stirred at room temperature for 12h. TLC showed the raw materials disappeared. The reaction solution was poured into ice water, extracted with DCM, and the organic phase were combined, washed with saturated salt water, dried over anhydrous sodium sulfate, and concentrated to obtain crude product which was slurried with 100mL PE:EA = 5:1, filtered, and the filter cake was washed and dried to obtain C110-2(7.5g, yield 67%) as a white solid. LC-MS:m/z = 409.9 [M + H]⁺.

### Step 3:

C110-2(3.0g,7.3 mmol), bromocyclopentane (1.6g,10.7 mmol) and potassium carbonate (2.0g,14.5 mmol) were dispersed into DMF(50mL), and the mixture was heated to 100°C and stirred for 2h. TLC showed the raw materials disappeared. The reaction solution was cooled to room temperature, added with water/EA, extracted and separated, and the organic phases were combined, washed with saturated salt water, dried over anhydrous sodium sulfate, and concentrated to obtain C110-3 (crude) which was directly used in the next step. LC-MS:m/z = 477.9 [M + H]⁺.

### Step 4:

The C110-3 (crude) was dissolved in ethanol (20mL) and NaOH aqueous solution (10mL,10M), and the mixture was heated to 80°C to react for 1h. TLC showed the raw materials disappeared. The reaction solution was cooled to room temperature, added with water/EA, extracted and separated, and the organic phases were combined, washed with saturated salt water, dried over anhydrous sodium sulfate, and concentrated to obtain C110-4 (crude) which was directly used in the next step. LC-MS:m/z =381.9 [M+H]⁺

### Step 5:

C110-4 (crude, 100% by theoretical calculation, 2.7g,7.3 mmol), ethyl acetoacetate (5.7g,43.8 mmol), tetrazoleacetic acid (200 mg,1.46 mmol), cuprous iodide (138 mg,0.73 mmol) and cesium carbonate (4.7g,14.6 mmol) were dispersed into dimethyl sulfoxide (100mL), and the mixture was heated to 100°C and stirred for 2 h under the protection of nitrogen. TLC showed the raw materials disappeared. The reaction solution was cooled to room temperature, added with water/EA, extracted and separated, and the organic phases were combined, washed with saturated salt water, dried over anhydrous sodium sulfate, and concentrated to obtain C110-5 (crude) which was directly used in the next step.

### Step 6:

C110-5 (crude, 100% by theoretical calculation, 2.6g,7.3mmol) was dissolved in DMF, 60% sodium hydride (0.4g,11 mmol) was added in batches at 0°C, stirred for 30min after addition, methyl iodide (1.6g,11 mmol) was added at 0°C, and the mixture was heated to room temperature and stirred for 1h. TLC showed the raw materials disappeared. The reaction solution was poured into ice water, extracted with EA and separated, and the organic phases were combined, washed with saturated salt water, dried over anhydrous sodium sulfate, and concentrated, and the crude product was separated and purified by silica gel column chromatography to obtain C110-6(2.2g, yield for four steps: 79%) as an oil. LC-MS:m/z =380.1 [M+H]⁺

### Step 7:

C110-6(2.2g,5.8 mmol), NBS(1.08g,6 mmol) and BPO(140 mg,0.58 mmol) were dispersed into carbon tetrachloride (50mL), and the mixture was heated to 80°C to react for 1h, and half of the raw materials were left, then additional NBS(0.5g,3 mmol) was added, and continued to react for 1h. TLC showed the raw materials disappeared. The reaction solution was filtered while hot, and the filtrate was concentrated to obtain C110-7(2.6g, crude), which was directly used in the next step.

### Step 8:

C110-7(2.6g, crude) was dissolved in DMF(20mL), NaN₃(500 mg,7.7 mmol) was added, and stirred at room temperature for 30min. TLC showed the raw materials disappeared. The reaction solution was added with water, extracted with EA, and the organic phases were combined, washed with saturated salt water, dried over anhydrous sodium sulfate, and concentrated to obtain C110-8(2.4 g, crude), which was directly used in the next step.

### Step 9:

C1 10-8(2.4g, crude) and PPh₃(2.2g,8.4 mmol) were dispersed into a mixed solvent of THF(50mL) and H₂O(5 mL), and the mixture was heated to 70°C and stirred for 1h. TLC showed the raw materials disappeared. The reaction solution was cooled to room temperature, diluted with 1N dilute HCL, extracted with EA, washed with dilute HCl twice, the organic phase was discarded, water layers were combined, adjusted to be alkaline with NaOH, extracted with EA, the organic phases were combined, washed with saturated salt water, dried over anhydrous sodium sulfate, and concentrated to obtain C1 10-9(1.8g, crude) as an oil. LC-MS:m/z = 377.0 [M-NH₂]⁺.

### Step 10:

C110-9(1.8g,4.5 mmol) was dissolved in DCM(50mL), (BOC)₂O(1.0g,4.5 mmol) was added, and stirred at room temperature for 1h. TLC showed the raw materials disappeared. The reaction solution was added with water, extracted with DCM, and the organic phases were combined, washed with saturated salt water, dried over anhydrous sodium sulfate, and concentrated, and the crude product was separated and purified by silica gel column chromatography to obtain solid C110-10(0.8 g, yield for four steps: 28%).

### Step 11:

C110-10(300 mg,0.61 mmol), 1-naphthaleneboronic acid(135 mg,0.78 mol), cesium carbonate (591 mg,1.8 mmol) and Pd(dppf)Cl₂(49 mg,0.06 mmol) were dispersed into a mixed solvent of dioxane (20mL) and water (2mL), and the mixture was heated to 100°C and stirred for 3 h under the protection of nitrogen. TLC showed the raw materials disappeared. The reaction solution was cooled to room temperature, quenched with water, extracted with ethyl acetate, and the organic phases were combined, washed with saturated salt water, dried over anhydrous sodium sulfate, and concentrated, and the crude product was purified by silica gel column chromatography to obtain C110-11 (202 mg, yield 61%). LC-MS: Not available

### Step 12:

C110-11(202 mg,0.37 mmol) was dissolved in DCM (10 mL), TFA(5mL) was added, and the mixture was stirred at room temperature for 30min. TLC showed the raw materials disappeared. The reaction solution was concentrated to obtain C110-12(crude), which was directly used in the next step.

### Step 13:

C110-12 (crude) was dissolved in acetonitrile (10mL), 1H-pyrazol-1-formamidine hydrochloride (90 mg,0.62 mmol) and DIPEA(184 mg,1.4 mmol) were added, the mixture was stirred at room temperature for 48h, and solids were precipitated. The reaction solution was filtered, and the filter cake was washed and dried to obtain solid SYY-C110(105 mg, yield for two steps: 58%). ¹H NMR: (DMSO-*d₆*, 400MHz): 1.03-1.16 (m, 1H), 1.22-1.35 (m, 4H), 1.43 (t, J = 6.8 Hz, 3H), 1.48-1.56 (m, 1H), 1.56-1.77 (m, 2H), 3.83 (s, 3H), 4.34-4.44 (m, 2H), 4.68-4.74 (m, 1H), 4.95 (d, J = 5.2 Hz, 2H), 7.23-7.72 (m, 10H), 7.93 (t, J = 8.8 Hz, 2H), 8.42 (t, J = 5.2 Hz, 1H). LC-MS:m/z =485.3 [M+H]⁺ (98.30% purity, 220 nm)

### Example 96: Preparation of Compound SYY-C111

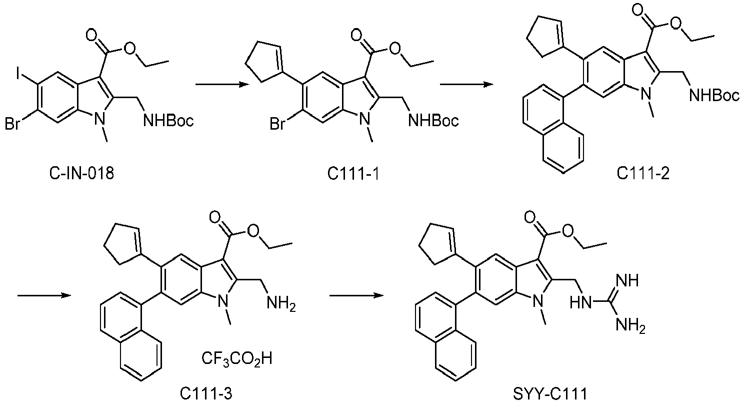

### Step 1:

C-IN-018(1.5g,2.8 mmol), cyclopentenylboric acid (468 mg,4.18 mmol), Pd(dppf)Cl2(150mg,0.18 mmol) and sodium carbonate (592 mg,5.59 mmol) were dispersed into a mixed solvent of dioxane (20 mL) and water (10 mL), and the mixture was heated to 80 °C to react for 16 hours under the protection of nitrogen. TLC showed the reaction was completed. The reaction solution was cooled to room temperature, added with water, extracted with ethyl acetate, and the organic phases were combined, washed with saturated salt water, dried over anhydrous sodium sulfate, and concentrated, and the crude product was purified by silica gel column chromatography to obtain C111-1(550 mg, yield 41%) as a light yellow solid, LC-MS:m/z = 423.0 [M-55]⁺.

### Step 2:

C111-1(550 mg,1.15 mmol), naphthylboronic acid (194 mg,1.73 mmol), Pd(dppf)Cl2(98 mg,0.12 mmol) and sodium carbonate (251 mg,2.37 mmol) were dispersed into a mixed solvent of dioxane (20 mL) and water (10 mL), and the mixture was heated to 100 °C to react for 1 hour under the protection of nitrogen. TLC showed the reaction was completed. The reaction solution was cooled to room temperature, added with water, extracted with ethyl acetate, and the organic phases were combined, washed with saturated salt water, dried over anhydrous sodium sulfate, concentrated, separated and purified by silica gel column chromatography to obtain C111-2(460 mg, yield 76%) as a light yellow solid. LC-MS:m/z= 509.3 [M-17]⁺

### Step 3:

A solution of C111-2(230 mg,0.483 mmol) and TFA(6 mL) in DCM(6 mL) were stirred at room temperature for 30 min. TLC showed the reaction was completed. The reaction solution was concentrated to obtain C112-3 (crude ) as a white solid, which was directly used for the next reaction, LC-MS:m/z = 408.2 [M- NH₂]⁺.

### Step 4:

C112-3(crude) was dissolved in acetonitrile (6 mL), DIPEA(121 mg,0.93 mmol) and 1H-pyrazol-1-formamidine hydrochloride(72 mg,0.45 mmol) were added at room temperature, and the mixture was reacted at room temperature for 16 hours, and white solids were precipitated. The reaction solution was filtered, and the filter cake was washed and dried to obtain SYY-C111(120 mg, yield for two steps: 57%) as a white solid. ¹H NMR: (DMSO-*d₆*, 400MHz): δ1.44 (t, J = 7.2 Hz, 3H), 2.22 (br, 6H), 4.07 (s, 3H), 4.39 (q, J = 7.2 Hz, 2H), 4.99 (s, 2H), 7.32-8.06 (m, 9H), 8.20 (s, 1H), 8.39-8.59 (m, 3H), 9.01 (d, J = 8.4 Hz, 1H). LC-MS:m/z= 467.2[M+H]⁺ (99.13% purity, 220 nm)

### Example 97: Preparation of Compound SYY-C112

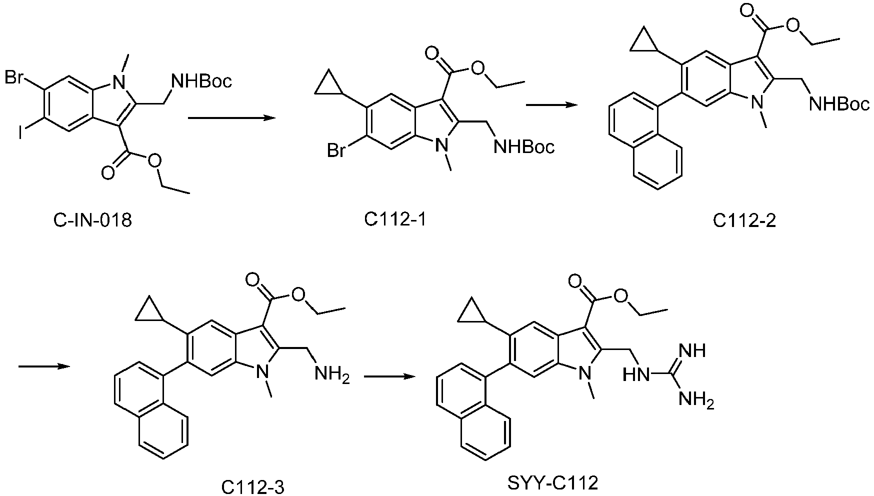

### Step 1:

C- IN-018(500 mg,0.93 mmol), cyclopropylboric acid (160 mg,1.86 mmol), Pd(dppf)Cl₂(90 mg, 0.11 mmol) and sodium carbonate (300 mg,2.83 mmol) were dispersed into a mixed solvent of dioxane (20 mL) and water (10 mL), and the mixture was heated to 80°C to react overnight under the protection of nitrogen. TLC showed the reaction was completed. The reaction solution was cooled to room temperature, added with water, extracted with ethyl acetate, and the organic phases were combined, washed with saturated salt water, dried over anhydrous sodium sulfate, and concentrated, and the crude product was separated and purified by silica gel column chromatography to obtain C1 12-1(220 mg, yield 52%) as a light yellow solid, LC-MS:m/z = 397.1[M-55]⁺.

### Step 2:

C112-1(220 mg,0.49 mmol), naphthylboronic acid (110 mg,0.64 mmol), Pd(dppf)Cl₂(37 mg,0.045 mmol) and sodium carbonate (155 mg,1.46 mmol) were dispersed into a mixed solvent of dioxane (20 mL) and water (10 mL), and the mixture was heated to 100 °C to react for 1 hour under the protection of nitrogen. TLC showed the reaction was completed. The reaction solution was cooled to room temperature, added with water, extracted with ethyl acetate, and the organic phases were combined, washed with saturated salt water, dried over anhydrous sodium sulfate, and concentrated, and the crude product was separated and purified by silica gel column chromatography to obtain C112-2(230 mg, yield 94%) as a light yellow solid. LC-MS:m/z= 481.3[M-55]⁺

### Step 3:

C112-2(230 mg, 0.46 mmol) and TFA(6 mL) were dissolved in DCM (6 mL) and stirred at room temperature for 30min. TLC showed the reaction was completed. The reaction solution was concentrated to obtain C112-3 (230 mg,crude) as a white solid, which was directly used for the next reaction, LC-MS:m/z = 382.2 [M- NH₂]⁺.

### Step 4:

C112-3(230 mg,0.46 mmol) was dissolved in acetonitrile (6 mL), DIPEA(180 mg,1.39 mmol) and 1H-pyrazol-1-formamidine hydrochloride (75 mg,0.51 mmol) were added at room temperature, and the mixture was stirred overnight at room temperature. LCMS showed the reaction was completed. The reaction solution was concentrated and purified by pre-HPLC to obtain a white solid (96 mg ,TAF salt, yield for two steps: 38%) . ¹H NMR: (DMSO-*d₆*, 400MHz): δ0.47-0.63 (m, 4H), 1.39-1.43 (m, 4H), 3.78 (s, 3H), 4.38 (q, J = 7.2 Hz, 2H), 4.87-4.97 (m, 2H), 7.49 (m, 11H), 7.99 (t, J = 8.0 Hz, 2H), 8.07-8.12 (m, 1H). LC-MS:m/z= 441.2[M+H]⁺ (98.42% purity, 254 nm)

### Example 98: Preparation of Compound SYY-C113

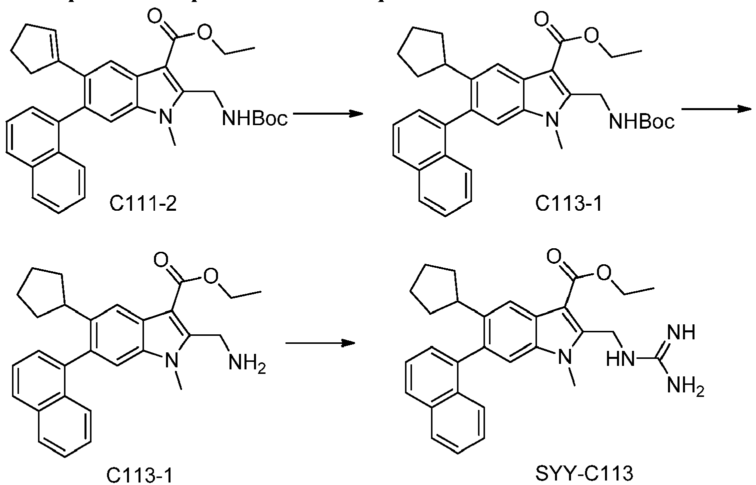

### Step 1:

C111-2(350 mg,0.67 mmol) was dissolved in ethyl acetate (20 mL), Pd/C(50 mg) was added at room temperature, replaced with hydrogen for 3 times, and the mixture was reacted at room temperature for 16 hours. lcms showed the reaction was completed. The reaction liquid was filtered by diatomite, and the filtrate was concentrated to obtain C113-1(350 mg, crude) as a white solid, which was directly used for the next reaction. LC-MS:m/z= 509.3 [M-17]⁺

### Step 2:

A solution of C113-1(350 mg, crude) and TFA(6 mL) in DCM(6 mL) was stirred at room temperature for 30 min. TLC showed the reaction was completed. The reaction solution was concentrated to obtain C113-2 (250 mg,crude) as a white solid, which was directly used for the next reaction, LC-MS:m/z = 410.2 [M- NH₂]⁺.

### Step 3:

C113-2(250 mg,0.59 mmol) was dissolved in CH3CN (6 mL), DIPEA(189 mg,1.47 mmol) and 1H-pyrazol-1-formamidine hydrochloride (128 mg,0.87 mmol) were added at room temperature, and the mixture was reacted at room temperature for 16 hours. LCMS showed the reaction was completed. The reaction solution was concentrated and purified by pre-HPLC to obtain a white solid (140 mg ,TAF salt, yield for three steps: 37%). ¹H NMR: (DMSO-*d₆*, 400MHz): δ1.31-1.49 (m, 6H), 1.60-1.79 (m, 5H), 2.65-2.73 (m, 1H), 3.79 (s, 3H), 4.40 (q, J = 7.2 Hz, 2H), 4.89-5.00 (m, 2H), 7.24-7.67 (m, 9H), 8.01 (t, J = 8.8 Hz, 3H), 8.16 (s, 1H). LC-MS:m/z= 469.3 [M+H]⁺ (98.61% purity, 220 nm)

### Example 99: Preparation of Compound SYY-C114

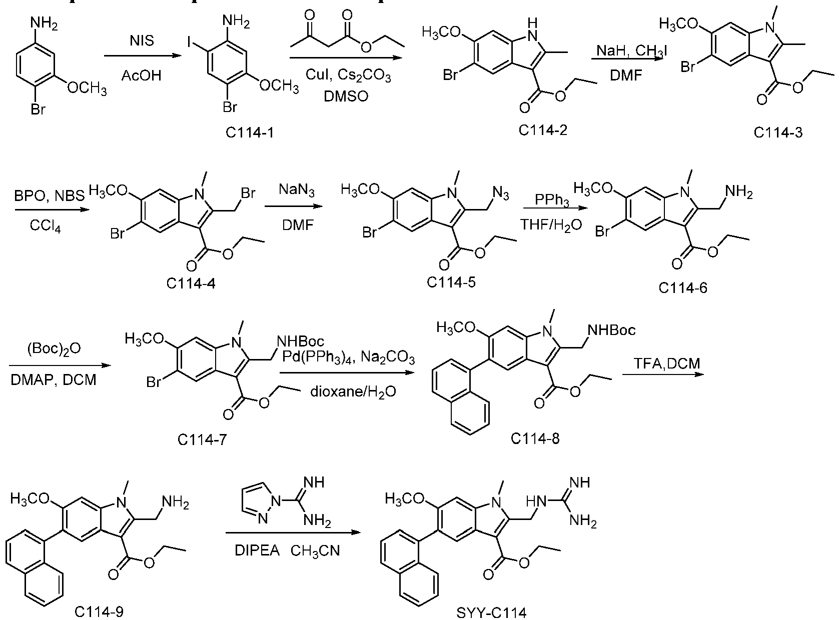

### Step 1:

4-Bromo-3-methoxyaniline (20.0g,99 mmol) was dissolved in AcOH(50 mL), NIS(24.5g,108 mmol) was added in batches under an ice bath, and the mixture was stirred for 1 h under an ice bath after addition. TLC showed the reaction was completed. The reaction solution was filtered, and the filter cake was washed with water, dissolved with EA, dried over anhydrous sodium sulfate, and concentrated to obtain C114-1(28.3g, crude).

### Step 2:

C1 14-1(28.3g, crude) was dissolved in DMSO(100 ml), ethyl acetoacetate (66.6g,512 mmol), tetrazoleacetic acid(2.2g,17.1 mmol), CuI(1.7g,8.54 mmol) and Cs₂CO₃(55.6g, 170.7 mmol) were added sequentially, replaced with N₂ for three times, and the mixture was heated to 100 °C to react overnight. TLC showed the reaction was completed. The reaction solution was cooled to room temperature, added with water, extracted with EA, and the organic phases were combined, washed with saturated salt water, dried over anhydrous sodium sulfate, and concentrated to obtain C114-2(16.1 g, crude). HNMR(DMSO-d6, 400Hz):11.80 (s, 1H), 8.02 (s, 1H), 6.99 (s, 1H), 4.26 (q, J=7.2 Hz, 2H), 3.85 (s, 3H), 2.61 (s, 3H), 1.33 (t, J=7.2 Hz, 3H). LC-MS:m/z= 314.0 [M+H]⁺

### Step 3:

C114-2(16.1g, crude) was dissolved in DMF(80 ml), 60% NaH(3.1g,77.2 mmol) was added in batches under an ice bath, stirred for 20 min after addition, CH₃I(10.9g,77.2 mmol) was added, and the mixture was stirred for 20 min, slowly heated to room temperature and stirred for 30 min. TLC showed the reaction was completed. The reaction solution was added with water, extracted with EA, and the organic phases were combined, washed with saturated salt water, dried over anhydrous sodium sulfate, and concentrated to obtain C114-3(14.0 g, crude). HNMR(DMSO-d6, 400Hz):8.05 (s, 1H), 7.23 (s, 1H), 4.28 (q, J=7.2 Hz, 2H), 3.90 (s, 3H) , 3.70 (s, 3H) , 2.67 (s, 3H), 1.34 (t, J=7.2 Hz, 3H). LC-MS:m/z= 328.0 [M+H]⁺

### Step 4:

C114-3(2.0 g, crude) was dissolved in CCl₄ (20 ml), NBS(1.2 g,6.77 mmol) and BPO(150 mg,0.62 mmol) were added at room temperature, and the mixture was heated to 70 °C to react for 2 h. TLC showed the reaction was completed. The reaction solution was concentrated, slurried with DCM, filtered, and the filter cake was washed and dried to obtain C114-4(1.5g, crude), LC-MS:m/z = 405.9 [M + H]⁺

### Step 5:

C1 14-4(1.5g, crude) was dissolved in DMF(10 ml), NaN₃(0.3g,4.1 mmol) was added, and the mixture was stirred overnight at room temperature. TLC showed the reaction was completed. The reaction solution was added with water, extracted with EA, and the organic phases were combined, washed with saturated salt water, dried over anhydrous sodium sulfate, and concentrated to obtain C114-5(1.1 g, crude), which was directly used in the next step.

### Step 6:

C114-5(1.1 g, crude) was dissolved in THF(20 ml) and water (5 ml), PPh₃(1.2 g,4.69 mmol) was added at room temperature, and the mixture was heated to 80°C to react for 3 h. TLC showed the reaction was completed. The reaction solution was cooled to room temperature, 1N dilute hydrochloric acid was added to adjust pH to be acidity, extracted with EA, the organic phase was discarded, and the water phase was adjusted to weakly alkaline with saturated NaHCO₃ solution, extracted with EA, and the organic phases were combined, washed with saturated salt water, dried over anhydrous sodium sulfate, and concentrated to obtain C114-6(500 mg, crude). LC-MS:m/z=326.0 [M-NH₂]⁺.

### Step 7:

C114-6(500 mg, crude) was dissolved in DCM(10 ml), DMAP(18 mg,0.15mmol) was added, BOC anhydride (384 mg,1.76 mmol) was slowly added, and the mixture was reacted at room temperature for 30 min after addition. TLC showed the reaction was completed. The reaction solution was added with water, extracted with DCM, and the organic phases were combined, washed with saturated salt water, dried over anhydrous sodium sulfate, and concentrated to obtain C114-7(360 mg, crude).

### Step 8:

C114-7(360 mg, crude) was dissolved in dioxane(20 ml) and water (5 ml), 2-Naphthylboronic acid(180 mg,0.98 mmol), Na₂CO₃(180 mg,1.64 mmol) and PdCl₂(dppf)(70 mg,0.082 mmol) were added at room temperature, replaced with N₂ for three times, and the mixture was heated to 100 °C to react for 2 h. TLC showed the reaction was completed. The reaction solution was cooled to room temperature, added with water, extracted with EA, and the organic phases were combined, washed with saturated salt water, dried over anhydrous sodium sulfate, and concentrated, and the crude product was purified by silica gel column chromatography to obtain C114-8(240 mg, yield for eight steps: 3%). LC-MS:m/z=433.1 [M-55]⁺

### Step 9:

C114-8(240 mg,0.49 mmol) was dissolved in DCM(5 ml), TFA(3 ml) was added and stirred at room temperature for 30 min. TLC showed the reaction was completed. The reaction solution was concentrated to obtain C114-9 (crude), which was directly used in the next step.

### Step 10:

C114-9 (crude) was dissolved in acetonitrile (5 ml), 1H-pyrazol-1-formamidine hydrochloride (110 mg,0.75 mmol) was added, and then DIPEA(260 mg,2 mmol) was added, the mixture was stirred at room temperature for a weekend, and solids were precipitated. The reaction solution was filtered, and the filter cake was washed and dried to obtain solid SYY-C114(68 mg, yield for two steps: 32%). 1H NMR (400 MHz, DMSO-d6) δ1.23 (t, J = 7.2 Hz, 3H), 3.72 (s, 3H), 3.93 (s, 3H), 4.28 (q, J = 7.2 Hz, 2H), 4.95 (s, 2H), 7.22-7.65 (m, 9H), 7.81 (s, 1H), 7.92-7.98 (m, 2H), 8.35 (br, 1H). LC-MS:m/z=431.2 [M+H]⁺

### Example 100: Preparation of Compound SYY-C115

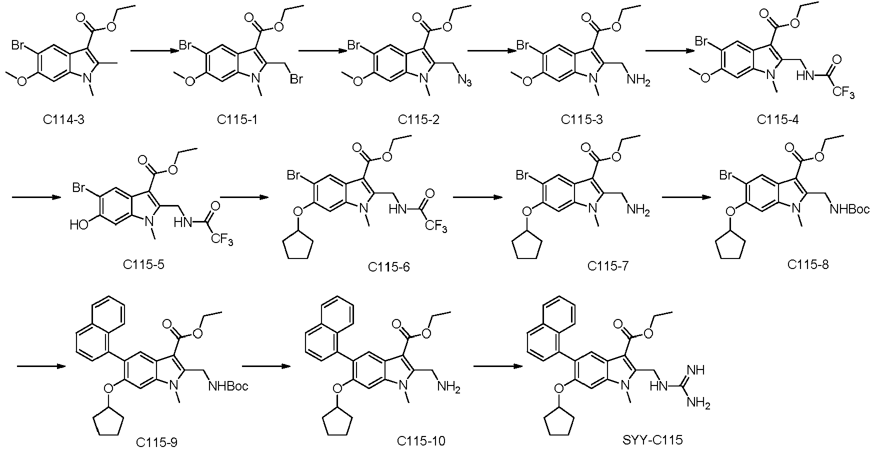

### Step 1:

C114-3(4.3g, 13.18 mmol) and NBS(3.5g, 19.67 mmol) were suspended in CCl₄(150 mL), BPO(318 mg, 1.31 mmol) was added at room temperature, and the mixture was heated to 80 °C to react for 3 hours. TLC showed the raw materials were basically reacted completely. The reaction solution was cooled to room temperature, filtered, and the filtrate was concentrated to obtain C115-1(6.0g, crude) as a dark green solid.

### Step 2:

C115-1(6.0g, crude) was dissolved in DMF(40 mL), NaN₃(962 mg, 14.80 mmol) was added at room temperature, and the mixture was reacted at room temperature for 10 minutes. TLC showed the raw materials were reacted completely. The reaction solution was added with water, extracted with ethyl acetate. The organic phases were combined, washed with saturated salt water, dried over anhydrous sodium sulfate, and concentrated to obtain C115-2(5.5 g, crude) as a brown solid.

### Step 3:

C115-2(5.5g, crude) was dissolved in THF(80 mL) and H₂O(40 mL), PPh₃(4.7g, 17.92 mmol) was added at room temperature, and the mixture was heated to 80 °C to react for half an hour. TLC showed the raw materials were reacted completely. The reaction solution was cooled to room temperature, added with water, extracted with ethyl acetate, and washed with 1N dilute hydrochloric acid. The aqueous phase was added with 3N NaOH to adjust the pH to be alkaline, extracted with ethyl acetate, and the organic phases were combined, washed with saturated salt water, dried over anhydrous sodium sulfate, and concentrated, and the crude product was slurried with PE/EA/DCM = 3/1/1 to obtain C115-3(1.8g, crude) as a white solid. LC-MS:m/z = 326.0 [M-NH₂]⁺.

### Step 4:

C115-3(1.8g, crude), DMAP(64 mg, 0.52 mmol) and triethylamine (1.1g, 10.87 mmol) were dissolved in DCM(50 mL), TFAA(2.2g, 10.46 mmol) was added under an ice bath, and the mixture was reacted under an ice bath for 1 hour after addition. TLC showed the raw materials were reacted completely. The reaction solution was concentrated, added with water, extracted with ethyl acetate, and the organic phases were combined, washed with saturated salt water, dried over anhydrous sodium sulfate, and concentrated, and the crude product was purified by silica gel column chromatography to obtain C1 15-4(1.5g, yield for four steps: 26%) as a white solid. LC-MS:m/z=439.0 [M+H]⁺, ¹H NMR(CDCl₃, 400Hz):δ8.22 (s, 1H), 7.97(br, 1H), 6.81(s, 1H), 4.88 (d, J=6.4 Hz, 2H), 4.50 (q, J=7.2 Hz, 2H), 3.97(s, 3H), 3,90(s, 3H), 1.49(t, J=7.2 Hz, 3H).

### Step 5:

C115-4(1.5g,3.43 mmol) was dissolved in DCM(10 mL), and BBr₃(15 mL, 1N in DCM) was added dropwise under an ice bath. The mixture was reacted under an ice bath for 2 hours. TLC showed the raw materials were reacted completely. The reaction solution was concentrated to dryness, the crude product was dissolved with dichloromethane and methanol, washed with saturated salt water, dried over anhydrous sodium sulfate, and concentrated to obtain C115-5(1.6g, crude) as a gray-white solid. LC-MS: Not available

### Step 6:

C115-5(1.6g, crude) and bromocyclopentane (789 mg,5.29 mmol) were dissolved in DMF(30 mL), K₂CO₃ (1.0g, 7.24 mmol) was added at room temperature, and the mixture was heated to 70 °C to react overnight. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C115-6(750 mg, yield 45%) as a white solid. LC-MS:m/z = 491.1 [M + H]⁺.

### Step 7:

C115-6(750 mg,1.53 mmol) was dissolved in ethanol (6 mL) and sodium hydroxide (2.2 mL, 1M), and the mixture was heated to 75 °C to react for 1 hour. TLC showed the reaction was completed. The reaction solution was cooled to room temperature, concentrated, added with water, extracted with ethyl acetate, and the organic phases were combined, washed with saturated salt water, dried over anhydrous sodium sulfate, and concentrated to obtain C115-7(630 mg, crude). LC-MS:m/z=380.1 [M-NH₂]⁺.

### Step 8:

C115-7(630 mg, crude) was dissolved in DCM(8 mL), Boc anhydride (417 mg,1.91 mmol) was added dropwise at room temperature, and the mixture was reacted for half an hour at room temperature after the addition. TLC showed the raw materials were reacted completely. The reaction solution was purified by silica gel column chromatography to obtain C115-8(630 mg, yield for two steps: 80%) as a white solid.

### Step 9:

C115-8(300 mg,0.61 mmol), 1-naphthylboronic acid (156 mg,0.91 mmol) and cesium carbonate (592 mg, 1.82 mmol) were dissolved in dioxane (6 mL) and water (1 mL), PdCl₂(dppf) (45 mg, 0.055 mmol) was added at room temperature, and the mixture was heated to 100 °C to react for 2 hours under the protection of nitrogen. TLC showed the reaction was completed. The reaction solution was cooled to room temperature, added with water, extracted with ethyl acetate, and the organic phases were combined, washed with saturated salt water, dried over anhydrous sodium sulfate, and concentrated, and the crude product was purified by silica gel column chromatography to obtain C1 15-9(340 mg, crude) as a colorless oil.

### Step 10:

C115-9(340 mg,0.63 mmol) was dissolved in DCM(6 mL), TFA (6 mL) was added at room temperature, and the mixture was reacted at room temperature for half an hour. TLC showed the raw materials were reacted completely. The reaction solution was concentrated and dried, the crude product was dissolved with dichloromethane, washed with saturated sodium carbonate, and washed with saturated salt water, dried over anhydrous sodium sulfate, and concentrated to obtain C115-10(230 mg, crude). LC-MS:m/z=426.2 [M-NH₂]⁺.

### Step 11:

C115-10(230 mg,0.52 mmol),, 1H-pyrazol-1-formamidine hydrochloride (84 mg, 0.57 mmol) and DIPEA(135 mg,1.05 mmol) were dissolved in acetonitrile (6 mL), and the mixture was reacted at room temperature for 16 hours. LCMS showed the raw materials were reacted completely. The reaction solution was filtered, and the filter cake was washed and dried to obtain SYY-C115(230 mg, yield for three steps: 78%) as a white solid. ¹H NMR: (DMSO-*d₆*, 400MHz): δ1.04-1.76 (m, 11H), 3.86 (s, 3H), 4.26 (q, J = 7.2 Hz, 2H), 4.83-4.95 (m, 3H), 7.30-7.56 (m, 9H), 7.82 (s, 1H), 7.89-7.95 (m, 2H), 8.07 (m, 1H). LC-MS:m/z=485.2 [M+H]⁺(98.47% purity, 220 nm)

### Example 101: Preparation of Compound SYY-C116

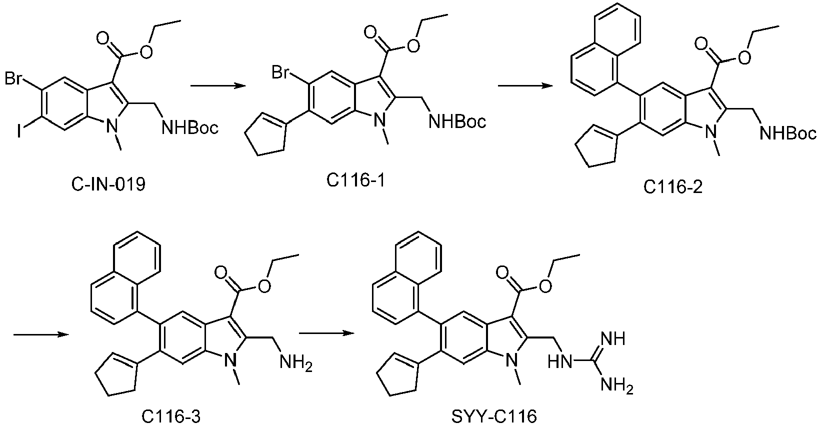

### Step 1:

C- IN-019(1.3g, 2.42 mmol), cyclopenten-1-ylboronic acid (271 mg, 2.42 mmol), sodium carbonate (513 mg, 4.84 mmol) and PdCl₂(dppf)(177 mg, 0.22 mmol) were dispersed into 1,4-dioxane (18 mL) and H₂O(6 mL), and heated to 85 °C to react overnight under the protection of nitrogen. LCMS showed the raw materials were basically reacted completely. The reaction solution was cooled to room temperature, added with water and ethyl acetate, extracted and separated, and the organic phases were combined, washed with saturated salt water, dried over anhydrous sodium sulfate, and concentrated, and the crude product was purified by silica gel column chromatography to obtain C116-1(400 mg, yield 35%) as a white solid. LC-MS:m/z= 423.1 [M-55]⁺

### Step 2:

C116-1(400 mg,0.838 mmol), 1-naphthaleneboronic acid (216 mg, 1.26 mmol), sodium carbonate (178 mg, 1.68 mmol) and PdCl₂(dppf)(61 mg, 0.075 mmol) were dispersed into 1,4-dioxane (8 mL) and H₂O(2 mL), and heated to 85 °C to react for 2 hours under the protection of nitrogen. LCMS showed the raw materials were reacted completely. The reaction solution was cooled to room temperature, added with water and ethyl acetate, extracted and separated, and the organic phases were combined, washed with saturated salt water, dried over anhydrous sodium sulfate, and concentrated, and the crude product was purified by silica gel column chromatography to obtain C1 16-2(360 mg, yield 82%) as a white solid. LC-MS: Not available

### Step 3:

C116-2(180 mg,0.343 mmol) was dissolved in DCM(4 mL), trifluoroacetic acid (2 mL) was added, and reacted at room temperature for 1 hour. TLC showed the raw materials were reacted completely. The reaction solution was concentrated, and the crude product was dissolved with ethyl acetate, washed with saturated sodium carbonate solution, and washed with saturated salt water, dried over anhydrous sodium sulfate, and concentrated to obtain C116-3(137 mg, crude) as a yellow solid, LC-MS : m/z = 408.2 [M-NH₂]⁺.

### Step 4:

C116-3(137 mg,0.32 mmol), 1H-pyrazol-1-formamidine hydrochloride (52 mg, 0.35 mmol) and DIPEA(83 mg, 0.65 mmol) were dissolved in acetonitrile (3 mL) and methanol (1mL), and the mixture was stirred overnight at room temperature. The reaction solution was filtered, and the filter cake was washed and dried to obtain SYY-C116(100 mg, yield for two steps: 62%) as a light yellow solid. ¹H NMR: (DMSO-*d₆*, 400MHz): δ1.50 (t, J = 7.2 Hz, 3H), 2.05-2.15 (m, 4H), 2.19-2.29 (m, 2H), 3.88 (s, 3H), 4.39 (q, J = 7.2 Hz, 2H), 4.90 (s, 2H), 7.16-7.54 (m, 4H), 7.63-7.71 (m, 2H), 7.76 (s, 1H), 7.88 (d, J = 8.8 Hz, 1H), 8.00 (d, J = 8.0 Hz, 1H), 8.12 (br, 1H), 8.71 (d, J = 8.4 Hz, 1H), 8.95 (s, 1H). LC-MS:m/z= 467.2 [M+1]⁺(97.23% purity, 254 nm)

### Example 102: Preparation of Compound SYY-C117

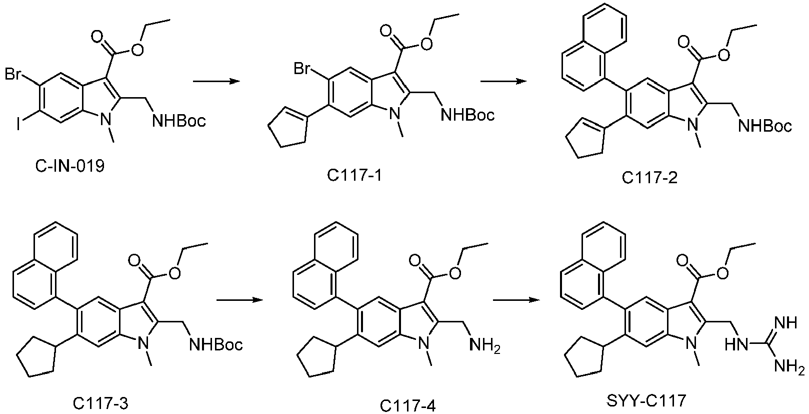

### Step 1:

C- IN-019(1.3g, 2.42 mmol) was dissolved in a mixed solvent of 1, 4-dioxane (18 mL) and H₂O(6 mL), cyclopenten-1-ylboronic acid (271 mg, 2.42 mmol), sodium carbonate (513 mg, 4.84 mmol) and PdCl₂(dppf)(177 mg, 0.22 mmol) were added at room temperature, heated to 85 °C to react overnight under the protection of nitrogen. TLC showed the raw materials were basically reacted completely. The reaction solution was cooled to room temperature, added with water and ethyl acetate, extracted and separated, and the organic phases were combined, washed with saturated salt water, dried over anhydrous sodium sulfate, and concentrated, and the crude product was purified by silica gel column chromatography to obtain C117-1(400 mg, yield 35%) as a white solid. LC-MS:m/z = 423.1 [M-55]⁺.

### Step 2:

C117-1(400 mg,0.838 mmol) was dissolved in a mixed solvent of 1, 4-dioxane (8 mL) and H₂O(2 mL), 1-naphthylboronic acid (216 mg, 1.26 mmol), sodium carbonate (178 mg, 1.68 mmol) and PdCl₂(dppf)(61 mg, 0.075 mmol) were added at room temperature, and the mixture was heated to 85 °C to react for 2 hours under the protection of nitrogen. LCMS showed the raw materials were reacted completely (MS no peak). The reaction solution was cooled to room temperature, added with water and ethyl acetate, extracted and separated, and the organic phases were combined, washed with saturated salt water, dried over anhydrous sodium sulfate, and concentrated, and the crude product was purified by silica gel column chromatography to obtain C117-2(360 mg, yield 82%) as a white solid.

### Step 3:

C117-2(180 mg,0.343 mmol) was dissolved in ethyl acetate (4 mL), 10% Pd/C (100 mg) was added at room temperature, replaced with hydrogen for 3 times, and stirred overnight at room temperature. LCMS showed the raw materials were reacted completely (MS no peak). The reaction solution was filtered with diatomite, and the filtrate was concentrated to obtain C117-3(150 mg, crude) as a white solid.

### Step 4:

C117-3(150 mg,0.343 mmol) was dissolved in DCM(6 mL) and trifluoroacetic acid (2 mL) was added. The mixture was reacted at room temperature for 1h. TLC showed the raw materials were reacted completely. The reaction solution was concentrated to dryness, and the crude product was dissolved by adding ethyl acetate, washed with saturated sodium carbonate solution, and washed with saturated salt water, dried over anhydrous sodium sulfate, and concentrated to obtain C117-4(120 mg, crude) as a yellow solid, LC-MS : m/z = 410.2 [M-NH₂]⁺.

### Step 5:

C117-4(120 mg,0.28 mmol), 1H-pyrazol-1-formamidine hydrochloride (45 mg, 0.31 mmol) and DIPEA(73 mg, 0.56 mmol) were dissolved in acetonitrile (3 mL) and stirred at room temperature for a weekend. The reaction solution was purified by pre-HPLC to obtain SYY-C117(TFA salt)(110 mg, yield for three steps: 56%) as a white solid. ¹H NMR: (DMSO-*d₆*, 400MHz): δ1.20 (t, J = 7.2 Hz, 3H), 1.25-1.36 (m, 2H), 1.49-1.77 (m, 6H), 2.55-2.69 (m, 1H), 3.92 (s, 3H), 4.23-4.30 (m, 2H), 4.90-4.99 (m, 2H), 7.30 (d, J = 8.0 Hz, 2H), 7.37-7.42 (m, 3H), 7.51-7.54 (m, 1H), 7.58-7.62 (m, 1H), 7.72 (s, 1H), 7.75 (s, 1H), 7.97-8.04 (m, 3H). LC-MS:m/z = 469.3 [M+H]⁺ (99.06% purity, 220 nm)

### Example 103: Preparation of Compound SYY-C118

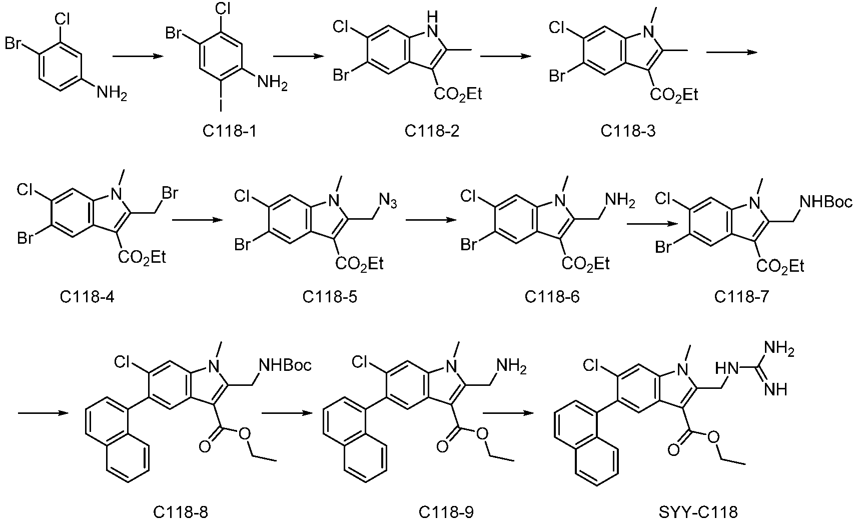

### Step 1:

4-Bromo-3-chloroaniline (5.0g,24.2 mmol) was dissolved in acetic acid (25 mL), NIS (6.5g,28.9 mmol) was added, and stirred at room temperature for 1h. TLC showed the reaction was completed. The reaction solution was filtered, the filtrate was added with water, extracted with ethyl acetate, and the organic phases were combined, washed with saturated salt water, dried over anhydrous sodium sulfate, and concentrated to obtain solid 118-1(4 .0g, crude) which was directly used in the next step.

### Step 2:

C181-1(3.0 g,9.0 mmol), ethyl acetoacetate (7.0 g,53.8 mmol), tetrazoleacetic acid (0.2 g,1.8 mmol), cuprous iodide (0.17 g,0.9 mmol) and cesium carbonate (6 g,18.1 mmol) were dispersed into DMSO(150mL), and heated to 100°C to react for 3h under the protection of nitrogen. TLC detection showed the reaction was completed. The reaction solution was cooled to room temperature, added with saturated salt water, extracted with ethyl acetate, dried over anhydrous sodium sulfate, and concentrated to obtain C1 18-2(2.5g, crude) as a gray solid which was directly used in the next step. LC-MS:m/z = 315.9 [M + H]⁺.

### Step 3:

C1 18-2(2.5g,7.9 mmol) was dissolved in DMF, 60% sodium hydride (0.5g,11.9 mmol) was added at 0°C, and the mixture was reacted for 30min, methyl iodide (1.7g, 11.9 mmol) was added, and reacted for 1h. TLC showed the reaction was completed. The reaction solution was quenched with ice water, extracted with ethyl acetate, and the organic phases were combined, washed with saturated salt water, dried over anhydrous sodium sulfate, and concentrated, and the crude product was separated and purified by silica gel column chromatography to obtain solid C118-3(2.0 g, yield for three steps : 67%).

### Step 4:

C118-3(2.0g,6.0 mmol), NBS(1.2g,6.38mmo l) and BPO(145 mg,0.6 mmol) were dispersed into carbon tetrachloride (80 mL), and heated to 80°C to react for 2h. TLC showed the reaction was completed. The reaction solution was concentrated to obtain C118-4(crude), which was directly used in the next step.

### Step 5:

C118-4 (crude) was dissolved in DMF(20mL), sodium azide was added, and stirred at room temperature for 30min. TLC showed the reaction was completed. The reaction solution was added with water, extracted with ethyl acetate, and the organic phases were combined, washed with saturated salt water, dried over anhydrous sodium sulfate, and concentrated to obtain C118-5 (crude) which was directly used in the next step.

### Step 6:

C118-5 (crude) was dissolved in a mixed solvent of THF(50mL) and water (5mL), triphenylphosphine (2.3g,8.7mmol) was added, and heated to 70°C to react for 3 h. TLC showed the reaction was completed. The reaction solution was cooled to room temperature, diluted with 1M dilute hydrochloric acid, extracted with ethyl acetate, the organic layer was discarded, the water layer was adjusted to be alkaline by adding 1M sodium hydroxide aqueous solution, extracted with ethyl acetate, and the organic phases were combined, washed with saturated salt water, dried over anhydrous sodium sulfate, and concentrated to obtain solid C118-6(1.6g, crude). LC-MS:m/z = 345.0 [M + H]⁺.

### Step 7:

C1 18-6(1.6 g, 4.6 mmol) was dissolved in DCM (50 mL), BOC anhydride (1.1 g,5 mmol) was added, and stirred at room temperature for 1h. TLC showed the reaction was completed. The reaction solution was concentrated, and the crude product was separated and purified by silica gel column chromatography to obtain solid C118-7(1.5g, yield for four steps: 55%). LC-MS:m/z =389.0 [M-55]⁺

### Step 8:

C118-7(400 mg,0.9 mmol), naphthylboronic acid (232 mg,1.35 mmol), and sodium carbonate (372 mg,2.7 mmol) were dispersed into a mixed solvent of dioxane (50 mL) and water (5 mL), and tetratriphenylphosphine palladium (104 mg,0.09 mmol) was added, and the mixture was heated to 100 °C to react for 4 h under the protection of nitrogen. TLC showed the reaction was completed. The reaction solution was cooled to room temperature, added with water, extracted with ethyl acetate, the organic phases were combined, washed with saturated salt water, dried over anhydrous sodium sulfate, and concentrated to obtain C118-8 (crude) which was directly used in the next step.

### Step 8

C118-8 (crude) was dissolved in DCM (10mL), TFA(5mL) was added, and stirred at room temperature for 30min. The reaction solution was concentrated and the crude product was purified by pre-TLC to obtain C118-9(200 mg, yield for two steps: 57%).

### Step 9

C118-9(200 mg, 0.51 mmol), 1H-pyrazol-1-formamidine hydrochloride (90 mg,0.61mmol) and DIPEA(264 mg,2 mmol) were dispersed into acetonitrile (10 ml) and stirred at room temperature for 18h, and solids were precipitated. The reaction solution was filtered, and the filter cake was washed and dried to obtain SYY-C118(120 mg, yield 54%) as a white solid. ¹H NMR: (DMSO-*d₆*, 400MHz): δ1.22 (t, J = 7.2 Hz, 3H), 3.95 (s, 3H), 4.29 ((q, J = 7.2 Hz, 2H), 4.98 (s, 2H), 7.33-7.62 (m, 8H), 7.97-8.05 (m, 4H), 8.38 (br, 1H). LC-MS:m/z =435.1 [M+H]⁺ (99.66% purity, 220 nm)

### Example 104: Preparation of Compound SYY-C119

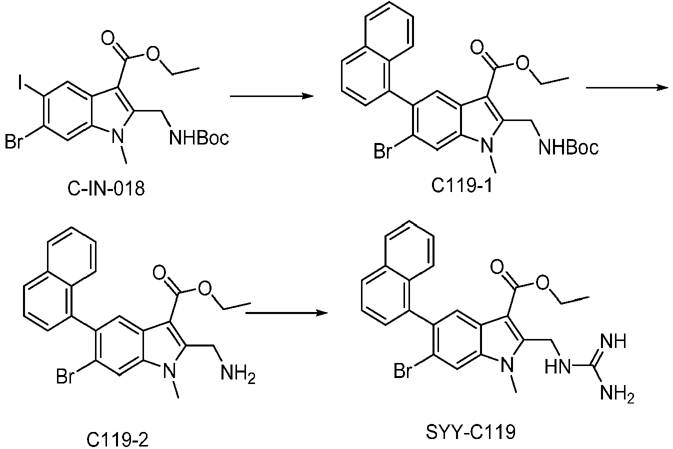

### Step 1:

C-IN-018(200 mg,0.37 mmol), naphthaleneboronic acid (76.8 mg,0.45 mmol), Pd(dppf)Cl₂(33 mg,0.04 mmol) and sodium carbonate (79 mg,0.75 mmol) were dissolved into a mixed solvent of 1,4-dioxane (15 mL) and water (5 mL), and heated to 80°C to react for 1h under the protection of nitrogen. LCMS showed the raw materials disappeared. The reaction solution was cooled to room temperature, added with water, extracted with ethyl acetate, and the organic phases were combined, washed with saturated salt water, dried over anhydrous sodium sulfate, concentrated, separated and purified by silica gel column chromatography to obtain C119-1(180 mg, yield 91%) as a light yellow solid. LC-MS:m/z=437.0 [M-Boc+H]⁺

### Step 2:

C119-1(340 mg, 0.63 mmol) and TFA(6 mL) were dissolved in DCM (6 mL) and stirred at room temperature for 30min. TLC showed the reaction was completed. The reaction solution was concentrated, dissolved with dichloromethane, washed with saturated sodium bicarbonate aqueous solution, dried over anhydrous sodium sulfate, and concentrated to obtain C119-2(260 mg, crude) as a white solid which was directly used in the next step. LC-MS:m/z = 437.1 [M + H]⁺.

### Step 3:

C119-2(260 mg,0.59 mmol) and DIPEA(230 mg,1.78 mmol) were dissolved in acetonitrile (6 mL), 1H-pyrazol-1-formamidine hydrochloride,(96 mg,0.65 mmol) was added, stirred at room temperature overnight, and white solids were precipitated. The reaction solution was filtered, and the filter cake was washed and dried to obtain a white solid (190 mg, yield for two steps: 63%). 1H NMR: (DMSO-d6, 400MHz): δ1.20 (t, J = 6.8 Hz, 3H), 3.94 (s, 3H), 4.26 (q, J = 6.8 Hz, 2H), 4.97 (s, 2H), 7.28-7.59 (m, 8H), 7.90-8.10 (m, 3H), 8.17 (s, 1H), 8.42 (br, 1H). LC-MS: m/z=479.1 [M+H]⁺

### Example 105: Preparation of Compound SYY-C120

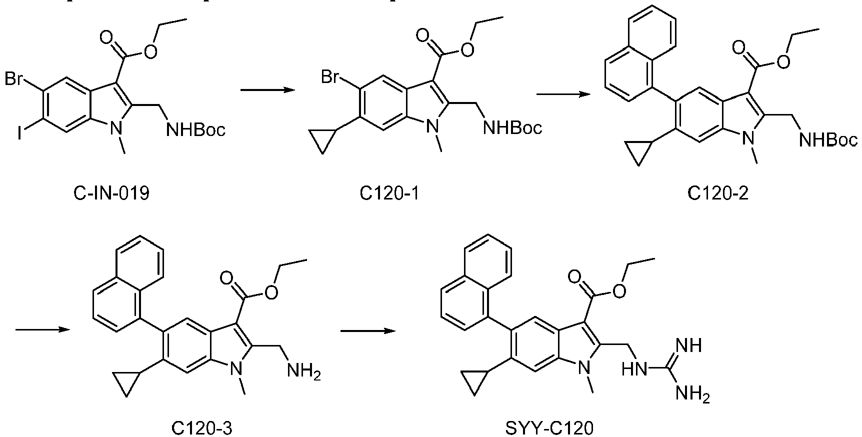

### Step 1:

C- IN-019(1.5g, 2.79 mmol), cyclopropylboric acid (240 mg, 2.79 mmol), potassium carbonate (591 mg, 5.58 mmol) and Pd(PPh3)4(323 mg, 0.28 mmol) were dispersed into DMSO(20 mL), and heated to 90 °C to react overnight under the protection of nitrogen. LCMS showed the raw materials were reacted completely. The reaction solution was cooled to room temperature, added with water and ethyl acetate, extracted and separated, the organic phase was washed with saturated salt water, dried over anhydrous sodium sulfate, and concentrated, and the crude product was purified by silica gel column chromatography to obtain C120-2(190 mg, yield 15%) as a colorless oil. LC-MS:m/z = 395.1 [M-55]⁺.

### Step 2:

C120-1(210 mg,0.465 mmol), 1-naphthaleneboronic acid (120 mg, 0.698 mmol), sodium carbonate (99 mg, 0.93 mmol) and PdCl₂(dppf)(34 mg, 0.047 mmol) were dispersed into a mixed solvent of 1,4-dioxane (6 mL) and H₂O(2 mL), and heated to 85 °C to react for 6 hours under the protection of nitrogen. LCMS showed the raw materials were reacted completely. The reaction solution was cooled to room temperature, added with water and ethyl acetate, extracted and separated, the organic phase was washed with saturated salt water, dried over anhydrous sodium sulfate, and concentrated, and the crude product was purified by silica gel column chromatography to obtain C120-2(230 mg, yield 99%) as a colorless oil. LC-MS:m/z= 443.2 [M-55]⁺

### Step 3:

C120-2(230 mg,0.46 mmol) was dissolved in DCM(6 mL), trifluoroacetic acid (3 mL) was added, and the mixture was reacted at room temperature for 1 hour. TLC showed the raw materials were reacted completely. The reaction solution was concentrated, and the crude product was dissolved with ethyl acetate, washed with saturated sodium carbonate solution, and washed with saturated salt water, dried over anhydrous sodium sulfate, and concentrated to obtain C120-3 (170 mg, crude product) as a brown oil. LC-MS:m/z = 382.2 [M-NH₂]⁺.

### Step 4:

C120-3(170 mg, crude), 1H-pyrazol-1-formamidine hydrochloride (100 mg, 0.68 mmol) and DIPEA(165 mg, 1.28 mmol) were dissolved in a mixed solvent of acetonitrile (3 mL), methanol (1mL) and dichloromethane (2 mL), and stirred at room temperature for 2 d. TLC showed the raw materials were reacted completely. The reaction solution was concentrated and purified by pre-HPLC to obtain SYY-C120 TFA salt (66 mg) as a white solid. ¹H NMR: (DMSO-*d₆*, 400MHz): δ 0.53-0.78(m, 4H), 1.21 (t, J = 7.2 Hz, 3H), 1.37-1.44(m, 1H), 4.27 (q, J = 7.2 Hz, 2H), 4.94 (d, J = 4.4 Hz, 2H), 7.28(s, 1H),7.37-7.54(m, 6H), 7.60-7.63 (m, 1H), 7.80 (s, 1H), 7.96-8.01 (m, 1H), 8.16 (br, 2H).LC-MS:m/z= 441.3 [M+1]⁺ (95.20% purity, 220 nm)

### Example 106: Preparation of Compound SYY-C121

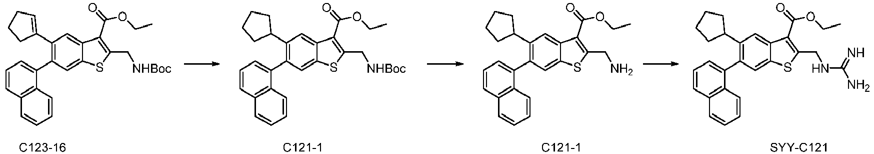

### Step 1:

C123-16(80 mg,0.15 mmol) was dissolved in methanol (2 mL), palladium carbon (catalytic amount) was added, replaced with hydrogen for three times, and heated to 30°C and stirred for 4 hours. LCMS showed the raw materials were reacted completely. The reaction solution was filtered with diatomite, the filtrate was concentrated, and the crude product was purified by pre-TLC(PE/EA = 15/1) to obtain C121-1(75 mg, yield 93%) as a yellow solid.

### Step 2:

C121-1(75 mg,0.14 mmol) was dissolved in DCM(2 mL), TFA(2 mL) was added, and stirred at room temperature for 30 minutes. LCMS detection showed the reaction was completed. The reaction solution was concentrated and dried to obtain C121-2(61 mg, crude) as a yellow oil. LC-MS:m/z=430.2 [M+H]⁺

### Step 3:

C121-2(61 mg, crude) was dissolved in acetonitrile (3 mL), DIPEA(55 mg,0.43 mmol) and 1H-pyrazol-1-formamidine hydrochloride (31.4 mg, 0.21 mmol) were added in sequence, and the mixture was stirred at room temperature for 3 days. LCMS detection showed the reaction was completed. The reaction solution was filtered, and the filter cake was washed with acetonitrile and purified by pre-HPLC to obtain SYY-C121(23 mg, yield for two steps: 28%, TFA salt) as a white solid. ¹H NMR: (DMSO-*d₆*, 400MHz): δ1.27-1.36 (m, 2H), 1.41-1.51 (m, 4H), 1.57-1.79 (m, 5H), 2.61-2.72 (m, 1H), 4.41 (q, J = 7.2 Hz, 2H), 5.02 (d, J = 6.4 Hz, 2H), 7.26 (d, J = 8.4 Hz, 1H), 7.34-7.70 (m, 7H), 7.84 (s, 1H), 7.99 (t, J = 7.6 Hz, 2H), 8.39 (t, J = 6.4 Hz, 1H), 8.51 (s, 1H). LC-MS:m/z=472.2 [M+H]⁺ (99.58% purity, 254 nm)

### Example 107: Preparation of Compound SYY-C122

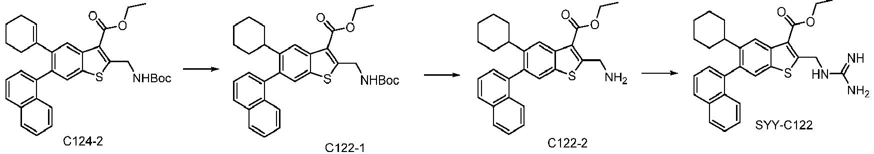

### Step 1:

C124-2(100 mg, 0.185 mmol) was dissolved in methanol (6 mL), replaced with hydrogen for 3 times, and the mixture was heated to 45 °C to react for 2 days. LCMS showed the reaction was completed. The reaction liquid was filtered with diatomite, the filtrate was concentrated, and the crude product was purified by pre-TLC to obtain C122-1(60 mg, yield 60%) as a white solid. 1H NMR (CDCl₃, 400 Hz): δ 8.54 (s, 1H), 7.90 (t, J =7.6 Hz, 2H), 7.60 (s, 1H), 7.46-7.55 (m, 2H), 7.31-7.42 (m, 3H), 5.44-5.48 (m, 1H), 4.77-4.87(m, 2H), 4.51 (q, J=7.2 Hz, 2H), 2.23-2.33 (m, 1H), 1.65-1.79(m, 2H), 1.52-162(m, 7H), 1.39 (s,9H), 1.11-1.13 (m, 2H), 0.83-0.95 (m, 2H). LC-MS:m/z= 444.2 [M-Boc+H] ⁺

### Step 2:

C122-1(60 mg, 0.11 mmol) was dissolved in DCM (3 mL), TFA(1 mL) was added dropwise, and the mixture was reacted at room temperature for half an hour. TLC showed the raw materials were reacted completely. The reaction solution was concentrated to obtain C122-2(crude), which was directly used in the next step. LC-MS:m/z= 444.2[M+H]⁺

### Step 3:

C122-2 (crude product from the previous step), 1H-pyrazol-1-formamidine hydrochloride (21 mg, 0.14 mmol) and DIPEA(57 mg, 0.44 mmol) were dissolved in acetonitrile (3 mL) and methanol (1 mL), and the mixture was heated to 50 °C to react for 2 days. LCMS showed the reaction was completed. The reaction solution was filtered, and the filter cake was washed with acetonitrile, and dried to obtain a white solid (25 mg, yield for two steps: 47%). ¹H NMR: (DMSO-*d₆*, 400MHz): δ 0.66-0.88 (m, 2H), 1.04-1.31 (m, 2H), 1.43-1.73 (m, 9H), 2.18-2.24 (m, 1H), 4.44 (q, J = 7.2 Hz, 2H), 5.04 (d, J = 3.2 Hz, 2H), 7.27 (d, J = 8.4 Hz, 1H), 7.39-7.64 (m, 7H), 7.86 (s, 1H), 8.02 (t, J = 7.2 Hz, 2H), 8.39 (br, 1H), 8.50 (s, 1H). LC-MS:m/z=486.2 [M+H]⁺ (95.94% purity, 220 nm)

### Example 108: Preparation of Compound SYY-C123

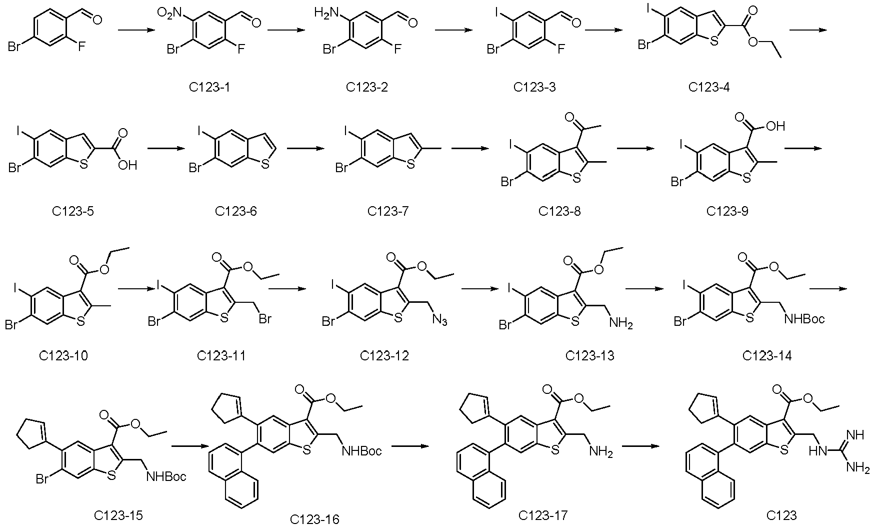

### Step 1:

Potassium nitrate (29.8g,295.8 mmol) was dispersed in concentrated sulfuric acid (200 mL), cooled under an ice water bath, 4-bromo-2-fluorobenzaldehyde (50.0g,246.3 mmol) was added in batches, and the mixture was stirred under an ice water bath for 30 minutes after the addition. TLC detection showed the reaction was completed. The reaction solution was slowly poured into the vigorously stirred ice water, a large amount of solid was precipitated, stirred for 30 minutes and then filtered, and the filter cake was washed with water and dried to obtain C123-1(61.0g, crude) as a yellow solid. HNMR(CDCl₃, 400Hz):10.30 (s, 1H), 8.40 (d, J=6.4 Hz, 1H),7.66 (d, J=9.2 Hz, 1H).

### Step 2:

C123-1(61.0g, crude) was dispersed in ethanol (600 mL) and water (300 mL), ammonium chloride (65.9g,1.23 mol) and iron powder (69.0g,1.24 mol) were added at room temperature, and the mixture was heated to 80°C and stirred for 30 minutes. TLC detection showed the reaction was completed. The reaction solution was filtered while hot, the filter cake was washed with ethanol, the filtrate was concentrated to remove ethanol, diluted with water, extracted with ethyl acetate, and the organic phases were combined, washed with saturated salt water, dried over anhydrous sodium sulfate, and concentrated to obtain C123-2(44.0g, crude) as a brown solid.

### Step 3:

C123-2(44.0g, crude) was dispersed in hydrochloric acid (6 M, 600 mL), cooled to about 0°C under an ice salt bath, a solution of sodium nitrite (15.3g,221.7mmol) in water (20 mL) was added dropwise, and the mixture was stirred at this temperature for 30 minutes after addition, a solution of potassium iodide (67.0g,403.6 mmol) in water (50 mL) was added dropwise, the mixture was stirred for 1 hour after the addition. TLC detection showed the reaction was completed. The reaction solution was quenched with saturated sodium sulfite aqueous solution, extracted with ethyl acetate, the organic phases were combined, washed with saturated sodium sulfite until clarified, washed with saturated salt water, dried over anhydrous sodium sulfate, and concentrated, and the crude was purified by silica gel column chromatography (pure PE) to obtain C123-3(20.0g, yield for three steps: 21%) as a yellow solid, HNMR(DMSO-d6, 400Hz):10.07 (s, 1H), 8.25 (d, J=7.2 Hz, 1H), 7.95 (d, J=10.4Hz, 1H).

### Step 4:

C123-3(20.0g,60.81 mmol), ethyl mercaptoacetate (7.3g,60.75 mmol) and potassium carbonate (25.2g,182.3 mmol) were dispersed in DMF(200 mL), and the mixture was heated to 120°C and stirred for 3 hours. TLC detection showed that reaction was completed. The reaction solution was diluted with water, extracted with ethyl acetate, the organic phases were combined, washed with water, washed with saturated salt water, dried over anhydrous sodium sulfate, and concentrated to obtain C123-4(25.0g, crude) as a brown solid.

### Step 5:

C123-4(25.0g, crude) was dispersed in ethanol (200 mL) and water (200 mL), sodium hydroxide solid (12.2g,305.1 mmol) was added at room temperature, and the mixture was heated to 120 °C and stirred for 3 hours after addition. The reaction solution was concentrated to remove the organic solvent, the pH was adjusted to acidity with 1M hydrochloric acid, and a large amount of solid was precipitated, stirred for 30 minutes, filtered, and the filter cake was washed with water, and washed with petroleum ether, and dried to obtain C123-5(24.0g, crude) as a yellow solid.

### Step 6:

C123-5(24.0g, crude) and silver carbonate (8.7g,31.55 mmol) were dispersed in DMSO (150 mL), acetic acid (24 drops) was added dropwise at room temperature, and then the mixture was heated to 140 °C and stirred for 4 hours after the addition. TLC detection showed the reaction was completed. The reaction solution was cooled to room temperature, excess silver carbonate was precipitated with 1M hydrochloric acid, filtered, the filter cake was washed with ethyl acetate, the filtrate was diluted with water, extracted with ethyl acetate, and the organic phases were combined, washed with saturated salt water, dried over anhydrous sodium sulfate, and concentrated, and the crude product was purified by silica gel column chromatography (pure PE) to obtain C123-6(12.0g, yield for three steps: 58%) as a yellow solid. LC-MS:m/z=338.4 [M+H]⁺, HNMR(DMSO-d6, 400Hz):8.32 (s, 1H), 8.15 (s, 1H), 7.43 (d, J=5.6 Hz, 1H), 7.21 (d, J=5.2 Hz, 1H).

### Step 7:

C123-6(11.5g,33.92 mmol) was dispersed in THF(200 mL), cooled to about -60°C under a dry ice-ethanol bath, LDA(22.1 mL,44.2 mmol) was added dropwise, and the mixture was stirred at this temperature for 1 hour after addition, methyl iodide (7.7g,54.2 mmol) was added dropwise, and the mixture was heated to room temperature naturally and stirred overnight. TLC detection showed the reaction was completed. The reaction solution was diluted with water, extracted with ethyl acetate, and the organic phases were combined, washed with saturated salt water, dried over anhydrous sodium sulfate, and concentrated to obtain C123-4(12.0 g, crude) as a dark yellow solid.

### Step 8:

C123-7(11.0g, crude) and acetyl chloride (4.9g,62.42 mmol) were dispersed in 1,2-dichloroethane (150 mL), a solution of tin tetrachloride (16.2g,62.18 mmol) in 1,2-dichloroethane (50 mL) was added dropwise at room temperature, and the mixture was stirred overnight at room temperature after the addition. TLC detection showed the reaction was completed. The reaction solution was diluted with water, extracted with dichloromethane, and the organic phases were combined, washed with saturated salt water, dried over anhydrous sodium sulfate, and concentrated to obtain dark brown solid C123-8(12.0g, crude).

### Step 9:

Sodium hydroxide (14.6g,365.1 mmol) was dissolved in water (150 mL), cooled under an ice bath, bromine (17.0g,106.4 mmol) was added dropwise, the mixture was stirred for 10 minutes, a solution of C123-8(12.0g, crude) in dioxane (150 mL) was added dropwise, and the mixture was stirred for 1 hour after addition. The reaction was completed. The reaction solution was quenched with saturated sodium sulfite aqueous solution, concentrated to remove organic solvent, 1M hydrochloric acid was added to adjust pH to acidity, solids were precipitated, filtered, and the filter cake was washed with water, and washed with PE, and dried to obtain C123-9(10.7g, crude) as a yellow solid.

### Step 10:

C123-9(10.7g, crude) was dispersed in DMF(100 mL), potassium carbonate (7.4g,54 mmol) and iodoethane (8.4g,54 mmol) were added in sequence, and the mixture was stirred at room temperature for 30 minutes after the addition. TLC detection showed the reaction was completed. The reaction solution was diluted with water, extracted with ethyl acetate, the organic phases were combined, washed with water, and washed with saturated salt water, dried over anhydrous sodium sulfate, and concentrated, and the crude product was purified by silica gel column chromatography (PE to PE/EA = 200/1) to obtain C123-10(10.0g, yield for four steps: 69%) as a light yellow solid. HNMR(CDCl₃, 400Hz):8.96 (s, 1H), 7.98 (s, 1H), 4. 34 (q, J=7.2 Hz, 2H), 2.83 (s, 3H), 1.46 (t, J=7.2 Hz, 3H).

### Step 11:

C123-10(9.2g,21.64 mmol) was dispersed in CCl₄(100 mL), BPO(512 mg,2.11 mmol) was added at room temperature, the mixture was heated to 75°C, NBS(4.1g,23.04 mmol) was added in batches, the mixture was stirred at the temperature for 1 hour after addition. LCMS showed half of the raw materials did not react completely. Additional NBS(770 mg,4.33 mmol) was added, continued to stir for one hour and the reaction was completed. The reaction solution was filtered while hot, the filter cake was washed with carbon tetrachloride, and the filtrate was concentrated to obtain C123-11(12.0g, crude) as a brown solid.

### Step 12:

C123-11(12.0g, crude) was dispersed in DMF(100 mL), sodium azide (3.1g,47.68 mmol) was added, and the mixture was stirred at room temperature for 1 hour. The reaction solution was diluted with saturated potassium carbonate aqueous solution, extracted with ethyl acetate, the organic phases were combined, washed with saturated salt water, dried over anhydrous sodium sulfate, and concentrated, and the crude product was mixed and purified by silica gel column chromatography (PE to PE/EA = 50/1) to obtain C123-12(8.0g, containing about 40% brominated raw materials) as a brown solid.

### Step 13:

C123-12(4.8g, crude) and triphenylphosphine (4.1g,15.63 mmol) were dispersed in THF(100 mL) and water (10 mL), and the mixture was heated to 80 °C and refluxed for 3 hours, and the reaction was completed. The reaction solution was cooled to room temperature, diluted with ethyl acetate, washed with 1M dilute hydrochloric acid, the aqueous phase was adjusted to pH=8 with saturated sodium carbonate, extracted with ethyl acetate, and the organic phases were combined, washed with saturated salt water, dried over anhydrous sodium sulfate, and concentrated to obtain C123-13(4.5g, crude) as a brown solid. LC-MS:m/z = 439.9 [M + H]⁺.

### Step 14:

C123-13(4.5g, crude) was dispersed in dichloromethane (80 mL), Boc anhydride (4.5g,20.62 mmol) and triethylamine (2.09g,20.62 mmol) were added, and the mixture was stirred at room temperature for 30 minutes, and the reaction was completed. The mixture was quenched with water, extracted with ethyl acetate, washed with saturated salt water, dried over anhydrous sodium sulfate, and concentrated, and the crude product was purified by silica gel column chromatography (PE to PE/EA = 10/1) to obtain C123-14(1.5g, yield for four steps: 13%) as a brown solid. LC-MS:m/z=483.9 [M-55]⁺

### Step 15:

C123-14(750 mg,1.39 mmol), cyclopentenylboronic acid (156 mg,1.39 mmol), sodium carbonate (295 mg,2.78 mmol) and Pd(dppf)Cl₂(102 mg,0.14 mmol) were dispersed in dioxane (20 mL) and water (5 mL), replaced with nitrogen for three times, and the mixture was heated to 80°C and stirred for 2 hours. TLC detection showed the reaction was completed. The reaction solution was cooled to room temperature, diluted with water, extracted with ethyl acetate, the organic phases were combined, washed with saturated salt water, dried over anhydrous sodium sulfate, and concentrated, and the crude product was purified by silica gel column chromatography (PE to PE/EA = 40/1) to obtain C123-15(540 mg, yield 81%) as a yellow solid.

### Step 16:

C123-15(540 mg,1.12 mmol), naphthylboronic acid (290 mg,1.69 mmol), sodium carbonate (239 mg,2.25 mmol) and Pd(dppf)Cl₂(80 mg,0.11 mmol) were dispersed in dioxane (20 mL) and water (15 mL), replaced with nitrogen for three times, and the mixture was heated to 80°C and stirred for 2 hours. LCMS detection showed the reaction was completed. The reaction solution was cooled to room temperature, diluted with water, extracted with ethyl acetate, the organic phases were combined, washed with saturated salt water, dried over anhydrous sodium sulfate, and concentrated, and the crude product was purified by silica gel column chromatography (PE to PE/EA = 40/1), the pure product was collected, and the mixture was purified by pre-TLC (PE/EA = 10/1; four times), then the pure products were combined to obtain C123-16(240 mg, yield 41%) as a yellow solid.

### Step 17:

C123-16(120 mg,0.23 mmol) was dispersed in DCM(2 mL), TFA(2 mL) was added, and the mixture was stirred at room temperature for 30 minutes. LCMS detection showed the reaction was completed. The reaction solution was concentrated to obtain C123-17(98 mg, crude) as a yellow oil. LC-MS:m/z=411.1 [M-NH₂]⁺.

### Step 18:

C123-17(98 mg, crude) was dispersed in acetonitrile (3 mL), DIPEA(89 mg,0.69 mmol) and 1H-pyrazol-1-formamidine hydrochloride (51 mg, 0.35 mmol) were added in sequence, and the mixture was stirred at room temperature for 2 days after addition. LCMS detection showed the reaction was completed. The reaction solution was filtered, and the filter cake was washed with acetonitrile and purified by pre-HPLC to obtain SYY-C123(28.2 mg, yield for two steps: 21%, TFA salt) as a white solid. LC-MS:m/z=470.2 [M+H]⁺ (99.83% purity, 254 nm)

¹H NMR: (DMSO-*d₆*, 400MHz): δ1.44 (t, J = 7.2 Hz, 3H), 2.22-2.24(m, 6H), 4.41 (q, J = 7.2 Hz, 2H), 5.02 (d, J = 6.4 Hz, 2H), 7.46-7.76 (m, 7H), 7.98 (d, J =8.4Hz, 1H), 8.05(d, J =7.6 Hz, 1H), 8.28(t, J = 6.4Hz, 1H), 8.54(s, 1H), 8.87(d, J=8.4Hz, 1H), 9.12(s, 1H).

### Example 109: Preparation of Compound SYY-C124

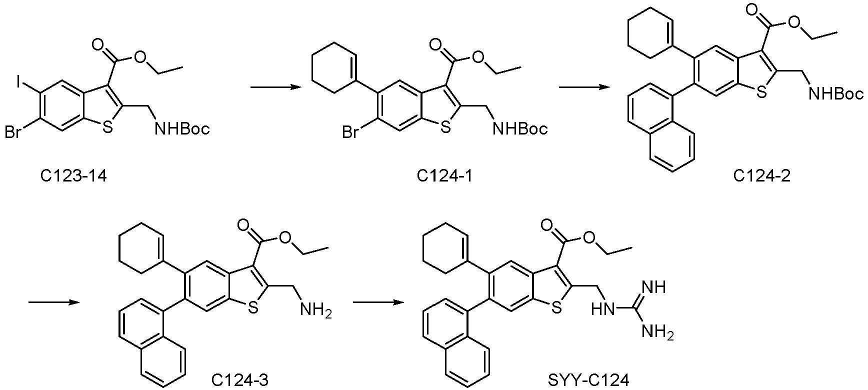

### Step 1:

C123-14(750 mg,1.39 mmol), cyclohexenylboronic acid (175 mg,1.39 mmol), sodium carbonate (295 mg,2.78 mmol) and Pd(dppf)Cl₂(102 mg,0.14mmol) were dispersed in dioxane (20 mL) and water (5 mL), replaced with nitrogen for three times, and the mixture was heated to 80°C and stirred for 2 hours. TLC detection showed the reaction was completed. The reaction solution was cooled to room temperature, diluted with water, extracted with ethyl acetate, the organic phases were combined, washed with saturated salt water, dried over anhydrous sodium sulfate, and concentrated, and the crude product was purified by silica gel column chromatography (PE to PE/EA = 40/1) to obtain C124-1(650 mg, yield 95%) as a yellow solid. LC-MS: no peak.

### Step 2:

C124-1(650 mg,1.31 mmol), naphthylboronic acid (339 mg,1.97 mmol), sodium carbonate (279 mg,2.63 mmol) and Pd(dppf)Cl₂(95 mg,0.13 mmol) were dispersed in 1.4-dioxane (20 mL) and water (5 mL), replaced with nitrogen for more than three times, and the mixture was heated to 80°C and stirred for 2 hours. LCMS detection showed the reaction was completed. The reaction solution was cooled to room temperature, diluted with water, extracted with ethyl acetate, the organic phases were combined, washed with saturated salt water, dried over anhydrous sodium sulfate, and concentrated, and the crude product was purified by silica gel column chromatography (PE to PE/EA = 40/1) to obtain C124-2(330 mg, yield 46%) as a yellow solid. LC-MS: no peak.

### Step 3:

C124-2(150 mg,0.28 mmol) was dissolved in DCM(2 mL), TFA(2 mL) was added, and the mixture was stirred at room temperature for 30 minutes. LCMS detection showed the reaction was completed. The reaction solution was concentrated and dried to obtain C124-3(150 mg, crude) as a yellow oil. LC-MS:m/z = 442.2 [M + H] ⁺.

### Step 4:

C124-3(150 mg, crude) was dissolved in acetonitrile (3 mL), DIPEA(132 mg,1.02 mmol) and 1H-pyrazol-1-formamidine hydrochloride (75 mg, 0.51 mmol) were added, and the mixture was stirred at room temperature for 2 weeks. LCMS detection showed the reaction was completed. The reaction solution was filtered, and the filter cake was washed with acetonitrile and purified by pre-HPLC to obtain SYY-C124(120 mg, TFA salt, yield for two steps: 74%) as a white solid. ¹H NMR (400 MHz, DMSO_d6) : δ 1.46 (t, J = 7.2Hz, 3H),1.85-2.05(m, 8H), 4.43(q, J = 7.2Hz, 2H), 5.05(s, 2H), 7.56-8.05(m, 9H), 8.46(br, 1H), 8.91-8.94(m, 2H), 9.18(s, 1H). LC-MS:m/z= 484.2 [M+H] ⁺

### Example 110: Preparation of Compound SYY-C125

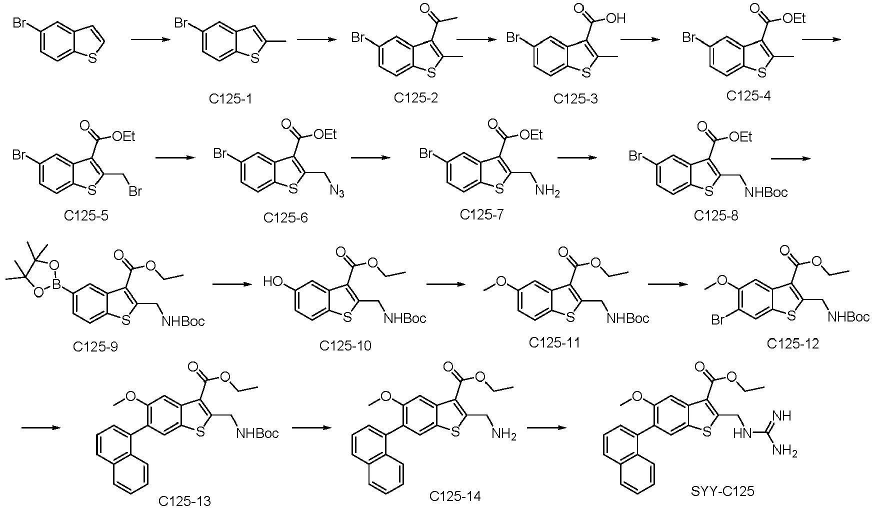

### Step 1:

5-Bromobenzothiophene (120.0g,0.56 mol) was dissolved in anhydrous THF(1L), cooled to -60 °C in the dry ice/ethanol system, LDA(425 ml,0.85 mol) was added dropwise through a constant pressure dropping funnel in 1 hour under the protection of nitrogen, the mixture was stirred at this temperature for 1 hour, methyl iodide (128.0g,0.90 mol) was slowly added dropwise, and the mixture was naturally heated to room temperature after addition, and stirred for a weekend. TLC monitored the reaction was completed. The reaction solution was added with water, extracted with ethyl acetate, and the organic phases were combined, washed with saturated salt water, dried over anhydrous sodium sulfate, and concentrated to obtain C125-1(126.0g, crude) as a light yellow solid.

### Step 2:

Aluminum trichloride (149.0g, 1.12 mol) was dissolved in dichloromethane (1L), the system was cooled to -10 °C, stirred for 15 minutes, acetyl chloride (48.0g,0.61 mol) was added dropwise through a constant pressure dropping funnel, the mixture was stirred at -10 °C for 20 minutes after the addition, a solution of C125-1(126.0g, crude) in dichloromethane (500ml) was slowly added, and the mixture was naturally heated to room temperature after addition, and stirred overnight. TLC monitored the reaction was completed. The reaction solution was slowly poured into ice water, extracted with dichloromethane, the organic phase were combined, washed with saturated salt water, dried over anhydrous sodium sulfate, and concentrated, the crude product was slurried with PE, filtered, and the filter cake was washed and dried to obtain C125-2(128.0g, crude) as a brown solid. HNMR(CDCl₃, 400Hz):δ 8.41 (d, J=2.0 Hz, 1H), 7.57 (d, J=8.8 Hz, 1H), 7.42 (dd, J₁=2.0, J₂=8.4 Hz, 1H), 2.79 (s, 3H), 2.63 (s, 3H).

### Step 3:

Sodium hydroxide solid (173.0g,4.33 mol) was dissolved in water (1L), cooled to 0 °C, bromine (269.0g,1.68 mol) was slowly added dropwise, kept at 0 °C to 5 °C, the mixture was stirred for 30 minutes after addition, a solution of C125-2(128.0g, crude) in 1,4-dioxane (120 ml) was added dropwise, and the mixture was reacted overnight at room temperature after addition. TLC showed the reaction was completed. The reaction solution was firstly quenched with sodium sulfite, then 6N hydrochloric acid was slowly added to adjust the PH to 3-4 under the condition of cooling under an ice water bath, extracted with ethyl acetate, the organic phases were combined, washed with saturated salt water, dried over anhydrous sodium sulfate, and concentrated, the crude product was slurried with a mixed solvent (200ml) of PE:EA = 10:1, filtered, and the filter cake was washed and dried to obtain C125-3(120.0g, crude) as a light brown solid.

### Step 4:

C125-3(120.0g, crude) was dissolved in DMF(700 ml), potassium carbonate (91.0g,0.66 mol) was added, the mixture was stirred for 15 minutes, iodoethane (83.0g,0.53 mol) was slowly added through a constant pressure dropping funnel, kept at 30-35 °C, and the mixture was heated to 40 °C to react for 2 hours after the addition. TLC showed the reaction was completed. The reaction solution was cooled to room temperature, slowly poured into water, filtered, and the filter cake was washed and dried to obtain C125-4(128.0g, crude) as a beige solid. HNMR(CDCl₃, 400Hz):δ 8.59 (d, J=2.0 Hz, 1H), 7.57 (d, J=8.4 Hz, 1H), 7.42 (dd, J1=2.0, J2=8.4 Hz, 1H), 4.44 (q, J=7.2 Hz, 2H), 2.84 (s, 3H), 1.46 (t, J=7.2 Hz, 3H).

### Step 5:

C125-4(20.0 g,66.85 mmol) was dissolved in carbon tetrachloride (150ml), BPO(1.6 g,6.60 mmol) and NBS(12.5 g,70.23 mmol) were added at room temperature, the mixture was heated to 80°C to react for 2h. TLC monitored the reaction was completed. The reaction solution was cooled to room temperature, filtered, the filtrate was concentrated to obtain 20.0g of crude product as a dark yellow solid which was slurried with mixed solvent (PE:EA = 10:1,100 ml), filtered, and the filter cake was washed and dried to obtain C125-5(16.0g, crude product) as a yellow solid.

### Step 6:

C125-5(16.0g crude) was dissolved in DMF(100ml), sodium azide (2.7g,41.53 mmol) was added, and the mixture was reacted at room temperature for 2 hours. TLC monitored the reaction was completed. The reaction solution was quenched with ice water, extracted with ethyl acetate, the organic phases were combined, washed with water, and washed with saturated salt water, dried over anhydrous sodium sulfate, and concentrated to obtain crude product which was slurred with pure PE, filtered, and the filter cake was washed and dried to obtain C125-6(14.0g, crude) as a dark yellow solid.

### Step 7:

C125-6(14.0g, crude) was dissolved in a mixed solvent of THF/H₂O(140 ml/70 ml), triphenylphosphorus (12.9g,49.18 mmol) was added at room temperature, and the mixture was heated to 80 °C to react for 1 hour. TLC showed the raw materials were reacted completely with a lot of impurity spots. The reaction solution was cooled to room temperature, added with water, adjusted PH to 2-3 with 1M hydrochloric acid solution, extracted with ethyl acetate, the aqueous phase was adjusted to 10-11 with saturated sodium carbonate aqueous solution, extracted with ethyl acetate, the organic phases were combined, washed with saturated salt water, dried over anhydrous sodium sulfate, and concentrated to obtain C125-7(8.0g, crude) as a yellow-brown solid-liquid mixture. LC-MS: m/z= 314.0 [M+H]⁺

### Step 8:

C125-7(8.0g, crude) was dissolved in dichloromethane (100 ml), triethylamine (5.2g,51.39 mmol) and Boc anhydride (6.7g,30.70 mmol) were added, and the mixture was reacted at room temperature for 2 hours. TLC showed the reaction was completed. The reaction solution was added with water, extracted with EA, and the organic phases were combined, washed with saturated salt water, dried over anhydrous sodium sulfate, and concentrated, and the crude product was purified by silica gel column chromatography to obtain C125-8(6.3 g, yield for four steps: 23%) as a light yellow solid. HNMR(DMSO-d6, 400Hz):δ 8.47 (d, J=1.6 Hz, 1H), 7.99 (d, J=8.8 Hz, 1H), 7.87 (t, J=6.4 Hz, 1H),7.52 (dd, J1=2.0, J2=8.4 Hz, 1H), 4.70 (d, J=2.0 Hz, 2H), 4.36 (q, J=7.2 Hz, 2H), 1.41 (s, 9H), 1.36 (t, J=7.2 Hz, 3H). LC-MS:m/z= 358.0 [M-55]⁺

### Step 9:

C125-8(5.4g,13.03 mmol), bis(pinacolato)diboron(4.0g,15.75 mmol), AcOK(1.9g,19.36 mmol) and PdCl₂(dppf)(190 mg,0.23 mmol) were dissolved in 1,4-dioxane(60 mL), and the mixture was heated to 90 °C to react for 3 hours under the protection of nitrogen. TLC showed the reaction was completed. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C125-9(6.8 g, crude) as a colorless oil. HNMR(DMSO-d6, 400Hz):δ 8.83 (s, 1H), 7.73-7.78 (m, 2H), 5.46 (t, J=6.0 Hz, 1H),4.82 (d, J=6.4 Hz, 2H), 4.46 (q, J=7.2 Hz, 2H), 1.48 (t, J=7.2 Hz, 3H), 1.46 (s, 9H), 1.36 (s, 12H). LC-MS:m/z= 484.1 [M+Na]⁺

### Step 10:

C125-9(6.8g, crude) was dissolved in acetonitrile (50 mL), 30% hydrogen peroxide (16 mL) was added, and the mixture was stirred overnight at room temperature. The reaction solution was concentrated to half the volume, filtered, and the filter cake was washed and dried to obtain C125-10(4.0g, crude) as a white solid. HNMR(DMSO-d6, 400Hz):δ 9.57 (s, 1H), 7.73-7.80 (m, 3H), 6.87(dd, J1=2.4, J2=8.4Hz, 1H), 4.67 (d, J=6.4 Hz, 2H), 4.35 (q, J=7.2 Hz, 2H), 1.42 (s, 9H), 1.38 (t, J=7.2 Hz, 3H). LC-MS:m/z= 374.1 [M+Na]⁺

### Step 11:

C125-10 (1.0g, crude) and potassium carbonate (591 mg,4.28 mmol) were dissolved in DMF (8 mL), methyl iodide (485 mg, 3.42 mmol) was added dropwise at room temperature, and the mixture was heated to 30 °C to react overnight after the addition. TLC showed the raw materials did not react completely. The reaction solution was added with water and ethyl acetate, the organic phases were combined, washed with saturated salt water, dried over anhydrous sodium sulfate, and concentrated, and the crude product was purified by silica gel column chromatography to obtain C125-11(380 mg, yield for three steps : 32%) as a light yellow solid. HNMR(CDCl₃, 400Hz):δ 7.96 (d, J=2.8 Hz, 1H), 7.63 (d, J=8.8 Hz, 1H), 7.00 (dd, J1=2.4, J2=8.8Hz, 1H), 5.43 (t, J=6.4 Hz, 1H), 4.82(d, J=6.4Hz, 2H), 4.44 (q, J=7.2 Hz, 2H), 3.89 (s, 3H), 1.48 (t, J=7.2 Hz, 3H) , 1.46 (s, 9H). LC-MS:m/z=310.1 [M-55]⁺

### Step 12:

C125-11(720 mg,1.97 mmol) was dissolved in acetonitrile (6mL), heated to 60 °C, NBS(351 mg,1.97 mmol) was added, and the mixture was reacted at 60 °C for 1 hour after the addition. TLC showed the raw materials disappeared. The reaction solution was concentrated to dryness, purified by silica gel column chromatography, and the crude product was purified by TLC to obtain C125-12(130 mg, yield 15%) and by-product C125-12-1(730 mg). HNMR(DMSO-d6, 400Hz): 8.30 (s, 1H), 7.95 (s, 1H), 7.85 (t, J=5.6 Hz, 1H), 4.68 (d, J=6.0 Hz, 2H), 4.35 (q, J=7.2 Hz, 2H), 3.88 (s, 3H), 1.36-1.41 (m, 12H). LC-MS: m/z=389.0/390.0 [M-55]⁺

### Step 13:

C125-12(130 mg,0.29 mmol), 1-naphthaleneboronic acid (76 mg, 0.44 mmol), Na₂CO₃(62 mg,0.58 mmol) and PdCl₂(dppf)(24 mg,0.03 mmol) were dispersed in a mixed solvent of 1,4-dioxane(6 mL) and H₂O(2 mL), the mixture was heated to 100 °C to react for 5 hours under the protection of nitrogen. LCMS showed the raw materials were reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C125-13(130 mg, yield 90%) as a white solid. HNMR(DMSO-d6, 400Hz):δ 8.12 (s, 1H), 7.88-7.91 (m, 2H), 7.65 (s, 1H), 7.35-7.56 (m, 5H), 5.45 (t, J=6.4 Hz, 1H), 4.88 (d, J=6.4 Hz, 2H), 4.49 (q, J=7.2 Hz, 2H), 3.77 (s, 3H), 1.52 (t, J=7.2 Hz, 3H) , 1.47 (s, 9H). LC-MS:m/z=392.0 [M-Boc+H]⁺

### Step 14:

C125-13(130 mg,0.26 mmol) was dissolved in DCM (3 mL), TFA(1.5 mL) was added dropwise at room temperature, and the mixture was heated to 25 °C to react for half an hour. TLC showed the reaction was completed. The reaction solution was concentrated to obtain C125-14(crude) which was directly used in the next step. LC-MS:m/z = 392.2 [M + H]+.

### Step 15:

C125-14 (crude product from the previous step), 1H-pyrazol-1-formamidine hydrochloride (58 mg, 0.40 mmol) and DIPEA(136 mg, 1.05 mmol) were dissolved in acetonitrile (2 mL) and stirred at room temperature overnight. LCMS showed most of the raw materials did not react, and the mixture was heated to 30 °C to continue to react for 48 hours. TLC and LCMS showed the raw materials were reacted completely. The reaction solution was cooled to room temperature, filtered, and the filter cake was washed with a small amount of acetonitrile, and dried to obtain SYY-C125(70 mg, yield for two steps: 61%) as a white solid. ¹H NMR: (DMSO-*d₆*, 400MHz): δ1.44 (t, J = 7.2 Hz, 3H), 3.69 (s, 3H), 4.42 (q, J = 7.2 Hz, 2H), 5.04 (d, J = 6.0 Hz, 2H), 7.38-7.71 (m, 8H), 7.92-7.98 (m, 3H), 8.08 (m, 1H), 8.42 (t, J = 6.4 Hz, 1H). LC-MS:m/z=434.1 [M+H]⁺ (99.10% purity, 254 nm)

### Example 111: Preparation of Compound SYY-C126

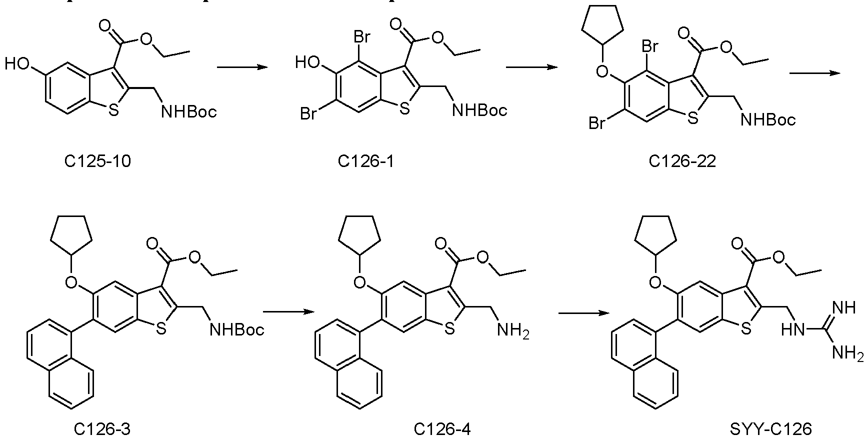

### Step 1:

C125-10(3.0g,8.54 mmol) was dissolved in acetonitrile (50 mL), heated to 40 °C, NBS(3.0g,16.86 mmol) was added, and the mixture was continued to be stirred for 2 hours. TLC showed the reaction was completed. The reaction solution was concentrated and the crude product was purified by silica gel column chromatography to obtain C126-1(2.5g, yield 57%) as a red solid. LC-MS:m/z = 453.8 [M-55]⁺

### Step 2:

C126-1(600 mg,1.18 mmol) was dissolved in anhydrous THF(5mL), cyclopentanol (155 mg, 1.80 mmol) and PPh₃(472 mg, 1.80 mmol) were added under the protection of nitrogen, cooled to 0 °C under an ice bath, DIAD(485 mg, 2.40 mmol) was added dropwise, the mixture was naturally heated to room temperature after addition, and stirred for 2 hours. TLC showed the raw materials disappeared. The reaction solution was quenched with water, extracted with ethyl acetate, the organic phases were combined, washed with saturated salt water, dried over anhydrous sodium sulfate, and concentrated, and the crude product was purified by silica gel column chromatography to obtain C126-2(600 mg, yield 88%) as a colorless transparent viscous liquid. HNMR(CDCl₃, 400Hz):δ 7.93 (s, 1H), 5.08-5.13 (m, 1H), 4.95-5.00 (m, 1H), 4.50 (d, J=6.0 Hz, 2H), 4.44 (d, J=7.2 Hz, 2H), 1.95-2.11 (m, 4H), 1.55-1.87 (m, 4H), 1.46 (s, 9H), 1.42 (t, J=7.2 Hz, 3H). LC-MS: m/z=477.0 [M-Boc+H]⁺

### Step 3:

C126-2(500 mg,0.87 mmol) was dissolved in 1,4-dioxane(15 mL) and H₂O(3 mL), 1-naphthylboronic acid (224 mg, 1.30mmol), Na₂CO₃(180 mg,1.70 mmol) and PdCl₂(dppf)(82 mg,0.10 mmol) were added under the protection of nitrogen, and the mixture was heated to 90 °C to react for 3 hours. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C126-3(400 mg, crude) as a white solid. LC-MS:m/z=446.0 [M-Boc+H]⁺

### Step 4:

C126-3(400 mg, crude) was dissolved in DCM(10 mL), TFA(2 mL) was slowly added dropwise, and the mixture was reacted at room temperature for half an hour. TLC showed the raw materials were reacted completely. The reaction solution was concentrated, saturated sodium carbonate aqueous solution was added to adjust PH to be weakly alkaline, extracted with DCM, and the organic phases were combined, washed with saturated salt water, dried over anhydrous sodium sulfate, and concentrated, and the crude product was purified by TLC to obtain C126-4(120 mg, yield for two steps: 31%) as a light yellow viscous liquid. LC-MS:m/z=446.2 [M+H]⁺

### Step 5:

C126-4(120mg, 0.27mmol) was dissolved in acetonitrile (2mL) and methanol (3 drops), 1H-pyrazol-1-formamidine hydrochloride(51.3mg, 0.35mmol) and DIPEA(142mg, 1.10mmol) were added at room temperature, and the mixture was heated to 40 °C and stirred overnight. The reaction solution was filtered, and the filter cake was washed with a small amount of acetonitrile, and dried to obtain SYY-C126(60mg, yield 46%) as a white solid. ¹H NMR: (DMSO-*d₆*, 400MHz): δ1.08-1.78 (m, 9H), 1.63-1.79 (m, 2H), 4.41 (q, J = 7.2 Hz, 2H), 4.74-4.80 (m, 1H), 5.02 (s, 2H), 7.35-7.74 (m, 8H), 7.92-7.96 (m, 3H), 8.05 (s, 1H), 8.49-8.25 (m, 1H). LC-MS: m/z=488.2 [M+H]⁺ (98.98% purity, 220 nm)

### Example 112: Preparation of Compound SYY-C127

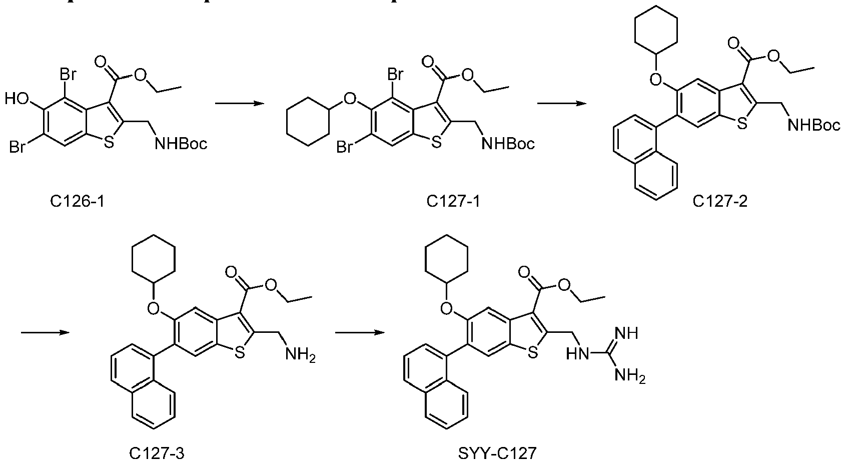

### Step 1:

Under the protection of nitrogen, C126-1(700 mg,1.37 mmol), cyclohexanol (207 mg, 2.07 mmol) and PPh₃(540 mg, 2.06 mmol) were dissolved in anhydrous THF(4 mL), cooled to 0 °C under an ice bath, DIAD(554 mg, 2.74 mmol) was added dropwise, and the mixture was stirred overnight at room temperature after the addition. TLC showed the raw materials disappeared. The reaction solution was quenched with water, extracted with ethyl acetate, the organic phases were combined, washed with saturated salt water, dried over anhydrous sodium sulfate, and concentrated, and the crude product was purified by silica gel column chromatography to obtain C127-1(730 mg, yield 90%) as a withe solid. LC-MS:m/z=535.9 [M-55]⁺

### Step 2:

C127-1(580 mg,0.98 mmol), 1-naphthaleneboronic acid (253 mg, 1.47 mmol), Na₂CO₃(208 mg,1.96 mmol) and PdCl₂(dppf)(80 mg,0.10 mmol) were dispersed in a mixed solvent of 1,4-dioxane(12 mL) and H₂O(4 mL), and the mixture was heated to 90 °C to react for 3 hours under the protection of nitrogen. The reaction solution was cooled to room temperature, added with water, extracted with ethyl acetate, the organic layers were combined, washed with saturated salt water, dried over anhydrous sodium sulfate, and concentrated, and the crude product was purified by silica gel column chromatography to obtain (370 mg, mixture), and purified by TLC to obtain C127-2(160 mg, yield 29%) as a white solid. ¹H NMR(CDCl₃, 400Hz):δ 8.11 (s, 1H), 7.87 (t, J=7.6 Hz, 2H), 7.76 (s, 1H), 7.61 (d, J=8.0 Hz, 1H), 7.50-7.54 (m, 1H), 7.41-7.47 (m, 2H), 7.34-7.37 (m, 1H), 5.44-5.52 (m, 1H), 4.86 (d, J=5.6 Hz, 2H), 4.48 (t, J=7.2 Hz, 2H), 4.18-4.24 (m, 1H), 1.67-1.75 (m, 2H), 1.53-1.60 (m, 2H), 1.51 (t, J=7.2 Hz, 3H), 1.47 (s, 9H), 1.36-1.43 (m, 2H) , 1.26-1.29 (m, 2H) , 1.11-1.18 (m, 2H). LC-MS:m/z=460.0 [M-Boc+H]⁺

### Step 3:

C127-2(160 mg, 0.28 mmol) was dissolved in DCM (4 mL), TFA(1 mL) was added dropwise, and the mixture was reacted at room temperature for half an hour after addition. TLC showed the raw materials were reacted completely. The reaction solution was concentrated, and the crude product was purified by silica gel column chromatography to obtain C127-3 (120 mg, yield for two steps: 77%) as a white solid. HNMR(CDCl₃, 400Hz):δ 8.06 (s, 1H), 7.86 (t, J=7.2 Hz, 2H), 7.69 (s, 1H), 7.57 (d, J=9.2 Hz, 1H), 7.45 (t, J=9.2 Hz, 1H), 7.44 (t, J=7.2 Hz, 1H), 7.39 (d, J=7.2 Hz, 1H) 7.34 (t, J=7.2 Hz, 1H), 4.48-4.53 (m, 4H), 4.12-4.253 (m, 1H), 1.67-1.75 (m, 2H), 1.51 (t, J=7.2 Hz, 3H), 1.06-1.42 (m, 8H). LC-MS: m/z=460.2 [M+H]⁺

### Step 4:

C127-3(120 mg, 0.22 mmol), 1H-pyrazol-1-formamidine hydrochloride (56 mg, 0.38 mmol) and DIPEA(153 mg, 1.18 mmol) were dissolved in acetonitrile (4 mL) and methanol (0.5 mL) and the mixture was stirred overnight at room temperature. LCMS showed there were still many raw materials, and the mixture was heated to 45 °C and continued to react for 2 days. LCMS showed there were still some raw materials that had not been reacted. The reaction solution was filtered, and the filter cake was washed with acetonitrile, and dried to obtain SYY-C127(60 mg, yield 56%) as a light yellow solid. ¹H NMR: (DMSO-*d₆*, 400MHz): δ1.00-1.56(m, 5H), 1.30-1.32(m, 3H), 1.43(t, J = 7.2Hz, 3H), 1.64-1.71(m, 2H), 4.23-4.27(m, 1H), 4.40(q, J = 7.2Hz, 2H), 5.02(s, 2H), 7.38-7.56(m, 8H), 7.93-7.96(m, 3H), 8.06(s, 1H), 8.32(br, 1H). LC-MS:m/z=502.2 [M+H]⁺

### Example 113: Preparation of Compound SYY-C128

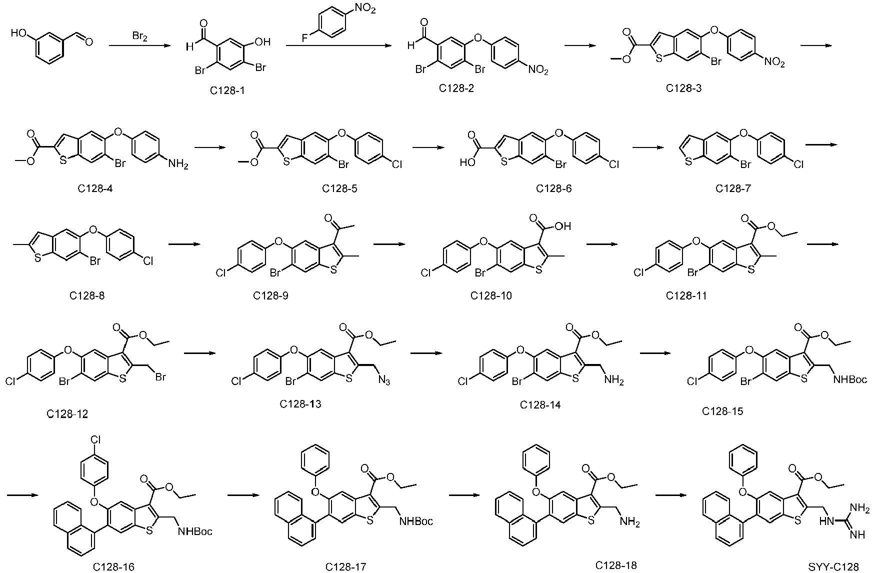

### Step 1:

m-Hydroxybenzaldehyde(20.0g,163.8 mmol) was dissolved in chloroform (400 mL), and stirred for 5 minutes at room temperature, bromine (52.3g,327.3 mmol) was added dropwise, and the mixture was stirred at room temperature overnight after addition. TLC showed the raw materials disappeared. A large amount of solids were precipitated form the reaction solution, filtered, and the filter cake was washed and dried to obtain C128-1(15.0g, crude) as a gray solid. LC-MS:m/z= 278.8[M-H]⁻

### Step 2:

C128-1(500 mg, crude), p-fluoronitrobenzene (378 mg,2.68 mmol) and potassium carbonate (495 mg,3.58 mmol) were dispersed into DMF(10 mL), and the mixture was heated to 80°C to react for 2 hours. TLC showed the raw materials disappeared. The reaction solution was cooled to room temperature, quenched with water, extracted with ethyl acetate, the organic phases were combined, washed with saturated salt water, dried over anhydrous sodium sulfate, and concentrated, and the crude product was separated and purified by silica gel column chromatography (PE/EA = 20/1) to obtain C128-2(530 mg, yield for two steps: 24%) as a white solid. ¹H NMR(400MHz, CDCl₃): δ 10.26 (s, 1H), 8.25 (d, J=8.8 Hz, 2H), 8.03 (s, 1H), 7.61 (s, 1H), 7.01 (d, J=9.2 Hz, 2H).

### Step 3:

C128-2(200 mg,0.499 mmol), ethyl mercaptoacetate (53 mg,0.441 mmol), DIPEA(129.3 mg,1.00 mmol), Pd₂(dba)₃(23 mg,0.025 mmol), and Xantphos(29 mg,0.05 mmol) were dispersed into 1, 4-dioxane (10 mL), the mixture was heated to 95°C to react overnight under the protection of nitrogen. TLC showed there was a small amount of raw material left. The reaction solution was cooled to room temperature, quenched with water, extracted with ethyl acetate, the organic phases were combined, washed with saturated salt water, dried over anhydrous sodium sulfate, and concentrated, and the crude product was separated and purified by silica gel column chromatography (PE/EA = 30/1) to obtain C128-3(70 mg, yield: 34%) as a white solid. ¹H NMR(400MHz, CDCl₃):δ 8.23 (d, *J* =9.2 Hz, 2H), 8.20 (s, 1H), 7.98 (s, 1H), 7.63 (s, 1H), 6.99 (d, J=9.2 Hz, 2H), 3.96 (s, 3H).

### Step 4:

C128-3(2.0g,4.90 mmol), iron powder (1.65g,29.6 mmol) and ammonium chloride (1.57g,29.4 mmol) were dispersed into ethanol (20 mL) and water (10 mL), and the mixture was heated to 80°C to react overnight. TLC showed the raw materials disappeared. The reaction solution was filtered while hot, the filtrate was added with water, extracted with ethyl acetate, and the organic phases were combined, washed with saturated salt water, dried over anhydrous sodium sulfate, and concentrated to obtain C128-4(1.5g, crude) which was directly used in the next step.

### Step 5:

Cuprous chloride (392 mg,3.96 mmol) was dispersed into acetonitrile (30 mL), cooled under an ice bath, isoamyl nitrite (619 mg,5.28 mmol) was added under the protection of nitrogen, stirred under an ice bath for half an hour, C128-4(1.0g, crude) was added, and the mixture was heated to room temperature and stirred overnight. TLC showed raw materials disappeared. The reaction solution was quenched with water, extracted with ethyl acetate, and the organic phases were combined, washed with saturated salt water, dried over anhydrous sodium sulfate, and concentrated, and the crude product was separated and purified by silica gel column chromatography to obtain C128-5(830 mg, yield for two steps : 64%).

### Step 6:

C128-5(830 mg,2.09 mmol) was dissolved in a mixed solvent of tetrahydrofuran/methanol/water (9 mL/3 mL/3 mL), sodium hydroxide (167 mg,4.17 mmol) was added, and the mixture was stirred at room temperature for half an hour. TLC showed the raw materials disappeared. The reaction solution was concentrated, added with water, extracted with ethyl acetate, the organic phases were combined, washed with saturated salt water, dried over anhydrous sodium sulfate, and concentrated to obtain C128-6(810 mg, crude) as a white solid which was directly used in the next step. LC-MS: M/z = 380.9 [M-H]⁻.

### Step 7:

C128-6(6.3g, crude) was dissolved in DMSO(50 mL), silver carbonate (2.3g,8.3 mmol) and acetic acid (10 drops) were added, the mixture was heated to 100°C, a large amount of solids were precipitated, continued to heat, the solids slowly disappeared, and the mixture was heated to 140°C to react for 4 hours. The reaction solution was cooled to room temperature, added with water, extracted with ethyl acetate, the organic phases were combined, washed with saturated salt water, dried over anhydrous sodium sulfate, and concentrated, and the crude product was separated and purified by silica gel column chromatography(100% petroleum ether) to obtain C128-7(5.0g, yield for two steps: 91%) as a white solid.

### Step 8:

C128-7(160 mg,0.47 mmol) was dissolved in anhydrous tetrahydrofuran (4 mL), cooled to -65 °C under a dry ice bath, LDA(0.35 mL,2.65mmol,2M) was added dropwise under the protection of nitrogen, the mixture was continued to be stirred for half an hour after addition, methyl iodide (100 mg,0.7 mmol) was added, and the mixture was stirred overnight at room temperature. The reaction solution was quenched with water, extracted with ethyl acetate, and the organic phases were combined, washed with saturated salt water, dried over anhydrous sodium sulfate, and concentrated, and the crude product was separated and purified by silica gel column chromatography to obtain C128-8(160 mg, yield 96%).

### Step 9:

Aluminum trichloride (120 mg,0.90 mmol) was dispersed into dichloromethane (2 mL), acetyl chloride (39 mg,0.50 mmol) was added dropwise under an ice bath, the mixture was stirred for half an hour after addition, a solution of C128-8(160 mg,0.45 mmol) in dichloromethane (2 mL) was added dropwise, the mixture was heated to room temperature, and stirred for half an hour. TLC showed the raw materials disappeared. The reaction solution was quenched with saturated ammonium chloride solution, extracted with ethyl acetate, the organic phases were combined, washed with saturated salt water, dried over anhydrous sodium sulfate, and concentrated, and the crude product was separated and purified by silica gel column chromatography to obtain C128-9(160 mg, yield 92%). ¹H NMR (400MHz, CDCl₃): δ 7.99 (s, 2H), 7.27 (d, *J* =9.2 Hz, 2H), 6.88 (d, *J* =9.2 Hz, 2H), 2.80 (s, 3H), 2.57 (s, 3H).

### Step 10:

Sodium hydroxide (1.7g,42.5 mmol) was dissolved in water (20 mL), cooled under an ice bath, bromine (2.7g, 16.9 mmol) was added dropwise, the mixture was stirred for 20 minutes under an ice bath after addition, a solution of C128-9(1.9g,4.80 mmol) in 1,4-dioxane (15 mL) was added dropwise, and the mixture was stirred at room temperature for half an hour. TLC showed the raw materials disappeared. The reaction solution was added with 1N hydrochloric acid to adjust the pH to 1-2, filtered, and the filter cake was washed and dried to obtain C128-10(1.9g, crude) as a white solid, which was directly used in the next step.

### Step 11:

C128-10(1.9g, crude) and potassium carbonate (991 mg,7.17 mmol) were dispersed into DMF(20 mL), iodoethane (894 mg,5.73 mmol) was added dropwise at room temperature, and the mixture was stirred overnight at room temperature after addition. TLC showed the raw materials disappeared. The reaction solution was quenched with water, extracted with ethyl acetate, the organic phases were combined, washed with saturated salt water, dried over anhydrous sodium sulfate, and concentrated, and the crude product was separated and purified by silica gel column chromatography to obtain C128-11(1.5g, crude) as a white solid, which was directly used in the next step.

### Step 12:

C128-11(1.4g, crude) was dissolved in carbon tetrachloride (20 mL), BPO(73 mg,0.21 mmol,70%) and NBS(0.6g,3.4 mmol) were added, and the mixture was heated to 80°C to react for 2 hours. TLC monitored the reaction was completed. The reaction solution was cooled to room temperature, filtered, the filtrate was concentrated, the crude product was added with 20ml of mixed solvent (petroleum ether: ethyl acetate = 20:1) for slurrying, and filtered to obtain C128-12(1.0g, crude product) as a yellow solid, which was directly used in the next step. ¹H NMR (400MHz, CDCl₃) δ 8.04 (s, 1H), 8.03 (m, 1H), 7.32 (d, *J* =8.8 Hz, 2H), 6.94 (d, *J* =8.8 Hz, 2H), 5.12 (s, 2H), 4.37 (q, J =7.2 Hz, 2H), 1.31 (t, *J* =7.2 Hz, 2H).

### Step 13:

C128-12(500 mg, crude) was dissolved in DMF(10 mL), the system was cooled to 0°C, sodium azide (72 mg,1.1 mmol) was added, and the mixture was reacted at 0 °C for 10 minutes. The reaction solution was quenched with water, extracted with ethyl acetate, the organic phases were combined, dried over anhydrous sodium sulfate, and concentrated to obtain C128-13(400 mg, crude) as a yellow solid which was directly used in the next step.

### Step 14:

C128-13(800 mg, crude) was dissolved in tetrahydrofuran/water (5 mL/2.5 mL), triphenylphosphorus (647 mg,2.13 mmol) was added at room temperature, and the mixture was heated to 80°C to react overnight. The reaction solution was cooled to room temperature, added with water, extracted with ethyl acetate, the organic phases were combined, washed with saturated salt water, dried over anhydrous sodium sulfate, and concentrated to obtain C128-14(1.1g, crude) as a yellow-brown solid-liquid mixture which was directly used in the next step.

### Step 15:

C128-14(1.1 g, crude) was dissolved in dichloromethane (15mL), triethylamine (0.5 g,5.0 mmol) and Boc anhydride (1.1 g,5.0 mmol) were added, and the mixture was reacted at room temperature for 2 hours. TLC showed the reaction was completed. The reaction solution was quenched with water, extracted with ethyl acetate, the organic phases were combined, washed with saturated salt water, dried over anhydrous sodium sulfate, and concentrated, and the crude product was separated and purified by silica gel column chromatography (PE:EA = 10:1) to obtain C128-15(500 mg, yield for six steps: 23%) as a light yellow solid.

### Step 16:

C128-15(400 mg,0.74 mmol) was dissolved in a mixed solvent of 1, 4-dioxane (15 mL) and water(3 mL), 1-naphthylboronic acid (191 mg, 1.11 mmol), sodium carbonate (157 mg, 1.48 mmol) and PdCl₂(dppf)(65 mg, 0.08 mmol) were added at room temperature, and the mixture was heated to 90 °C to react 4 hours under the protection of nitrogen. The reaction solution was cooled to room temperature, added with water, extracted with ethyl acetate, the organic phases were combined, washed with saturated salt water, dried over anhydrous sodium sulfate, and concentrated, and the crude product was separated and purified by silica gel column chromatography (100% petroleum ether-petroleum ether: ethyl acetate = 10:1) to obtain C128-16(400 mg, yield 92%) as a white solid. LC-MS:m/z = 488.3[M-Boc + H]⁺.

### Step 17:

C128-16(250 mg,0.43mmol) was dissolved in ethanol (15 mL), ammonium formate (50 mg,0.793mmol) and 10% Pd/C(20 mg) were added, replaced with hydrogen for three times, and the mixture was heated to 60°C to react overnight. The reaction solution was cooled to room temperature, filtered, the filtrate was added with water, extracted with ethyl acetate, the organic phases were combined, washed with saturated salt water, dried over anhydrous sodium sulfate, and concentrated to obtain C128-17(170 mg, crude) as a solid-liquid mixture.

### Step 18:

C128-17(150 mg, crude) was dissolved in dichloromethane (5ml), trifluoroacetic acid (1ml) was added at room temperature, and the mixture was stirred for 30 minutes. The reaction solution was concentrated, added with water, saturated sodium bicarbonate aqueous solution was added to adjust pH = 8, extracted with dichloromethane, the organic phases were combined, washed with saturated salt water, dried over anhydrous sodium sulfate, and concentrated, and the crude product was separated and purified by Pre-TLC to obtain C128-18(100 mg, yield for two steps: 59%) as an off-white solid, which was directly used in the next step.

### Step 19:

C128-18(100 mg, 0.22 mmol), 1H-pyrazol-1-formamidine hydrochloride (41.0 mg, 0.28 mmol) and DIPEA(114 mg,0.88 mmol) were dispersed into acetonitrile (2 mL) and the mixture was heated to 50°C and stirred overnight. The reaction solution was filtered and the filter cake was separated and purified by pre-HPLC to obtain SYY-C128(14 mg, yield 13%) as a white solid. LC-MS:m/z=496.2[M+H]⁺, ¹H NMR (400 MHz, DMSO) δ 8.30 (t, *J* = 6.4 Hz, 1H), 8.14 (s, 1H), 7.98 (t, *J* = 8.0 Hz, 2H), 7.93 (s, 1H), 7.65 (d, *J* = 8.0 Hz, 1H), 7.61-7.42 (m, 7H), 7.32-7.28 (m, 2H), 7.08 (t, *J =* 7.2 Hz, 1H), 6.91 (d, *J =* 8.0 Hz, 2H), 5.02 (d, *J* = 6.8 Hz, 2H), 4.26 (q, *J* = 7.2 Hz, 2H), 1.17 (t, *J* = 7.2 Hz, 3H).

### Example 115: Preparation of Compound SYY-C130

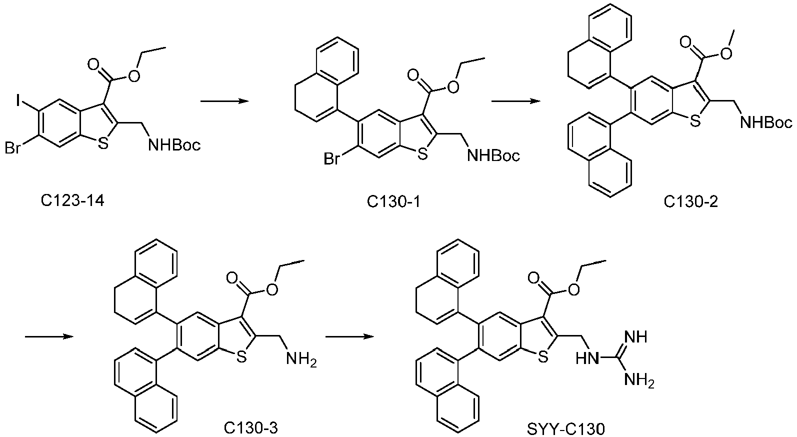

### Step 1:

C123-14(400 mg,0.74 mmol), 2-(3,4-dihydronaphthalen-1-yl)-4,4,5,5-tetramethyl-1,3,2-dioxaborolane (200 mg, 0.78 mmol) and sodium carbonate (157 mg, 1.48 mmol) were dissolved in a mixed solvent of 1, 4-dioxane (15 mL) and H₂O(5 mL), PdCl₂(dppf)(57 mg,0.07 mmol) was added under the protection of nitrogen, and the mixture was heated to 80 °C to react for 3 hours. TLC showed the raw materials were reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C130-1(320 mg, yield 80%) as a colorless oil.

### Step 2:

C130-1(320 mg,0.59 mmol), 1-naphthylboronic acid (152 mg, 0.88 mmol) and sodium carbonate (156 mg, 1.47 mmol) were dissolved in a mixed solvent of 1, 4-dioxane (15 mL) and H₂O(5 mL), PdCl₂(dppf)(49 mg,0.06 mmol) was added under the protection of nitrogen, and the mixture was heated to 100 °C to react for 2 hours. LCMS showed the raw materials were reacted completely. The reaction solution was cooled to room temperature, added with water, extracted with ethyl acetate, the organic phases were combined, washed with saturated salt water, dried over anhydrous sodium sulfate, and concentrated, and the crude product was purified by silica gel column chromatography to obtain C130-2(210 mg, yield 60%) as a colorless oil.

### Step 3:

C130-2(105 mg,0.18 mmol) was dissolved in DCM(4 mL), trifluoroacetic acid (1 mL) was added, and the mixture was reacted at room temperature for 30 minutes. TLC showed the raw materials were reacted completely. The reaction solution was concentrated to obtain C130-3(87 mg, crude), which was directly used in the next step. LC-MS:m/z = 490.2 [M + H]⁺.

### Step 4:

C130-3(87 mg, crude) and DIPEA(62 mg, 0.48 mmol) were dissolved in a mixed solvent of acetonitrile (3 mL) and methanol (1 mL), 1H-pyrazol-1-formamidine hydrochloride (40 mg, 0.27 mmol) was added at room temperature, and the mixture was heated to 30 °C and stirred overnight. lcms showed the reaction was completed. The reaction solution was purified by pre-HPLC to obtain SYY-C130(15 mg, yield for two steps : 13%, TFA salt) as a white solid. ¹H NMR: (DMSO-*d₆*, 400MHz): δ1.31 (t, *J* = 7.2 Hz, 3H), 1.84- 2.02 (m, 2H), 2.17-2.29 (m, 2H), 3.10-3.17 (m, 1H), 4.23-4.36 (m, 2H), 4.99 (d, *J* = 6.4 Hz, 2H), 6.05 (d, *J* = 8.0 Hz, 1H), 6.76 (t, *J* = 8.0 Hz, 1H), 7.07 (t, *J* = 7.2 Hz, 1H),7.19-7.79 (m, 9H), 7.90 (d, *J* = 8.4 Hz, 1H), 8.04 (d, *J* = 8.4 Hz, 1H), 8.25 (s, 1H), 8.35 (t, *J* = 6.0 Hz, 1H), 8.96 (d, *J* = 8.4 Hz, 1H), 9.21 (s, 1H). LC-MS: m/z=532.2 [M+H]⁺ (97.25% purity, 254 nm)

### Example 116: Preparation of Compound SYY-C131

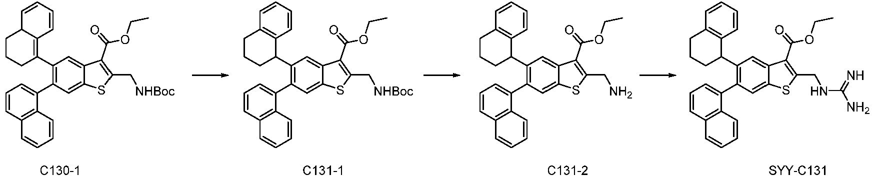

### Step 1:

C130-1(270 mg,0.46 mmol) was dissolved in methanol (10 mL), 10% palladium carbon (100 mg) was added at room temperature, replaced with hydrogen for 3 times, and the mixture was heated to 40 °C to react for 2 days. LCMS showed the reaction was basically completed. The reaction liquid was filtered with diatomite, the filtrate was concentrated, and the crude product was purified by pre-TLC to obtain C131-1(65 mg, yield 24%) as a white solid. LC-MS: no peak.

### Step 2:

C131-1(65 mg, 0.11 mmol) was dissolved in DCM (3 mL), TFA(1 mL) was added dropwise, and the mixture was reacted at room temperature for half an hour after addition. TLC showed the reaction was completed. The reaction solution was concentrated to obtain C131-2(crude), which was directly used in the next step. LC-MS:m/z = 492.2[M + H]⁺.

### Step 3:

C131-2 (crude), 1H-pyrazol-1-formamidine hydrochloride (40 mg, 0.27 mmol) and DIPEA(85 mg,0.66 mmol) were dissolved in a mixed solvent of acetonitrile (4 mL), methanol (1 mL) and dichloromethane (1 mL) and the mixture was heated to 50 °C to react for 3 days. LCMS showed the reaction was completed. The reaction solution was concentrated and the crude product was purified by pre-HPLC to obtain a white solid (27 mg, yield for two steps: 46%). ¹H NMR: (DMSO-*d₆*, 400MHz): δ1.08-1.21 (m, 3H), 1.34-1.45 (m, 1H), 1.57-1.86 (m, 3H), 2.57-2.84 (m, 2H), 3.72-3.92 (m, 1H), 4.15-4.24 (m, 2H), 4.97 (d, J = 6.4 Hz, 2H), 6.84-6.73 (m, 1H), 8.32-8.23 (m, 1H), 6.96-7.11 (m, 3H), 7.38-7.65 (m, 8H), 7.92-8.06 (m, 4H). LC-MS:m/z = 534.2 [M+H]⁺. (96.90% purity, 254 nm).

### Section of biological activity test

### Example 1: Determination of the dissociation constants of the compounds of the invention with protein SH3 in surface plasmon resonance experiment.

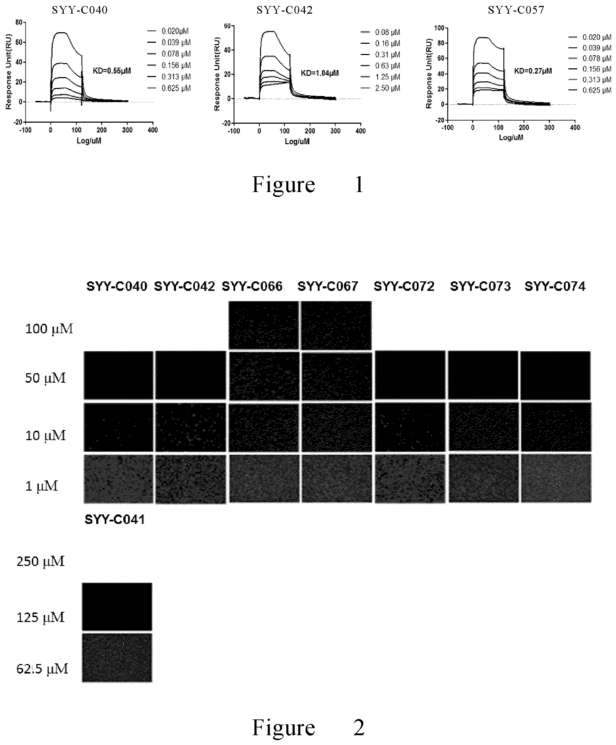

1. Experimental method: Surface plasmon resonance test was carried out on BIACORE T200 instrument (GE Company). Fresh purified grade Src kinase SH3 domain protein (concentration 2 mg/ml) was diluted to 0.1 mg/ml with 10 mM CH3COONa (pH 4.2) and then SH3 protein was coupled to the CM5 chip by standard amino coupling methods. The compounds of the invention were diluted in a gradient using a buffer solution (20 mM Tris-HCl, PH 8.0, 100 mM NaCl) for 60 seconds and dissociated for 120 seconds at a flow rate of 20 µl/s, respectively. The changes in response values over time were recorded and analyzed by the BIA Evaluation Software (GE Healthcare) program to obtain the dissociation constants KD for the different compounds with SH3 protein.
2. Experimental results and conclusions: The dissociation constants KD of different compounds and SH3 protein were described in Table 2, and the surface plasmon resonance (SPR) experimental results of some compounds were shown in Figure 1.

**Table 2 Binding Constants of Compound and SH3 Measured by Surface Plasmon Resonance (SPR) Experiment**

| No. | Structure | KD value (µM) | No. | Structure | KD value (µM) |
|---|---|---|---|---|---|
| SYY-C001 | 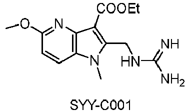 | A | SYY-C072 | 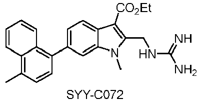 | B |
| SYY-C002 | 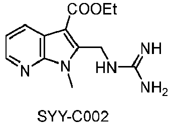 | B | SYY-C073 | 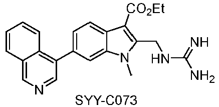 | A |
| SYY-C003 | 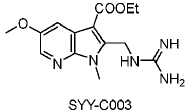 | A | SYY-C074 | 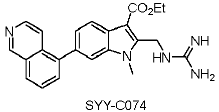 | C |
| SYY-C004 | 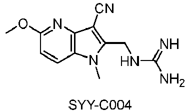 | B | SYY-C075 | 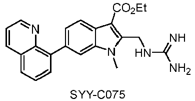 | A |
| SYY-C005 | 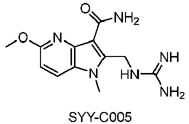 | B | SYY-C076 | 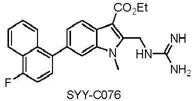 | B |
| SYY-C006 | 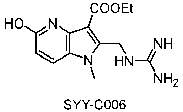 | A | SYY-C077 | 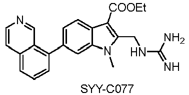 | A |
| SYY-C007 | 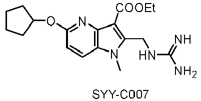 | A | SYY-C078 | 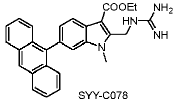 | A |
| SYY-C008 | 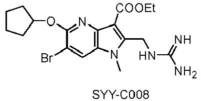 | C | SYY-C079 | 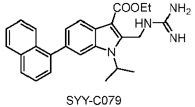 | A |
| SYY-C009 | 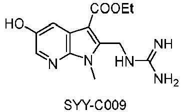 | A | SYY-C080 | 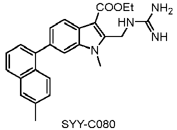 | A |
| SYY-C010 | 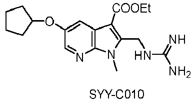 | C | SYY-C081 | 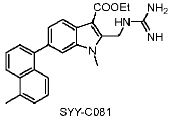 | A |
| SYY-C014 | 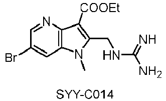 | A | SYY-C082 | 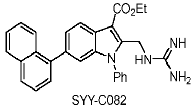 | A |
| SYY-C015 | 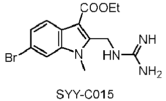 | A | SYY-C083 | 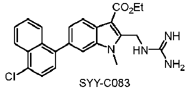 | B |
| SYY-C016 | 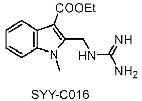 | C | SYY-C084 | 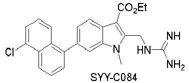 | A |
| SYY-C017 | 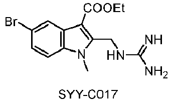 | A | SYY-C085 | 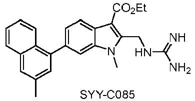 | A |
| SYY-C020 | 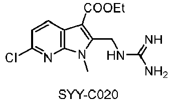 | B | SYY-C086 | 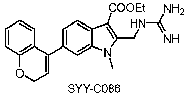 | A |
| SYY-C022 | 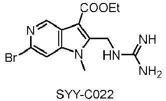 | B | SYY-C087 | 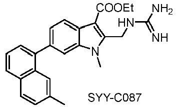 | C |
| SYY-C023 | 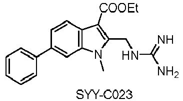 | C | SYY-C088 | 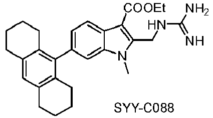 | C |
| SYY-C024 | 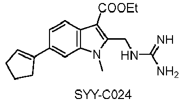 | A | SYY-C089 | 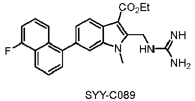 | A |
| SYY-C025 | 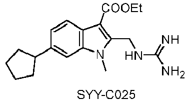 | A | SYY-C090 | 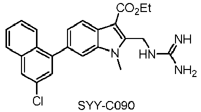 | B |
| SYY-C026 | 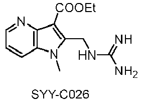 | B | SYY-C091 | 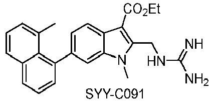 | A |
| SYY-C028 | 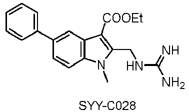 | A | SYY-C092 | 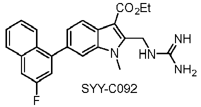 | C |
| SYY-C029 | 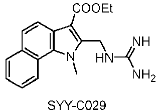 | A | SYY-C093 | 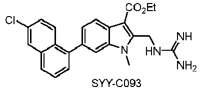 | A |
| SYY-C030 | 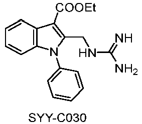 | C | SYY-C094 | 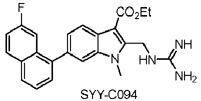 | A |
| SYY-C031 | 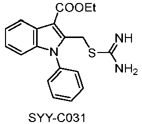 | A | SYY-C095 | 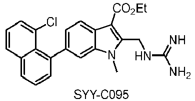 | C |
| SYY-C032 | 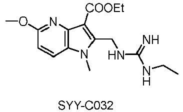 | A | SYY-C096 | 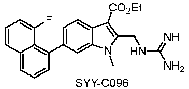 | A |
| SYY-C034 | 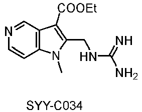 | B | SYY-C097 | 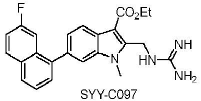 | C |
| SYY-C035 | 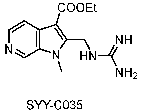 | B | SYY-C098 | 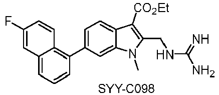 | A |
| SYY-C036 | 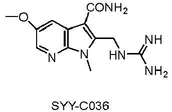 | A | SYY-C099 | 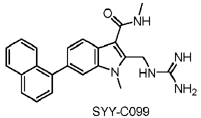 | A |
| SYY-C037 | 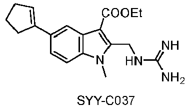 | B | SYY-C 100 | 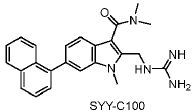 | B |
| SYY-C039 | 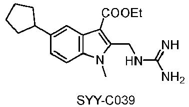 | A | SYY-C101 | 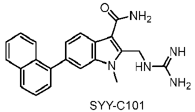 | A |
| SYY-C040 | 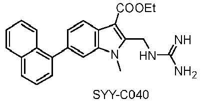 | A | SYY-C102 | 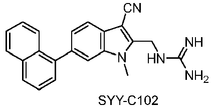 | A |
| SYY-C041 | 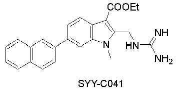 | C | SYY-C 103 | 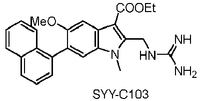 | B |
| SYY-C042 | 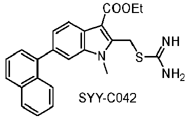 | B | SYY-C104 | 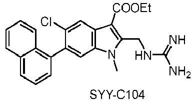 | A |
| SYY-C043 | 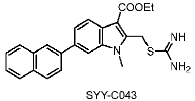 | B | SYY-C105 | 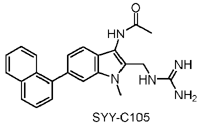 | A |
| SYY-C045 | 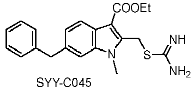 | A | SYY-C106 | 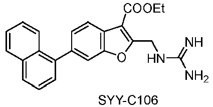 | B |
| SYY-C046 | 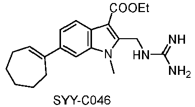 | C | SYY-C107 | 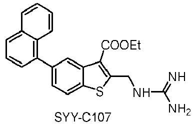 | A |
| SYY-C047 | 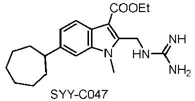 | A | SYY-C108 | 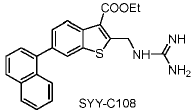 | C |
| SYY-C048 | 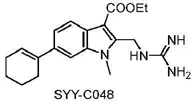 | C | SYY-C 109 | 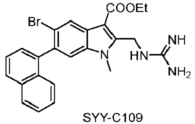 | A |
| SYY-C049 | 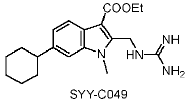 | A | SYY-C110 | 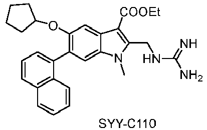 | A |
| SYY-C050 | 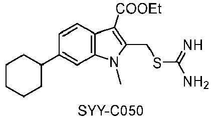 | A | SYY-C111 | 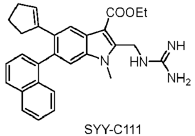 | B |
| SYY-C051 | 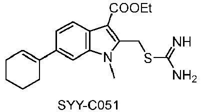 | B | SYY-C 112 | 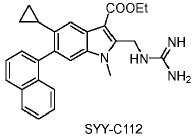 | A |
| SYY-C052 | 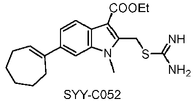 | B | SYY-C 113 | 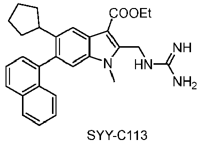 | A |
| SYY-C053 | 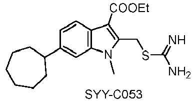 | A | SYY-C 114 | 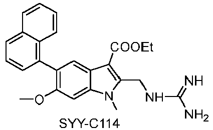 | C |
| SYY-C054 | 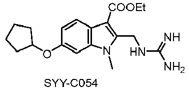 | A | SYY-C115 | 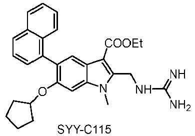 | A |
| SYY-C055 | 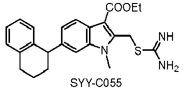 | A | SYY-C 116 | 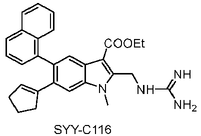 | A |
| SYY-C056 | 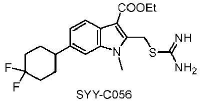 | C | SYY-C117 | 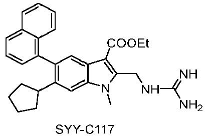 | C |
| SYY-C057 | 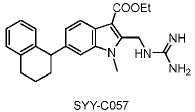 | A | SYY-C118 | 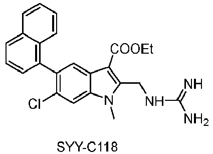 | A |
| SYY-C058 | 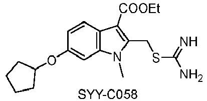 | C | SYY-C 119 | 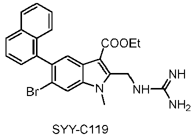 | A |
| SYY-C059 | 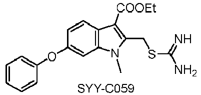 | A | SYY-C 120 | 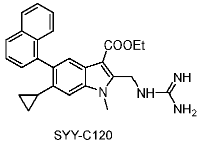 | A |
| SYY-C060 | 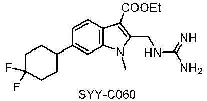 | A | SYY-C121 | 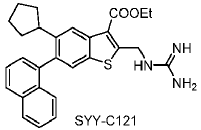 | B |
| SYY-C061 | 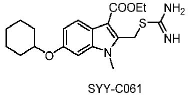 | C | SYY-C 122 | 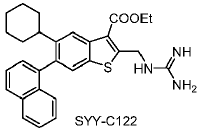 | A |
| SYY-C062 | 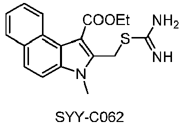 | A | SYY-C 123 | 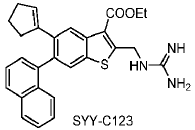 | C |
| SYY-C063 | 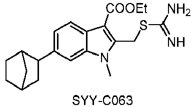 | A | SYY-C 124 | 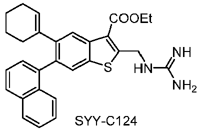 | A |
| SYY-C065 | 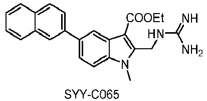 | C | SYY-C 125 | 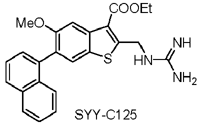 | A |
| SYY-C066 | 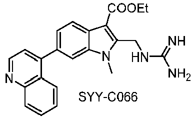 | A | SYY-C 126 | 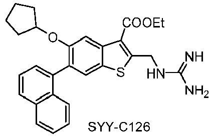 | A |
| SYY-C067 | 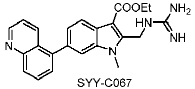 | A | SYY-C 127 | 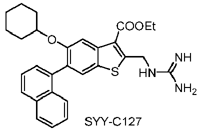 | A |
| SYY-C068 | 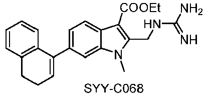 | B | SYY-C 128 | 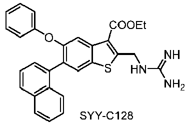 | C |
| SYY-C069 | 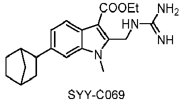 | A | SYY-C 129 | 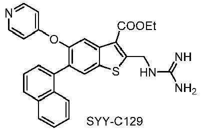 | A |
| SYY-C070 | 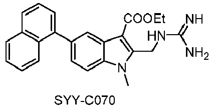 | A | SYY-C130 | 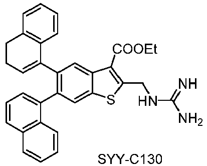 | C |
| SYY-C071 | 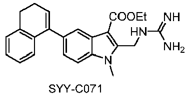 | A | SYY-C131 | 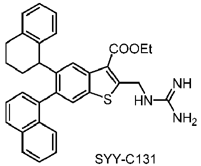 | C |

| | | | | | |
|---|---|---|---|---|---|
| Note: A indicates that the range of KD value was 0-1 µM, B indicates that the range of KD value was 1-10 µM, and C indicates that the range of KD value was 10-100 µM. | | | | | |

As can be seen from Table 2 and Figure 1, the compounds of the present invention all bind to the SH3 protein, and the binding of the compounds to the SH3 protein indicates that the compounds have an antithrombotic effect. The smaller the dissociation constant KD is, the stronger the binding ability of the compounds to SH3 protein is, the stronger the antithrombotic effect of the compounds is.

The compound, when binded to the SH3 structural domain protein of Src kinase, is able to interfere with the binding of integrin αIIbβ3 to Src kinase, thereby selectively inhibiting outside-in signaling without affecting inside-out signaling, allowing the compound of the present invention to be anti-thrombotic without affecting normal physiological hemostasis and avoiding bleeding side effects.

### Example 2: Effect of Compound on Platelet Stretching on Solid Phase Fibrinogen.

1. Experimental method: Firstly, 50 µl fibrinogen (0.1M, diluted with pH 8.3 sodium bicarbonate , 20 µg/ml) was added to 96-well plate, and coated overnight at 4°C. After being washed by PBS for 3 times in the morning, sealed with bovine serum albumin (20 mg/ml) at 37°C for 60min, then 100 µl of washed platelet suspension was taken, and added into compounds in each group to make the final concentration 250 µM, and incubated at 37°C for 60min. 50 µl of incubated platelets were added to 96-well plate, adhered in a temperature box at 37°C for 60min, unadhered platelets were removed by PBS washing for 3 times, and adhered platelets were stably fixed with 4% paraformaldehyde, and washed with PBS for 3 times. Then the platelet membrane was perforated with 0.5% Triton X-100, and then the platelet was stained with 0.5 µg/ml phalloidin-rhodamine for 60 min at 37°C and washed 3 times (10 min each) with PBS. After washing, the fluorescent color development was observed with a fluorescent microscope (Leica), the concentration of drug action was reduced, and the effect of different concentration gradients of the compound on the platelet stretching function on solid phase fibrinogen was observed.
2. Experimental results and conclusions: The concentration of different compounds on platelet stretching on fibrinogen was shown in Table 3, and the inhibitory effect of some compounds on platelet stretching was shown in Figure 2.

**Table 3. Concentration range for compounds that inhibit platelet stretching on fibrinogen**

| No. | Structure | Concentratio n (µM) | No. | Structure | KD value (µM) |
|---|---|---|---|---|---|
| SYY-C00 1 | 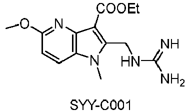 | A | SYY-C07 2 | 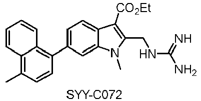 | A |
| SYY-C00 2 | 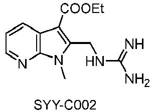 | B | SYY-C07 3 | 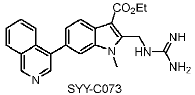 | A |
| SYY-C00 3 | 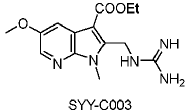 | A | SYY-C07 4 | 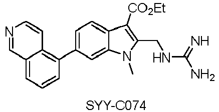 | A |
| SYY-C00 4 | 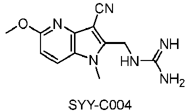 | B | SYY-C07 5 | 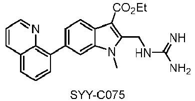 | A |
| SYY-C00 5 | 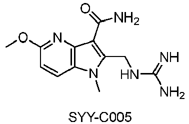 | B | SYY-C07 6 | 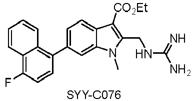 | B |
| SYY-C00 6 | 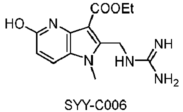 | A | SYY-C07 7 | 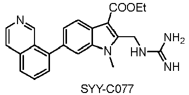 | A |
| SYY-C00 7 | 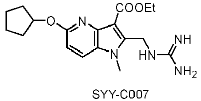 | A | SYY-C07 8 | 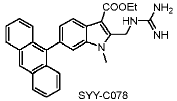 | A |
| SYY-C00 8 | 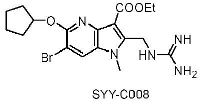 | B | SYY-C07 9 | 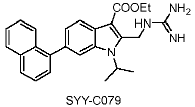 | A |
| SYY-C00 9 | 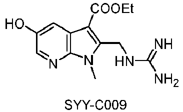 | A | SYY-C08 0 | 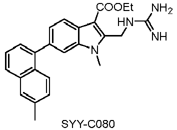 | A |
| SYY-C01 0 | 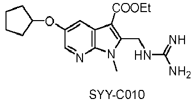 | A | SYY-C08 1 | 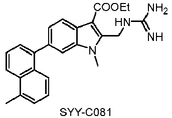 | A |
| SYY-C01 4 | 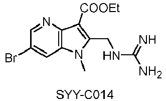 | A | SYY-C08 2 | 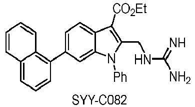 | A |
| SYY-C01 5 | 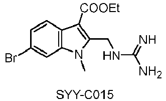 | A | SYY-C08 3 | 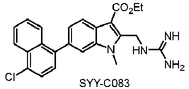 | B |
| SYY-C01 6 | 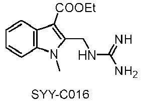 | A | SYY-C08 4 | 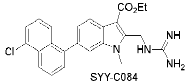 | A |
| SYY-C01 7 | 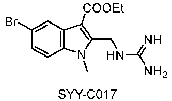 | A | SYY-C08 5 | 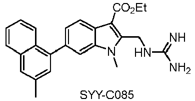 | A |
| SYY-C02 0 | 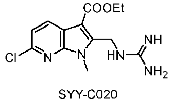 | B | SYY-C08 6 | 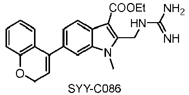 | A |
| SYY-C02 2 | 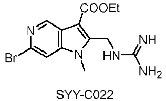 | B | SYY-C08 7 | 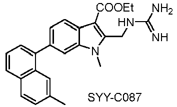 | B |
| SYY-C02 3 | 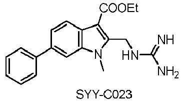 | A | SYY-C08 8 | 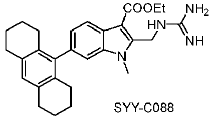 | B |
| SYY-C02 4 | 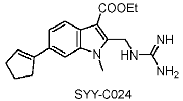 | A | SYY-C08 9 | 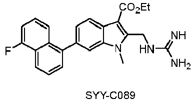 | A |
| SYY-C02 5 | 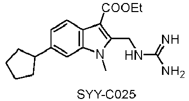 | A | SYY-C09 0 | 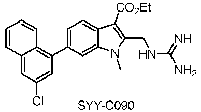 | B |
| SYY-C02 6 | 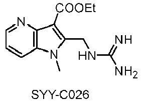 | B | SYY-C09 1 | 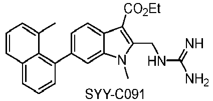 | A |
| SYY-C02 8 | 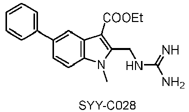 | A | SYY-C09 2 | 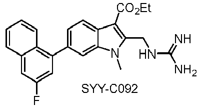 | B |
| SYY-C02 9 | 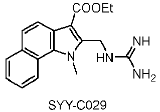 | A | SYY-C09 3 | 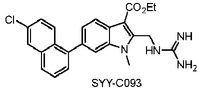 | A |
| SYY-C03 0 | 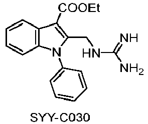 | B | SYY-C09 4 | 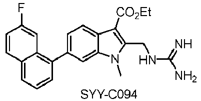 | A |
| SYY-C03 1 | 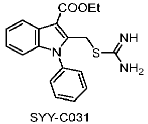 | A | SYY-C09 5 | 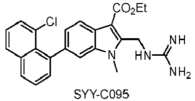 | A |
| SYY-C03 2 | 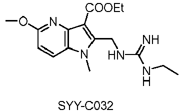 | A | SYY-C09 6 | 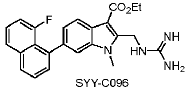 | A |
| SYY-C03 4 | 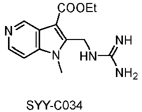 | B | SYY-C09 7 | 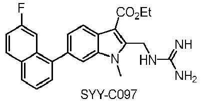 | A |
| SYY-C03 5 | 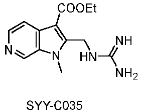 | B | SYY-C09 8 | 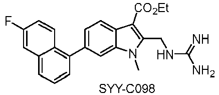 | A |
| SYY-C03 6 | 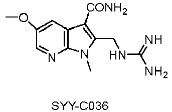 | A | SYY-C09 9 | 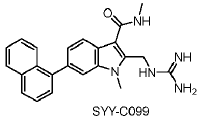 | A |
| SYY-C03 7 | 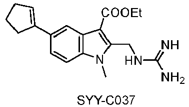 | B | SYY-C10 0 | 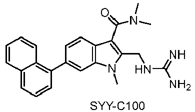 | B |
| SYY-C03 9 | 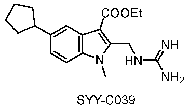 | A | SYY-C10 1 | 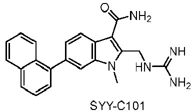 | A |
| SYY-C04 0 | 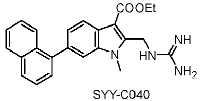 | A | SYY-C10 2 | 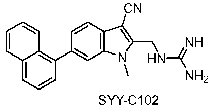 | A |
| SYY-C04 1 | 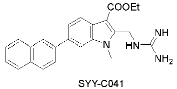 | B | SYY-C10 3 | 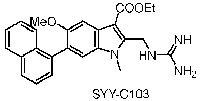 | B |
| SYY-C04 2 | 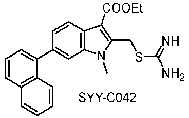 | A | SYY-C10 4 | 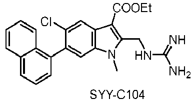 | A |
| SYY-C04 3 | 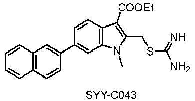 | B | SYY-C10 5 | 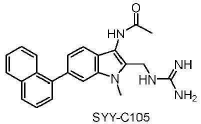 | A |
| SYY-C04 5 | 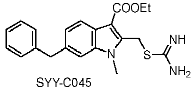 | A | SYY-C10 6 | 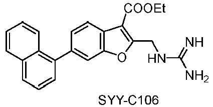 | B |
| SYY-C04 6 | 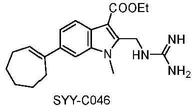 | B | SYY-C10 7 | 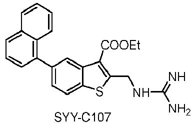 | A |
| SYY-C04 7 | 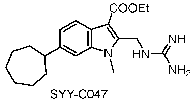 | A | SYY-C10 8 | 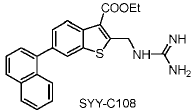 | B |
| SYY-C04 8 | 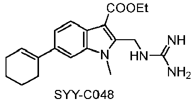 | A | SYY-C10 9 | 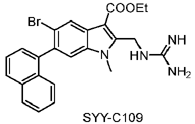 | A |
| SYY-C04 9 | 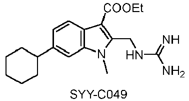 | A | SYY-C11 0 | 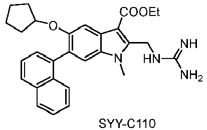 | A |
| SYY-C05 0 | 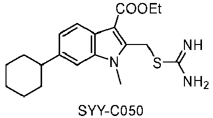 | A | SYY-C11 1 | 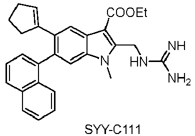 | B |
| SYY-C05 1 | 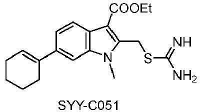 | B | SYY-C11 2 | 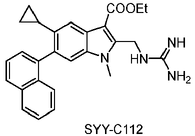 | A |
| SYY-C05 2 | 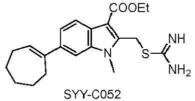 | B | SYY-C11 3 | 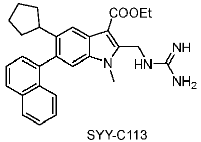 | A |
| SYY-C05 3 | 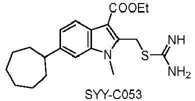 | A | SYY-C11 4 | 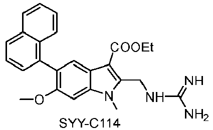 | A |
| SYY-C05 4 | 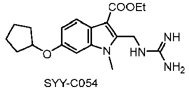 | A | SYY-C11 5 | 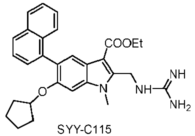 | A |
| SYY-C05 5 | 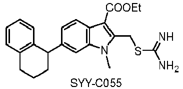 | A | SYY-C11 6 | 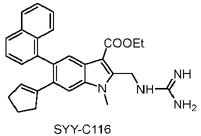 | A |
| SYY-C05 6 | 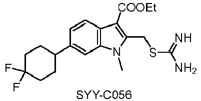 | B | SYY-C11 7 | 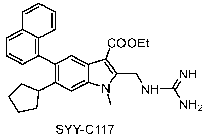 | B |
| SYY-C05 7 | 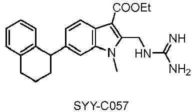 | A | SYY-C11 8 | 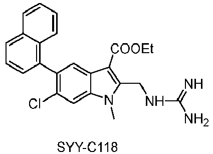 | A |
| SYY-C05 8 | 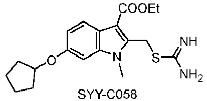 | B | SYY-C11 9 | 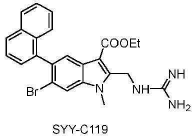 | A |
| SYY-C05 9 | 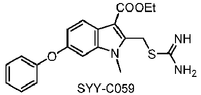 | A | SYY-C12 0 | 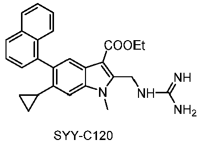 | A |
| SYY-C06 0 | 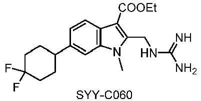 | A | SYY-C12 1 | 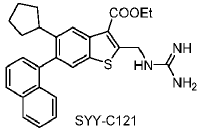 | B |
| SYY-C06 1 | 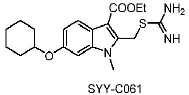 | A | SYY-C12 2 | 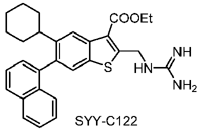 | A |
| SYY-C06 2 | 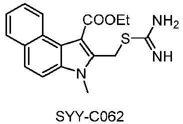 | A | SYY-C12 3 | 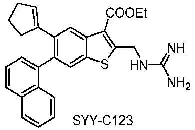 | B |
| SYY-C06 3 | 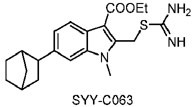 | A | SYY-C12 4 | 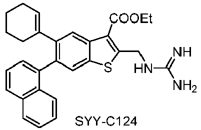 | A |
| SYY-C06 5 | 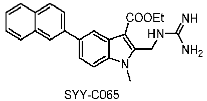 | A | SYY-C12 5 | 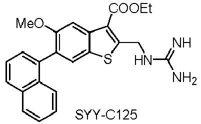 | A |
| SYY-C06 6 | 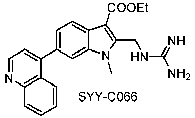 | A | SYY-C12 6 | 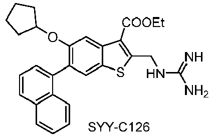 | A |
| SYY-C06 7 | 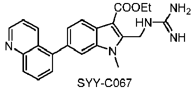 | A | SYY-C12 7 | 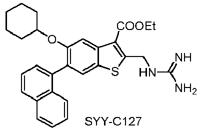 | A |
| SYY-C06 8 | 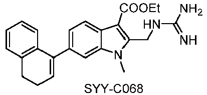 | B | SYY-C12 8 | 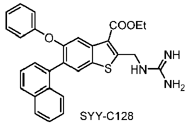 | B |
| SYY-C06 9 | 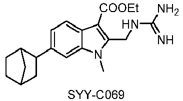 | A | SYY-C12 9 | 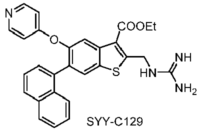 | A |
| SYY-C07 0 | 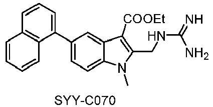 | A | SYY-C13 0 | 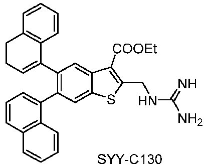 | B |
| SYY-C07 1 | 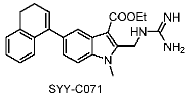 | A | SYY-C13 1 | 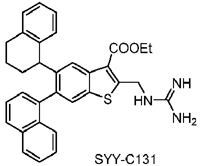 | B |

| | | | | | |
|---|---|---|---|---|---|
| Note: A indicates that the concentration range of compound to inhibit platelet stretching on fibrinogen is 10-100 µ m, and B indicates that the concentration range of compound to inhibit platelet stretching on fibrinogen is 100-250 µ m. | | | | | |

It can be seen from Table 3 and Figure 2 that the compound at a concentration gradient of 10-250 µM can variously inhibit the stretching function of platelets on the solid phase fibrinogen, and inhibiting the stretching function of platelets on the solid phase fibrinogen has an antithrombotic effect. Therefore, Table 3 and Figure 2 show that the compound of the present invention has an antithrombotic effect. Some of the compounds shown in Figure 2 are capable of concentration-dependent inhibition of platelet stretching and thus concentration-dependent antithrombotic effects.

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof: 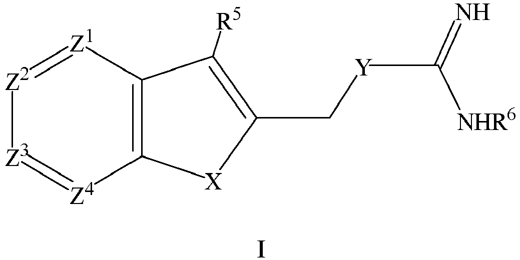
Z¹ is N or CR¹;
Z² is N or CR²;
Z³ is N or CR³;
Z⁴ is N or CR⁴;
with the proviso that 0, 1, or 2 of Z¹, Z², Z³ and Z⁴ are each independently N;
R¹, R², R³ and R⁴ are each independently hydrogen, hydroxyl, amino, C₁-C₆ alkyl, substituted or unsubstituted C₃-C₁₀ cycloalkyl, substituted or unsubstituted C₃-C₁₀ cycloalkenyl, C₂-C₆ alkenyl, C₂-C₆ alkynyl, halogen, C₁-C₆ haloalkyl, G^{a}, -OR^{a}, -OC(O)R^{d}, -OC(O)NR^{b}R^{c}, -SR^{a}, -S(O)₂R^{d}, -S(O)₂NR^{b}R^{c}, -C(O)R^{d}, -C(O)OR^{a}, -C(O)NR^{b}R^{c}, -NR^{b}R^{c}, -N(R^{e})C(O)R^{d}, -N(R^{e})S(O)₂R^{d}, -N(R^{e})C(O)OR^{d}, -N(R^{e})C(O)NR^{b}R^{c}, -N(R^{e})S(O)₂NR^{b}R^{c}, -(C₁-C₆ alkylene)-G^{a}, -(C₁-C₆ alkylene)-OR^{a}, -(C₁-C₆ alkylene)-OC(O)R^{d}, -(C₁-C₆ alkylene)-OC(O)NR^{b}R^{c}, -(C₁-C₆ alkylene)-S(O)₂R^{d}, -(C₁-C₆ alkylene)-S(O)₂NR^{b}R^{c}, -(C₁-C₆ alkylene)-C(O)R^{d}, -(C₁-C₆ alkylene)-C(O)OR^{a}, -(C₁-C₆ alkylene)-C(O)NR^{b}R^{c}, -(C₁-C₆ alkylene)-NR^{b}R^{c}, -(C₁-C₆ alkylene)-N(R^{e})C(O)R^{d}, -(C₁-C₆ alkylene)-N(R^{e})S(O)₂R^{d}, - (C₁-C₆ alkylene)-N(R^{e})C(O)OR^{a}, -(C₁-C₆ alkylene)-N(R^{e})C(O)NR^{b}R^{c}, -(C₁-C₆ alkylene)-N(R^{e})S(O)₂NR^{b}R^{c}, -(C₁-C₆ alkylene)-CN, substituted or unsubstituted C₆-C₁₆ aryl, substituted or unsubstituted 5-16-membered heteroaryl, substituted or unsubstituted C₆-C₁₆ aryl-C₁-C₄ alkyl-, substituted or unsubstituted 5-16-membered heteroaryl-C₁-C₄ alkyl-, wherein the substituted means that one or more (preferably 1, 2, 3 or 4) hydrogens on the group are each independently substituted by a substituent selected from the group consisting of C₁-C₆ alkyl, halogen, C₁-C₆ haloalkyl, and C₃-C₆ cycloalkyl; or, Z³ is CR³, Z⁴ is CR⁴, R³ and R⁴ are connected to form a C6-C12 aromatic ring; or, Z¹ is CR¹, Z² is CR², and R¹ and R² are connected to form a C6-C12 aromatic ring;
R⁵ is -CN, -C(O)OR^{a}, -C(O)NR^{b}R^{c}, or -N(R^{e})C(O)R^{d};
X is independently -NR^{x}-, -O-, or -S-; wherein, R^{x} is hydrogen, C₁-C₆ alkyl, C₂-C₆ alkenyl, C₂-C₆ alkynyl, C₁-C₆ haloalkyl, C₆-C₁₂ aryl, 5-12 membered heteroaryl, G^{b}, or -(C₁-C₆ alkylene)-G^{b};
Y is independently -NH-, -O- or -S-;
R⁶ is hydrogen, C₁-C₆ alkyl, C₁-C₆ haloalkyl, or G^{b};
R^{a}, R^{b}, R^{c} and R^{e} are each independently hydrogen, C₂-C₆ alkenyl, C₂-C₆ alkynyl, C₁-C₆ haloalkyl, G^{b}, C₁-C₆ alkyl, C₃-C₈ cycloalkyl, C₆-C₁₂ aryl, 5-12 membered heteroaryl, or C₁-C₆ alkyl substituted by a substituent, wherein the substituent is -OR^{z1}, -NR^{z1}R^{z2}, -C(O)OR^{z1}, -C(O)NR^{z1}R^{z2}, -S(O)₂R^{z1}, -S(O)₂NR^{z1}R^{z2} or G^{b};
R^{d} is C₂-C₆ alkenyl, C₂-C₆ alkynyl, C₁-C₆ haloalkyl, G^{b}, C₁-C₆ alkyl, or C₁-C₆ alkyl substituted by a substituent, and the substituent is selected from -OR^{z1}, -NR^{z1}R^{z2}, -C(O)OR^{z1}, -C(O)NR^{z1}R^{z2}, -S(O)₂R^{z1}, -S(O)₂NR^{z1}R^{z2} or G^{b};
R^{z1} and R^{z2} are each independently hydrogen, C₁-C₆ alkyl, or C₁-C₆ haloalkyl;
G^{a} and G^{b} are each independently C₆-C₁₂ aryl, 5-12-membered heteroaryl, 3-10-membered heterocyclyl, C₃-C₁₀ cycloalkyl, or C₃-C₁₀- cycloalkenyl, and each is independently unsubstituted or substituted by 1, 2, 3, 4, or 5 R^{v};
R^{v} is C₁-C₆ alkyl, C₂-C₆ alkenyl, C₂-C₆ alkynyl, halogen, C₁-C₆ haloalkyl, -CN, oxo, -OR^{h}, -OC(O)R', -OC(O)NR^{j}R^{k}, -SR^{h}, -S(O)₂R^{h}, -S(O)₂NR^{j}R^{k}, -C(O)R^{h}, -C(O)-5-12 membered monocyclic heterocyclyl, -C(O)-5-12 membered monocyclic heteroaryl, -C(O)OR^{h}, -C(O)NR^{j}R^{k}, -NR^{j}R^{k}, -N(R^{h})C(O)Rⁱ, -N(R^{h})S(O)₂Rⁱ, -N(R^{h})C(O)ORⁱ, -N(R^{h})C(O)NR^{j}R^{k}, -(C₁-C₆ alkylene)-OR^{h}, -(C₁-C₆ alkylene)-OC(O)R', -(C₁-C₆ alkylene)-OC(O)NR^{j}R^{k}, -(C₁-C₆ alkylene)-S(O)₂R^{h}, -(C₁-C₆ alkylene)-S(O)₂NR^{j}R^{k}, -(C₁-C₆ alkylene)-C(O)R^{h}, -(C₁-C₆ alkylene)-C(O)OR^{h}, -(C₁-C₆ alkylene)-C(O)NR^{j}R^{k}, -(C₁-C₆ alkylene)-NR^{j}R^{k}, -(C₁-C₆ alkylene)-N(R^{h})C(O)Rⁱ, -(C₁-C₆ alkylene)-N(R^{h})S(O)₂Rⁱ, -(C₁-C₆ alkylene)-N(R^{h})C(O)ORⁱ, -(C₁-C₆ alkylene)-N(R^{h})C(O)NR^{j}R^{k}, or -(C₁-C₆ alkylene)-CN;
R^{z1} and R^{z2} are each independently hydrogen, C₁-C₆ alkyl, or C₁-C₆ haloalkyl;
R^{h}, R^{j} and R^{k} are each independently hydrogen, C₁-C₆ alkyl, or C₁-C₆ haloalkyl; and R' is independently C₁-C₆ alkyl, or C₁-C₆ haloalkyl at each occurrence;
Rⁱ is C₁-C₆ alkyl, or C₁-C₆ haloalkyl;
each heterocycle of the heterocyclyl and heteroaryl independently has 1 to 4 (preferably 1, 2, 3 or 4) heteroatoms selected from N, O and S.

2. The compound of claim 1, wherein the compound has one or more characteristics selected from the group consisting of:
R¹, R², R³ and R⁴ are each independently hydrogen, hydroxyl, amino, methoxy, monomethylnaphthalenyl, isoquinolinyl, quinolinyl, mono-halogenated naphthyl, phenyl, naphthyl, cyclopentyl-O-, cyclohexyl-O-, anthryl, halogen (such as bromine), benzopyranyl, 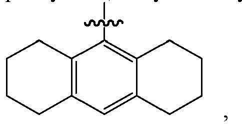 cyclopentenyl, cyclohexenyl, cycloheptenyl, cyclopropyl, cyclopentyl, cyclohexyl, cycloheptyl, benzyl, benzocyclopentyl, benzocycloheptenyl, dihalocyclohexyl, pyridyl, pyridyl-O-; or, Z³ is CR³, Z⁴ is CR⁴, R³ and R⁴ are connected to form benzene ring; or, Z¹is CR1, Z²is CR², and R¹ and R² are connected to form benzene ring.
X is independently -NR^{x}-, -O-, or -S-; R^{x} is hydrogen, methyl, propyl, or phenyl;
R^{a} is C₁-C₆ alkyl;
R^{b}, R^{c} and R^{e} are each independently hydrogen, C₁-C₆ alkyl;
R^{d} is C₁-C₆ alkyl;
R⁵ is -CN, -C(O)OR^{a}, -C(O)NR^{b}R^{c}, -N(R^{e})C(O)R^{d}; wherein R^{a} is C₁-C₄ alkyl; R^{b}, R^{c} and R^{e} are each independently hydrogen, C₁-C₄ alkyl; R^{d} is C₁-C₄ alkyl;
Y is independently -NH- or -S-;
R⁶ is hydrogen, C₁-C₄ alkyl, C₁-C₄ haloalkyl.

3. The compound of claim 1, wherein the compound is selected from the group consisting of:

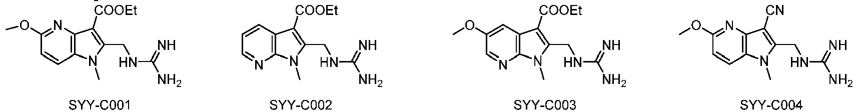

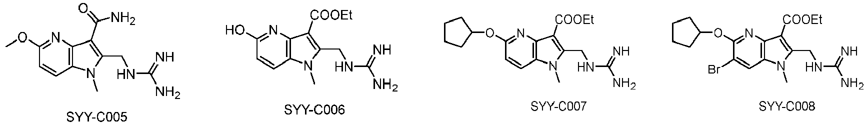

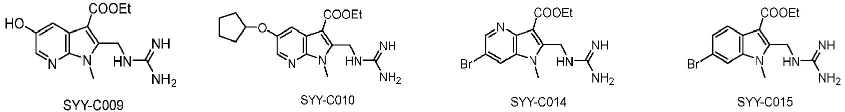

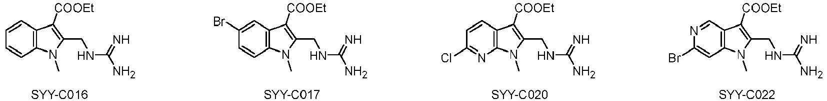

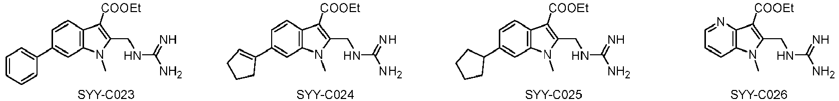

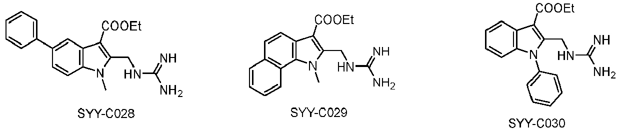

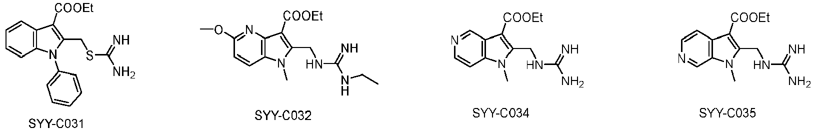

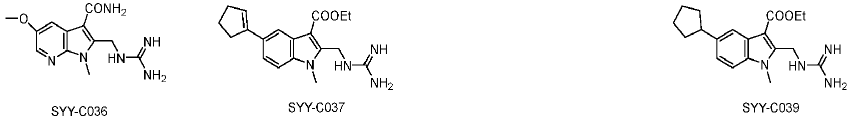

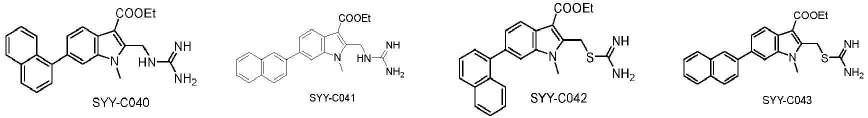

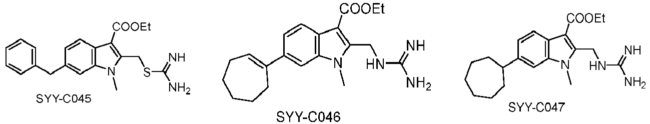

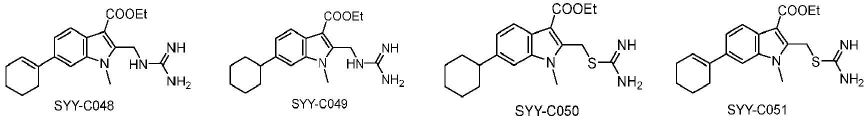

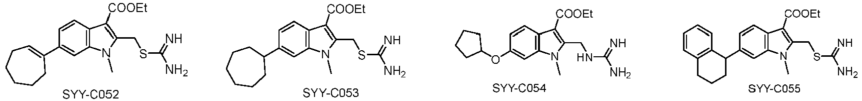

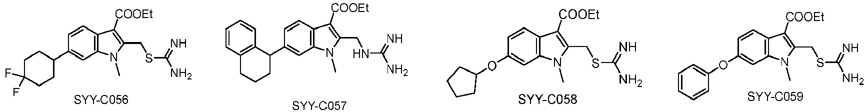

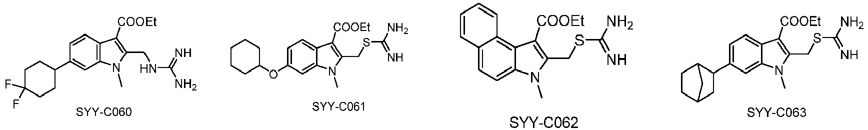

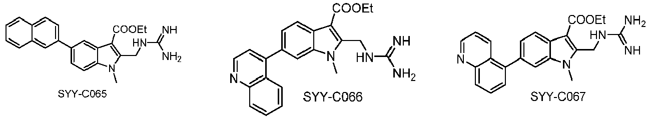

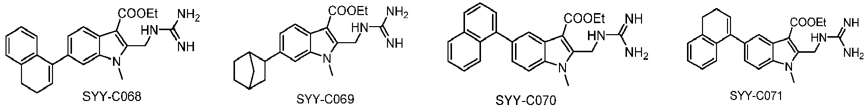

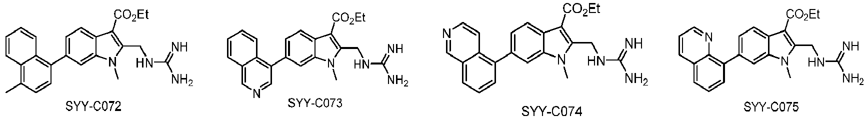

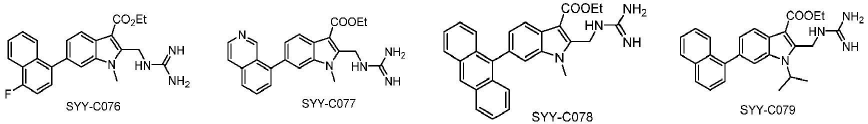

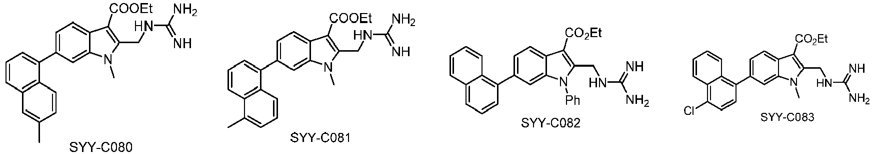

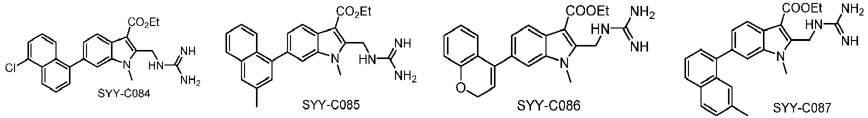

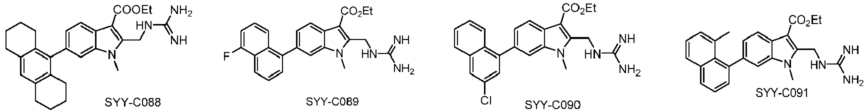

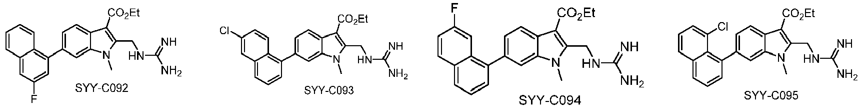

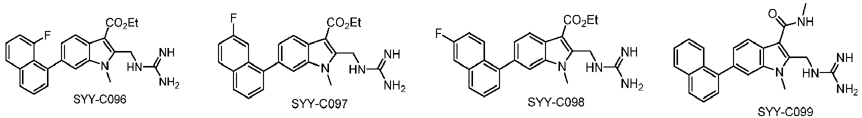

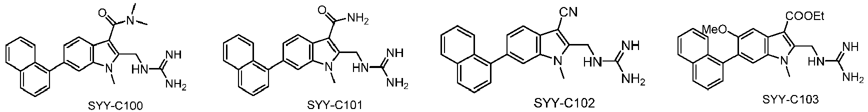

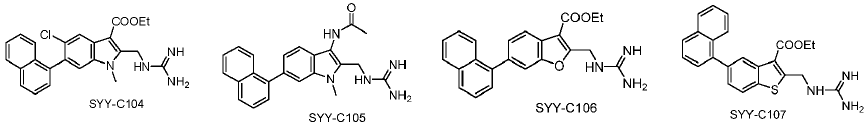

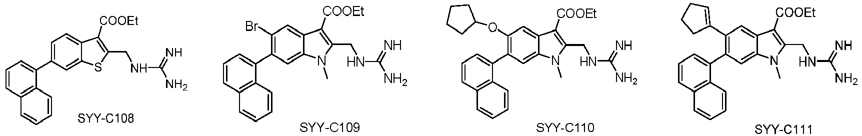

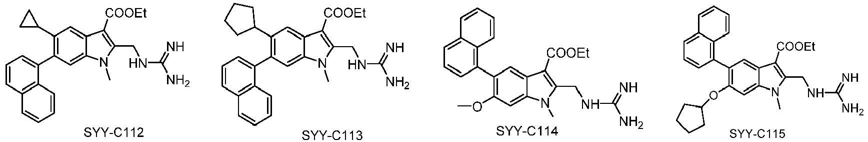

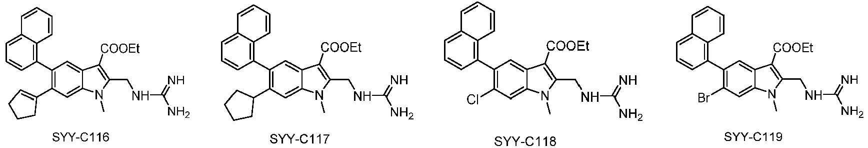

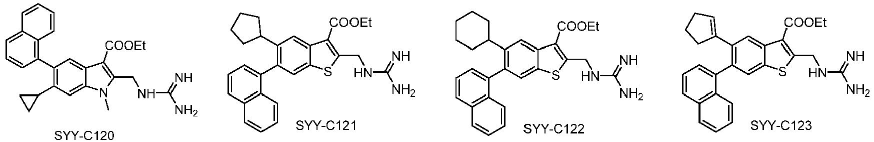

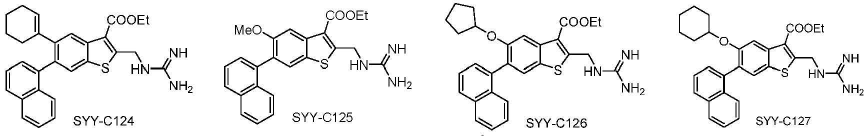

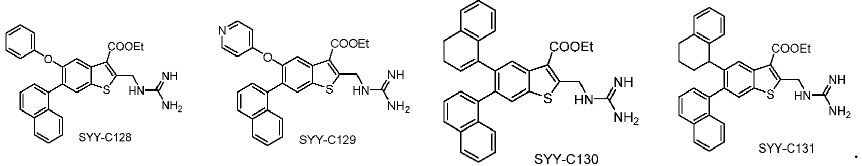

4. A pharmaceutical composition comprising:
(a) the compound of formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof of claim 1; and
(b) pharmaceutically acceptable carriers.

5. The pharmaceutical composition of claim 4, wherein, the content of the compound of formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof is 0.01-99.99 wt.%, preferably 0.1-99.9 wt.%, more preferably 1-99 wt.%, more preferably 5-95 wt.%, more preferably 10-90 wt.%, more preferably 20-80 wt.%, most preferably 30-70 wt.%, by the weight of the composition.

6. A use of the compound of formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof of claim 1 for the preparation of a composition or a formulation for one or more uses selected from the group consisting of: (1) selective inhibition of outside-in signaling mediated by the interaction of integrin β3 with Src kinase; (2) prevention and/or treatment of diseases related to outside-in signaling mediated by the interaction of integrin β3 with Src kinase; and (3) prevention and/or treatment of thrombosis.

7. The use of claim 6, wherein the diseases related to outside-in signaling mediated by the interaction of integrin β3 with Src kinase is selected from the group consisting of thrombus, tumor, osteoporosis, endothelial cell-mediated angiogenesis, and a combination thereof.

8. The use of claim 6, wherein the prevention and/or treatment of thrombosis does not affect bleeding or improve bleeding while achieving anti-thrombosis.

9. The use of claim 8, wherein the improve bleeding includes inhibiting bleeding, not increasing bleeding risk, reducing bleeding risk, not causing bleeding side effects, and/or not affecting hemostatic function.

10. The use of claim 6, wherein the thrombosis is a cardio-cerebrovascular thrombus, and the cardio-cerebrovascular thrombus is selected from the group consisting of: myocardial infarction thrombus, cerebral infarction thrombus, ischemic stroke, atherosclerotic thrombus, and a combination thereof.

11. The use of claim 6, wherein the prevention and/or treatment of thrombosis includes one or more ways selected from the group consisting of:
(3-1) inhibition of the extension function of platelets on solid phase fibrinogen;
(3-2) inhibition of platelet aggregation, adhesion and/or extension;
(3-3) inhibition of fibrin clot retraction;
(3-3) not affecting the binding function of platelet binding to free fibrinogen.

12. The use of claim 6, wherein the composition or formulation further comprises other antithrombotic drugs, other anti-tumor drugs, other drugs for treating osteoporosis, and/or other anti-angiogenic drugs.

13. A method for preventing and/or treating thrombosis comprising the steps of: administrating of the compound of formula I, or a pharmaceutically acceptable salt thereof, or an enantiomer thereof, or a diastereomer thereof, or an atropisomer thereof, or a racemate thereof, or a polymorph thereof, or a solvate thereof or an isotopically labeled derivative thereof of claim 1 to a subject in need thereof so as to prevent and/or treat thrombosis.
